(19) Europäisches Patentamt
European Patent Office
Office européen des brevets

(11) EP 0 900 198 B1

(12) EUROPÄISCHE PATENTSCHRIFT

(45) Veröffentlichungstag und Bekanntmachung des Hinweises auf die Patenterteilung:
**12.03.2003 Patentblatt 2003/11**

(21) Anmeldenummer: **97921683.5**

(22) Anmeldetag: **21.04.1997**

(51) Int Cl.⁷: **C07C 401/00**, A61K 31/59

(86) Internationale Anmeldenummer:
**PCT/EP97/02013**

(87) Internationale Veröffentlichungsnummer:
**WO 97/041096 (06.11.1997 Gazette 1997/47)**

(54) **NEUE VITAMIN-D-DERIVATE MIT CARBO- ODER HETEROCYCLISCHEN SUBSTITUENTEN AN C-25, VERFAHREN ZU IHRER HERSTELLUNG, ZWISCHENPRODUKTE UND DIE VERWENDUNG ZUR HERSTELLUNG VON ARZNEIMITTELN**

NEW VITAMIN D DERIVATIVES WITH CARBO- OR HETEROCYCLIC SUBSTITUENTS AT C-25, A PROCESS FOR THEIR PRODUCTION, INTERMEDIATE PRODUCTS AND THEIR USE FOR PRODUCING MEDICAMENTS

NOUVEAUX DERIVES DE VITAMINE D AVEC DES SUBSTITUANTS CARBOCYCLIQUES OU HETEROCYCLIQUES EN C-25, PROCEDE DE PRODUCTION DESDITS DERIVES, PRODUITS INTERMEDIAIRES ET LEUR UTILISATION POUR LA PRODUCTION DE MEDICAMENTS

(84) Benannte Vertragsstaaten:
**AT BE CH DE DK ES FI FR GB GR IE IT LI LU MC NL PT SE**
Benannte Erstreckungsstaaten:
**SI**

(30) Priorität: **30.04.1996 DE 19619036**

(43) Veröffentlichungstag der Anmeldung:
**10.03.1999 Patentblatt 1999/10**

(73) Patentinhaber: **Schering Aktiengesellschaft 13342 Berlin (DE)**

(72) Erfinder:
- **STEINMEYER, Andreas, Dr. D-13581 Berlin (DE)**
- **KIRSCH, Gerald, Dr. D-14199 Berlin (DE)**
- **NEEF, Günter, Dr. D-10771 Berlin (DE)**
- **SCHWARZ, Katica D-10585 Berlin (DE)**
- **THIEROFF-EKERDT, Ruth, Dr. D-13469 Berlin (DE)**
- **WIESINGER, Herbert, Dr. D-10781 Berlin (DE)**
- **HABEREY, Martin, Dr. D-12169 Berlin (DE)**
- **FÄHNRICH, Marianne, Dr. D-13409 Berlin (DE)**

(56) Entgegenhaltungen:
**EP-A- 0 549 318**
**EP-A- 0 619 305**
**WO-A-95/33716**
**WO-A-97/00242**
**DE-A- 4 200 783**

- **G.Grue -Sörensen et al., Biorg. Med. Chem. 6, 2029-2039 (1998)**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 900 198 B1

Printed by Jouve, 75001 PARIS (FR)

## Beschreibung

Die vorliegende Erfindung betrifft Vitamin D-Derivate mit Substituenten an C-25 der allgemeinen Formel **I**,

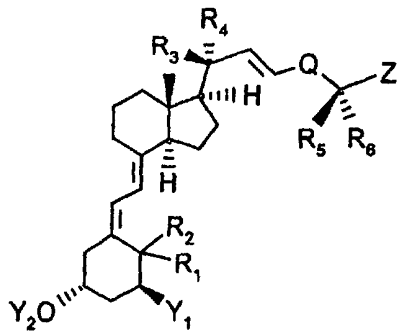

I

worin

$Y_1$ ein Wasserstoffatom, eine Hydroxylgruppe, eine Alkanoyloxygruppe mit 1 bis 12 C-Atomen oder eine Aroyloxygruppe,
$Y_2$ ein Wasserstoffatom oder eine Alkanoylgruppe mit 1 bis 12 C-Atomen oder eine Aroylgruppe,
$R_1$ und $R_2$ je ein Wasserstoffatom oder gemeinsam eine exocyclische Methylengruppe,
$R_3$ und $R_4$ unabhängig voneinander ein Wasserstoffatom, ein Chlor- oder Fluoratom, eine Alkylgruppe mit 1 bis 4 Kohlenstoffatomen, gemeinsam eine Methylengruppe oder gemeinsam mit dem quartären Kohlenstoffatom 20 einen 3-7-gliedrigen, gesättigten oder ungesättigten carbocyclischen Ring,
Q eine geradkettige oder verzweigte Kohlenstoffeinheit mit bis zu 10 Kohlenstoffatomen, die an beliebigen Positionen Hydroxylgruppen (α- oder β-ständig), die ihrerseits verethert oder verestert sein können, Ketogruppen, Aminogruppen oder Halogenatome aufweisen kann,
$R_5$ und $R_6$ gemeinsam mit dem Kohlenstoffatom 25 einen 3-7-gliedrigen, gesättigten oder ungesättigten carbocyclischen Ring und
Z einen fünf- oder sechsgliedrigen carbo- oder heterocyclischen Ring, der gesättigt,

ungesättigt oder aromatisch sein kann und an beliebigen Positionen eine oder mehrere Alkylketten, die geradkettig oder verzweigt, gesättigt oder ungesättigt sein können und an beliebigen Stellen durch Oxa-, Thiä- oder Azafunktionen (substituiert oder unsubstituiert) oder Sulfoxid oder Sulfongruppen unterbrochen sein können oder Substituenten (Hydroxygruppen, Halogenatome) tragen können,
bedeuten,
Verfahren zu ihrer Herstellung, Zwischenprodukte im Herstellungsverfahren sowie deren Verwendung zur Herstellung von Arzneimitteln.

Die für die Reste $Y_1$ und $Y_2$ möglichen Alkanoyl- bzw. Alkanoyloxygruppen mit 1 bis 12 C-Atomen leiten sich insbesondere von gesättigten Carbonsäuren ab. Diese Reste können cyclisch, acyclisch, carbocyclisch oder heterocyclisch sein. Die bevorzugten Reste leiten sich von $C_1$- bis $C_9$-, insbesondere $C_2$- bis $C_5$-Alkancarbonsäuren wie beispielsweise Acetyl(oxy)-, Propionyl(oxy)-, Butyryl(oxy)- ab.
Als Aroyl(oxy)gruppen sind die Benzoyl(oxy)- und substituierte Benzoyl(oxy)gruppen bevorzugt.

Für $R_3$ und $R_4$ gelten die folgenden bevorzugten Kombinationen: $R_3$= H, $R_4$= Methyl oder $R_3$= Methyl, $R_4$=H; $R_3$= F, $R_4$= Methyl oder $R_3$= Methyl, $R_4$=F; $R_3$, $R_4$= Methyl; $R_3$ und $R_4$ bilden zusammen eine Methylengruppe oder gemeinsam mit dem tertiären Kohlenstoffatom 20 einen Cyclopropylring.

Für Q gelten die folgenden Bevorzugungen:

Q ist eine unsubstituierte, unverzweigte Alkyleinheit mit 1, 2 oder 3 Kohlenstoffatomen oder
Q ist eine Hydroxymethylengruppe (α- oder β-ständige Hydroxylgruppe) oder
Q = $-CH(OH)-CH_2-$ oder $-CH(OH)-CH_2-CH_2-$ (α- oder β-ständige Hydroxylgruppen),

Substituenten vorzugsweise Alkylgruppen für alle beschriebenen Varianten an C-24a sind möglich.

Für $R_5$ und $R_6$ gelten folgende Bevorzugungen:

$R_5$=$R_6$=Methyl oder Ethyl, $R_5$ und $R_6$ bilden gemeinsam mit dem Kohlenstoffatom C-25 einen Cyclopropyl-, Cyclobutyl-, Cyclopentyl- oder Cyclohexylring.

Besonders bevorzugt sind die Fälle: $R_5$=$R_6$=Methyl sowie $R_5$ und $R_6$ bilden gemeinsam mit dem Kohlenstoffatom C-25 einen Cyclopropylring.

Für Z gelten folgende Bevorzugungen:

Für den funfgliedrigen Fall sind Furan-, Tetrahydrofuran-, Thiophen-, Pyrrol-, Pyrrolidin-, Imidazol-, Pyrazol-, Oxazol-, Thiazol-, Isoxazol-, Triazol-, Oxadiazol- oder Thiadiazol-Ringe, die an beliebigen Positionen eine oder mehrere Alkylketten mit 1 bis 12 Kohlenstoffatomen tragen können bevorzugt. Diese Alkylketten können geradkettig oder verzweigt, gesättigt oder ungesättigt sein und durch Heteroatome (O, S, N auch substituiert) unterbrochen sein. Sie können auch weitere Substituenten (Hydroxylgruppen, Halogenatome) tragen.
Für den sechsgliedrigen Fall sind der Phenyl-, Pyridin-, Pyrazin-, Pyrimidin-, Pyridazin-, Piperidin- oder Tetrahydropyran-Ringe bevorzugt, die wie die fünfgliedrigen Ringe mit den genannten Alkylketten einfach oder mehrfach substituiert sein können.
Besonders bevorzugt sind: Imidazol-, Oxazol-, Thiazol-, Furan-, Thiophen-, Pyrrol-, Isoxazol-, Pyrazol-, Triazol-, Pyridin-, Pyrimidin- und Phenyl-Ringe, die je eine geradkettige, gesättigte C1 bis C12-Alkylkette tragen.

Besonders bevorzugt gemäß vorliegender Erfindung sind die folgenden Verbindungen

(5*Z*,7*E*,22*E*)-(1*S*,3*R*,24*R*)-25-(5-Propyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Propyloxazol-2-yl)-26,27-cyclo-9,10-secocholesta-5,7,10( 19),22-tetraen-1,3,24-triol
(5*Z*,7*E*,22*E*)-(1*S*,3*R*,24*R*)-25-(5-Methyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Methyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Ethyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Ethyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Butyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Butyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Pentyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Pentyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Propylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Propylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Methylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Methylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Ethylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Ethylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Butylthiazo]-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Butylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Pentylthiazol-2-yl)-26,27-cyclo-9,10-secocholesta-5,7,10(19),22-tetraen-1,3,24-triol

(5*Z*,7*E*,22*E*)-(1*S*,3*R*,24*S*)-25-(5-Pentylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Propylimidazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Propylimidazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Methylimidazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Methylimidazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Ethylimidazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Ethylimidazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Butylimidazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Butylimidazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Pentylimidazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Pentylimidazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Propylfuran-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Propylfuran-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Methylfuran-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Methylfuran-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Ethylfuran-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Ethylfuran-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Butylfuran-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Butylfuran-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Pentylfuran-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Pentylfuran-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Propylthiophen-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Propylthiophen-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Methylthiophen-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Methylthiophen-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Ethylthiophen-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Ethylthiophen-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Butylthiophen-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Butylthiophen-2-yl)-26,27-cyclo-9,10-secocholesta-5,7,10(19),22-tetraen-1,3,24-triol

(5*Z*,7*E*,22*E*)-(1*S*,3*R*,24*R*)-25-(5-Pentylthiophen-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Pentylthiophen-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Propylpyrrol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Propylpyrrol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Methylpyrrol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Methylpyrrol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Ethylpyrrol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Ethylpyrrol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Butylpyrrol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Butylpyrrol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Pentylpyrrol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Pentylpyrrol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Propyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Propyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Methyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Methyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Ethyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Ethyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Butyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Butyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Pentyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Pentyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Propylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Propylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Methylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Methylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Ethylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Ethylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Butylthiazol-2-yl)-26,27-cyclo-9,10-secocholesta-5,7,10(19),22-tetraen-1,3,24-triol

(5*Z*,7*E*,22*E*)-(1*S*,3*R*,24*S*)-25-(4-Butylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Pentylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Pentylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Propylimidazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Propylimidazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Methylimidazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Methylimidazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Ethylimidazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Ethylimidazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Butylimidazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Butylimidazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Pentylimidazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Pentylimidazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Propylfuran-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Propylfuran-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Methylfuran-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Methylfuran-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Ethylfuran-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Ethylfuran-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Butylfuran-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Butylfuran-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Pentylfuran-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Pentylfuran-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Propylthiophen-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Propylthiophen-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Methylthiophen-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Methylthiophen-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Ethylthiophen-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Ethylthiophen-2-yl)-26,27-cyclo-9,10-secocholesta-5,7,10(19),22-tetraen-1,3,24-triol

(5*Z*,7*E*,22*E*)-(1*S*,3*R*,24*R*)-25-(4-Butylthiophen-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Butylthiophen-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Pentylthiophen-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Pentylthiophen-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Propylpyrrol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Propylpyrrol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Methylpyrrol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Methylpyrrol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Ethylpyrrol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Ethylpyrrol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Butylpyrrol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Butylpyrrol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Pentylpyrrol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Pentylpyrrol-2-yl)-26,27-cyclo-9,10-secocholesta-5,7,10(19),22-tetraen-1,3,24-triol
(5*Z*,7*E*,22*E*)-(1*S*,3*R*,24*R*)-25-(3-Methyl-1,2,4-oxadiazol-5-yl)-26,27-cyclo-9,10-secocholesta-5,7,10(19),22-tetraen-1,3,24-triol
(5*Z*,7*E*,22*E*)-(1*S*,3*R*,24*S*)-25-(3-Methyl-1,2,4-oxadiazol-5-yl)-26,27-cyclo-9,10-secocholesta-5,7,10(19),22-tetraen-1,3,24-triol
(5*Z*,7*E*,22*E*)-(1*S*,3*R*,24*R*)-25-(3-Ethyl-1,2,4-oxadiazol-5-yl)-26,27-cyclo-9,10-secocholesta-5,7,10(19),22-tetraen-1,3,24-triol
(5*Z*,7*E*,22*E*)-(1*S*,3*R*,24*S*)-25-(3-Ethyl-1,2,4-oxadiazol-5-yl)-26,27-cyclo-9,10-secocholesta-5,7,10(19),22-tetraen-1,3,24-triol
(5*Z*,7*E*,22*E*)-(1*S*,3*R*,24*R*)-25-(3-Propyl-1,2,4-oxadiazol-5-yl)-26,27-cyclo-9,10-secocholesta-5,7,10(19),22-tetraen-1,3,24-triol
(5*Z*,7*E*,22*E*)-(1*S*,3*R*,24*S*)-25-(3-Propyl-1,2,4-oxadiazol-5-yl)-26,27-cyclo-9,10-secocholesta-5,7,10(19),22-tetraen-1,3,24-triol
(5*Z*,7*E*,22*E*)-(1*S*,3*R*,24*R*)-25-(3-Butyl-1,2,4-oxadiazol-5-yl)-26,27-cyclo-9,10-secocholesta-5,7,10(19),22-tetraen-1,3,24-triol
(5*Z*,7*E*,22*E*)-(1*S*,3*R*,24*S*)-25-(3-Butyl-1,2,4-oxadiazol-5-yl)-26,27-cyclo-9,10-secocholesta-5,7,10(19),22-tetraen-1,3,24-triol
(5*Z*,7*E*,22*E*)-(1*S*,3*R*,24*R*)-25-(3-Pentyl-1,2,4-oxadiazol-5-yl)-26,27-cyclo-9,10-secocholesta-5,7,10(19),22-tetraen-1,3,24-triol
(5*Z*,7*E*,22*E*)-(1*S*,3*R*,24*S*)-25-(3-Pentyl-1,2,4-oxadiazol-5-yl)-26,27-cyclo-9,10-secocholesta-5,7,10(19),22-tetraen-1,3,24-triol
(5*Z*,7*E*,22*E*)-(1*S*,3*R*,24*R*)-25-(5-Methyl-1,3,4-oxadiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Methyl-1,3,4-oxadiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Ethyl-1,3,4-oxadiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Ethyl-1,3,4-oxadiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Propyl-1,3,4-oxadiazol-2-yl)-26,27-cyclo-9,10-secocholesta-5,7,10(19),22-tetraen-1,3,24-triol

(5*Z*,7*E*,22*E*)-(1*S*,3*R*,24*S*)-25-(5-Propyl-1,3,4-oxadiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Butyl-1,3,4-oxadiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Butyl-1,3,4-oxadiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Pentyl-1,3,4-oxadiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Pentyl-1,3,4-oxadiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-Phenyl-26,27-cyclo-9,10-secocholesta-5,7,10(19),22-tetraen-1,3,24-triol
(5*Z*,7*E*,22*E*)-(1*S*,3*R*,24*S*)-25-Phenyl-26,27-cyclo-9,10-secocholesta-5,7,10(19),22-tetraen-1,3,24-triol
(5*Z*,7*E*,22*E*)-(1*S*,3*R*,24*R*)-25-(4-Methylphenyl)-26,27-cyclo-9,10-secocholesta-5,7,10(19),22-tetraen-1,3,24-triol
(5*Z*,7*E*,22*E*)-(1*S*,3*R*,24*S*)-25-(4-Methylphenyl)-26,27-cyclo-9,10-secocholesta-5,7,10(19),22-tetraen-1,3,24-triol
(5*Z*,7*E*,22*E*)-(1*S*,3*R*,24*R*)-25-(4-Ethylphenyl)-26,27-cyclo-9,10-secocholesta-5,7,10(19),22-tetraen-1,3,24-triol
(5*Z*,7*E*,22*E*)-(1*S*,3*R*,24*S*)-25-(4-Ethylphenyl)-26,27-cyclo-9,10-secocholesta-5,7,10(19),22-tetraen-1,3,24-triol
(5*Z*,7*E*,22*E*)-(1*S*,3*R*,24*R*)-25-(4-Propylphenyl)-26,27-cyclo-9,10-secocholesta-5,7,10(19),22-tetraen-1,3,24-triol
(5*Z*,7*E*,22*E*)-(1*S*,3*R*,24*S*)-25-(4-Propylphenyl)-26,27-cyclo-9,10-secocholesta-5,7,10(19),22-tetraen-1,3,24-triol
(5*Z*,7*E*,22*E*)-(1*S*,3*R*,24*R*)-25-(4-Butylphenyl)-26,27-cyclo-9,10-secocholesta-5,7,10(19),22-tetraen-1,3,24-triol
(5*Z*,7*E*,22*E*)-(1*S*,3*R*,24*S*)-25-(4-Butylphenyl)-26,27-cyclo-9,10-secocholesta-5,7,10(19),22-tetraen-1,3,24-triol
(5*Z*,7*E*,22*E*)-(1*S*,3*R*,24*R*)-25-(4-Pentylphenyl)-26,27-cyclo-9,10-secocholesta-5,7,10(19),22-tetraen-1,3,24-triol
(5*Z*,7*E*,22*E*)-(1*S*,3*R*,24*S*)-25-(4-Pentylphenyl)-26,27-cyclo-9,10-secocholesta-5,7,10(19),22-tetraen-1,3,24-triol
(5*Z*,7*E*,22*E*)-(1*S*,3*R*,24*R*)-25-(3-Methylphenyl)-26,27-cyclo-9,10-secocholesta-5,7,10(19),22-tetraen-1,3,24-triol
(5*Z*,7*E*,22*E*)-(1*S*,3*R*,24*S*)-25-(3-Methylphenyl)-26,27-cyclo-9,10-secocholesta-5,7,10(19),22-tetraen-1,3,24-triol
(5*Z*,7*E*,22*E*)-(1*S*,3*R*,24*R*)-25-(3-Ethylphenyl)-26,27-cyclo-9,10-secocholesta-5,7,10(19),22-tetraen-1,3,24-triol
(5*Z*,7*E*,22*E*)-(1*S*,3*R*,24*S*)-25-(3-Ethylphenyl)-26,27-cyclo-9,10-secocholesta-5,7,10(19),22-tetraen-1,3,24-triol
(5*Z*,7*E*,22*E*)-(1*S*,3*R*,24*R*)-25-(3-Propylphenyl)-26,27-cyclo-9,10-secocholesta-5,7,10(19),22-tetraen-1,3,24-triol
(5*Z*,7*E*,22*E*)-(1*S*,3*R*,24*S*)-25-(3-Propylphenyl)-26,27-cyclo-9,10-secocholesta-5,7,10(19),22-tetraen-1,3,24-triol
(5*Z*,7*E*,22*E*)-(1*S*,3*R*,24*R*)-25-(3-Butylphenyl)-26,27-cyclo-9,10-secocholesta-5,7,10(19),22-tetraen-1,3,24-triol
(5*Z*,7*E*,22*E*)-(1*S*,3*R*,24*S*)-25-(3-Butylphenyl)-26,27-cyclo-9,10-secocholesta-5,7,10(19),22-tetraen-1,3,24-triol
(5*Z*,7*E*,22*E*)-(1*S*,3*R*,24*R*)-25-(3-Pentylphenyl)-26,27-cyclo-9,10-secocholesta-5,7,10(19),22-tetraen-1,3,24-triol
(5*Z*,7*E*,22*E*)-(1*S*,3*R*,24*S*)-25-(3-Pentylphenyl)-26,27-cyclo-9,10-secocholesta-5,7,10(19),22-tetraen-1,3,24-triol
(5*Z*,7*E*,22*E*)-(1*S*,3*R*,24*R*)-25-[4-(1-Methylethyl)phenyl)-26,27-cyclo-9,10-secocholesta-5,7,10(19),22-tetraen-1,3,24-triol
(5*Z*,7*E*,22*E*)-(1*S*,3*R*,24*S*)-25-[4-(1-Methylethyl)phenyl)-26,27-cyclo-9,10-secocholesta-5,7,10(19),22-tetraen-1,3,24-triol
(5*Z*,7*E*,22*E*)-(1*S*,3*R*,24*R*)-25-[3-(1-Methylethyl)phenyl)-26,27-cyclo-9,10-secocholesta-5,7,10(19),22-tetraen-1,3,24-triol
(5*Z*,7*E*,22*E*)-(1*S*,3*R*,24*S*)-25-[3-(1-Methylethyl)phenyl)-26,27-cyclo-9,10-secocholesta-5,7,10(19),22-tetraen-1,3,24-triol
(5*Z*,7*E*,22*E*)-(1*S*,3*R*,24*R*)-25-(2-Pyridyl)-26, 27-cyclo-9,10-secocholesta-5, 7,10(19),22-tetraen-1,3,24-triol
(5*Z*,7*E*,22*E*)-(1*S*,3*R*,24*S*)-25-(2-Pyridyl)-26,27-cyclo-9,10-secocholesta-5,7,10(19),22-tetraen-1,3,24-triol
(5*Z*,7*E*,22*E*)-(1*S*,3*R*,24*R*)-25-(6-Methyl-2-pyridyl)-26,27-cyclo-9,10-secocholesta-5,7,10(19),22-tetraen-1,3,24-triol
(5*Z*,7*E*,22*E*)-(1*S*,3*R*,24*S*)-25-(6-Methyl-2-pyridyl)-26,27-cyclo-9,10-secocholesta-5,7,10(19),22-tetraen-1,3,24-triol
(5*Z*,7*E*,22*E*)-(1*S*,3*R*,24*R*)-25-(5-Methyl-2-pyridyl)-26,27-cyclo-9,10-secocholesta-5,7,10(19),22-tetraen-1,3,24-triol
(5*Z*,7*E*,22*E*)-(1*S*,3*R*,24*S*)-25-(5-Methyl-2-pyridyl)-26,27-cyclo-9,10-secocholesta-5,7,10(19),22-tetraen-1,3,24-triol
(5*Z*,7*E*,22*E*)-(1*S*,3*R*,24*R*)-25-(4-Methyl-2-pyridyl)-26,27-cyclo-9,10-secocholesta-5,7,10(19),22-tetraen-1,3,24-triol
(5*Z*,7*E*,22*E*)-(1*S*,3*R*,24*S*)-25-(4-Methyl-2-pyridyl)-26,27-cyclo-9,10-secocholesta-5,7,10(19),22-tetraen-1,3,24-triol
(5*Z*,7*E*,22*E*)-(1*S*,3*R*,24*R*)-25-(6-Ethyl-2-pyridyl)-26,27-cyclo-9,10-secocholesta-5,7,10(19),22-tetraen-1,3,24-triol
(5*Z*,7*E*,22*E*)-(1*S*,3*R*,24*S*)-25-(6-Ethyl-2-pyridyl)-26,27-cyclo-9,10-secocholesta-5,7,10(19),22-tetraen-1,3,24-triol

(5*Z*,7*E*,22*E*)-(1*S*,3*R*,24*R*)-25-(5-Ethyl-2-pyridyl)-26,27-cyclo-9,10-secocholesta-5,7,10(19),22-tetraen-1,3,24-triol

(5*Z*,7*E*,22*E*)-(1*S*,3*R*,24*S*)-25-(5-Ethyl-2-pyridyl)-26,27-cyclo-9,10-secocholesta-5,7,10(19),22-tetraen-1,3,24-triol

(5*Z*,7*E*,22*E*)-(1*S*,3*R*,24*R*)-25-(4-Ethyl-2-pyridyl)-26,27-cyclo-9,10-secocholesta-5,7,10(19),22-tetraen-1,3,24-triol

(5*Z*,7*E*,22*E*)-(1*S*,3*R*,24*S*)-25-(4-Ethyl-2-pyridyl)-26,27-cyclo-9,10-secocholesta-5,7,10(19),22-tetraen-1,3,24-triol

(5*Z*,7*E*,22*E*)-(1*S*,3*R*,24*R*)-25-(6-Propyl-2-pyridyl)-26,27-cyclo-9,10-secocholesta-5,7,10(19),22-tetraen-1,3,24-triol

(5*Z*,7*E*,22*E*)-(1*S*,3*R*,24*S*)-25-(6-Propyl-2-pyridyl)-26,27-cyclo-9,10-secocholesta-5,7,10(19),22-tetraen-1,3,24-triol

(5*Z*,7*E*,22*E*)-(1*S*,3*R*,24*R*)-25-(5-Propyl-2-pyridyl)-26,27-cyclo-9,10-secocholesta-5,7,10(19),22-tetraen-1,3,24-triol

(5*Z*,7*E*,22*E*)-(1*S*,3*R*,24*S*)-25-(5-Propyl-2-pyridyl)-26,27-cyclo-9,10-secocholesta-5,7,10(19),22-tetraen-1,3,24-triol

(5*Z*,7*E*,22*E*)-(1*S*,3*R*,24*R*)-25-(4-Propyl-2-pyridyl)-26,27-cyclo-9,10-secocholesta-5,7,10(19),22-tetraen-1,3,24-triol

(5*Z*,7*E*,22*E*)-(1*S*,3*R*,24*S*)-25-(4-Propyl-2-pyridyl)-26,27-cyclo-9,10-secocholesta-5,7,10(19),22-tetraen-1,3,24-triol

(5*Z*,7*E*,22*E*)-(1*S*,3*R*,24*R*)-25-(6-Butyl-2-pyridyl)-26,27-cyclo-9,10-secocholesta-5,7,10(19),22-tetraen-1,3,24-triol

(5*Z*,7*E*,22*E*)-(1*S*,3*R*,24*S*)-25-(6-Butyl-2-pyridyl)-26,27-cyclo-9,10-secocholesta-5,7,10(19),22-tetraen-1,3,24-triol

(5*Z*,7*E*,22*E*)-(1*S*,3*R*,24*R*)-25-(5-Butyl-2-pyridyl)-26,27-cyclo-9,10-secocholesta-5,7,10(19),22-tetraen-1,3,24-triol

(5*Z*,7*E*,22*E*)-(1*S*,3*R*,24*S*)-25-(5-Butyl-2-pyridyl)-26,27-cyclo-9,10-secocholesta-5,7,10(19),22-tetraen-1,3,24-triol

(5*Z*,7*E*,22*E*)-(1*S*,3*R*,24*R*)-25-(4-Butyl-2-pyridyl)-26,27-cyclo-9,10-secocholesta-5,7,10(19),22-tetraen-1,3,24-triol

(5*Z*,7*E*,22*E*)-(1*S*,3*R*,24*S*)-25-(4-Butyl-2-pyridyl)-26,27-cyclo-9,10-secocholesta-5,7,10(19),22-tetraen-1,3,24-triol

(5*Z*,7*E*,22*E*)-(1*S*,3*R*,24*R*)-25-(5,5-Dimethyl-2-oxazolin-2-yl)-26,27-cyclo-9,10-secocholesta-5,7,10(19),22-tetraen-1,3,24-triol

(5*Z*,7*E*,22*E*)-(1*S*,3*R*,24*S*)-25-(5,5-Dimethyl-2-oxazolin-2-yl)-26,27-cyclo-9,10-secocholesta-5,7,10(19),22-tetraen-1,3,24-triol

(5*Z*,7*E*,22*E*)-(1*S*,3*R*,24*R*)-25-(5,5-Diethyl-2-oxazolin-2-yl)-26,27-cyclo-9,10-secocholesta-5,7,10(19),22-tetraen-1,3,24-triol

(5*Z*,7*E*,22*E*)-(1*S*,3*R*,24*S*)-25-(5,5-Diethyl-2-oxazolin-2-yl)-26,27-cyclo-9,10-secocholesta-5,7,10(19),22-tetraen-1,3,24-triol

(5*Z*,7*E*,22*E*)-[1*S*,3*R*,24*R*,25(*R*)]-25-(5-Methyl-2-oxazolin-2-yl)-26,27-cyclo-9,10-secocholesta-5,7,10(19),22-tetraen-1,3,24-triol

(5*Z*,7*E*,22*E*)-[1*S*,3*R*,24*S*,25(*R*)]-25-(5-Methyl-2-oxazolin-2-yl)-26,27-cyclo-9,10-secocholesta-5,7,10(19),22-tetraen-1,3,24-triol

(5*Z*,7*E*,22*E*)-[1*S*,3*R*,24*R*,25(S)]-25-(5-Methyl-2-oxazolin-2-yl)-26,27-cyclo-9,10-secocholesta-5,7,10(19),22-tetraen-1,3,24-triol

(5*Z*,7*E*,22*E*)-[1*S*,3*R*,24*S*,25(*S*)]-25-(5-Methyl-2-oxazolin-2-yl)-26,27-cyclo-9,10-secocholesta-5,7,10(19),22-tetraen-1,3,24-triol

(5*Z*,7*E*,22*E*)-[1*S*,3*R*,24*R*,25(*R*)]-25-(5-Ethyl-2-oxazolin-2-yl)-26,27-cyclo-9,10-secocholesta-5,7,10(19),22-tetraen-1,3,24-triol

(5*Z*,7*E*,22*E*)-[1*S*,3*R*,24*S*,25(*R*)]-25-(5-Ethyl-2-oxazolin-2-yl)-26,27-cyclo-9,10-secocholesta-5,7,10(19),22-tetraen-1,3,24-triol

(5*Z*,7*E*,22*E*)-[1*S*,3*R*,24*R*,25(*S*)]-25-(5-Ethyl-2-oxazolin-2-yl)-26,27-cyclo-9,10-secocholesta-5,7,10(19),22-tetraen-1,3,24-triol

(5*Z*,7*E*,22*E*)-[1*S*,3*R*,24*S*,25(*S*)]-25-(5-Ethyl-2-oxazolin-2-yl)-26,27-cyclo-9,10-secocholesta-5,7,10(19),22-tetraen-1,3,24-triol

(5*Z*,7*E*,22*E*)-[1*S*,3*R*,24*R*,25(*R*)]-25-(5-Propyl-2-oxazolin-2-yl)-26,27-cyclo-9,10-secocholesta-5,7,10(19),22-tetraen-1,3,24-triol

(5*Z*,7*E*,22*E*)-[1*S*,3*R*,24*S*,25(*R*)]-25-(5-Propyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*R*,25(*S*)]-25-(5-Propyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*S*,25(*S*)]-25-(5-Propyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*R*,25(*R*)]-25-(5-Butyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*S*,25(*R*)]-25-(5-Butyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*R*,25(*S*)]-25-(5-Butyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*S*,25(*S*)]-25-(5-Butyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*R*,25(*R*)]-25-(5-Phenyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*S*,25(*R*)]-25-(5-Phenyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*R*,25(*S*)]-25-(5-Phenyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*S*,25(*S*)]-25-(5-Phenyl-2-oxazolin-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(5-Propyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(5-Propyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(5-Methyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(5-Methyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(5-Ethyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(5-Ethyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(5-Butyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(5-Butyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(5-Pentyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(5-Pentyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(5-Propylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(5-Propylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(5-Methylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(5-Methylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(5-Ethylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(5-Ethylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(5-Butylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(5-Butylthiazol-2-yl)-26,27-cyclo-9,10-secocholesta-5,7,10(19),22-tetraen-1,3,24-triol

(5*Z*,7*E*,22*E*)-(1*S*,3*R*,20*S*,24*R*)-25-(5-Pentylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(5-Pentylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(4-Propyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(4-Propyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(4-Methyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(4-Methyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(4-Ethyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(4-Ethyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(4-Butyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(4-Butyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(4-Pentyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(4-Pentyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(4-Propylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(4-Propylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(4-Methylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(4-Methylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(4-Ethylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(4-Ethylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(4-Butylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(4-Butylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(4-Pentylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(4-Pentylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(5-propyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(5-propyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(5-methyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(5-methyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Ethyloxazol-2-yl)-20-methyl-26,27-cyclo-9,10-secocholesta-5,7,10(19),22-tetraen-1,3,24-triol
(5*Z*,7*E*,22*E*)-(1*S*,3*R*,24*S*)-25-(5-Ethyloxazol-2-yl)-20-methyl-26,27-cyclo-9,10-secocholesta-5,7,10(19),22-tetraen-1,3,24-triol
(5*Z*,7*E*,22*E*)-(1*S*,3*R*,24*R*)-25-(5-Butyloxazol-2-yl)-20-methyl-26,27-cyclo-9,10-secocholesta-5,7,10(19),22-tetraen-1,3,24-triol

(5*Z*,7*E*,22*E*)-(1*S*,3*R*,24*S*)-25-(5-Butyloxazol-2-yl)-20-methyl-26,27-cyclo-9,10-secocholesta-5,7,10(19),22-tetraen-1,3,24-triol
(5*Z*,7*E*,22*E*)-(1*S*,3*R*,24*R*)-20-Methyl-25-(5-pentyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(5-pentyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(5-propylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(5-propylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(5-methylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(5-methylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Ethylthiazol-2-yl)-20-methyl-26,27-cyclo-9,10-secocholesta-5,7,10(19),22-tetraen-1,3,24-triol
(5*Z*,7*E*,22*E*)-(1*S*,3*R*,24*S*)-25-(5-Ethylthiazol-2-yl)-20-methyl-26,27-cyclo-9,10-secocholesta-5,7,10(19),22-tetraen-1,3,24-triol
(5*Z*,7*E*,22*E*)-(1*S*,3*R*,24*R*)-25-(5-Butylthiazol-2-yl)-20-methyl-26,27-cyclo-9,10-secocholesta-5,7,10(19),22-tetraen-1,3,24-triol
(5*Z*,7*E*,22*E*)-(1*S*,3*R*,24*S*)-25-(5-Butylthiazol-2-yl)-20-methyl-26,27-cyclo-9,10-secocholesta-5,7,10(19),22-tetraen-1,3,24-triol
(5*Z*,7*E*,22*E*)-(1*S*,3*R*,24*R*)-25-(5-Pentylthiazol-2-yl)-20-methyl-26,27-cyclo-9,10-secocholesta-5,7,10(19),22-tetraen-1,3,24-triol
(5*Z*,7*E*,22*E*)-(1*S*,3*R*,24*S*)-25-(5-Pentylthiazol-2-yl)-20-methyl-26,27-cyclo-9,10-secocholesta-5,7,10(19),22-tetraen-1,3,24-triol
(5*Z*,7*E*,22*E*)-(1*S*,3*R*,24*R*)-20-Methyl-25-(4-propyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(4-propyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(4-methyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(4-methyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Ethyloxazol-2-yl)-20-methyl-26,27-cyclo-9,10-secocholesta-5,7,10(19),22-tetraen-1,3,24-triol
(5*Z*,7*E*,22*E*)-(1*S*,3*R*,24*S*)-25-(4-Ethyloxazol-2-yl)-20-methyl-26,27-cyclo-9,10-secocholesta-5,7,10(19),22-tetraen-1,3,24-triol
(5*Z*,7*E*,22*E*)-(1*S*,3*R*,24*R*)-25-(4-Butyloxazol-2-yl)-20-methyl-26,27-cyclo-9,10-secocholesta-5,7,10(19),22-tetraen-1,3,24-triol
(5*Z*,7*E*,22*E*)-(1*S*,3*R*,24*S*)-25-(4-Butyloxazol-2-yl)-20-methyl-26,27-cyclo-9,10-secocholesta-5,7,10(19),22-tetraen-1,3,24-triol
(5*Z*,7*E*,22*E*)-(1*S*,3*R*,24*R*)-20-Methyl-25-(4-pentyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(4-pentyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(4-propylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(4-propylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(4-methylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(4-methylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Ethylthiazol-2-yl)-20-methyl-26,27-cyclo-9,10-secocholesta-5,7,10(19),22-tetraen-1,3,24-triol
(5*Z*,7*E*,22*E*)-(1*S*,3*R*,24*S*)-25-(4-Ethylthiazol-2-yl)-20-methyl-26,27-cyclo-9,10-secocholesta-5,7,10(19),22-tetraen-1,3,24-triol

(5*Z*,7*E*,22*E*)-(1*S*,3*R*,24*R*)-25-(4-Butylthiazol-2-yl)-20-methyl-26,27-cyclo-9,10-secocholesta-5,7,10(19),22-tetraen-1,3,24-triol
(5*Z*,7*E*,22*E*)-(1*S*,3*R*,24*S*)-25-(4-Butylthiazol-2-yl)-20-methyl-26,27-cyclo-9,10-secocholesta-5,7,10(19),22-tetraen-1,3,24-triol
(5*Z*,7*E*,22*E*)-(1*S*,3*R*,24*R*)-25-(4-Pentylthiazol-2-yl)-20-methyl-26,27-cyclo-9,10-secocholesta-5,7,10(19),22-tetraen-1,3,24-triol
(5*Z*,7*E*,22*E*)-(1*S*,3*R*,24*S*)-25-(4-Pentylthiazol-2-yl)-20-methyl-26,27-cyclo-9,10-secocholesta-5,7,10(19),22-tetraen-1,3,24-triol

Die natürlichen Vitamine $D_2$ und $D_3$ (vergl. allgemeine Formel Vitamin D) sind an sich biologisch inaktiv und werden erst nach Hydroxylierung am C-Atom 25 in der Leber und am C-Atom 1 in der Niere in biologisch aktive Metaboliten [1α,25-Dihydroxyvitamin $D_3$ (Calcitriol) bzw. -$D_2$] umgewandelt. Die Wirkung der aktiven Metaboliten besteht in der Regulation der Calcium- und Phosphatkonzentration im Serum; sie wirken einem Absinken der Calciumkonzentration im Serum entgegen, indem sie die Calciumabsorption im Darm erhöhen und unter bestimmten Umständen die Calciummobilisation aus dem Knochen fördern.

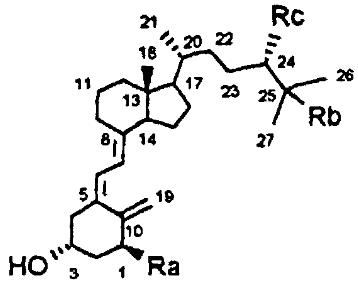

Ergocalciferol: Ra=Rb=H, Rc=$CH_3$ — Vitamin $D_2$
Doppelbindung C-22/23

Cholecalciferol: Ra=Rb=Rc=H — Vitamin $D_3$
25-Hydroxycholecalciferol: Ra=Rc=H, Rb=OH
1α-Hydroxycholecalciferol: Ra=OH, Rb=Rc=H
1α,25-Dihydroxycholecalciferol: Ra=Rb=OH, Rc=H — Calcitriol

Neben ihrer ausgeprägten Wirkung auf den Calcium- und Phosphatstoffwechsel besitzen die aktiven Metaboliten von Vitamin $D_2$ und $D_3$ und seine synthetischen Abkömmlinge eine proliferationshemmende und differenzierungsstimulierende Wirkung auf Tumorzellen und normale Zellen, wie zum Bespiel Hautzellen. Weiterhin wurde eine ausgeprägte Wirkung auf Zellen des Immunsystems (Hemmung der Proliferation und Interleukin 2-Synthese von Lymphocyten, Steigerung der Cytotoxizität und Phagocytose in vitro von Monocyten) gefunden, die sich in einer immunmodulatorischen Wirkung äußert, schließlich wird infolge einer fördernden Wirkung auf knochenbildende Zellen eine vermehrte Knochenbildung bei normalen und osteoporotischen Ratten gefunden [R. Bouillon et al. "Short term course of 1,25$(OH)_2D_3$ stimulates osteoblasts but not osteoclasts". *Calc. Tissue Int* **49,** 168 (1991)]

Alle Wirkungen werden durch Bindung an den Vitamin D-Rezeptor vermittelt. Infolge der Bindung wird die Aktivität von spezifischen Genen reguliert.

Bei Anwendung der biologisch aktiven Metaboliten von Vitamin $D_2$ und $D_3$ wird eine toxische Wirkung auf den Calciumstoffwechsel hervorgerufen (Hypercalcämie).

Durch strukturelle Manipulationen der Seitenkette können therapeutisch nutzbare Wirkqualitäten von der unerwünschten hypercalcamischen Aktivität abgetrennt werden. Eine geeignete Strukturvariante ist die Einführung von 24-Hydroxy-Derivaten.

In 24-Stellung hydroxylierte 1α-Cholecalciferole gehen bereits aus der DE 25 26 981 hervor. Sie besitzen eine geringere Toxizität als das entsprechende nicht-hydroxylierte 1α-Cholecalciferol. Darüberhinaus sind 24-Hydroxy-Derivate in folgenden Patentanmeldungen beschrieben: DE 39 33 034, DE 40 03 854, DE 40 34 730, EP 0 421 561, EP 0 441 467, WO 91/12238.

Aus DE-A-42 00 783 sind 24-Benzyl-substituierte Vitamin D Derivate bekannt.

Schließlich werden in der WO 94/07853 an C 24 hydroxylierte 25-Carbonsäure-Derivate von Calcitriol beschrieben, die ein günstigeres Wirkspektrum als Calcitriol aufweisen. Während die Fähigkeit zur Auslösung einer Hypercalcämie deutlich abgeschwächt ist, bleiben die proliferationshemmenden und differenzierungsstimuliernden Wirkungen erhalten.

Gegenüber diesen strukturell verwandten Verbindungen zeichnen sich einige der erfindungsgemäßen Substanzen dadurch aus, daß sie eine stärkere Wirkung auf die Zelldifferenzierung zeigen, wobei die Wirkung auf den Calciumhaushalt nicht zunimmt. Andere der erfindungsgemäßen Substanzen dagegen weisen ein antagonistisches

Wirkprofil auf, daß neue Anwendungen ermöglichen kann.

Die Vitamin D-Aktivität der erfindungsgemäßen Substanzen wird mittels des Calcitriol-Rezeptortests bestimmt. Er wird unter Verwendung eines spezifischen Rezeptorproteins aus dem Darm von jungen Schweinen durchgeführt.

Rezeptorhaltiges Bindungsprotein wird mit $^3$H-Calcitriol ($5x10^{-10}$ mol/l) in einem Reaktionsvolumen von 0,270 ml in Abwesenheit und in Anwesenheit der Prüfsubstanzen für zwei Stunden bei 4°C in einem Teströhrchen inkubiert. Zur Trennung von freiem und rezeptorgebundenem Calcitriol wird eine Charcoal-Dextran-Absorption durchgeführt. Dazu werden 250 µl einer Charcoal-Dextran-Suspension jedem Teströhrchen zugeführt und bei 4°C für 20 Min. inkubiert. Anschließend werden die Proben bei 10 000 x g 5 Minuten bei 4°C zentrifugiert. Der Überstand wird dekantiert und nach Istündiger Äquilibrierung in Picofluor 15 ™ in einem β-Zähler gemessen.

Die mit verschiedenen Konzentrationen der Prüfsubstanz sowie der Referenzsubstanz (unmarkiertes Calcitriol) bei konstanter Konzentration der Bezugssubstanz ($^3$H-Calcitriol) erhaltenen Kompetitionskurven werden in Beziehung zueinander gesetzt und ein Kompetitionsfaktor (KF) ermittelt.

Er ist definiert als Quotient aus den Konzentrationen der jeweiligen Prüfsubstanz und der Referenzsubstanz, die für 50%ige Kompetition erforderlich sind:

$$KF = \frac{\text{Konzentration Prüfsubstanz bei 50\% Kompetition}}{\text{Konzentration Referenzsubstanz bei 50\% Kompetition}}$$

Den erfindungsgemäßen Verbindungen ist gemein, daß sie alle über eine beträchtliche Affinität zum Calcitriol-Rezeptor verfügen.

Zur Bestimmung der akuten hypercalcämischen Wirkung verschiedener Calcitriolderivate wird nachfolgend beschriebener Test durchgeführt:

Die Wirkung von Kontrolle (Lösungsgrundlage), Referenzsubstanz ($1,25(OH)_2$-$D_3$=Calcitriol) und Testsubstanz wird jeweils nach einmaliger subcutaner Applikation in Gruppen von 10 gesunden männlichen Ratten (140-170 g) getestet. Die Ratten werden während der Versuchszeit in speziellen Käfigen zur Bestimmung der Exkretion von Wasser und Mineralstoffen gehalten. Der Harn wird in 2 Fraktionen (0-16 h und 16-22 h) gesammelt. Eine orale Calciumgabe (0.1 mM Calcium in 6,5% Alpha-hydroxypropylcellulose, 5 ml/Tier) ersetzt zum Zeitpunkt 16 h die durch Futterentzug fehlende Calciumaufnahme. Zu Versuchsende werden die Tiere durch Dekapitieren getötet und für die Bestimmung der Serum-Calciumwerte entblutet. Für die primäre Screen-Untersuchung in vivo wird eine einzelne Standarddosis (200 µg/kg) getestet. Für ausgewählte Substanzen wird das Ergebnis durch Erstellung einer Dosis-Wirkungs-Beziehung abgesichert.

Eine hypercalcämische Wirkung zeigt sich in im Vergleich zur Kontrolle erhöhten Serum-Calciumspiegel-Werten.

Die Signifikanz aufiretender Unterschiede zwischen Substanzgruppen und Kontrollen sowie zwischen Testsubstanz und Referenzsubstanz werden mit geeigneten statistischen Verfahren abgesichert. Das Ergebnis wird als Dosisrelation DR (DR = Faktor Testsubstanzdosis/Referenzsubstanzdosis für vergleichbare Wirkungen) angegeben.

Die differenzierungsstimulierende Wirkung von Calcitriolanaloga wird ebenfalls quantitativ erfaßt.

Es ist literaturbekannt [Mangelsdorf, D.J. et al., *J. Cell. Biol.* **98,** 391 (1984)], daß die Behandlung humaner Leukämiezellen (Promyelozytenzellinie HL 60) in vitro mit Calcitri-ol die Differenzierung der Zellen zu Makrophagen induziert.

HL 60-Zellen werden in Gewebekulfurmedium (RPMI 10% fetales Kälberserum) bei 37°C in einer Atmosphäre 5% $CO_2$ in Luft kultiviert.

Zur Substanztestung werden die Zellen abzentrifugiert und $2,0 \times 10^5$ Zellen/ml in phenolrotfreiem Gewebekulturmedium aufgenommen. Die Testsubstanzen werden in Ethanol gelöst und mit Gewebekulturmedium ohne Phenolrot auf die gewünschte Konzentration verdünnt. Die Verdünnungsstufen werden mit der Zellsuspension in einem Verhältnis von 1:10 gemischt und je 100 µl dieser mit Substanz versetzten Zellsuspension in eine Vertiefung einer 96-Loch-Platte pipettiert. Zur Kontrolle wird eine Zellsuspension analog mit dem Lösungsmittel versetzt.

Nach Inkubation über 96 Stunden bei 37°C in 5% $CO_2$ in Luft wird in jede Vertiefung der 96-Loch-Platte zu der Zellsuspension 100 µl einer NBT-TPA-Lösung (Nitroblautetrazolium (NBT), Endkonzentration im Ansatz 1 mg/ml, Tetradecanoylphorbolmyristat-13-acetat (TPA), Endkonzentration im Ansatz $2 \times 10^{-7}$ mol/l) pipettiert.

Durch Inkubation über 2 Stunden bei 37°C und 5% $CO_2$ in Luft wird infolge der intrazellulären Sauerstoffradikalfreisetzung, stimuliert durch TPA, in den zu Makrophagen differenzierten Zellen NBT zu unlöslichem Formazan reduziert.

Zur Beendigung der Reaktion werden die Vertiefungen der 96-Loch-Platte abgesaugt und die Zellen durch Zugabe von Methanol am Plattenboden fixiert und nach Fixation getrocknet. Zur Lösung der gebildeten intrazellularen Formazankristalle werden in jede Vertiefung 100 µl Kaliumhydroxid (2 mol/l) und 100 µl Dimethylsulfoxid pipettiert und 1 Minute ultrabeschallt. Die Konzentration von Formazan wird spektralphotometrisch bei 650 nm gemessen.

Als Maß für die Differenzierungsinduktion der HL 60-Zellen zu Makrophagen gilt die Konzentration an gebildetem

Formazan. Das Ergebnis wird als Dosisrelation (DR = Faktor Testsubstanzdosis/Referenzsubstanzdosis für vergleichbare halbmaximale Wirkungen) angegeben.

Die Ergebnisse des Calcitriol-Rezeptortests sowie der Bestimmung der Dosisrelation der Differenzierungsinduktion von HL 60-Zellen und der Dosisrelation für Hypercalcämie sind nachfolgend zusammengefaßt:

Testverbindungen:

(5*Z*,7*E*,22*E*)-(1*S*,3*R*,24*R*)-25-(5-Propyloxazol-2-yl)-26,27-cyclo-9,10-secocholesta-5,7,10(19),22-tetraen-1,3,24-triol **12b**
(5*Z*,7*E*,22*E*)-(1*S*,3*R*,24*R*)-25-(5-Methyloxazol-2-yl)-26,27-cyclo-9,10-secocholesta-5,7,10(19),22-tetraen-1,3,24-triol **13b**
(5*Z*,7*E*,22*E*)-(1*S*,3*R*,24*R*)-25-(5-Ethyloxazol-2-yl)-26,27-cyclo-9,10-secocholesta-5,7,10(19),22-tetraen-1,3,24-triol **14b**
(5*Z*,7*E*,22*E*)-(1*S*,3*R*,24*R*)-25-(5-Pentyloxazol-2-yl)-26,27-cyclo-9,10-secocholesta-5,7,10(19),22-tetraen-1,3,24-triol **15b**

Vergleichsverbindung:

Calcitriol

| Verbindung | Kompetitionsfaktor KF für Rezeptorbindung | Dosisrelation für Differenzierungsinduktion in HL 60-Zellen |
|---|---|---|
| **12b** | 2 | 1.9 |
| **13b** | 2 | 3.4 |
| **14b** | 3 | 1.3 |
| **15b** | 4 | >100 |
| Calcitriol | 1 | 1 |

Die aufgeführten Verbindungen zeigen neben einer Affinität zum Vitamin D-Rezeptor, die der von Calcitriol vergleichbar ist, zum Teil eine ebenso vergleichbare zelldifferenzierende Aktivität.

Die Induktion einer Hypercalcämie erfolgt dagegen erst bei sehr viel höheren Dosen als bei Calcitriol (Dosisrelation für **14** = 300; Calcitriol DR =1).
Durch die verminderte Eigenschaft, eine Hypercalcämie auszulösen, eignen sich die erfindungsgemäßen Substanzen in besonderer Weise zur Herstellung von Arzneimitteln für die Behandlung von Erkrankungen, die durch eine Hyperproliferation und fehlende Zelldifferenzierung gekennzeichnet sind. Dazu zählen zum Beispiel hyperproliferative Erkrankungen der Haut (Psoriasis, Pituriasis subia pilasis, Akne, Ichthyosis) sowie Tumorerkrankungen und Präkanzerosen (zum Beispiel Darmtumoren, Mammakarzinom, Lungentumoren, Prostatakarzinom, Leukämien, T-Zell-Lymphome, Melanome, Batazell Karzinom, Squamous Carcinoma, aktinische Keratosen, Cervixdysplasien, metastasierende Tumore jeglicher Art).

Auch zur Behandlung und Prophylaxe von Erkrankungen, die durch eine Störung des Gleichgewichts des Immunsystems gekennzeichnet sind, eignen sich die erfindungsgemäßen Substanzen. Hierzu zählen Ekzeme und Erkrankungen des atopischen Formonkreises, sowie Autoimmunerkrankungen wie zum Beispiel Multiple Sklerose, Diabetes mellitus Typ I, Myasthenia gravis, Lupus erythematodes, Sklerodermie, bullöse Hauterkrankungen (Pemphigus, Pemphigoid), weiterhin Abstoßungsreaktionen bei autologen, allogenen oder xenogenen Transplantaten sowie AIDS. Bei all diesen Erkrankungen können die neuen Verbindung der allgemeinen Formel I vorteilhaft mit anderen immunsuppressiv wirksamen Stoffen wie Cyclosporin A, FK 506, Rapamycin und Anti-CD 4-Antikörpern kombiniert werden.

Ebenso sind die Substanzen geeignet zur Therapie von sekundärem Hyperparathyreoidismus und renaler Osteodystrophie infolge der Eigenschaft von Calcitriolen, die Parathormonsynthese zu senken.

Aufgrund der Präsenz des Vitamin D-Rezeptor in den insulinproduzierenden Zellen der Bauchspeicheldrüse eignen sich die Substanzen durch Erhöhung der Insulinsekretion zur Therapie des Diabetes mellitus Typ II.

Weiterhin wurde überraschenderweise gefunden, daß durch topische Applikation der erfindungsgemäßen

Verbindungen auf die Haut von Mäusen, Ratten und Meerschweinchen eine vermehrte Hautrötung und Zunahme der Epidermisdicke induziert werden kann. Die Zunahme der Hautrötung wird anhand der Erhöhung des mit einem Farbmeßgerät quantifizierbaren Rotwertes der Hautoberfläche ermittelt Der Rotwert ist nach dreimaliger Substanzapplikation (Dosis 0,003%) im Abstand von 24 Stunden typischerweise um das 1,5-fache erhöht. Die Zunahme der Epidermisdicke wird im histologischen Präparat quantifiziert. Sie ist typischerweise um das 2,5-fache erhöht. Die Anzahl der proliferierenden Epidermiszellen (Zellen in der S-Phase des Zellcyclus) wird durchflußcytometrisch ermittelt und ist typischerweise um den Faktor 6 erhöht. Diese Eigenschaften der erfindungsgemäßen Derivate in der Vitamin D-Reihe läßt sie zum therapeutischen Einsatz bei atrophischer Haut, wie sie bei natürlicher Hautalterung infolge erhöhter Lichtexposition oder medikamentös induzierter Hautatrophie durch Behandlung mit Glucocorticoiden auftritt, geeignet erscheinen.

Weiterhin ist anzunehmen, daß die Wundheilung durch topische Applikation mit den neuen Verbindungen beschleunigt werden kann.

In Zellpopulationen das Haarfollikels, die entscheidend zum Haarwachstum bzw. der Haarzyklusregulation beitragen, konnten Vitamin $D_3$-Rezeptorproteine nachgewiesen werden [Stumpf, W. E. et al., *Cell Tissue Res.* **238,** 489 (1984); Milde, P. et al., *J. Invest. Dermatol.* **97,** 230 (1991)]. Außerdem zeigen in vitro-Befunde an isolierten Haarfollikelkeratinozyten einen proliferationsinhibierenden und differenzierungsstimmulierenden Einfluß von 1,25-$(OH)_2$-$D_3$.

Aus klinischen Beobachtungen ist bekannt, daß die Vitamin $D_3$-resistente Rachitis häufig mit einer Alopezie einhergeht, die sich im frühen Kindesalter ausprägt. Experimentelle Befunde zeigen, daß die Vitamin $D_3$-Bindungsstelle des VDR bei dieser Erkrankung mutiert, d. h. defekt ist [Kristjansson. K. et al., *J. Clin. Invest.* **92,** 12 (1993)]. Keratinozyten, die aus den Haarfollikeln dieser Patienten isoliert wurden, reagieren in vitro nicht auf die Zugabe von 1,25-$(OH)_2$-$D_3$ [Arase, S. et al., *J. Dermatol. Science* **2***, 353* 1991)].

Aus diesen Befunden läßt sich eine entscheidende Rolle von 1,25-$(OH)_2$-$D_3$ auf die Regulation des Haarwuchstums ableiten.

Daher eignen sich diese Analoga besonders zur Herstellung von Arzneimitteln zur Behandlung von Erkrankungen, die mit einem gestörten Haarwachstum einhergehen (androgenetische Alopezie, Alopezia areata/totalis, chemotherapie-induzierte Alopezie), oder zur Unterstützung des physiologischen Haarwachstums.

Die senile und postmenopausale Osteoporose ist gekennzeichnet durch einen erhöhten Knochenumsatz mit einer insgesamt negativen Bilanz. Aufgrund des Knochenschwundes insbesondere von trabekulärem Knochen kommt es in verstärktem Maße zu Knochenbrüchen. Aufgrund der fördernden Wirkung von Calcitriol sowohl auf die Anzahl als auch die Syntheseleistung von knochenneubildenden Zellen (Osteoblasten) eignen sich die erfindungsgemäßen Substanzen zur Therapie und Prophylaxe der senilen und postmenopausalen Osteoporose (EP 0 634 173 A1), der steroidinduzierten Osteoporose sowie zur beschleunigten Einheilung von Gelenkplastiken. Für die Therapie der verschiedenen Formen der Osteoporose können sie vorteilhaft mit Estradiol oder anderen Abkommlingen des Östrogens kombiniert werden.

Schließlich konnte gezeigt werden, daß Calcitriol die Synthese eines Wuchsstoffes für Nervenzellen (nerve growth factor) steigert [M.S. Saporito et al. *Brain Res.* **633**, 189 (1994)]. Daher eignen sich die erfindungsgemäßen Verbindungen auch zur Behandlung von degenerativen Erkrankungen des peripheren und zentralen Nervensystems, wie der Alzheimerschen Erkrankung und der amyotrophen Lateralsklerose.

Es wurde außerdem gefunden, daß bestimmte Verbindungen der allgemeinen Formel **I** in HL 60-Zellen überraschenderweise die Wirkung von Calcitriol antagonisieren. In der Reihe der 25-Oxazol-Derivate zeigen die Verbindungen mit zunehmender Kettenlänge am Heterocyclus bei gleichbleibend guter Rezeptoraffinität deutlich schwächere differenzierungsstimulierende agonistische Aktivität in HL 60-Zellen (Tab.1). Die Verbindungen **16** und **17** antagonisieren die Wirkung von Calcitriol in HL 60-Zellen. Diese Eigenschaft setzt sich mit zunehmender Kettenlänge im Rest Z der allgemeinen Formel I fort.

Solche Verbindungen, die die Wirkung von Calcitriol antagonisieren, können bei der Therapie von Hypercalcämien eingesetzt werden, wie zum Beispiel bei Hypervitaminose D oder Intoxikation mit Calcitriol und calcitriolartig wirksamen Substanzen, oder bei erhöhter extrarenaler Calcitriolsynthese bei granulomatösen Erkrankungen (Sarkoidose, Tuberkulose). Auch paraneoplastische Hypercalcämien (zum Beispiel bei osteolytischen Metastasen und Tumoren mit erhöhter Synthese von Parathormon-related peptide) sowie bei Hypercalcämie bei Hyperparathyreoidismus.

Weiterhin sind Calcitriolantagonisten zur Fertilitätskontrolle einzusetzen. Im Reproduktionstrakt weiblicher und mannlicher Tiere wird der Vitamin D-Rezeptor exprimiert. Es ist bekannt, daß die weibliche und männliche Fertilität Vitamin D-defizienter Tiere herabgesetzt ist. Durch kurzfristige Substitution von Calcitriol kann die Reproduktionsleistung erhöht werden. Daher sind Calcitriolantagonisten in der Lage, die weibliche und männliche Fertilität zu beeinflussen.

Da Calcitriol unter bestimmten Bedingungen eine immunsuppressive Wirkung zeigt, sind Calcitriolrezeptorantagonisten auch als Immunstimulantien, z. B. bei Infektabwehrschwäche, einzusetzen.

Von Calcitriol ist bekannt, daß es das Haarwachstum modulieren kann. Calcitriolantagonisten können daher

bei unerwünschtem Haarwachstum, z. B. beim Hirsutismus, therapeutische Verwendung finden.

Eine fördernde Rolle von Vitamin D auf die Bildung von arteriosklerotischen Plaques ist seit langem bekannt. In solchen Gefäßläsionen wird ein Calcitriol-reguliertes Protein, das Osteopontin, vermehrt gefunden, dem eine Rolle bei der Gefäßverkalkung zugeschrieben wird [R. Eisenstein et al. *Arch. Path.* **77**, 27 (1964), L.A. Fitzpatrick et al. *J. Clin. Invest.* **94**, 1597 (1994)]. Deshalb eignen sich Calcitriolantagonisten zur Therapie und Prophylaxe aller Erscheinungsformen der Arteriosklerose.

Schließlich eignen sich Calcitriolantagonisten infolge der Eigenschaft von Calcitriol, unspezifische Immunreaktionen von monocytären Zellen zu steigern, zur Therapie von entzündlichen Erkrankungen insbesondere chronischer Natur, wie rheumatoide Arthritis, Morbus Crohn, Colitis ulcerosa, und granulomatösen Erkrankungen wie Sarkoidose und anderen Fremdkörperreaktionen.

Die vorliegende Erfindung bezieht sich somit auf pharmazeutische Präparate, die mindestens eine Verbindung gemäß der allgemeinen Formel I zusammen mit einem pharmazeutisch vertäglichen Träger enthalten.

Die Verbindungen können formuliert werden als Lösungen in pharmazeutisch verträglichen Solventien oder als Emulsionen, Suspensionen oder Dispensionen in geeigneten pharmazeutischen Solventien oder Trägern oder als Pillen, Tabletten oder Kapseln, die in an sich bekannter Weise feste Trägerstoffe enthalten. Für eine topische Anwendung werden die Verbindungen vorteilhafterweise als Cremes oder Salben oder in einer ähnlichen, zur topischen Anwendung geeigneten Arzneimittelform formuliert. Jede derartige Formulierung kann auch andere pharmazeutisch verträgliche und nichttoxische Hilfsstoffe enthalten, wie z. B. Stabilisatoren, Antioxidantien, Bindemittel, Farbstoffe, Emulgatoren oder Geschmackskorrigentien. Die Verbindungen werden vorteilhafterweise durch Injektion oder intravenöse Infusion geeigneter steriler Lösungen oder als orale Dosierung über den Emährungstrakt oder topisch in der Form von Cremes, Salben, Lotions oder geeigneter transdermaler Pflaster appliziert, wie in der EP-A 0 387 077 beschrieben ist.

| | |
|---|---|
| Die tägliche Dosis liegt bei | 0,1 µg/Patient/Tag - 1000 µg (1 mg)/Patient/Tag, |
| vorzugsweise | 1,0 µg/Patient/Tag - 500 µg/Patient/Tag. |

In der Patentanmeldung WO 97/00242 (Schering AG) sind bereits Vitamin D-Derivate mit Substituenten an C-25 beschrieben. In allen Fällen ist der Substituent aber durch eine Carbonylgruppe, eine Hydroxymethylgruppe oder eine Doppelbindung an das Kohlenstoffatom 25 geknüpft. In keinem Fall erfolgte die Anknüpfung des Kohlenstoffatoms 25 direkt an einen Carbo- oder Heterocyclus. Die beschriebenen Synthesewege erlauben den Aufbau derartiger Substitutionsmuster nicht, so daß neue Verfahren entwickelt werden mußten.

Die Herstellung der Vitamin D-Derivate der allgemeinen Formel **I** erfolgt erfindungsgemäß aus einer Verbindung der allgemeinen Formel **II**,

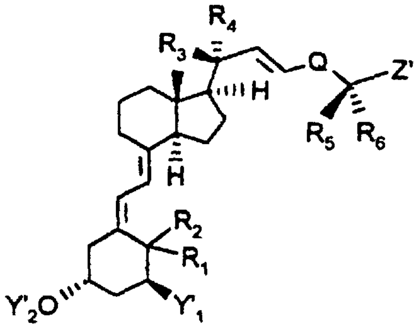

**II**

worin $Y'_1$ ein Wasserstoffatom oder eine geschützte Hydroxylgruppe und $Y'_2$ eine Hydroxyschutzgruppe bedeuten.
Z' unterscheidet sich von Z dadurch, daß eventuell vorliegende Hydroxylgruppen in geschützter Form vorliegen können. Bei den Schutzgruppen handelt es sich vorzugsweise um alkyl-, aryl- oder gemischt alkylarylsubstituierte Silylgruppen, z.B. die Trimethylsilyl- (TMS), Triethylsilyl- (TES), *tert.*-Butyldimethylsilyl- (TBDMS), *tert.*-Butyldiphenylsilyl- (TBDPS) oder Triisopropylsilylgruppen (TIPS) oder eine andere gängige Hydroxyschutzgruppe (Methoxymethyl-, Methoxyethoxymethyl-, Ethoxyethyl-, Tetrahydrofuranyl-Tetrahydropyranyl-Gruppen oder siehe T.W. Greene, P.G.M. Wuts "Protective Groups in Organic Synthesis", 2nd Edition, John Wiley & Sons, 1991).

Durch gleichzeitige oder sukzessive Abspaltung der Hydroxyschutzgruppen und gegebenenfalls durch partielle, sukzessive oder vollständige Veresterung der freien Hydroxylgruppen wird **II** in eine Verbindung der allgemeinen

Formel **I** überführt.
Im Falle der Silylschutzgruppen oder der Trimethylsilylethoxymethylgruppe verwendet man zu deren Abspaltung Tetrabutylammoniumfluorid, Fluorwasserstoffsäure oder Fluorwasserstoffsäure/Pyridin; im Falle von Ethergruppen (Methoxymethyl-, Methoxyethoxymethyl-, Ethoxyethyl-, Tetrahydropyranylether) werden diese unter katalytischer Einwirkung von Säure, beispielsweise p-Toluolsulfonsäure, Pyridinium-ptoluolsulfonat, Essigsäure, Salzsäure, Phosphorsäure oder einem sauren Ionenaustauscher abgespalten.
Die Veresterung der freien Hydroxygruppen kann nach gängigen Verfahren mit den entsprechenden Carbonsäurechloriden, -bromiden oder -anhydriden erfolgen.
Die Herstellung der Ausgangsverbindungen für die allgemeine Formel **II** geht je nach letztendlich gewünschtem Substitutionsmuster in 10- und 20-Position von verschiedenen Startverbindungen aus.

Für die Herstellung von Verbindungen der allgemeinen Formel **II,** worin $R_1$ und $R_2$ gemeinsam eine exocyclische Methylengruppe bedeuten, wird von dem bekannten Aldehyd **III** ausgegangen [M. Calverley Tetrahedron **43,** 4609 (1987), WO 87/00834].

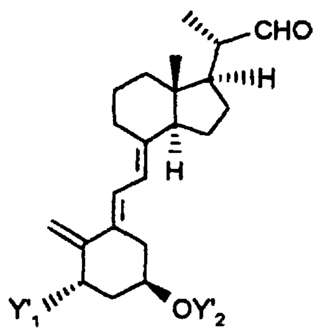

**III**

Für $Y'_1$ und $Y'_2$ gelten die schon erwähnten Definitionen. Andere Schutzgruppen als die in den Literaturstellen erwähnten lassen sich durch analoge Vorgehensweise unter Verwendung entsprechend modifizierter Silylchloride (z. B. *tert.*-Butyldiphenylsilylchlorid anstelle von *tert.*-Butyldimethylsilylchlorid) erhalten. Durch Verzicht auf die entsprechenden Stufen zur 1α-Hydroxylierung lassen sich Derivate vom Typ $Y'_1$=H erhalten.
Die Verbindungen der allgemeinen Formel **III** werden nun analog bekannter Verfahren in Aldehyde der allgemeinen Formel **IV** überführt [EP 647 219, WO 94/07853, M.J. Calverley, L. Binderup *Bioorg. Med. Chem. Lett.* **3,** 1845-1848 (1993)].

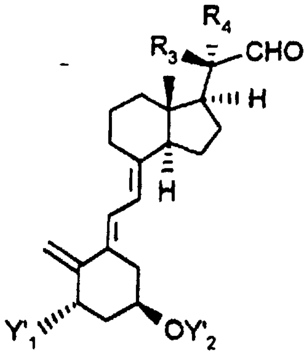

**IV**

Für $R_3$ und $R_4$ gelten die schon eingangs erwähnten Definitionen.
Zum Aufbau der Seitenkette können nun sowohl Verbindungen der allgemeinen Formel **III** als auch Verbindungen der allgemeinen Formel **IV** eingesetzt werden.
Analog der etablierten Sequenz (WO 94/07853) können so Carbonsäureamide der allgemeinen Formel **V** generiert werden,

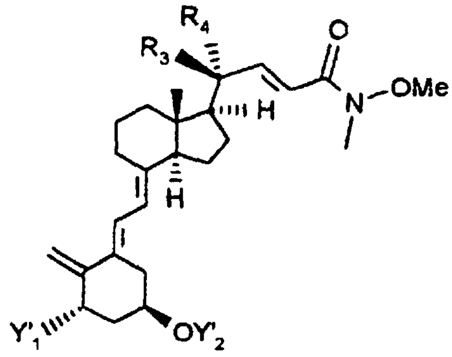

**V**

wobei für $Y'_1$, $Y'_2$, $R_3$ und $R_4$ die bereits gegebenen Definitionen gelten.

Zur Etablierung des natürlichen Vitamin D-Triensystems wird eine photochemische Isomerisierung von Verbindungen der allgemeinen Formel **V** vorgenommen. Bestrahlung mit ultraviolettem Licht erfolgt in Gegenwart eines sogenannten Triplettsensibilisators. Im Rahmen der vorliegenden Erfindung wird dafür Anthracen verwendet. Durch Spaltung der $\pi$-Bindung der 5,6-Doppelbindung, Rotation des A-Ringes um 180° um die 5,6-Einfachbindung und Reetablierung der 5,6-Doppelbindung wird die Stereoisomerie an der 5,6-Doppelbindung umgekehrt, wobei Verbindungen der allgemeinen Formel **VI** anfallen,

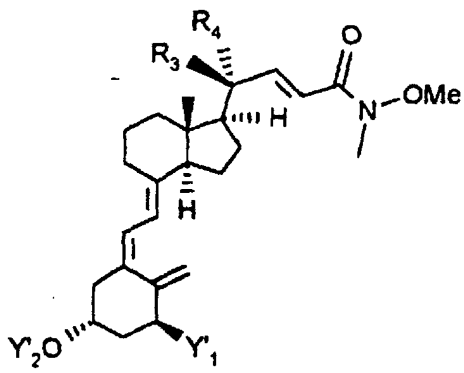

**VI**

wobei $Y'_1$, $Y'_2$, $R_3$ und $R_4$ die genannten Bedeutungen haben. Mit einem Reduktionsmittel (z.B. Lithiumaluminiumhydrid oder Diisobutylaluminiumhydrid) wird bei tiefer Temperatur (-60°C bis -100°C) in einem Lösungsmittel wie Tetrahydrofuran oder einem anderen Ether die Amidfunktion in der Verbindung der allgemeinen Formel **VI** zum Aldehyd reduziert, wobei eine Verbindung der allgemeinen Formel **VII** anfällt,

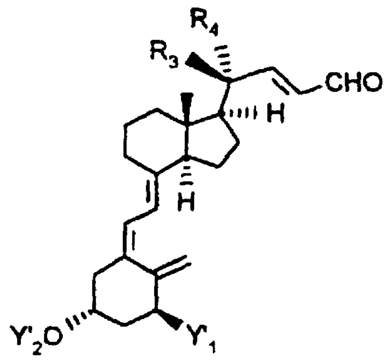

**VII**

wobei die Reste $Y'_1$, $Y'_2$, $R_3$ und $R_4$ die bereits genannten Bedeutungen haben.
An der Verbindung der allgemeinen Formel **VII** erfolgt nun der weitere Aufbau der Seitenkette. Exemplarisch wird im Folgenden die Verwendung des Aldehyds der allgemeinen Formel **VII** beschrieben für den gilt: $R_3$=H und $R_4$=Methyl. Sinngemäß gelten die nachfolgenden Umsetzungsmöglichkeiten aber auch für alle anderen Definitionen für $R_3$ und $R_4$.
Für die Synthese von Calcitriol-Derivaten mit 26,27-Cyclomodifikation, die daneben Oxazol-Substituenten an C-25 besitzen, kann der folgende Syntheseweg beschritten werden.
Geeignete Seitenkettenfragmente können ausgehend von 1-Bromcyclopropancarbonsäure **VIII** [H.M.R. Hoffmann et al. *J. Org. Chem.* **54,** 6096 (1989)] präpariert werden.

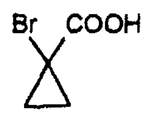

**VIII**

Zuerst erfolgt die Umsetzung mit Aminoketonen oder deren Hydrochloriden [M. Jackson et al. *J. Am. Chem. Soc.* **70,** 2884 (1948), J.D. Hepworth *Org. Synth.* **45,** 1 (1965)] der allgemeinen Formel **IX** unter Kondensationsbedingungen (z.B. N,N'-Dicyclohexylcarbodiimid, Triethylamin),

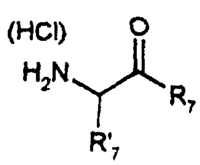

**IX**

wobei $R_7$ und $R'_7$ unabhängig voneinander ein Wasserstoffatom, einen geradkettigen oder verzweigten, gesättigten oder ungesättigten Alkylrest mit bis zu 12 Kohlenstoffatomen bedeuten, der an beliebigen Stellen durch Oxa-, Thia- oder Azafunktionen (substituiert oder unsubstituiert) oder Sulfoxid oder Sulfongruppen unterbrochen sein kann oder weitere Substituenten (freie oder geschützte Hydroxygruppen, Halogenatome) tragen kann. So entstehen Derivate der allgemeinen Formel X.

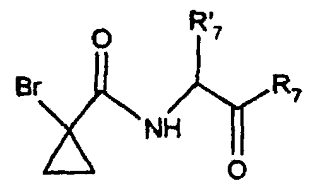

X

Alternativ können auch Aminoalkohole bzw geschützte Aminocarbonylverbindungen mit der Carbonsäure **VIII** umgesetzt werden. Die Bildung von Derivaten der allgemeinen Formel X erfolgt dann durch Oxidation oder Schutzgruppenspaltung.
Die Bildung des Oxazolringes kann nun unter Einwirkung von Säure erfolgen (z. B. Schwefelsäure, Phosphorsäure, Polyphosphorsäure), wobei Derivate der allgemeinen Formel **XI** anfallen. Speziell für den Fall $R_7$=H sind schonendere Methoden zur Oxazol-Bildung vorzuziehen [z.B. $PPh_3$, $NEt_3$, $I_2$ oder $C_2Cl_6$, C.J. Moody et al. *Synlett* 825 (1996), W. Steglich et al. *Lieb. Ann.* 1916 (1978)].

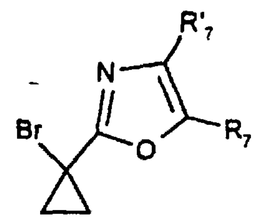

XI

Der Aufbau der Seitenkette erfolgt nun durch Reaktion der durch Brom-Metallaustausch aus **XI** generierten Cyclopropyl-Metallverbindung und dem Vitamin D-C-24-Aldehyd **VII**.
Günstig ist hier die Verwendung von *n*-Butyllithium oder *tert*.-Butyllithium in Hexan, Diethylether, Tetrahydrofuran oder Gemischen dieser Lösungsmittel bei Temperaturen zwischen -100°C und -50°C. So erhält man Derivate der allgemeinen Formel **XII**.

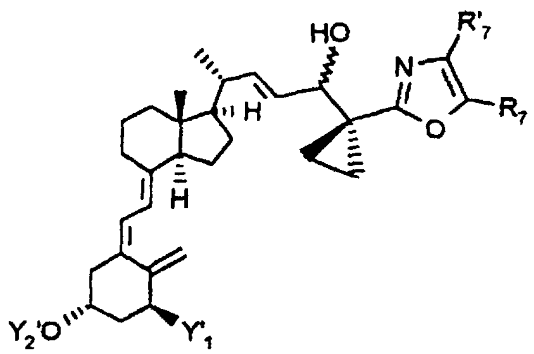

XII

Diese können als Spezialfälle der allgemeinen Formel **II** angesehen werden, deren weitere Umsetzung bereits beschrieben wurde und fiir die gilt, daß Q eine Hydroxymethylengruppe ist, $R_1$ und $R_2$ gemeinsam eine Methylengruppe bilden, $R_5$ und $R_6$ gemeinsam mit dem Kohlenstoffatom C-25 einen Cyclopropylring bilden und Z' ein Oxazol mit dem Substiuenten $R_7$ oder $R'_7$, die zuvor definiert worden sind, ist.
Die Diastereomeren bezüglich dem Kohlenstoffatom C-24 können auf dieser oder einer späteren Stufe chromatographisch getrennt werden.

Ein ähnlicher Syntheseweg kann zu Calcitriol-Derivaten führen, die Thiazol-Substituenten an C-25 tragen. Das Derivat der allgemeinen Formel **X** muß dann in Gegenwart eines Schwefelreagenzes wie z. B. Phosphorpentasulfid umgesetzt werden, wobei ThiazolDerivate der allgemeinen Formel **XIII** anfallen

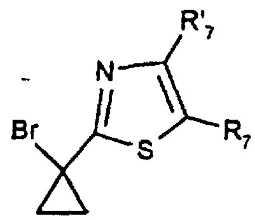

**XIII**

Alternativ könnte man Derivate der allgemeinen Formel **XIII** für die $R_7$=Wasserstoff ist und $R'_7$ die zuvor genannte Bedeutung hat durch Umsetzung des zur Carbonsäure **VIII** korrespondierenden Amides **VIII'** [H.M.R. Hoffmann et al. *J. Org. Chem.* **54,** 6096 (1989)]

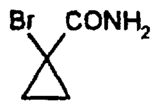

**VIII'**

mit 1-Bromketonen ($BrCH_2$-CO-$R'_7$) in Gegenwart eines Schwefelreagenzes wie Phoshorpentasulfid erhalten [R. Kurkjy et al. *J. Am. Chem. Soc.* **74,** 5778 (1952), G. Schwarz *Org. Synth.* Coll. Vol. III, 332].
Zur Synthese von Derivaten der allgemeinen Formel **XIII** für die $R_7$ die zuvor genannte Bedeutung haben kann und $R'_7$=Wasserstoff ist muß dagegen das Amid **VIII'** mit 2-Bromaldehyden (OHC-CHBr-$R_7$) in Gegenwart von Phosphorpentasulfid umgesetzt werden.

Zur Synthese von Calcitriol-Derivaten, die Imidazol-Substituenten an C-25 tragen, kann das Derivat der allgemeinen Formel **X** in Gegenwart von primären Aminen vom Typ $R_8NH_2$ umgesetzt werden, wobei Imidazol-Derivate der allgemeinen Formel **XIV** entstehen.

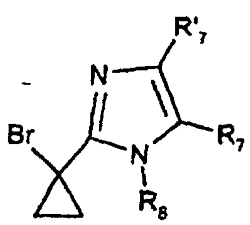

**XIV**

Der Substituent $R_8$ kann ein Wasserstoffatom oder ein geradkettiger oder verzweigter, gesättigter oder ungesättigter Alkylrest mit bis zu 12 Kohlenstoffatomen sein.
Daneben kann der Aufbau von Imidazolsystemen auch aus dem zur Carbonsäure **VIII** korrespondierenden Amidin **VIII''**

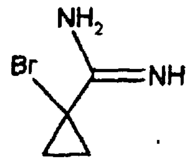

VIII''

mit 1-Bromketonen (Br-$CH_2$-CO-$R_7$) oder 2-Bromaldehyden (OHC-CHBr-$R'_7$) erfolgen.

Die Anknüpfung der Thiazol- oder Imidazol-Fragmente kann wie fiir den Oxazol-Fall beschrieben erfolgen, wobei Derivate der allgemeinen Formel **XV** entstehen. Im Fall mehrere azider Wasserstoffatome muß entweder eine geeignete Schutzgruppentechnik zur Anwendung kommen oder durch Einstellen sehr tiefer Temperaturen (-130 bis -100°C) der selektive Halogen/Lithiumaustausch am Bromcyclopropan durchgeführt werden.

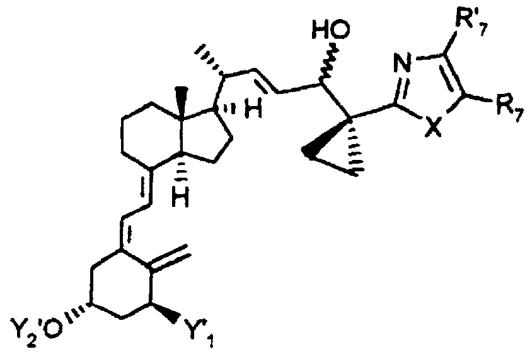

XV

In der allgemeinen Formel **XV** kann **X** ein Schwefelatom oder die zuvor definierte Einheit N-$R_8$ bedeuten. Alle weiteren Reste und die Weiterbehandlung der Derivate der allgemeinen Formel **XV** sind zuvor beschrieben worden.

Ausgehend von der Carbonsäure **VIII** oder Derivaten davon (Ester, Amide, Säurechloride) kann man unter Standardreaktionsbedingungen [Einsatz funktionalisierter metallorganischer Reagenzien, siehe z.B. M. Yus et al *J. Org. Chem*. **56,** 3825 (1991), J. Barluenga et al. *J. Chem. Soc. Perk. I* 3113 (1988), gefolgt von Manipulation der funktionellen Gruppen wie z.B. Ketalspaltungen] 1,4-Diketone der allgemeine Formel **XVI** generieren.

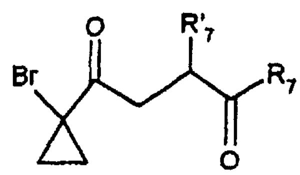

XVI

Durch Einsatz der schon für Umsetzung der Derivate der allgemeinen Formel **X** verwendeten Reaktionsbedingungen (Einwirkung von Säure, Phosphorpentasulfid o.ä. bzw. primären Aminen vom Typ $R_8NH_2$) können Furan-, Thiophen oder Pyrrol-Derivate der allgemeinen Formel **XVII** erhalten werden,

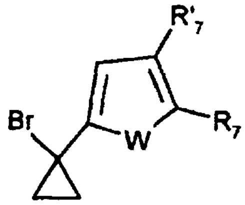

XVII

wobei W ein Sauerstoffatom, ein Schwefelatom oder die Gruppe N-$R_8$ bedeuten kann.
Die Anknüpfung an das Vitamin D-System erfolgt analog den zuvor beschriebenen Fällen, wobei Derivate der allgemeinen Formel **XVIII** anfallen.

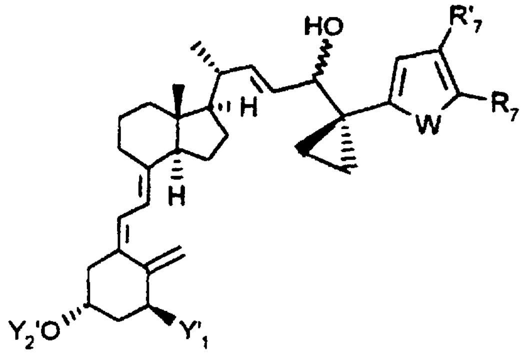

XVIII

Die Weiterreaktionen sowie die Reste sind bereits vorher beschrieben worden.
Die Carbonsäure **VIII** kann auch in andere geeignete Bausteine überführt werden. So kann durch Reduktion der Carbonsäure oder eines Derivates (Säurechlorid, Ester, Amid- speziell N-Methoxy-N-methylamid) und gegebenenfalls Reoxidation der Aldehyd **IXX** erzeugt werden.

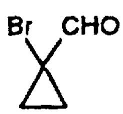

IXX

Durch Umsetzung mit dem Seyferth-Reagenz [S. Schreiber et al. *J. Am, Chem. Soc.* **112**, 5583 (1990)] oder unter Corey-Fuchs-Bedingungen [P. Ma et al. *Synth. Comm.* **25,** 3641 (1995)] erhält man das Acetylen-Derivat **XX**.

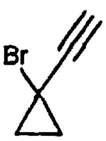

**XX**

Durch 1,3-dipolare Cycloadditionen mit Nitriloxiden ($R_7$-C=N-$O^-$), Diazoverbindungen ($R_7$-$CH^-$-N=$N^+$) oder Alkylaziden ($R_7$-$N^-$-N=$N^+$) lasse sich so Heterocyclen der allgemeinen Formeln **XXIa** und **XXIb** (Isoxazole), **XXIIa** und **XXIIb** (Pyrazole) sowie **XXIIIa** und **XXIIIb** (Triazole) herstellen.

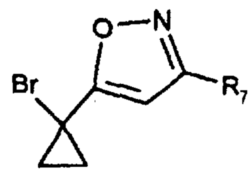

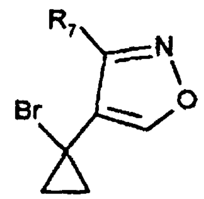

**XXIa** **XXIb**

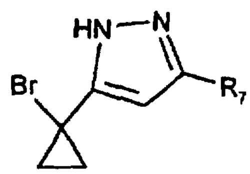

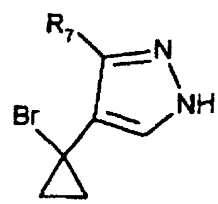

**XXIIa** **XXIIb**
**a**

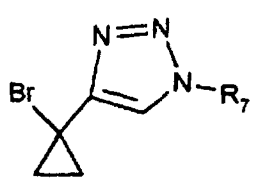

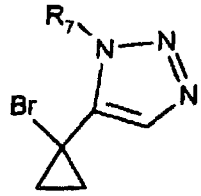

**XXIIIa** **XXIIIb**

Analog den zuvor beschriebenen Synthesen können diese Heterocyclen - gegebenfalls nach Schutz der funktionellen Gruppen - mit dem Vitamin D-Aldehyd **VII** verknüpft werden, wobei Derivate der allgemeinen Formel **II** mit den entsprechenden heterocyclischen Substituenten für Z ' anfallen.
Überführt man den Aldehyd **IXX** in einer Wittig-Reaktion in die korrespondierende Vinylverbindung, so können ebenso die 1,3-dipolaren Cycloadditionen vollzogen werden, die dann die entsprechenden Isoxazoline, Pyrazoline oder Triazoline liefern würden. Deren Behandlung kann analog zu den heteroaromatischen Derivaten erfolgen.
Ebenso ist es möglich die Carbonsäure **VIII** oder ein entsprechendes Derivat (Ester, Amid, Säurechlorid) unter Standardbedingungen (z.B. Claisen-Kondensation) in 1,3-Diketone der allgemeinen Formel **XXIV** zu überführen.

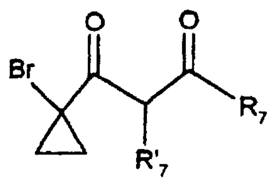

XXIV

In Gegenwart von Hydroxylamin können nun Isoxazole der allgemeinen Formel **XXV** und mit Hydrazinen vom Typ $R_8$-NH-$NH_2$ können Pyrazole der allgemeinen Formel **XXVI** entstehen.

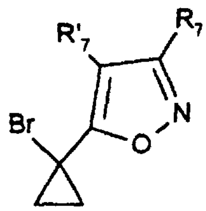

XXV

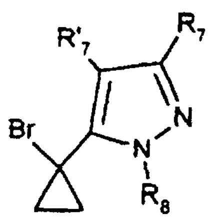

XXVI

Analog den zuvor beschriebenen Synthesen können diese Heterocyclen - gegebenfalls nach Schutz der funktionellen Gruppen - mit dem Vitamin D-Aldehyd **VII** verknüpft werden, wobei Derivate der allgemeinen Formel **II** mit den entsprechenden heterocyclischen Substituenten anfallen.

Zur Synthese von 1,2,4-Oxadiazolen können Derivate der Carbonsäure **VIII** (Säurechloride, -ester, -orthoester) mit Amidoximen der allgemeinen Formel **XXVII**

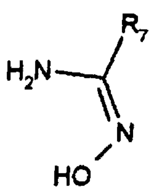

XXVII

umgesetzt und thermisch (eventuell Zusatz von Trifluoressigsäureanhydrid) in 1,2,4-Oxadiazole der allgemeinen Formel **XXVIII** überführt werden [L.B. Clapp *Adv. Heterocycl. Chem.* 20, 65 (1976)]. Die Bildung der 1,2,4-Oxadiazole kann ebenso unter basischen Bedingungen (z.B. Natriummethanolat) erfolgen.

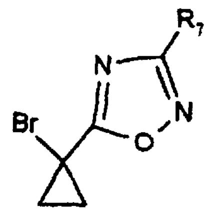

XXVIII

Analog den zuvor beschriebenen Synthesen können Heterocyclen der allgemeinen Formel **XXVIII** - gegebenfalls nach Schutz der funktionellen Gruppen - mit dem Vitamin D-Aldehyd **VII** verknüpft werden, wobei Derivate der allgemeinen Formel **II** mit den entsprechenden heterocyclischen Substituenten anfallen.

Es können auch Carbonsäure-Derivate (z.B. Ester), die bereits das Calcitriol-Gerust besitzen (DE 42 34 382), direkt in die entsprechenden 1,2,4-Oxadiazole überführt werden.

Zur Synthese von 1,3,4-Oxadiazolen können Derivate der Carbonsäure **VIII** in 1,2-Diacylhydrazine der allgemeinen Formel **XXIX** überführt werden,

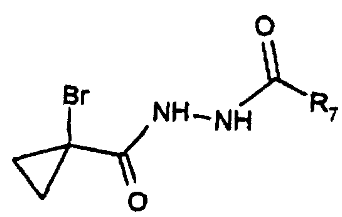

XXIX

welche thermisch oder unter Säurekatalyse (z.B. Essigsäure, Salzsäure o. ä.) zu 1,3,4-Oxazolinen der allgemeinen Formel **XXX** umgesetzt werden können (DE 28 08 842).

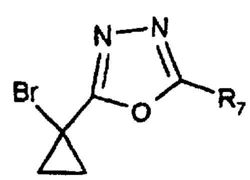

XXX

Analog den zuvor beschriebenen Synthesen können Heterocyclen der allgemeinen Formel **XXX -** gegebenfalls nach Schutz der funktionellen Gruppen - mit dem Vitamin D-Aldehyd **VII** verknüpft werden, wobei Derivate der allgemeinen Formel **II** mit den entsprechenden heterocyclischen Substituenten anfallen.

Die Herstellung von Verbindungen, die 6-gliedrige Ringe an C-25 tragen kann ganz analog erfolgen. Die notwendigen Reagenzien der allgemeinen Formel **XXXI** müssen dazu unter Einsatz der bekannten Methoden zum Aufbau von 6-Ring-Heterocyclen synthetisiert werden (R.M. Acheson "An Introduction to the Chemistry of Heterocyclic Compounds", 3. Aufl., John Wiley & Sons, New York, 1976, A.R. Katritzky, J.M. Lagowsky "The Principles of Heterocyclic Chemistry", Chapman & Hall, London, 1971).

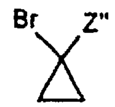

XXXI

Z" bedeutet Pyridin-, Pyrazin-, Pyrimidin-, Pyridazin-, Piperidin-, Tetrahydropyran-Ringe, die wie die 5-gliedrigen Ringe einfach oder mehrfach mit Substituenten vom Typ $R_7$ substituiert sein können.

Eine andere generelle Methode der Seitenketteneinführung bedient sich des Aldehydes der allgemeinen Formel **XXXII,**

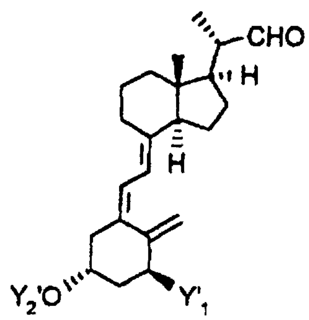

XXXII

der durch photochemische Isomerisierung des Aldehydes **III** (Bedingungen analog der Umsetzung **V** reagiert zu **VI)** zugänglich ist. Sinngemäß gelten die Isomerisierung sowie weiteren Umsetzungen auch für Aldehyde der allgemeinen Formel **IV.** Exemplarisch ist aber nur die weitere Behandlung von **XXXII** aufgeführt.

Die Einführung der Seitenkette erfolgt hier durch Umsetzung mit Bausteinen der allgemeinen Formel **XXXIII,**

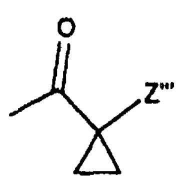

XXXIII

wobei Z'" alle bereits gegebenen Definitionen für Z, Z' und Z" haben kann. Die Synthese der entsprechenden Bausteine gelingt nach Standardmethoden. Die Anküpfung an den Aldehyd **XXXII** erfolgt durch Deprotonierung des Ketons **XXXIII** mit einer Base (z. B. Lithiumdiisopropylamid, Lithium-, Natrium-, Kaliumhexamethyldisilazid o.ä.) in einer Aldol-Reaktion, wobei Derivate der allgemeinen Formel **XXXIV** anfallen.

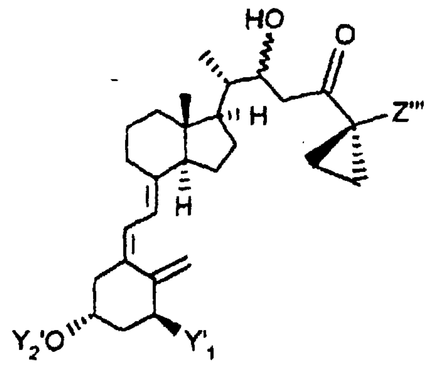

**XXXIV**

Überführung der freien Hydroxygruppe in eine Fluchtgruppe (z.B. Acetat, Trifluoracetat, Methansulfonat, Toluolsulfonat, Trifluormethansulfonat) und Eliminierung unter basischen Bedingungen (z.B. Diazabicycloundecan, Diazabicyclononan, Triethylamin o. ä.) liefert dann Enon-Derivate der allgemeinen Formel **XXXV**

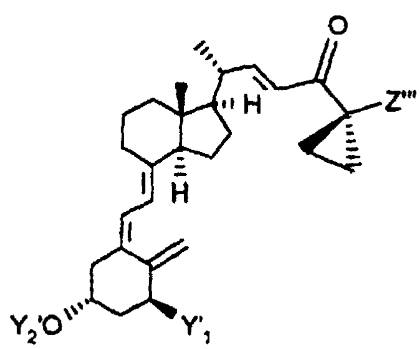

**XXXV**

Reduktion der Ketogruppen mit einem Reduktionsmittel (z.B. Natriumborhydrid, Natriumborhydrid/Certrichlorid, Lithiumaluminiumhydrid, Diisobutylaluminiumhydrid o ä.) führt dann zu Derivaten der allgemeinen Formel **II,** für die gilt, daß Q eine Hydroxymethylengruppe darstellt und Z''' gleich Z' ist, die wie beschrieben weiter umgesetzt werden.

Die Herstellung von Verbindungen der allgemeinen Formel **I** für den Fall, daß $R_1$ und $R_2$ Wasserstoffatome bedeuten, erfolgt dadurch, daß eine Verbindung der allgemeinen Formel **II'**.

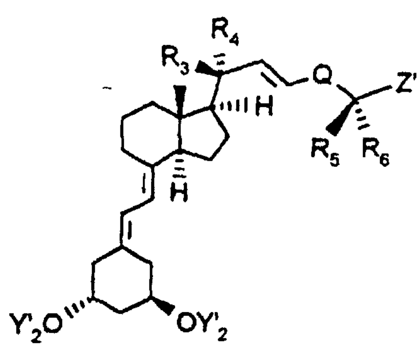

**II'**

wobei für $Y'_2$, $R_3$, $R_4$, $R_5$, $R_6$, Q und Z' die bereits genannten Bedeutungen bestehen, analog den für die Umsetzung von **II** beschriebenen Bedingungen behandelt wird.

Die Herstellung von Verbindungen der allgemeinen Formel **II'** erfolgt auf einem konvergenten Syntheseweg, wobei CD- und A-Ring-Fragmente separat aufgebaut werden. Zur Synthese der CD-Fragmente wird der literaturbekannte Aldehyd **XXXVI** [H.H. Inhoffen et al. *Chem. Ber.* **91,** 780 (1958), *Chem. Ber.* **92,** 1772 (1959), W.G. Dauben *Tetrahedron Lett.* **30,** 677 (1989)] verwendet,

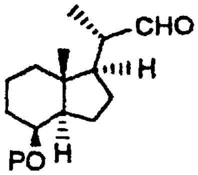

**XXXVI**

worin P eine acyl-, alkyl- oder arylsubstituierte Silyl- oder Tetrahydropyranyl-, Tetrahydrofuranyl-, Methoxymethyl-, Ethoxyethyl- oder eine Acylgruppe (z.B. Acetyl-, Benzoylgruppe) oder eine andere Alkoholschutzgruppe bedeuten (siehe T.W. Greene, P.G.M. Wuts "Protective Groups in Organic Synthesis", $2^{nd}$ Edition, John Wiley & Sons Inc., 1991).

Nach bekannten Verfahren, die schon für die Normalreihe beschrieben worden sind (siehe vorn und siehe auch WO 94/07853), können ebenso am CD-Fragment die entsprechenden Seitenketten aufgebaut werden, wobei Derivate der allgemeinen Formel **XXXVII** anfallen

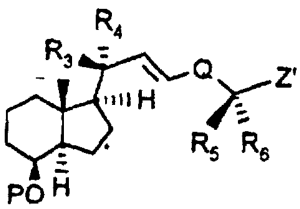

**XXXVII**

Die Reste sind zuvor beschrieben worden. Die Schutzgruppe P wird nun durch geeignete Reagenzien entfernt. Für Silylschutzgruppen verwendet man Tetrabutylammoniumfluorid, Fluorwasserstoffsäure oder Fluorwasserstoffsäure/Pyridin. Im Fall der übrigen Ethergruppen verwendet man Säuren (z.B. p-Toluolsulfonsäure, Pyridinium-p-Toluolsulfonat, Essigsäure, Oxalsäure, Salzsäure, Phosphorsäure, saure Ionentauscher). Die Acylgruppen werden unter basischen Bedingungen abgespalten (Kaliumcarbonat, Kalium-, Natrium-, Lithiumhydroxid in Alkoholen, Wasser, THF oder entsprechenden Lösungsmittelgemischen), wobei Derivate der allgemeinen Formel **XXXVIII** anfallen.

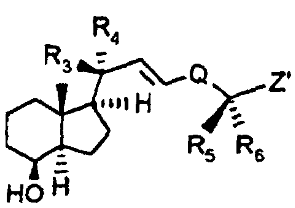

**XXXVIII**

Die freie Hydroxygruppe wird nun mit einem Oxidationsmittel (Pyridiniumdichromat, Pyridiniumchlorochromat, Bariummanganat, Swern-Bedingungen, Dess-Martin-Reagenz) in ein Keton der allgemeinen Formel **XXXIX** überführt.

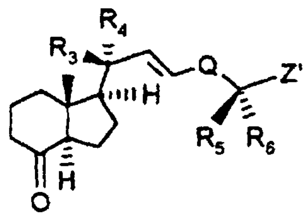

XXXIX

Die Verbindungen der allgemeinen Formel **XXXIX** werden nun durch Reaktion mit dem durch eine Base wie *n*-Butyllithium oder Lithiumdiisopropylamin erzeugten Anion des literaturbekannten Phosphinoxides der allgemeinen Formel **XL** [H.F. DeLuca et al. *Tetrahedron Lett.* **32**, 7663 (1991)]

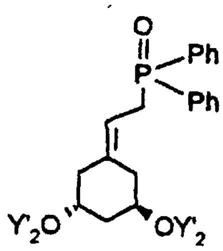

XL

in eine Verbindung der allgemeinen Formel **II'** überführt.

Für die Synthese von Calcitriol-Derivaten der allgemeinen Formel **II** mit 26,27-Cyclomodifikation, die daneben Pyridyl-Substituenten an C-25 besitzen, kann der folgende Syntheseweg beschritten werden.

Die Carbonsäure **VIII** wird mit Pyridinthiol in Gegenwart von N,N'-Dicyclohexylcarbodiimid in den Thioester **XLI** überführt,

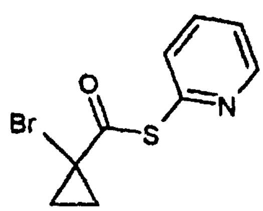

XLI

welcher mit Grignardreagenzien vom Typ **XLII** [Beispiele: D. Wenkert et al. *J. Org. Chem.* **50,** 4114 (1985); , S. Borelly, L.A. Paquette *J. Am. Chem. Soc.* **118,** 727 (1996); T.E. Bellas *Tetrahedron* **25,** 5149 (1969)]

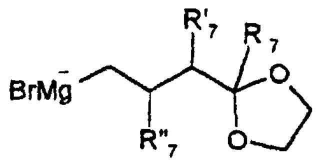

**XLII**

zu Ketonen der allgemeinen Formel **XLIII** umgesetzt werden. Für $R''_7$ gilt die gleiche Definition wie für $R_7$ und $R'_7$.

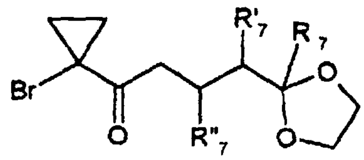

**XLIII**

Die Cyclisierung zu Pyridin-Derivaten der allgemeinen Formel **XLIV** kann nun unter Standardbedingungen (z.B. Essigsäure, Hydroxylamin Hydrochlorid) erfolgen [G. Chelucci *Synth. Comm*. **15,** 808 (1985)].

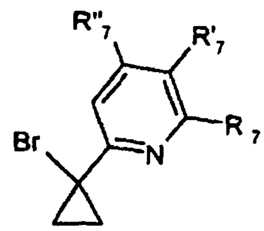

**XLIV**

Der Aufbau der Seitenkette erfolgt dann wie im Fall der Oxazol-Derivate durch Reaktion der durch Brom-Metallaustausch generierten Cyclopropyl-Metallverbindung und dem Vitamin D-C-24-Aldehyd **VII**, wobei Derivate der allgemeinen Formel **XLV** erhalten werden.

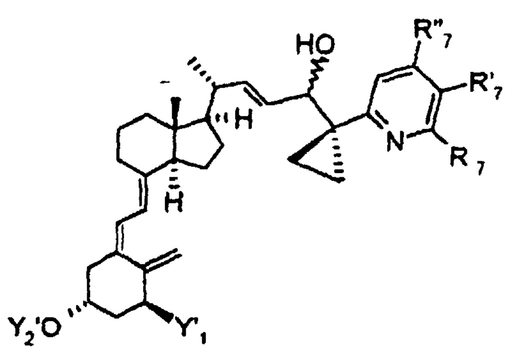

**XLV**

Diese können als Spezialfälle der allgemeinen Formel **II** angesehen werden, deren weitere Umsetzung bereits beschrieben ist.

Für die Synthese von Calcitriol-Derivaten mit 26,27-Cyclomodifikation, die daneben Oxazolin-Substituenten an C-25 besitzen, kann der folgende Syntheseweg beschritten werden.
Die Carbonsäure **VIII** wird in das Säurechlorid der allgemeinen Formel **XLVI** überführt.

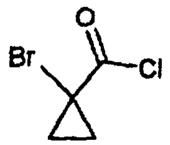

**XLVI**

Die Carbonsäure kann nun mit Aminoalkoholen der allgemeinen Formel **XLVII** zur Reaktion gebracht werden,

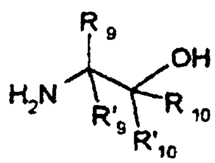

**XLVII**

wobei $R_9$, $R'_9$, $R_{10}$ und $R'_{10}$ unabhängig voneinander ein Wasserstoffatom, einen geradkettig oder verzweigten, gesättigten oder ungesättigten auch cyclischen (aromatisch, aliphatisch) Alkylrest mit bis zu 12 Kohlenstoffatomen bedeuten, der an beliebiger Stelle durch Oxa-, Thia- oder Azafunktionen (substituiert oder unsubstituiert) oder durch Sulfoxid oder Sulfongruppen unterbrochen sein kann oder weitere Substituenten (freie oder geschützte Hydroxylgruppen, Halogenatome) tragen kann.

Dabei entstehen Amide der allgemeinen Formel **XLVIII**,

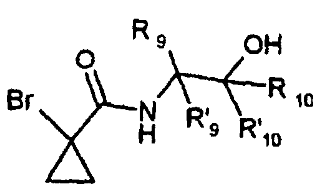

**XLVIII**

welche unter Standardbedingungen (z.B. Phosphoroxychlorid) in die Oxazoline der allgemeinen Formel **XLIX** überführt werden [N. Langlois et al. *Heterocycles* **42,** 635 1996)].

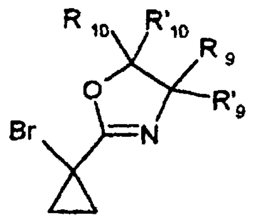

XLIX

Der Aufbau der Seitenkette erfolgt dann wie im Fall der Oxazol-Derivate durch Reaktion der durch Brom-Metallaustausch generierten Cyclopropyl-Metallverbindung und dem Vitamin D-C-24-Aldehyd **VII,** wobei Derivate der allgemeinen Formel **L** erhalten werden.

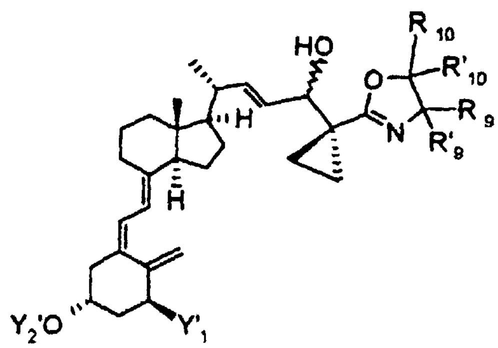

L

Diese können als Spezialfälle der allgemeinen Formel **II** angesehen werden, deren weitere Umsetzung bereits beschrieben ist.

Die Erfindung beinhaltet also auch Zwischenprodukte der allgemeinen Formeln **XI**, **XII** und **XLIV** innerhalb der Herstellung der erfindungsgemäßen Vitamin D-Derivate

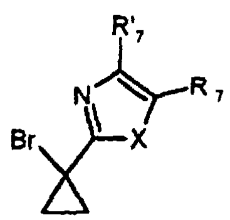

XI X=O
XIII X=S

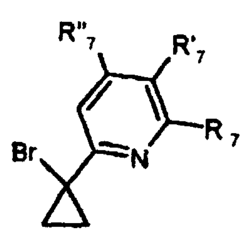

XLIV

worin $R_7$, $R'_7$ und $R''_7$ unabhängig voneinander ein Wasserstoffatom, einen geradkettigen oder verzweigten, gesättigten oder ungesättigten Alkylrest mit bis zu 12 Kohlenstoffatomen bedeuten, der an beliebigen Stellen durch Oxa-, Thia, oder Azafunktionen (substituiert oder unsubstituiert) oder Sulfoxid oder Sulfongruppen unterbrochen sein kann oder weitere Substituenten (freie oder geschützte Hydroxygruppen, Halogenatome) tragen kann.

Die nachstehenden Beispiele dienen der näheren Erläuterung der Erfindung.

**Synthese der Ausgangsverbindungen in der 5-Alkyloxazol-Reihe**

(5*Z*,7*E*,22*E*)-(1*S*,3*R*)-1,3-Bis[[dimethyl(1,1-dimethylethyl)silyl]oxy]-9,10-secochola-5,7,10(19),22-tetraen-24-al **3**

a) Eine Menge von 1,40 g (5*E*,7*E*,22*E*)-(1*S*,3*R*)-1,3-Bis[[dimethyl(1,1-dimethylethyl)-silyl]oxy]-N-methyl-N-methoxy-9,10-secochola-5,7,10(19),22-tetraen-24-amid **1** (WO 94/07853) werden in 200 ml Toluol gelöst und nach Zugabe von 232 mg Anthracen und 4 Tropfen Triethylamin 12 Minuten unter Stickstoff mit einer Quecksilberhochdrucklampe (Heraeus TQ 150) durch Pyrex-Glas bestrahlt. Danach wird filtriert und eingeengt. Nach mehrmaliger Durchführung dieser Prozedur (11 mal) erhält man 11,4 g (5*Z*,7*E*,22*E*)-(1*S*,3*R*)-1,3-Bis[[dimethyl(1,1-dimethylethyl)silyl]oxy]-N-methyl-N-methoxy-9,10-secochola-5,7,10(19),22-tetraen-24-amid **2** als gelbes Öl.

b) 7,8 g des Amides **2** werden in 39,15 ml THF gelöst und tropfenweise bei -78°C mit 49,62 ml Diisobutylaluminiumhydrid (1,2 M in Toluol) behandelt. Nach 70 Minuten werden 2,84 ml Methanol bei -78°C zugefügt und danach die Reaktionsmischung in eiskalte Natriumtartrat-Lösung eingerührt und mit 370 ml Diethylether 1,5 Stunden gut durchgerührt. Die Etherphase wird eingeengt und an Kieselgel mit Essigester/Hexan chromatographiert, wobei 5,44 g der Titelverbindung **3** als gelbe harzartige Masse anfallen.

$^1$H-NMR (300 MHz, $CDCl_3$): δ= 0,08 ppm (s, 12H); 0,58 (s, 3H); 0,88 (s, 18H); 1,17 (d, 3H); 4,20 (m, 1H); 4,38 (m, 1H); 4,85 (brs, 1H); 5,18 (brs, 1H); 6,02 (d, 1H); 6,08 (dd, 1H); 6,23 (d, 1H); 6,72 (dd, 1H); 9,48 (d, 1H)

2-(1-Bromcyclopropyl)-5-propylaxazol **7**

a) Man rührt eine Mischung aus 2,57 g 1-Bromcyclopropancarbonsäure **4** [H.M.R. Hoffmann et al. *J. Org. Chem.* **54**, 6096 (1989], 2,22 g 1-Amino-2-pentanon Hydrochlorid **5** [M. Jackman et al. *J. Am. Chem. Soc.* **70,** 2884 (1948)] und 3,36 g N,N'-Dicyclohexylcarbodiimid in 234 ml Methylenchlorid in Gegenwart von 2,46 ml Triethylamin über Nacht bei Raumtemperatur. Danach wird filtriert, das Filtrat eingeengt und der Rückstand an Kieselgel mit Essigester/Hexan chromatographiert. Man erhält 2,74 g 1-Brom-N-(2-oxopentyl)-cyclopropan-1-carbamid **6** als kristallisierendes Öl.

$^1$H-NMR (300 MHz, $CDCl_3$). δ= 0,92 ppm (t, 3H); 1,32 (m, 2H); 1,68 (m, 4H); 2,45 (t, 2H); 4,12 (d, 2H); 7,58 (brs, 1H)

Analog werden erhalten:

1-Brom-N-(2-oxopropyl)-cyclopropan-1-carbamid aus 1-Amino-2-propanon Hydrochlorid [J.D. Hepworth *Org. Synth.* **45,** 1 (1965)

1-Brom-N-(2-oxobutyl)-cyclopropan-1-carbamid aus 1-Amino-2-butanon Hydrochlorid [M. Jackman et al. *J. Am. Chem. Soc.* **70,** 2884 (1948)]

1-Brom-N-(2-oxoheptyl)-cyclopropan-1-carbamid aus 1-Amino-2-heptanon Hydrochlorid [M. Jackman et al. *J. Am. Chem. Soc.* **70,** 2884 (1948)]

b) 2,14 g **6** und 6,42 g Polyphosphorsäure werden 3,5 Stunden bei 140°C gehalten. Die Reaktionsmischung wird danach mit Eis/Natriumcarbonatlösung versetzt und mit Essigester extrahiert. Nach Trocknung über Natriumsulfat wird an Kieselgel mit Essigester/Hexan chromatographiert. Es werden 1,47 g der Titelverbindung **7** als gelbes Öl erhalten.

$^1$H-NMR (300 MHz, $CDCl_3$): δ= 0,98 ppm (t, 3H); 1,50 (m, 2H); 1,60 (m, 4H); 2,59 (t, 2H); 6,65 (s, 1H)

2-(1-Bromcyclopropyl)-5-methyloxazol **8**

4,62 g 1-Brom-N-(2-oxopropyl)-cyclopropan-1-carbamid werden in 35,47 ml konzentrierter Schwefelsäure 30 Minuten bei 60°C gehalten. Die abgekühlte Reaktionsmischung wird in Eiswasser eingerührt und mit Natriumcarbonat Dekahydrat portionsweise basisch gestellt. Anschließend wird mit Essigester extrahiert, über Natriumsulfat getrocknet und eingeengt. Chromatographie des öligen Rückstandes an Kieselgel mit Essigester/Hexan ergeben 2,79 g der Titelverbindung **8** als gelbliches Öl.

$^1$H-NMR (300 MHz, $CDCl_3$): δ= 1,50 ppm (m, 2H); 1,60 (m, 2H); 2,30 (s, 3H); 6,65 (s, 1H)

Analog werden erhalten:

2-(1-Bromcyclopropyl)-5-ethyloxazol **9** aus 1-Brom-N-(2-oxobutyl)-cyclopropan-1-carbamid

$^1$H-NMR (300 MHz, $CDCl_3$): δ= 1,24 ppm (t, 3H); 1,50 (m, 2H); 1,60 (m, 2H); 2,65 (q, 2H); 6,65 (s, 1H)
2-(1-Bromcyclopropyl)-5-pentyloxazol **10** aus 1-Brom-N-(2-oxoheptyl)-cyclopropan-1-carbamid
$^1$H-NMR (300 MHz, $CDCl_3$): δ= 0,90 ppm (t, 3H); 1,33 (m, 4H); 1,50 (m, 2H); 2,60 (t, 2H); 6,65 (s, 1H)

**Beispiel 1**

(5*Z*,7*E*,22*E*)-(1*S*,3*R*,24*S*)-25-(5-Propyloxazol-2-yl)-26,27-cyclo-9,10-secocholesta-5,7,10(19),22-tetraen-1,3,24-triol **12a** (5*Z*,7*E*,22*E*)-(1*S*,3*R*,24*R*)-25-(5-Propyloxazol-2-yl)-26,27-cyclo-9,10-secocholesta-5,7,10(19),22-tetraen-1,3,24-triol **12b**

Man gibt 1,0 g 2-(1-Bromcyclopropyl)-5-propyloxazol **7** in 2,0 ml Diethylether tropfenweise bei -78°C zu 4,78 ml *tert*.-Butyllithium (1,7 M in Pentan) in 13,6 ml Diethylether. Nach 5 Minuten werden 869 mg **3** in 5,44 ml Diethylether hinzugetropft. Nach 10 Minuten läßt man die Reaktionsmischung auf 0°C kommen und rührt dann in gesättigte Ammoniumchlorid-Lösung ein. Nach Extraktion mit Diethylether und Trocknung über Natriumsulfat werden 1,58 g gelbes Öl erhalten. Durch Chromatographie an Kieselgel mit Essigester/Hexan erhält man in der Elutionsreihenfolge 420 mg (5*Z*,7*E*,22*E*)-(1*S*,3*R*,24*S*)-1,3-Bis[[dimethyl(1,1-dimethyl-ethyl)silyl]oxy]-25-(5-propyloxazol-2-yl)-26,27-cyclo-9,10-secocholesta-5,7,10(19),22-tetra-en-24-ol **11a** und 380 mg (5*Z*,7*E*,22*E*)-(1*S*,3*R*,24*R*)-1,3-Bis[[dimethyl(1,1-dimethylethyl)-silyl]oxy]-25-(5-propyloxazol-2-yl)-26,27-cyclo-9,10-secocholesta-5,7,10(19),22-tetraen-24-ol **11b** als Öle.
410 mg **11a** werden in 15,9 ml THF mit 841 mg Tetrabutylammoniumfluorid (Trihydrat) über Nacht bei Raumtemperatur stehengelassen. Danach werden weitere 420 mg Tetrabutylammoniumfluorid (Trihydrat) zugesetzt und 5 Stunden gerührt. Nach Zugabe von gesättigter Natriumhydrogencarbonat-Lösung und gesättigter Natriumchlorid-Lösung wird mit Essigester extrahiert. Nach der Trocknung der organischen Phase mit Natriumsulfat wird eingeengt und der Rückstand an Kieselgel mit Essigester/Hexan chromatographiert. Es werden 110 mg der Titelverbindung **12a** als farbloses Öl erhalten.
$^1$H-NMR (300 MHz, $CDCl_3$): δ= 0,57 ppm (s, 3H); 1,00 (m, 8H); 1,15 (m, 2H); 2,55 (t, 2H); 4,12 (d, 2H); 4,23 (m, 1H); 4,43 (m, 1H); 5,00 (brs, 1H); 5,32 (brs, 1H); 5,42 (dd, 1H); 5,58 (dd, 1H); 6,00 (d, 1H); 6,38 (d, 1H); 6,58 (s, 1H)
Analog wird aus **11b** mit Tetrabutylammoniumfluorid (Trihydrat) die Titelverbindung **12b** als farbloser Schaum erhalten.
$^1$H-NMR (300 MHz, $CDCl_3$): δ= 0,57 ppm (s, 3H); 1,00 (m, 8H); 1,15 (m, 2H); 2,55 (t, 2H); 4,12 (d, 2H); 4,23 (m, 1H); 4,43 (m, 1H); 5,00 (brs, 1H); 5,32 (brs, 1H); 5,42 (dd, 1H); 5,53 (dd, 1H); 6,00 (d, 1H); 6,38 (d, 1H); 6,58 (s, 1H)

**Beispiel 2**

(5*Z*,7*E*,22*E*)-(1*S*,3*R*,24*R*)-25-(5-Methyloxazol-2-yl)-26,27-cyclo-9,10-secocholesta-5,7,10(19),22-tetraen-1,3,24-triol **13b**

Ausgehend vom Aldehyd **3** wird analog dem Beispiel 1 mit 2-(1-Bromcyclopropyl)-5-methyloxazol **8** die Titelverbindung **13b** als farbloser Schaum erhalten.
$^1$H-NMR (300 MHz, $CDCl_3$): δ= 0,57 ppm (s, 3H); 0,97 (m, 2H); 1,05 (m, 3H); 1,15 (m, 2H); 2,24 (s, 2H); 4,10 (d, 2H); 4,23 (m, 1H); 4,43 (m, 1H); 5,00 (brs, 1H); 5,32 (brs, 1H); 5,42 (dd, 1H); 5.53 (dd, 1H); 6,00 (d, 1H); 6,38 (d, 1H); 6,58 (s, 1H)

**Beispiel 3**

(5*Z*,7*E*,22*E*)-(1*S*,3*R*,24*R*)-25-(5-Ethyloxazol-2-yl)-26,27-cyclo-9,10-secocholesta-5,7,10(19),22-tetraen-1,3,24-triol **14b**

Ausgehend vom Aldehyd **3** wird analog dem Beispiel 1 mit 2-(1-Bromcyclopropyl)-5-ethyloxazol **9** die Titelverbindung **14b** als farbloser Schaum erhalten.
$^1$H-NMR (300 MHz, $CDCl_3$): δ= 0,57 ppm (s, 3H); 0,97 (m, 2H); 1,05 (m, 3H); 1,20 (m, 5H); 2,60 (q, 3H); 4,12 (d, 2H); 4,23 (m, 1H); 4,43 (m, 1H); 5,00 (brs, 1H); 5,32 (brs, 1H); 5,42 (dd, 1H); 5,53 (dd, 1H); 6,00 (d, 1H); 6,38 (d, 1H); 6,58 (s, 1H)

**Beispiel 4**

(5*Z*,7*E*,22*E*)-(1*S*,3*R*,24*R*)-25-(5-Pentyloxazol-2-yl)-26,27-cyclo-9,10-secocholesta-5,7,10(19),22-tetraen-1,3,24-triol **15b**

Ausgehend vom Aldehyd **3** wird analog dem Beispiel 1 mit 2-(1-Bromcyclopropyl)-5-pentyloxazol **10** die Titelverbindung **15b** als farbloser Schaum erhalten.

$^1$H-NMR (300 MHz, $CDCl_3$): δ= 0,57 ppm (s, 3H); 0,90 (t, 3H); 1,05 (m, 3H); 1,15 (m, 2H); 2,55 (s, 3H); 4,12 (d, 2H); 4,23 (m, 1H); 4,43 (m, 1H); 5,00 (brs, 1H); 5,32 (brs, 1H); 5,42 (dd, 1H); 5,53 (dd, 1H); 6,00 (d, 1H); 6,38 (d, 1H); 6,60 (s, 1H)

**Synthese der Ausgangsmaterialien in der 4-Alkylthiazol-Reihe**

2-(1-Bromcyclopropyl)-4-ethylthiazol **17**

Man rührt 10,0 g 1-Brompropancarbonsäure in **4** 173 ml Methylenchlorid mit 8,36 g N-Hydroxysuccinimid für 10 min bei Raumtemperatur unter Stickstoff. Bei 0°C werden 15,0 g N,N-Dicyclohexylcarbodiimid hinzugegeben und 3 h bei 0°C gerührt. Anschließend werden 10,0 ml einer 33%igen wässrigen Ammoniak-Lösung zugesetzt und weitere 3 h bei 0°C gerührt. Die Reaktionsmischung wird filtriert, das Filtrat eingeengt und der Rückstand (14,12 g farbloser Feststoff) an Kieselgel mit Essigester/Hexan chromatographiert. Es werden 9,01 g 1-Bromcyclopropancarbonsäureamid **16** als farbloser Feststoff (Fp. 104-107°C) erhalten.

4,64 g **16** in 2,0 ml Toluol werden mit 1,11 g Phosphorpentasulfid und 4,17 g 1-Brom-2-butanon (aus 2-Butanon und Brom in Gegenwart von Methanol) 13 min bei 50°C gerührt. Die abgekühlte Reaktionsmischung wird mit Eis und 100 ml gesättigter Natriumhydrogencarbonat-Lösung versetzt. Anschließend wird mit Essigester extrahiert und die organische Phase mit Natriumsulfat getrocknet. Nach Entfernen des Lösungsmittels wird der Rückstand an Kieselgel mit Essigester/Hexan chromatographiert, wobei 810 mg der Titelverbindung **17** als gelbes Öl anfallen.

$^1$H-NMR (300 MHz, $CDCl_3$): δ= 1,25 ppm (t, 3H); 1,60 (m, 2H); 1,75 (m, 2H); 2,72 (q, 2H); 6,78 (s, 1H)

Analog werden erhalten:

2-(1-Bromcyclopropyl)-4-methylthiazol **18** aus Chloraceton bei 100°C und 30 min Reaktionszeit.

$^1$H-NMR (300 MHz, $CDCl_3$): δ= 1,60 ppm (m, 2H); 1,75 (m, 2H); 2,38 (s, 3H); 6,78 (s, 1H)

2-(1-Bromcyclopropyl)-4-propylthiazol **19** aus 1-Brompentanon bei 50°C und 20 min Reaktionszeit.

$^1$H-NMR (300 MHz, $CDCl_3$): δ= 0,95 ppm (t, 3H); 1,60 (m, 2H); 1,69 (m, 2H); 1,75 (m, 2H); 2,65 (t, 2H); 6,78 (s, 1H)

2-(1-Bromcyclopropyl)-4-butylthiazol **20** aus 1-Brom-2-hexanon bei 100°C und 2 min Reaktionszeit.

$^1$H-NMR (300 MHz, $CDCl_3$): δ= 0,95 ppm (t, 3H); 1,38 (m, 2H); 1,60 (m, 2H); 1,75 (m, 2H); 2,68 (t, 2H); 6,78 (s, 1H)

**Beispiel 5**

(5*Z*,7*E*,22*E*)-(1*S*,3*R*,24*S*)-25-(4-Ethylthiazol-2-yl)-26,27-cyclo-9,10-secocholesta-5,7,10(19),22-tetraen-1,3,24-triol **22a** und (5*Z*,7*E*,22*E*)-(1*S*,3*R*,24*R*)-25-(4-Ethylthiazol-2-yl)-26,27-cyclo-9,10-secocholesta-5,7,10(19),22-tetraen-1,3,24-triol **22b**

2,94 ml *tert*.-Butyllithium (1.7 M in Pentan) werden bei -20°C unter Stickstoff zu 8 ml Trapp-Mischung (THF, Ether, Pentan = 4:1:1) gegeben und auf -116°C (Ether, fl. Stickstoff) gekühlt. Danach werden 580 mg **17** in 1 ml Trapp-Mischung zugetropft und 1 h bei -116°C gerührt. Anschließend werden 950 mg des Aldehydes **3** in 1 ml Trapp-Mischung zugetropft. Nach 30 min bei dieser Temperatur wird gesättigte Ammoniumchlorid-Lösung hinzugegeben und mit Essigester extrahiert. Nach Trocknung der organischen Phase über Natriumsulfat und Einengen des Lösungsmittels wird ein Rückstand von 890 mg als gelbes Öl erhalten. Durch Chromatographie an Kieselgel mit Essigester/Hexan erhält man in der Elutionsreihenfolge 210 mg (5*Z*,7*E*,22*E*)-(1*S*,3*R*,24*S*)-1,3-Bis[[dimethyl(1,1-dimethylethyl)silyl]oxy]-25-(4-ethyl-thiazol-2-yl)-26,27-cyclo-9,10-secocholesta-5,7,10(19),22-tetraen-24-ol **21a** und 200 mg (5*Z*,7*E*,22*E*)-(1*S*, 3*R*,24*R*)-1,3-Bis[[dimethyl(1,1-dimethylethyl)silyl]oxy]-25-(4-ethylthiazol-2-yl)-26,27-cyclo-9,10-secocholesta-5,7,10 (19),22-tetraen-24-ol **21b** als hellgelbe Öle.

210 mg **21a** werden in 7 ml THF gelöst und mit 420 mg Tetrabutylammoniumfluorid (Trihydrat) über Nacht bei Raumtemperatur unter Stickstoff stehengelassen. Danach gießt man in ein Gemisch von gesättigter Natriumchlorid- und gesättigter Natriumhydrogencarbonat-Lösung (50:1), extrahiert mit Essigester, wäscht die organische Phase mit gesättigter Natriumchlorid-Lösung, trocknet über Natriumsulfat und engt ein. Der Rückstand wird an Kieselgel mit Essigester/Hexan chromatographiert, wobei man 56 mg der Titelverbindung **22a** als farblosen Schaum erhält.

$^1$H-NMR (300 MHz, $CDCl_3$): δ= 0,50 ppm (s, 3H); 0,98 (d, 3H); 1,05 (m, 4H); 1,25 (t, 3H); 2,74 (q, 2H); 3,97 (d, 1H); 4,23 (m, 1H); 4,43 (m, 1H); 5,00 (brs, 1H); 5,32 (brs, 1H); 5,40 (dd, 1H); 5,56 (dd, 1H); 6,00 (d, 1H); 6,38 (d, 1H); 6,65 (s, 1H)

Analog wird aus **21b** mit Tetrabutylammoniumfluorid (Trihydrat) die Titelverbindung **22b** als farbloser Schaum erhalten.

$^1$H-NMR (300 MHz, $CDCl_3$): δ= 0,52 ppm (s, 3H); 0,98 (d, 3H); 1,05 (m, 4H); 1,25 (t, 3H); 2,74 (q, 2H); 3,98 (d, 1H); 4,23 (m, 1H); 4,43 (m, 1H); 5,00 (brs, 1H); 5,32 (brs, 1H); 5,38 (dd, 1H); 5,51 (dd, 1H); 6,00 (d, 1H); 6,38 (d, 1H); 6,65 (s, 1H)

**Beispiel 6**

(5*Z*,7*E*,22*E*)-(1*S*,3*R*,24*R*)-25-(4-Methylthiazol-2-yl)-26,27-cyclo-9,10-secocholesta-5,7,10(19),22-tetraen-1,3,24-triol **23b**

Ausgehend vom Aldehyd **3** wird analog Beispiel 5 mit 2-(1-Bromcyclopropyl)-4-methylthiazol **18** die Titelverbindung **23b** als farbloser Schaum erhalten.

$^1$H-NMR (300 MHz, $CDCl_3$): δ= 0,52 ppm (s, 3H); 1,00 (d, 3H); 1,05 (m, 4H); 2,40 (s, 3H); 3,98 (d, 1H); 4,23 (m, 1H); 4,43 (m, 1H); 5,00 (brs, 1H); 5,32 (brs, 1H); 5,38 (dd, 1H); 5,51 (dd, 1H); 6,00 (d, 1H); 6,38 (d, 1H); 6,65 (s, 1H)

**Beispiel 7**

(5*Z*,7*E*,22*E*)-(1*S*,3*R*,24*R*)-25-(4-Propylthiazol-2-yl)-26,27-cyclo-9,10-secocholesta-5,7,10(19),22-tetraen-1,3,24-triol **24b**

Ausgehend vom Aldehyd **3** wird analog Beispiel 5 mit 2-(1-Bromcyclopropyl)-4-propylthiazol **19** die Titelverbindung **24b** als farbloser Schaum erhalten.

$^1$H-NMR (300 MHz, $CDCl_3$): δ= 0,52 ppm (s, 3H); 0,98 (m, 7H); 1,05 (m, 4H); 2,68 (t, 2H); 3,98 (d, 1H); 4,23 (m, 1H); 4,43 (m, 1H); 5,00 (brs, 1H); 5,32 (brs, 1H); 5,38 (dd, 1H); 5,50 (dd, 1H); 6,00 (d, 1H); 6,38 (d, 1H); 6,65 (s, 1H)

**Beispiel 8**

(5*Z*,7*E*,22*E*)-(1*S*,3*R*,24*R*)-25-(4-Butylthiazol-2-yl)-26,27-cyclo-9,10-secocholesta-5,7,10(19),22-tetraen-1,3,24-triol **25b**

Ausgehend vom Aldehyd **3** wird analog Beispiel 5 mit 2-(1-Bromcyclopropyl)-4-butylthiazol **20** die Titelverbindung **25b** als farbloser Schaum erhalten.

$^1$H-NMR (300 MHz, $CDCl_3$): δ= 0,52 ppm (s, 3H); 0,92 (t, 3H); 0,98 (d, 3H); 1,05 (m, 4H); 2,69 (t, 2H); 3,98 (d, 1H); 4,23 (m, 1H); 4,43 (m, 1H); 5,00 (brs, 1H); 5,32 (brs, 1H), 5,38 (dd, 1H); 5,50 (dd, 1H); 6,00 (d, 1H); 6,38 (d, 1H); 6,65 (s, 1H)

**Synthese der Ausgangsmaterialien in der Phenyl-Reihe**

1-(Phenylcyclopropan-1-yl)-1-ethanon **28**

Man rührt 4,5 g 1-Phenyl-1-cyclopropancarbonsäure **26** in 80 ml Methylenchlorid mit 3,83 g N-Hydroxysuccinimid für 10 min bei Raumtemperatur unter Stickstoff. Bei 0°C werden 6,84 g N,N'-Dicyclohexylcarbodiimid hinzugegeben und es wird 1,5 h nachgerührt. Anschließend gibt man 7,74 ml einer wässrigen Dimethylamin-Lösung hinzu und rührt weitere 30 min bei 0°C und 12 h bei Raumtemperatur. Man engt ein und chromatographiert den Rückstand an Kieselgel mit Essigester/Hexan, wobei 4,34 g 1-Phenyl-1-cyclopropancarbonsäure-dimethylamid **27** als kristallisierendes Öl anfallen.

Man löst 4,34 g **27** in 189 ml THF, kühlt auf -10°C und tropft unter Stickstoff 21,5 ml Methyllithium-Lösung (1.6 M in Diethylether) zu. Man rührt 2 h bei dieser Temperatur und gießt die Reaktionsmischung dann in gesättigte Ammoniumchlorid-Lösung. Nach Extraktion mit Essigester wird die organische Phase über Natriumsulfat getrocknet und eingeengt. Der Rückstand wird an Kieselgel mit Essigester/Hexan chromatographiert, wobei man 3,7 g der Titelverbindung **28** als gelbliches Öl erhält.

(5*Z*,7*E*)-(1*S*,3*R*)-1,3-Bis[[dimethyl(1,1-dimethylethyl)silyl]oxy]-9,10-secopregna-5,7,10(19)-trien-20-carbaldehyd **30**

Man löst 7,5 g (5*E*,7*E*)-(1*S*,3*R*)-1,3-Bis[[dimethyl(1,1-dimethylethyl)silyl]oxy]-9,10-secopregna-5,7,10(19)-trien-20-carbaldehyd **29** [M.J. Calverley *Tetrahedron* **43,** 4609 (1987)] in 200 ml Toluol, fügt 2 g Anthracen und 0,5 ml Triethylamin hinzu und bestrahlt unter Stickstoffdurchleitung in einer Pyrex-Apparatur mit einer Quecksilberhochdrucklampe für 30 min. Anschließend filtriert man, engt ein und chromatographiert den Rückstand an Kieselgel mit Essigester/Hexan, wobei man die Titelverbindung **30** als farblosen Schaum erhält.
$^1$H-NMR (300 MHz, $CDCl_3$): δ=0,05 ppm (s, 12H); 0,55 (s, 3H); 0,88 (s, 18H); 1,11 (d, 3H); 2,37 (m, 1H), 4,18 (m, 1H); 4,37 (m, 1H); 4,84 (brs, 1H); 5,17 (brs, 1H), 6,00 (d, 1H); 6,22 (d, 1H)

**Beispiel 9**

(5*Z*,7*E*,22*E*)-(1*S*,3*R*,24*S*)-25-Phenyl-26,27-cyclo-9,10-secocholesta-5,7,10(19),22-tetraen-1,3,24-triol **35a** und (5*Z*,7*E*,22*E*)-(1*S*,3*R*,24*R*)-25-Phenyl-26,27-cyclo-9,10-secocholesta-5,7,10(19),22-tetraen-1,3,24-triol **35b**

Man tropft 17,4 ml n-Butyllithium-Lösung (1.6 M in Hexan) bei 0°C zu 3,86 ml Diisopropylamin in 20,7 ml THF unter Stickstoff. Man rührt 20 min bei dieser Temperatur, kühlt auf-78°C und tropft dann 4,42 g des Ketons **28** in 6,2 ml THF zu. Nach 1 h bei -78°C werden 2,3 g des Aldehydes **30** in 18,8 ml THF zugegeben und es wird 1 h nachgerührt. Die Reaktionsmischung wird anschließend in eiskalte Ammoniumchlorid-Lösung eingerührt und mit Essigester extrahiert. Nach Trocknung der organischen Phase über Natriumsulfat entfernt man das Solvens und chromatographiert an Kieselgel mit Essigester/Hexan, wobei man 1,57 g (5*Z*,7*E*)-(1*S*,3*R*)-1,3-Bis[[dimethyl(1,1-dimethylethyl)silyl]oxy]-22-hydroxy-25-phenyl-26,27-cyclo-9,10-secocholesta-5,7,10(19)-trien-24-on **31** als farblosen Schaum erhält.
1,57 g **31** werden in 6,3 ml Pyridin gelöst, mit 2,08 ml Essigsäureanhydrid versetzt und über Nacht unter Stickstoff bei Raumtemperatur geruhrt. Danach wird die Reaktionsmischung mit wässriger Oxalsäure (5%) versetzt und mit Essigester extrahiert. Nach Trocknung der organischen Phase über Natriumsulfat und Chromatographie an Kieselgel mit Essigester/Hexan werden 1,44 g (5*Z*,7*E*)-(1*S*,3*R*)-22-(Acetyloxy)-1,3-bis[[dimethyl(1,1-dimethylethyl)silyl]oxy]-25-phenyl-26,27-cyclo-9,10-secocholesta-5,7,10(19)-trien-24-on **32** als farbloser Schaum erhalten.
1,44 g **32** werden in 40 ml Toluol gelöst und mit 8,13 ml 1,8-Diazabicyclo[5.4.0]undec-7-en (DBU) 30 min bei 40°C unter Stickstoff gerührt. Man verdünnt die Reaktionsmischung mit 150 ml Essigester und rührt in 600 ml 0.01 N Salzsäure ein. Es wird mit Essigester extrahiert, über Natriumsulfat getrocknet und das Solvens entfernt, wobei 1,31 g (5*Z*,7*E*,22*E*)-(1*S*,3*R*)-1,3-Bis[[dimethyl(1,1-dimethylethyl)silyl]oxy]-25-phenyl-26,27-cyclo-9,10-secocholesta-5,7,10(19),22-tetraen-24-on **33** als farbloses Öl erhalten werden. 1,31 g des Enons **33** werden in 3,1 ml THF und 7,2 ml Methanol gelöst und bei 0°C unter Stickstoff mit 7,2 ml einer 0.4 molaren methanolischen Certrichlorid-Heptahydrat-Losung versetzt. Anschließen gibt man portionsweise 200 mg Natriumborhydrid zu und rührt 40 min bei 0°C nach. Es wird nun Eiswasser zugegeben, mit Essigester extrahiert und über Natriumsulfat getrocknet. Der Rückstand wird an Kieselgel mit Hexan/Essigester chromatographiert, wobei in der Elutionsreihenfolge 133 mg (5*Z*,7*E*,22*E*)-(1*S*,3*R*,24*S*)-1,3-Bis[[dimethyl(1,1-dimethylethyl)silyl]oxy]-25-phenyl-26,27-cyclo-9,10-secocholesta-5,7,10(19),22-tetraen-24-ol **34a** und 374 mg (5*Z*,7*E*,22*E*)-(1*S*,3*R*,24*R*)-1,3-Bis[[dimethyl(1,1-dimethylethyl)silyl]oxy]-25-phenyl-26,27-cyclo-9-10-secocholesta-5,7,10(19),22-tetraen-24-ol **34b** als farblose Schäume anfallen.
133 mg **34a** werden in 5,6 ml THF mit 295 mg Tetrabutylammoniumfluorid (Trihydrat) über Nacht bei Raumtemperatur unter Stickstoff stehengelassen. Die Reaktionsmischung wird dann in eiskalte gesättigte Natriumhydrogencarbonat-Lösung eingerührt und mit Essigester extrahiert. Die vereinigten organischen Phasen werden mit Wasser gewaschen, über Natriumsulfat getrocknet und eingeengt. Chromatographie des Rückstandes an Kieselgel mit Essigester/Hexan ergeben 38 mg der Titelverbindung **35a** als farblosen Schaum.
$^1$H-NMR (300 MHz, $CDCl_3$): δ= 0,57 ppm (s, 3H); 0,85 (m, 4H); 1,05 (d, 3H); 3,78 (d, 1H); 4,23 (m, 1H), 4,43 (m, 1H); 5,00 (brs, 1H); 5,30 (dd, 1H); 5,32 (brs, 1H); 5,42 (dd, 1H); 6,00 (d, 1H); 6,38 (d, 1H); 7,30 (m, 5H)
Analog zu **34a** erhält man aus **34b** die Titelverbindung **35b** als farblosen Schaum.
$^1$H-NMR (300 MHz, $CDCl_3$): δ= 0,57 ppm (s, 3H); 0,85 (m, 4H); 1,00 (d, 3H); 3,70 (d, 1H); 4,23 (m, 1H); 4,43 (m, 1H); 5,00 (brs, 1H); 5,30 (dd, 1H); *5,32* (brs, 1H); 5,42 (dd, 1H); 6,00 (d, 1H); 6,38 (d, 1H); 7,30 (m, 5H)

**Synthese der Ausgangsmaterialien in der 4-Alkyl-Phenylreihe**

4-Methylbenzolacetaldehyd **37**

Man legt 28,5 g Methoxymethyltriphenylphosphoniumchlorid in 350 ml Diethylether unter Stickstoff vor. Bei 0°C werden 39,95 ml *n*-Butyllithium-Lösung (1.6 M in Hexan) zugetropft und es wird eine Stunde bei Raumtemperatur gerührt. Nun gibt man 10 g 4-Methylbenzaldehyd in 50 ml Diethylether hinzu und rührt eine Stunde nach. Zur Reaktionsmischung wird Natriumchlorid-Lösung gegeben, mit Essigester extrahiert, über Natriumsulfat getrocknet und eingeengt. Nach Chromatographie über Kieselgel mit Essigester/Hexan erhält man 7.8 g 1-Methoxy-2-(4-Methylphenyl)-ethen **36** (*E*, *Z*-Gemisch) als farbloses Öl, welches in 200 ml Aceton gelöst und unter Stickstoff mit 10 ml 2 N Salzsäure über Nacht gerührt wird. Man gibt Natriumchlorid-Lösung hinzu, extrahiert mit Essigester, trocknet über Natriumsulfat und engt ein. Chromatographische Reinigung an Kieselgel mit Essigester/Hexan ergibt 4,5 g der Titelverbindung **37** als farbloses Öl.

$^1$H-NMR (300 MHz, $CDCl_3$): δ= 2,38 ppm (s, 3H); 3,68 (d, 2H); 7,12 (d, 2H); 7,22 (d, 2H); 9,74 (t, 1H)

2-(4-Methylphenyl)-2-propenal **38**

Man legt 2,57 g des Aldehyds **37** in 300 ml Methylenchlorid unter Stickstoff vor und gibt bei 0°C 6,5 ml Triethylamin und 7,1 g Eschenmoser's Salz hinzu. Nach 2,5 h bei 0°C wird gesättigte Ammoniumchlorid-Lösung zugegeben, mit Methylenchlorid extrahiert, mit Natriumchlorid-Lösung gewaschen, über Natriumsulfat getrocknet und eingeengt. Der Rückstand wird an Kieselgel mit Essigester/Hexan chromatographiert, wobei 1,94 g der Titelverbindung **38** als farbloses Öl anfallen.

$^1$H-NMR (300 MHz, $CDCl_3$): δ= 2,39 ppm (s, 3H); 6,17 (s, 1H); 6,62 (s, 1H); 7,22 (d, 2H); 7,39 (d, 2H); 9,82 (s, 1H)

3-(4-Methylphenyl)-3-buten-2-ol **39**

Man löst 2,34 g des Aldehydes **38** in 150 ml Diethylether und kühlt unter Stickstoff auf -78°C. Nun werden 20 ml einer Methyllithium-Lösung (1.6 M in Diethylether) zugetropft. Nach 1,5 h bei dieser Temperatur quencht man mit gesättigter Ammoniumchlorid-Lösung, extrahiert mit Essigester, wäscht mit Natriumchlorid-Lösung, trocknet über Natriumsulfat und entfernt das Lösungsmittel. Der Rückstand wird durch Chromatographie an Kieselgel mit Essigester/Hexan gereinigt, wobei neben 237 mg des Ausgangsmaterials 1,06 g der Titelverbindung **39** als farbloses Öl anfallen.

$^1$H-NMR (300 MHz, $CDCl_3$): δ= 2,38 ppm (s, 3H); 1,35 (d, 3H); 4,82 (q, 1H); 5,28 (s, 1H); 5,34 (s, 1H); 7,18 (d, 2H); 7,32 (d, 2H)

3-(4-Methylphenyl)-3-buten-2-on **40**

Es werden 2,91 g des Alkohols **39** in 300 ml Methylenchlorid gelöst und unter Stickstoff werden 43,5 g Mangandioxid hinzugegeben. Man rührt über Nacht bei Raumtemperatur nach, filtriert über Celite und chromatographiert den Rückstand an Kieselgel mit Essigester/Hexan, wobei 850 mg der Titelverbindung **40** neben 1,06 g des Ausgangsmaterials als farblose Öle erhalten werden.

$^1$H-NMR (300 MHz, $CDCl_3$): δ= 2,45 ppm (s, 3H); 2,60 (s, 3H), 5,94 (s, 1H); 6,12 (s, 1H); 7,28 (d, 2H); 7,88 (d, 2H)

1-[1-(4-Methylphenyl)cyclopropyl]ethanon **41**

Man legt 507 mg Natriumhydrid (55% in Paraffinöl) in 20 ml Dimethylformamid unter Stickstoff bei 0°C vor und gibt 1,36 g Trimethylsulfoniumiodid hinzu. Nach 30 min bei dieser Temperatur werden 845 mg des Ketons **40** in 4 ml Dimethylformamid zugetropft. Man rührt 1 h bei 0°C nach, quencht dann mit Natriumchlorid-Lösung, extrahiert mit Essigester, trocknet über Natriumsulfat und engt ein. Nach Chromatographie an Kieselgel mit Essigester/Hexan werden 447 mg der Titelverbindung **41** als farbloses Öl erhalten.

$^1$H-NMR (300 MHz, $CDCl_3$): δ= 1,14 ppm (m, 2H); 1,59 (m, 2H); 2,01 (s, 3H); 2,38 (s, 3H); 7,28 (d, 2H); 7,88 (d, 2H)

4-(1-Methylethyl)benzolacetaldehyd **43**

Man legt 27,7 g Methoxymethyltriphenylphosphoniumchlorid in 350 ml Diethylether unter Stickstoff vor. Bei 0°C werden 38,8 ml n-Butyllithium-Lösung (1.6 M in Hexan) zugetropft und es wird eine Stunde bei Raumtemperatur gerührt. Nun gibt man 12 g 4-Isopropylbenzaldehyd in 50 ml Diethylether hinzu und rührt eine Stunde nach. Zur Reaktionsmischung wird Natriumchlorid-Lösung gegeben, mit Essigester extrahiert, über Natriumsulfat getrocknet und eingeengt. Nach Chromatographie über Kieselgel mit Essigester/Hexan erhält man 8,1 g 1-Methoxy-2-[4-(1-Methylethyl)phenyl]-ethen **42** (*E*, *Z*-Gemisch) als farbloses Öl, welches in 200 ml Aceton gelöst und unter Stickstoff mit 10 ml 2 N Salzsäure über Nacht gerührt wird. Man gibt Natriumchlorid-Lösung hinzu, extrahiert mit Essigester, trocknet über Natriumsulfat und engt ein. Chromatographische Reinigung an Kieselgel mit Essigester/Hexan ergibt 5,1 g der Titelverbindung **43** als farbloses Öl.

$^1$H-NMR (300 MHz, $CDCl_3$): δ= 1,28 ppm (d, 6H); 2,92 (hept, 1H); 3,68 (d, 2H); 7,16 (d, 2H); 7,24 (d, 2H); 9,73 (t, 1H)

2-[4-(1-Methylethyl)phenyl]-2-propenal **44**

Man legt 4,65 g des Aldehyds **43** in 300 ml Methylenchlorid unter Stickstoff vor und gibt bei 0°C 7,9 ml Triethylamin und 6,9 g Eschenmoser's Salz hinzu. Nach 2,5 h bei 0°C wird gesättigte Ammoniumchlorid-Lösung zugegeben, mit Methylenchlorid extrahiert, mit Natriumchlorid-Lösung gewaschen, über Natriumsulfat getrocknet und eingeengt. Der Rückstand wird an Kieselgel mit Essigester/Hexan chromatographiert, wobei 3,84 g der Titelverbindung **44** als farbloses Öl anfallen.
$^1$H-NMR (300 MHz, $CDCl_3$): δ= 1,28 ppm (d, 6H); 2,93 (hept, 1H); 6,15 (s, 1H); 6,62 (s, 1H); 7,26 (d, 2H); 7,41 (d, 2H); 9,81 (s, 1H)

3-[4-(1-Methylethyl)phenyl]-3-buten-2-ol **45**

Man löst 6,1 g des Aldehydes **44** in 250 ml Diethylether und kühlt unter Stickstoff auf -78°C. Nun werden 35,8 ml einer Methyllithium-Lösung (1.6 M in Diethylether) zugetropft. Nach 1,5 h bei dieser Temperatur quencht man mit gesättigter Ammoniumchlorid-Lösung, extrahiert mit Essigester, wäscht mit Natriumchlorid-Lösung, trocknet über Natriumsulfat und entfernt das Lösungsmittel. Der Rückstand wird durch Chromatographie an Kieselgel mit Essigester/Hexan gereinigt, wobei 2,8 g der Titelverbindung **45** als farbloses Öl anfallen.
$^1$H-NMR (300 MHz, $CDCl_3$): δ= 1,27 ppm (d, 6H); 2,92 (hept, 1H); 1,33 (d, 3H); 4,83 (q, 1H); 5,29 (s, 1H); 5,34 (s, 1H); 7,20 (d, 2H); 7,34 (d, 2H)

3-[4-(1-Methylethyl)phenyl]-3-buten-2-on **46**

Es werden 2,75 g des Alkohols **45** in 300 ml Methylenchlorid gelöst und unter Stickstoff werden 25,1 g Mangandioxid hinzugegeben. Man rührt über Nacht bei Raumtemperatur nach, filtriert über Celite und chromatographiert den Rückstand an Kieselgel mit Essigester/Hexan, wobei 937 mg der Titelverbindung **46** neben 635 mg des Ausgangsmaterials als farblose Öle erhalten werden.
$^1$H-NMR (300 MHz, $CDCl_3$): δ= 1,28 ppm (d, 6H); 2,46 (s, 3H); 2,93 (hept, 1H); 5,97 (s, 1H); 6,14 (s, 1H); 7,33 (d, 2H); 7,98 (d, 2H)

1-[1-[4-(1-Methylethyl)phenyl]cyclopropyl]ethanon **47**

Man legt 474 mg Natriumhydrid (55% in Paraffinöl) in 20 ml Dimethylformamid unter Stickstoff bei 0°C vor und gibt 1,29 g Trimethylsulfoniumiodid hinzu. Nach 30 min bei dieser Temperatur werden 930 mg des Ketons **46** in 4 ml Dimethylformamid zugetropft. Man rührt 1 h bei 0°C nach, quencht dann mit Natriumchlorid-Lösung, extrahiert mit Essigester, trocknet über Natriumsulfat und engt ein. Nach Chromatographie an Kieselgel mit Essigester/Hexan werden 447 mg der Titelverbindung **47** als farbloses Öl erhalten.
$^1$H-NMR (300 MHz, $CDCl_3$): δ= 1,18 ppm (m, 2H); 1,28 (d, 6H); 1,60 (m, 2H); 2,92 (hept, 1H); 2,02 (s, 3H); 7,20 (d, 2H); 7,30 (d, 2H)

4-Butylbenzolacetaldehyd **49**

Man legt 21,1 g Methoxymethyltriphenylphosphoniumchlorid in 280 ml Diethylether unter Stickstoff vor. Bei 0°C werden 29,6 ml n-Butyllithium-Lösung (1.6 M in Hexan) zugetropft und es wird eine Stunde bei Raumtemperatur gerührt. Nun gibt man 10 g 4-Butylbenzaldehyd in 40 ml Diethylether hinzu und rührt eine Stunde nach. Zur Reaktionsmischung wird Natriumchlorid-Lösung gegeben, mit Essigester extrahiert, über Natriumsulfat getrocknet und eingeengt. Nach Chromatographie über Kieselgel mit Essigester/Hexan erhält man 6,7 g 2-(4-Butylphenyl)-1-methoxyethen **48** (*E*, *Z*-Gemisch) als farbloses Öl, welches in 200 ml Aceton gelöst und unter Stickstoff mit 10 ml 2 N Salzsäure über Nacht gerührt wird. Man gibt Natriumchlorid-Lösung hinzu, extrahiert mit Essigester, trocknet über Natriumsulfat und engt ein. Chromatographische Reinigung an Kieselgel mit Essigester/Hexan ergibt 3,9 g der Titelverbindung **49** als farbloses Öl.
$^1$H-NMR (300 MHz, $CDCl_3$): δ= 0,95 ppm (t, 3H); 1,37 (m, 2H); 1,62 (m, 2H); 3,68 (d, 2H); 7,14 (d, 2H); 7,20 (d, 2H); 9,74 (t, 1H)

3-(4-Butylphenyl)-3-buten-2-ol **51**

Man legt 3,86 g des Aldehyds **43** in 300 ml Methylenchlorid unter Stickstoff vor und gibt bei 0°C 6,0 ml Triethylamin und 5,3 g Eschenmoser's Salz hinzu. Nach 2,5 h bei 0°C wird gesättigte Ammoniumchlorid-Lösung zugegeben, mit Methylenchlorid extrahiert, mit Natriumchlorid-Lösung gewaschen, über Natriumsulfat getrocknet und eingeengt.

Der Rückstand wird an Kieselgel mit Essigester/Hexan chromatographiert, wobei 3,24 g 2-(4-Butylphenyl)-2-propen-1-al **50** als farbloses Öl anfallen, die in 200 ml Diethylether gelöst und unter Stickstoff auf -78°C gekühlt werden. Nun werden 27,4 ml einer Methyllithium-Lösung (1.6 M in Diethylether) zugetropft. Nach 1,5 h bei dieser Temperatur quencht man mit gesättigter Ammoniumchlorid-Lösung, extrahiert mit Essigester, wäscht mit Natriumchlorid-Lösung, trocknet über Natriumsulfat und entfernt das Lösungsmittel. Der Rückstand wird durch Chromatographie an Kieselgel mit Essigester/Hexan gereinigt, wobei 1,8 g der Titelverbindung **51** als farbloses Öl anfallen.

$^1$H-NMR (300 MHz, $CDCl_3$): δ= 0,97 ppm (t, 3H); 1,35 (d, 3H); 1,61 (m, 4H); 2,62 (t, 2H); 4,83 (q, 1H); 5,29 (s, 1H); 5,34 (s, 1H); 7,18 (d, 2H); 7,33 (d, 2H)

3-(4-Butylphenyl)-3-buten-2-on **52**

Es werden 1,07 g des Alkohols **51** in 100 ml Methylenchlorid gelöst und unter Stickstoff werden 9,1 g Mangandioxid hinzugegeben. Man rührt über Nacht bei Raumtemperatur nach, filtriert über Celite und chromatographiert den Rückstand an Kieselgel mit Essigester/Hexan, wobei 301 mg der Titelverbindung **52** neben 395 mg des Ausgangsmaterials als farblose Öle erhalten werden.

$^1$H-NMR (300 MHz, $CDCl_3$): δ= 0,95 ppm (t, 3H); 1,37 (m, 2H); 1,60 (m, 2H); 2,43 (s, 3H); 2,65 (t, 2H); 5,94 (s, 1H); 6,12 (s, 1H); 7,18 (d, 2H), 7,22 (d, 2H)

1-[1-(4-Butylphenyl)cyclopropyl]ethanon **53**

Man legt 507 mg Natriumhydrid (55% in Paraffinöl) in 20 ml Dimethylformamid unter Stickstoff bei 0°C vor und gibt 1,38 g Trimethylsulfoniumiodid hinzu. Nach 30 min bei dieser Temperatur werden 1,06 g des Ketons **52** in 4 ml Dimethylformamid zugetropft. Man rührt 1 h bei 0°C nach, quencht dann mit Natriumchlorid-Lösung, extrahiert mit Essigester, trocknet über Natriumsulfat und engt ein. Nach Chromatographie an Kieselgel mit Essigester/Hexan werden 702 mg der Titelverbindung **53** als farbloses Öl erhalten.

$^1$H-NMR (300 MHz, $CDCl_3$): δ= 0,96 ppm (m, 2H); 1,17 (m, 2H); 1,37 (hex, 2); 1,59 (m, 2H); 1,60 (m, 2H); 2,64 (t, 2H); 2,02 (s, 3H), 7,17 (d, 2H); 7,29 (d, 2H)

**Beispiel 10**

(5*Z*,7*E*)-(1*S*,3*R*,24*R*)-25-(4-Methylphenyl)-26,27-cyclo-9,10-secocholesta-5,7,10(19),20(22)-tetraen-1,3,24-triol **59b,** (5*Z*,7*E*,22*E*)-(1*S*,3*R*,24*S*)-25-(4-Methylphenyl)-26,27-cyclo-9,10-secocholesta-5,7,10(19),22-tetraen-1,3,24-triol **60a** und (5*Z*,7*E*,22*E*)-(1*S*,3*R*,24*R*)-25-(4-Methylphenyl)-26,27-cyclo-9,10-secocholesta-5,7,10(19),22-tetraen-1,3,24-triol **60b**

Man tropft 1,01 ml n-Butyllithium-Lösung (1.6 M in Hexan) bei 0°C zu 0,33 ml Diisopropylamin in 10 ml THF unter Stickstoff. Man rührt 20 min bei dieser Temperatur, kühlt auf-78°C und tropft dann 440 mg des Ketons **41** in 2 ml THF zu. Nach 1 h bei -78°C werden 500 mg des Aldehydes **30** in 10 ml THF zugegeben und es wird 1 h nachgerührt. Die Reaktionsmischung wird anschließend in eiskalte Ammoniumchlorid-Lösung eingerührt und mit Essigester extrahiert. Nach Trocknung der organischen Phase über Natriumsulfat entfernt man das Solvens und chromatographiert an Kieselgel mit Essigester/Hexan, wobei man 529 mg (5*Z*,7*E*)-(1*S*,3*R*)-1,3-Bis[[dimethyl(1,1-dimethylethyl)silyl]oxy]-22-hydroxy-25-(4-methyl-phenyl)-26,27-cyclo-9,10-secocholesta-5,7,10(19)-trien-24-on **54** als farblosen Schaum erhält.

529 mg **54** werden in 20 ml Toluol gelöst, mit 0,34 ml Essigsäureanhydrid, 0,49 ml Triethylamin sowie einer Spatelspitze Dimethylaminopyridin (DMAP) versetzt und über Nacht unter Stickstoff bei Raumtemperatur gerührt. Danach wird die Reaktionsmischung mit Natriumhydrogencarbonat-Lösung behandelt und mit Essigester extrahiert. Nach Trocknung der organischen Phase über Natriumsulfat und Chromatographie an Kieselgel mit Essigester/Hexan werden 439 mg (5*Z*,7*E*)-(1*S*,3*R*)-22-(Acetyloxy)-1,3-bis[[dimethyl(1,1-dimethylethyl)silyl]oxy]-25-(4-methylphenyl)-26,27-cyclo-9,10-secocholesta-5,7,10(19)-trien-24-on **55** als farbloser Schaum erhalten, der in 20 ml Toluol gelöst und mit 3 ml 1,8-Diazabicyclo[5.4.0]undec-7-en (DBU) 30 min bei 40°C unter Stickstoff gerührt wird. Man verdünnt die Reaktionsmischung mit Essigester und säuert mit 0.01 N Salzsäure an. Es wird mit Essigester extrahiert, über Natriumsulfat getrocknet und das Solvens entfernt, wobei 395 mg (5*Z*,7*E*,22*E*)-(1*S*,3*R*)-1,3-Bis[[dimethyl(1,1-dimethylethyl)silyl]oxy]-25-(4-methylphenyl)-26,27-cyclo-9,10-secocholesta-5,7,10(19),22-tetraen-24-on **56** als farbloses Öl erhalten werden.

**56**: $^1$H-NMR (300 MHz, $CDCl_3$): δ= 0,05 ppm (s, 6H); 0,49 (s, 3H); 0,89 (s, 18H); 0,97 (d, 3H); 1,11 (m, 4H); 2,38 (s, 3H); 4,19 (m, 1H); 4,38 (m, 1H); 4,86 (s, 1H); 5,18 (s, 1H); 5,99 (d, 1H); 6,01 (d, 1H); 6,22 (d, 1H); 6,63 (d, 1H); 7,15 (d, 2H); 7,22 (d, 2H)

395 mg des Enons **56** werden in 2 ml THF und 4 ml Methanol gelöst und bei 0°C unter Stickstoff mit 217 mg

Certrichlorid (Heptahydrat) behandelt. Anschließend gibt man 18 mg Natriumborhydrid zu und rührt 2 h bei 0°C nach. Es wird nun Eiswasser zugegeben, mit Essigester extrahiert und über Natriumsulfat getrocknet. Der Rückstand wird an Kieselgel mit Essigester/Hexan chromatographiert, wobei in der Elutionsreihenfolge 115 mg (5*Z*,7*E*)-(1*S*,3*R*,24*R*)-1,3-Bis[[dimethyl(1,1-dimethylethyl)silyl]oxy]-25-(4-methylphenyl)-26,27-cyclo-9,10-secocholesta-5,7,10(19),20(22)-tetraen-24-ol **57b,** 50 mg (5*Z*,7*E*,22*E*)-(1*S*,3*R*,24*S*)-1,3-Bis[[dimethyl(1,1-dimethylethyl)silyl]oxy]-25-(4-methylphenyl)-26,27-cyclo-9,10-secocholesta-5,7,10(19),22-tetraen-24-ol **58a** und 30 mg (5*Z*,7*E*,22*E*)-(1*S*,3*R*,24*R*)-1,3-Bis[[dimethyl(1,1-dimethylethyl)silyl]oxy]-25-(4-methylphenyl)-26,27-cyclo-9,10-secocholesta-5,7,10(19),22-tetraen-24-ol **58b** als farblose Schäume anfallen.

115 mg **57b** werden in 15 ml THF gelöst und unter Stickstoff mit 468 mg Tetrabutylammoniumfluorid (Trihydrat) über Nacht bei Raumtemperatur gerührt. Man gibt dann gesättigte Natriumhydrogencarbonat-Lösung zu und extrahiert mit Essigester. Die vereinigten organischen Phasen werden mit Wasser gewaschen, über Natriumsulfat getrocknet und eingeengt. Chromatographie des Rückstandes an Kieselgel mit Essigester/Hexan ergeben 31 mg der Titelverbindung **59b** als farblosen Schaum.

$^{1}$H-NMR (300 MHz, $CD_2Cl_2$): δ= 0,41 ppm (s, 3H); 1,56 (s, 3H); 2,30 (s, 3H); 3,17 (m, 1H); 4,18 (m, 1H); 4,38 (m, 1H); 4,96 (s, 1H); 5,22 (t, 1H); 5,28 (s, 1H); 6,01 (d, 1H); 6,34 (d, 1H); 7,08 (d, 2H); 7,22 (d, 2H)

Analog zu **59b** erhält man aus **58a** sowie **58b** die Titelverbindungen **60a** und **60b** als farblose Schäume.

**60a:** $^{1}$H-NMR (300 MHz, $CD_2Cl_2$): δ= 0,52 ppm (s, 3H); 0,99 (d, 3H); 2,28 (s, 3H); 3,67 (m, 1H); 4,18 (m, 1H); 4,38 (m, 1H); 4,96 (s, 1H), 5,28 (dd, 1H); 5,29 (s, 1H); 5,39 (dd, 1H); 6,01 (d, 1H); 6,36 (d, 1H); 7,08 (d, 1H); 7,19 (d, 1H)
**60b:** $^{1}$H-NMR (300 MHz, $CD_2Cl_2$): δ= 0,53 ppm (s, 3H); 0,98 (d, 3H); 3,28 (s, 3H); 3,70 (m, 1H); 4,18 (m, 1H); 4,38 (m, 1H); 4,96 (s, 1H); 5,27 (dd, 1H); 5,29 (s, 1H); 5,37 (dd, 1H); 6,01 (d, 1H); 6,36 (d, 1H); 7,09 (d, 1H); 7,20 (d, 1H)

**Beispiel 11**

(5*Z*,7*E*)-(1*S*,3*R*,24*R*)-25-[4-(1-Methylethyl)phenyl]-26,27-cyclo-9,10-secocholesta-5,7,10(19),20(22)-tetraen-1,3,24-triol **66b,** (5*Z*,7*E*,22*E*)-(1*S*,3*R*,24*S*)-25-[4-(1-Methylethyl)-phenyl]-26,27-cyclo-9,10-secocholesta-5,7,10(19),22-tetraen-1,3,24-triol **67a** und (5*Z*,7*E*,22*E*)-(1*S*,3*R*,24*R*)-25-[4-(1-Methylethyl)phenyl]-26,27-cyclo-9,10-secocholesta-5,7,10(19),22-tetraen-1,3,24-triol 67b

Man tropft 1,11 ml *n*-Butyllithium-Lösung (1.6 M in Hexan) bei 0°C zu 0,36 ml Diisopropylamin in 10 ml THF unter Stickstoff. Man rührt 20 min bei dieser Temperatur, kühlt auf -78°C und tropft dann 560 mg des Ketons **47** in 2 ml THF zu. Nach 1 h bei -78°C werden 500 mg des Aldehydes **30** in 10 ml THF zugegeben und es wird 1 h nachgerührt. Die Reaktionsmischung wird anschließend mit Ammoniumchlorid-Lösung behandelt und mit Essigester extrahiert. Nach Trocknung der organischen Phase über Natriumsulfat entfernt man das Solvens und chromatographiert an Kieselgel mit Essigester/Hexan, wobei man 497 mg (5*Z*,7*E*)-(1*S*,3*R*)-1,3-Bis[[dimethyl(1,1-dimethylethyl)silyl]oxy]-22-hydroxy-25-[4-(1-methyl-ethyl)phenyl]-26,27-cyclo-9,10-secocholesta-5,7,10(19)-trien-24-on **61** als farblosen Schaum erhält.

490 mg **61** werden in 20 ml Toluol gelöst, mit 0,30 ml Essigsäureanhydrid, 0,44 ml Triethylamin sowie einer Spatelspitze Dimethylaminopyridin (DMAP) versetzt und über Nacht unter Stickstoff bei Raumtemperatur gerührt. Danach wird die Reaktionsmischung mit Natriumhydrogencarbonat-Losung behandelt und mit Essigester extrahiert. Nach Trocknung der organischen Phase über Natriumsulfat und Chromatographie an Kieselgel mit Essigester/Hexan werden 379 mg (5*Z*,7*E*)-(1*S*,3*R*)-22-(Acetyloxy)-1,3-bis[[dimethyl(1,1-dimethylethyl)silyl]oxy]-25-[4-(1-methylethyl)phenyl]-26,27-cyclo-9,10-secocholesta-5,7,10(19)-trien-24-on **62** als farbloser Schaum erhalten, der in 20 ml Toluol gelöst und mit 3 ml 1,8-Diazabicyclo[5.4.0]undec-7-en (DBU) 30 min bei 40°C unter Stickstoff gerührt wird. Man verdünnt die Reaktionsmischung mit Essigester und säuert mit 0.01 N Salzsäure an. Es wird mit Essigester extrahiert, über Natriumsulfat getrocknet und das Solvens entfernt, wobei 355 mg (5*Z*,7*E*,22*E*)-(1*S*,3*R*)-1,3-Bis[[dimethyl(1,1-dimethylethyl)silyl]oxy]-25-[4-(1-methylethyl)phenyl]-26,27-cyclo-9,10-secocholesta-5,7,10(19),22-tetraen-24-on **63** als farbloses Öl erhalten werden.

**63:** $^{1}$H-NMR (300 MHz, $CDCl_3$): δ= 0,06 ppm (s, 6H); 0,48 (s, 3H); 0,90 (s, 18H); 0,98 (d, 3H); 1,29 (d, 6H); 2,92 (hept, 1H); 4,18 (m, 1H); 4,37 (m, 1H); 4,85 (s, 1H); 5,18 (s, 1H); 5,95 (d, 1H); 6,00 (d, 1H); 6,22 (d, 1H); 6,60 (d, 1H); 7,20 (d, 2H); 7,23 (d, 2H) 350 mg des Enons **63** werden in 2 ml THF und 4 ml Methanol gelöst und bei 0°C unter Stickstoff mit 190 mg Certrichlorid-Heptahydrat behandelt. Anschließend gibt man 16 mg Natriumborhydrid zu und rührt 2 h bei 0°C nach. Es wird nun Eiswasser zugegeben, mit Essigester extrahiert und über Natriumsulfat getrocknet. Der Rückstand wird an Kieselgel mit Essigester/Hexan chromatographiert, wobei in der Elutionsreihenfolge 27 mg (5*Z*,7*E*)-(1*S*,3*R*,24*R*)-1,3-Bis[[dimethyl(1,1-dimethylethyl)silyl]oxy]-25-[4-(1-methylethyl)phenyl]-26,27-cyclo-9,10-secocholesta-5,7,10(19),20(22)-tetraen-24-ol **64b,** 40 mg (5*Z*,7*E*,22*E*)-(1*S*,3*R*,24*S*)-1,3-Bis[[dimethyl(1,1-dimethylethyl) silyl]oxy]-25-[4-(1-methylethyl)phenyl]-26,27-cyclo-9,10-secocholesta-5,7,10(19),22-tetraen-24-ol **65a** und 48 mg (5*Z*,7*E*,22*E*)-(1*S*,3*R*,24*R*)-1,3-Bis[[dimethyl(1,1-dimethylethyl)silyl]oxy]-25-[4-(1-methylethyl)phenyl]-26,27-cyclo-9,10-secocholesta-5,7,10(19),22-tetraen-24-ol **65b** als farblose Schäume anfallen.

27 mg **64b** werden in 5 ml THF gelöst und unter Stickstoff mit 112 mg Tetrabutylammoniumfluorid (Trihydrat) über Nacht bei Raumtemperatur gerührt. Man gibt dann gesättigte Natriumhydrogencarbonat-Lösung zu und extrahiert mit Essigester. Die vereinigten organischen Phasen werden mit Wasser gewaschen, über Natriumsulfat getrocknet und eingeengt. Chromatographie des Rückstandes an Kieselgel mit Essigester/Hexan ergeben 12 mg der Titelverbindung **66b** als farblosen Schaum.

$^1$H-NMR (300 MHz, $CD_2Cl_2$): δ= 0,45 ppm (s, 3H), 1,23 (d, 6H); 1,56 (s, 3H); 2,86 (hept, 1H); 3,18 (m, 1H); 4,17 (m, 1H); 4,37 (m, 1H); 4,96 (s, 1H); 5,18 (s, 1H); 5,25 (t, 1H); 6,00 (d, 1H); 6,36 (d, 1H); 7,14 (d, 2H); 7,27 (d, 2H)

Analog zu **66b** erhält man aus **65a** sowie **65b** die Titelverbindungen **67a** und **67b** als farblose Schäume.

**67a**: $^1$H-NMR (300 MHz, $CD_2Cl_2$): δ= 0,52 ppm (s, 3H); 0,98 (d, 3H); 1,20 (d, 6H); 2,86 (hept, 1H); 3,71 (m, 1H); 4,17 (m, 1H); 4,37 (m, 1H); 4,95 (s, 1H); 5,28 (s, 1H); 5,30 (dd, 1H); 5,39 (dd, 1H); 5,99 (d, 1H); 6,34 (d, 1H); 7,10 (d, 1H); 7,22 (d, 1H)

**67b**: $^1$H-NMR (300 MHz, $CD_2Cl_2$): δ= 0,53 ppm (s, 3H); 0,99 (d, 3H); 1,21 (d, 6H); 2,86 (hept, 1H); 3,67 (m, 1H); 4,17 (m, 1H); 4,37 (m, 1H); 4,96 (s, 1H); 5,28 (s, 1H); 5,29 (dd, 1H); 5,38 (dd, 1H); 6,01 (d, 1H); 6,37 (d, 1H); 7,13 (d, 1H); 7,25 (d, 1H)

**Beispiel 12**

(5*Z*,7*E*)-(1*S*,3*R*,24*R*)-25-(4-Butylphenyl)-26,27-cyclo-9,10-secocholesta-5,7,10(19),20(22)-tetraen-1,3,24-triol **73b,** (5*Z*,7*E*,22*E*)-(1*S*,3*R*,24*S*)-25-(4-Butylphenyl)-26,27-cyclo-9,10-secocholesta-5,7,10(19),22-tetraen-1,3,24-triol **74a** und (5*Z*,7*E*,22*E*)-(1*S*,3*R*,24*R*)-25-(4-Butylphenyl)-26,27-cyclo-9,10-secocholesta-5,7,10(19),22-tetraen-1,3,24-triol **74b**

Man tropft 1,28 ml *n*-Butyllithium-Lösung (1.6 M in Hexan) bei 0°C zu 0,42 ml Diisopropylamin in 10 ml THF unter Stickstoff. Man rührt 20 min bei dieser Temperatur, kühlt auf -78°C und tropft dann 692 mg des Ketons **53** in 4 ml THF zu. Nach 1 h bei -78°C werden 484 mg des Aldehydes **30** in 10 ml THF zugegeben und es wird 1 h nachgerührt. Die Reaktionsmischung wird anschließend mit Ammoniumchlorid-Lösung behandelt und mit Essigester extrahiert. Nach Trocknung der organischen Phase über Natriumsulfat entfernt man das Solvens und chromatographiert an Kieselgel mit Essigester/Hexan, wobei man 456 mg (5*Z*,7*E*)-(1*S*,3*R*)-1,3-Bis[[dimethyl(1,1-dimethylethyl)silyl]oxy]-25-(4-butylphenyl)-22-hydroxy-26,27-cyclo-9,10-secocholesta-5,7,10(19)-trien-24-on **68** als farblosen Schaum erhält. 450 mg **68** werden in 18 ml Toluol gelöst, mit 0,27 ml Essigsäureanhydrid, 0,40 ml Triethylamin sowie einer Spatelspitze Dimethylaminopyridin (DMAP) versetzt und über Nacht unter Stickstoff bei Raumtemperatur gerührt. Danach wird die Reaktionsmischung mit Natriumhydrogencarbonat-Lösung behandelt und mit Essigester extrahiert. Nach Trocknung der organischen Phase über Natriumsulfat und Chromatographie an Kieselgel mit Essigester/Hexan werden 399 mg (5*Z*,7*E*)-(1*S*,3*R*)-22-(Acetyloxy)-1,3-bis[[dimethyl(1,1-dimethylethyl)silyl]oxy]-25-(4-butylphenyl)-26,27-cyclo-9,10-secocholesta-5,7,10(19)-trien-24-on **69** als farbloser Schaum erhalten, der in 18 ml Toluol gelöst und mit 2,7 ml 1,8-Diazabicyclo[5.4.0]undec-7-en (DBU) 30 min bei 40°C unter Stickstoff gerührt wird. Man verdünnt die Reaktionsmischung mit Essigester und säuert mit 0.01 N Salzsäure an. Es wird mit Essigester extrahiert, über Natriumsulfat getrocknet und das Solvens entfernt, wobei 357 mg (5*Z*,7*E*,22*E*)-(1*S*,3*R*)-1,3-Bis[[dimethyl(1,1-dimethylethyl)silyl]oxy]-25-(4-butylphenyl)-26,27-cyclo-9,10-secocholesta-5,7,10(19),22-tetraen-24-on **70** als farbloses Öl erhalten werden.

**70:** $^1$H-NMR (300 MHz, $CDCl_3$): δ= 0,08 ppm (s, 6H); 0,48 (s, 3H); 0,89 (s, 18H); 0,93 (t, 3H); 0,95 (d, 3H); 2,62 (t, 2H); 4,19 (m, 1H); 4,38 (m, 1H); 4,86 (s, 1H); 5,18 (s, 1H); 5,97 (d, 1H); 6,00 (d, 1H); 6,22 (d, 1H); 6,63 (d, 1H); 7,16 (d, 2H); 7,22 (d, 2H)

350 mg des Enons **70** werden in 2 ml THF und 4 ml Methanol gelöst und bei 0°C unter Stickstoff mit 186 mg Certrichlorid-Heptahydrat behandelt. Anschließend gibt man 16 mg Natriumborhydrid zu und rührt 2 h bei 0°C nach. Es wird nun Eiswasser zugegeben, mit Essigester extrahiert und über Natriumsulfat getrocknet. Der Rückstand wird an Kieselgel mit Essigester/Hexan chromatographiert, wobei in der Elutionsreihenfolge 24 mg (5*Z*,7*E*)-(1*S*,3*R*,24*R*)-1,3-Bis[[dimethyl(1,1-dimethylethyl)silyl]oxy]-25-(4-butylphenyl)-26,27-cyclo-9,10-secocholesta-5,7,10(19),20(22)-tetraen-24-ol **71b,** 40 mg (5*Z*,7*E*,22*E*)-(1*S*,3*R*,24*S*)-1,3-Bis[[dimethyl(1,1-dimethylethyl)silyl]oxy]-25-(4-butylphenyl)-26,27-cyclo-9,10-secocholesta-5,7,10(19),22-tetraen-24-ol **72a** und 51 mg (5*Z*,7*E*,22*E*)-(1*S*,3*R*,24*R*)-1,3-Bis[[dimethyl(1,1-dimethylethyl)silyl]oxy]-25-(4-butylphenyl)-26,27-cyclo-9,10-secocholesta-5,7,10(19),22-tetraen-24-ol **72b** als farblose Schäume anfallen.

24 mg **71b** werden in 5 ml THF gelöst und unter Stickstoff mit 97 mg Tetrabutylammoniumfluorid (Trihydrat) über Nacht bei Raumtemperatur gerührt. Man gibt dann gesättigte Natriumhydrogencarbonat-Lösung zu und extrahiert mit Essigester. Die vereinigten organischen Phasen werden mit Wasser gewaschen, über Natriumsulfat getrocknet und eingeengt. Chromatographie des Rückstandes an Kieselgel mit Essigester/Hexan ergeben 13 mg der Titelverbindung **73b** als farblosen Schaum.

$^1$H-NMR (300 MHz, $CD_2Cl_2$): δ= 0,42 ppm (s, 3H); 0,91 (t, 3H); 1,68 (s, 3H); 2,58 (t, 2H); 3,18 (m, 1H); 4,17 (m, 1H); 4,38 (m, 1H); 4,98 (s, 1H); 5,25 (t, 1H); 5,30 (s, 1H); 6,02 (d, 1H); 6,36 (d, 1H); 7,09 (d, 2H); 7,27 (d, 2H)

Analog zu **73b** erhält man aus **72a** sowie **72b** die Titelverbindungen **74a** und **74b** als farblose Schäume.
**74a:** $^1$H-NMR (300 MHz, $CD_2Cl_2$): δ= 0,55 ppm (s, 3H); 0,92 (t, 3H); 0,98 (d, 3H); 2,58 (t, 2H); 3,71 (m, 1H); 4,16 (m, 1H); 4,37 (m, 1H); 4,97 (s, 1H); 5,29 (s, 1H); 5,31 (dd, 1H); 5,39 (dd, 1H); 6,00 (d, 1H); 6,36 (d, 1H); 7,08 (d, 1H); 7,23 (d, 1H)
**74b:** $^1$H-NMR (300 MHz, $CD_2Cl_2$): δ= 0,56 ppm (s, 3H); 0,93 (t, 3H); 0,98 (d, 3H); 2,58 (t, 2H); 3,68 (m, 1H); 4,17 (m, 1H);-4,38 (m, 1H); 4,98 (s, 1H); 5,28 (dd, 1H); 5,29 (s, 1H); 5,38 (dd, 1H); 6,01 (d, 1H); 6,37 (d, 1H); 7,08 (d, 1H); 7,22 (d, 1H)

**Synthese der Ausgangsmaterialien in der 4-Alkyloxazol-Reihe**

2-(1-Bromcyclopropyl)-4-methyloxazol **77**

Man gibt 7,45 g N,N'-Dicyclohexylcarbodiimid bei 0°C unter Stickstoff zu einer Lösung von 5,0 g 1-Bromcyclopropancarbonsaure **4** und 4,18 g N-Hydroxysuccinimid in 87 ml Methylenchlorid und rührt 1,5 h nach. Anschließend werden 9,40 g 2-Aminopropionaldehyddimethylacetal hinzugefügt und 2 h bei Raumtemperatur gerührt. Nach Verdünnung mit Methylenchlorid wird filtriert, eingeengt und der Rückstand an Kieselgel mit Essigester/Hexan chromatographiert. Es werden 6,18 g 1-Brom-N-[(2,2-dimethoxy-1-methyl)ethyl]-cyclopropan-1-carboxamid **75** als farbloses trübes Öl erhalten, welches in 247 ml Acetonitril aufgenommen und mit 12,4 ml 2 N Salzsäure 7 h bei Raumtemperatur gerührt wird. Nach Zugabe von Natriumchlorid-Lösung wird mit Essigester extrahiert, über Natriumsulfat getrocknet und das Losungsmittel entfernt. Der Rückstand wird an Kieselgel mit Essigester/Hexan chromatographiert, wobei 3,83 g 1-Brom-N-[(1-formyl)ethyl]cyclopropyl-1-carboxamid **76** anfallen.
3,43 g des Amids **76**, 5,54 g Hexachlorethan und 7,62 ml Triethylamin werden in 47 ml Acetonitril gelöst und auf -25°C gekühlt. Man gibt 6,14 g Triphenylphosphin hinzu und rührt 4,5 h bei Raumtemperatur nach. Es wird nun filtriert, eingeengt und der Rückstand an Kieselgel mit Essigester/Hexan chromatographiert, wobei 2,06 g der Titelverbindung **77** als gelbes Öl erhalten werden.
$^1$H-NMR (300 MHz, $CDCl_3$): δ= 1,50 ppm (m, 2H); 1,65 (m, 2H); 2,13 (s, 3H); 7,32 (s, 1H)

2-(1-Bromcyclopropyl)-4-ethyloxazol **80**

Man gibt 7,45 g N,N'-Dicyclohexylcarbodiimid bei 0°C unter Stickstoff zu einer Lösung von 5,0 g 1-Bromcyclopropancarbonsäure **4** und 4,18 g N-Hydroxysuccinimid in 87 ml Methylenchlorid und rührt 1,5 h nach. Anschließend werden 7,46 ml 2-Amino-1-butanol hinzugefügt und 2 h bei Raumtemperatur gerührt. Nach Verdünnung mit Methylenchlorid wird filtriert, eingeengt und der Rückstand an Kieselgel mit Essigester/Hexan chromatographiert. Es werden 5,44 g 1-Brom-N-[(1-hydroxymethyl)propyl]-cyclopropan-1-carboxamid **78** als gelbliches Öl erhalten.
2,23 ml Oxalylchlorid in 102 ml Methylenchlorid werden bei -78°C unter Stickstoff mit 3,6 ml Dimethylsulfoxid in 41 ml Methylenchlorid tropfenweise versetzt. Anschließend werden 5,43 g des Amidalkohols **78** in 41 ml Methylenchlorid und nach 15 min 14,3 ml Triethylamin bei -78°C zugetropft. Nach weiteren 30 min gibt man Natriumchlorid-Lösung hinzu, extrahiert mit Methylenchlorid, trocknet über Natriumsulfat und engt ein. Der Rückstand wird an Kieselgel mit Essigester/Hexan chromatographiert, wobei 3,4 g 1-Brom-N-[(1-formyl)propyl]-cyclopropan-1-carboxamid **79** als gelbes Öl erhalten werden, welches analog der Reaktion des Amids **76** in die Titelverbindung **80** überführt wird.
$^1$H-NMR (300 MHz, $CDCl_3$): δ= 1,19 ppm (t, 3H); 1,50 (m, 2H); 1,62 (m, 2H); 2,50 (q, 2H); 7,29 (s, 1H)

2-(1-Bromcyclopropyl)-4-propyloxazol **81**

Analog der Synthese des Oxazols **80** wird unter Verwendung von 2-Aminopentanol die Titelverbindung **81** erhalten.
$^1$H-NMR (300 MHz, $CDCl_3$): δ= 0,92 ppm (t, 3H); 1,50 (m, 2H); 1,62 (m, 4H); 2,45 (t, 2H); 7,30 (s, 1H)

**Beispiel 13**

(5*Z*,7*E*,22*E*)-(1*S*,3*R*,24*S*)-25-(4-Methyloxazol-2-yl)-26,27-cyclo-9,10-secocholesta-5,7,10(19),22-tetraen-1,3,24-triol **83a** und (5*Z*,7*E*,22*E*)-(1*S*,3*R*,24*R*)-25-(4-Methyloxazol-2-yl)-26,27-cyclo-9,10-secocholesta-5,7,10(19),22-tetraen-1,3,24-triol **83b**

Man legt 2,75 ml *tert*.-Butyllithium (1.7 M in Pentan) bei -20°C unter Stickstoff in 8 ml Diethylether vor, kühlt auf -78°C und tropft 505 mg 2-(1-Bromcyclopropyl)-4-methyloxazol **77** in 1,15 ml Diethylether zu. Nach 5 min tropft man den Aldehyd **3** in 3 ml Diethylether hinzu. rührt 10 min bei -78°C und läßt dann in 1,5 h auf 0°C kommen. Anschließend gibt man Ammoniumchlorid-Lösung zu, extrahiert mit Essigester, trocknet über Natriumsulfat und engt ein.

Der Rückstand wird an Kieselgel mit Essigester/Hexan chromatographiert, wobei man in der Elutionsreihenfolge 180 mg (5*Z*,7*E*,22*E*)-(1*S*,3*R*,24*S*)-1,3-Bis-[[dimethyl(1,1-dimethylethyl)silyl]oxy]-25-(4-methyloxazol-2-yl)-26,27-cyclo-9,10-secocholesta-5,7,10(19),22-tetraen-24-ol **82a** und 180 mg (5*Z*,7*E*,22*E*)-(1*S*,3*R*,24*R*)-1,3-Bis-[[dimethyl(1,1-dimethylethyl)silyl]oxy]-25-(4-methyloxazol-2-yl)-26,27-cyclo-9,10-secocholesta-5,7,10(19),22-tetraen-24-ol **82b** als farblose Öle erhält.

180 mg des Disilylethers **82a** werden in 7,3 ml THF gelöst und mit 384 mg Tetrabutylammoniumfluorid (Trihydrat) über Nacht bei Raumtemperatur unter Stickstoff gerührt. Man gibt dann ein Gemisch von Natriumchlorid und Natriumhydrogencarbonat-Lösung hinzu, extrahiert mit Essigester, wäscht die organische Phase mit Natriumchlorid-Lösung, trocknet über Natriumsulfat und entfernt das Solvens. Der Rückstand wird an Kieselgel mit Essigester/Hexan chromatographiert, wobei man 63 mg der Titelverbindung **83a** als farblosen Schaum erhält.

$^1$H-NMR (300 MHz, $CDCl_3$): δ= 0,52 ppm (s, 3H); 1,00 (d, 3H); 1,05 (m, 4H); 2,11 (s, 3H); 4,10 (m, 2H); 4,23 (m, 1H); 4,43 (m, 1H); 5,00 (brs, 1H); 5,32 (brs, 1H); 5,42 (dd, 1H); 5,58 (dd, 1H); 6,00 (d, 1H); 6,38 (d, 1H); 7,18 (s, 1H)

Analog erhält man aus dem Disilylether **82b** die Titelverbindung **83b** als farblosen Schaum.

$^1$H-NMR (300 MHz, $CDCl_3$): δ= 0,52 ppm (s, 3H); 0,98 (d, 3H); 1,05 (m, 4H); 2,08 (s, 3H); 4,08 (m, 1H); 4,21 (m, 1H); 4,40 (m, 1H); 4,48 (brs, 1H); 5,30 (brs, 1H); 5,40 (dd, 1H); 5,51 (dd, 1H); 5,98 (d, 1H); 6,35 (d, 1H); 7,18 (s, 1H)

**Beispiel 14**

(5*Z*,7*E*,22*E*)-(1*S*,3*R*,24*R*)-25-(4-Ethyloxazol-2-yl)-26,27-cyclo-9,10-secocholesta-5,7,10(19),22-tetraen-1,3,24-triol **84b**

Analog Beispiel 13 wird aus dem Aldehyd **3** und dem Oxazol **80** die Titelverbindung **84b** als farbloser Schaum erhalten.

$^1$H-NMR (300 MHz, $CDCl_3$): δ= 0,52 ppm (s, 3H); 0,98 (d, 3H); 1,08 (t, 3H); 2,48 (q, 2H); 4,09 (d, 1H); 4,23 (m, 1H); 4,43 (m, 1H); 4,47 (brs, 1H); 5,30 (brs, 1H); 5,40 (dd, 1H); 5,52 (dd, 1H); 6,00 (d, 1H); 6,36 (d, 1H); 7,15 (s, 1H)

**Beispiel 15**

(5*Z*,7*E*,22*E*)-(1*S*,3*R*,24*R*)-25-(4-Propyloxazol-2-yl)-26,27-cyclo-9,10-secocholesta-5,7,10(19),22-tetraen-1,3,24-triol **85b**

Analog Beispiel 13 wird aus dem Aldehyd **3** und dem Oxazol **81** die Titelverbindung **85b** als farbloser Schaum erhalten.

$^1$H-NMR (300 MHz, $CDCl_3$): δ= 0,50 ppm (s, 3H); 0,91 (t, 3H); 0,98 (d, 3H); 1,20 (m, 4H); 2,40 (t, 2H); 4,08 (d, 1H); 4,20 (m, 1H); 4,40 (m, 1H); 4,45 (brs, 1H); 5,28 (brs, 1H); 5,38 (dd, 1H); 5,50 (dd, 1H); 5,95 (d, 1H); 6,45 (d, 1H); 7,15 (s, 1H)

**Beispiel 16**

(5*Z*,7*E*,22*E*)-(1*S*,3*R*,24*R*)-25-(5-Butyloxazol-2-yl)-26,27-cyclo-9,10-secocholesta-5,7,10(19),22-tetraen-1,3,24-triol **88b**

Analog Amid **6** wird 1-Brom-N-(2-oxohexyl)-cyclopropan-1-carbamid **86** hergestellt und wie für Verbindung **8** beschrieben in 2-(1-Bromcyclopropyl)-5-butyloxazol **87** überführt.

**87:** $^1$H-NMR (300 MHz, $CDCl_3$): δ= 0,92 ppm (t, 3H); 1,40 (m, 2H); 1,50 (m, 2H); 1,60 (m, 4H); 2,60 (t, 2H); 6,65 (s, 1H)

Analog dem Beispiel 1 wird aus dem Aldehyd **3** und dem Oxazol **87** die Titelverbindung **88b** als farbloser Schaum erhalten.

$^1$H-NMR (300 MHz, $CDCl_3$): δ= 0,57 ppm (s, 3H); 0,90 (t, 3H); 0,97 (m, 2H); 1,05 (d, 3H); 1,15 (m, 2H); 2,58 (m, 3H); 4,12 (d, 1H); 4,23 (m, 1H); 4,43 (m, 1H); 5,00 (brs, 1H); 5,32 (brs, 1H); 5,42 (dd, 1H); 5,53 (dd, 1H); 6,00 (d, 1H); 6,38 (d, 1H); 6,58 (s, 1H)

**Ausgangsmaterialien in der 5-Alkyl-Thiazolreihe**

2-(1-Bromcyclopropyl)-5-butylthiazol **89**

500 mg des Amides **86** und 212 mg Phosphorpentasulfid werden in 2 ml Dioxan 25 min auf 100°C erhitzt und nach Abkühlung in verdünnte Natronlauge gegossen. Nach Extraktion mit Essigester und Trocknung über Natriumsulfat

wird eingeengt und der ölige Rückstend an Kieselgel mit Essigester/Hexan chromatographiert. Man erhält 240 mg der Titelverbindung **89** als farbloses Öl.

$^1$H-NMR (300 MHz, $CDCl_3$): δ= 0,92 ppm (t, 3H); 1,48 (m, 2H); 1,65 (m, 6H), 2,75 (t, 2H); 7,32 (s, 1H)

2-(1-Bromcyclopropyl)-5-ethylthiazol **90**

Aus dem Amid **9** wird in Analogie zur Herstellung von Thiazol **89** die Titelverbindung **90** als farbloses Öl erhalten.

$^1$H-NMR (300 MHz, $CDCl_3$): δ= 1,28 ppm (t, 3H); 1,58 (m, 2H); 1,70 (m, 2H); 2,80 (q, 2H); 7,32 (s, 1H)

**Beispiel 17**

(5*Z*,7*E*,22*E*)-(1*S*,3*R*,24*S*)-25-(5-Butylthiazol-2-yl)-26,27-cyclo-9,10-secocholesta-5,7,10(19),22-tetraen-1,3,24-triol **92a** (5*Z*,7*E*,22*E*)-(1*S*,3*R*,24*R*)-25-(5-Butylthiazol-2-yl)-26,27-cyclo-9,10-secocholesta-5,7,10(19),22-tetraen-1,3,24-triol **92b**

Bei -78°C werden unter Stickstoff zu 16 ml Trapp-Mischung (THF/Diethylether/Pentan 4:1:1) 5,9 ml *tert*.-Butyllithium (1.7 M in Pentan) gegeben. Bei -116°C werden 1,3 g Thiazol **89** in 2,4 ml Trapp-Mischung zugetropft. Nach 1 h gibt man 1,0 g des Aldehydes **3** in 6,4 ml Trapp-Mischung hinzu und rührt 1 h bei -116°C und 1 h bei -78°C. Man quencht dann mit Ammoniumchlorid-Lösung, extrahiert mit Diethylether und trocknet über Natriumsulfat. Nach dem Einengen wird der Rückstand an Kieselgel mit Essigester/Hexan chromatographiert, wobei in der Elutionsreihenfolge 470 mg (5*Z*,7*E*,22*E*)-(1*S*,3*R*,24*S*)-1,3-Bis-[[dimethyl(1,1-dimethylethyl)silyl]oxy]-25-(5-butylthiazol-2-yl)-26,27-cyclo-9,10-secocholesta-5,7,10(19),22-tetraen-24-ol **91a** und **440** mg (5*Z*,7*E*,22*E*)-(1*S*,3*R*,24*R*)-1,3-Bis-[[dimethyl(1,1-dimethylethyl)silyl]oxy]-25-(5-butylthiazol-2-yl)-26,27-cyclo-9,10-secocholesta-5,7,10(19),22-tetraen-24-ol **91b** als gelbe Öle anfallen .

460 mg Disilylether **91a** werden in 17 ml THF gelöst und unter Stickstoff mit 900 mg Tetrabutylammoniumfluorid (Trihydrat) behandelt. Man rührt über Nacht und gibt dann ein Gemisch aus Natriumchlorid- und Natriumhydrogencarbonat-Lösung zu. Anschließend extrahiert man mit Essigester, wäscht die organische Phase mit Natriumchlorid-Lösung, trocknet über Natriumsulfat und engt ein. Der Rückstand wird an Kieselgel chromatographiert, wobei 139 mg der Titelverbindung **92a** als farbloser Schaum erhalten werden.

**92a**: $^1$H-NMR (300 MHz, $CDCl_3$): δ= 0,52 ppm (s, 3H); 0,92 (t, 3H); 0,98 (d, 3H); 1,08 (m, 4H); 2,75 (t, 2H); 4,00 (d, 1H); 4,23 (m, 1H); 4,43 (m, 1H); 5,00 (brs, 1H); 5,32 (brs, 1H); 5,40 (dd, 1H); 5,55 (dd, 1H); 6,00 (d, 1H); 6,38 (d, 1H), 7,25 (s, 1H)

Analog erhält man aus dem Disilylether **91b** die Titelverbindung **92b** als farblosen Schaum.

**92b:** $^1$H-NMR (300 MHz, $CDCl_3$): δ= 0,52 ppm (s, 3H); 0,92 (t, 3H); 0,98 (d, 3H); 1,08 (m, 4H); 2,75 (t, 2H); 4,00 (d, 1H); 4,23 (m, 1H); 4,43 (m, 1H); 5,00 (brs, 1H); 5,32 (brs, 1H); 5,40 (dd, 1H); 5.55 (dd, 1H); 6,00 (d, 1H); 6,38 (d, 1H); 7,25 (s, 1H)

**Beispiel 18**

(5*Z*,7*E*,22*E*)-(1*S*,3*R*,24*R*)-25-(5-Ethylthiazol-2-yl)-26,27-cyclo-9,10-secocholesta-5,7,10(19),22-tetraen-1,3,24-triol **93b**

Ausgehen vom Aldehyd **3** wird analog Beispiel 17 mit dem Thiazol **90** die Titelverbindung als farbloser Schaum erhalten.

$^1$H-NMR (300 MHz, $CDCl_3$): δ= 0,52 ppm (s, 3H); 0,98 (d, 3H); 1,08 (m, 4H); 1,28 (t, 3H); 2,80 (q, 2H); 4,01 (d, 1H); 4,23 (m, 1H); 4,43 (m, 1H); 5,00 (brs, 1H); 5,32 (brs, 1H); 5,40 (dd, 1H); 5,52 (dd, 1H); 6,00 (d, 1H); 6,37 (d, 1H); 7,28 (s, 1H)

**Beispiel 19**

(5*Z*,7*E*,22*E*)-(1*S*,3*R*,24*S*)-25-(3-Butyl-1,2,4-oxadiazol-5-yl)-26,27-cyclo-9,10-secocholesta-5,7,10(19),22-tetraen-1,3,24-triol **95a**

Man löst 28 mg Natrium in 0,8 ml Methanol und gibt unter Stickstoff 200 mg (5*Z*,7*E*,22*E*)-(1*S*,3*R*,24*S*)-1,3-Bis-[[dimethyl(1,1-dimethylethyl)silyl]oxy]-24-hydroxy-26,27-cyclo-9,10-secocholesta-5,7,10(19),22-tetraen-25-carbonsäuremethylester **94a** (Herstellung siehe DE 42 34 382) in 0,8 ml Methanol und 55 mg Valerylamidoxim [K. P. Flora et al. *Cancer Res*. 1291 (1978)] zu und erhitzt 9,25 h zum Sieden. Die Reaktionsmischung wird in Natriumchlorid-Lösung gegeben, mit Essigester extrahiert und die organische Phase mit Natriumchlorid-Lösung gewaschen. Nach Trocknung mit Natriumsulfat und Einengen wird der ölige Rückstand chromatographisch an Kieselgel mit Essigester/Hexan gereinigt, wobei man 40 mg der Titelverbindung **95a** als farblosen Schaum erhält.

$^1$H-NMR (300 MHz, $CDCl_3$): δ= 0,52 ppm (s, 3H); 0,95 (t, 3H); 1,02 (d, 3H); 1,20 (m, 4H); 2,68 (t, 2H), 4,23 (m, 2H); 4,43 (m, 1H); 5,00 (brs, 1H); 5,32 (brs, 1H); 5,42 (dd, 1H); 5,62 (dd, 1H); 6,00 (d, 1H); 6,38 (d, 1H)

**Beispiel 20**

(5*Z*,7*E*,22*E*)-(1*S*,3*R*,24*R*)-25-(3-Butyl-1,2,4-oxadiazol-5-yl)-26,27-cyclo-9,10-secocholesta-5,7,10(19),22-tetraen-1,3,24-triol **95b**

Ausgehend von (5*Z*,7*E*,22*E*)-(1*S*,3*R*,24*R*)-1,3-Bis-[[dimethyl(1,1-dimethylethyl)silyl]oxy]-24-hydroxy-26,27-cyclo-9,10-secocholesta-5,7,10(19),22-tetraen-25-carbonsäuremethylester **94b** (Herstellung siehe DE 42 34 382) wird analog Beispiel 20 die Titelverbindung **95b** als farbloser Schaum erhalten.

$^1$H-NMR (300 MHz, $CDCl_3$): δ= 0,52 ppm (s, 3H); 0,92 (t, 3H); 1,03 (d, 3H); 1,20 (m, 4H); 2,70 (t, 2H); 3,55 (brd, 1H); 4,23 (m, 2H); 4,43 (m, 1H); 5,00 (brs, 1H); 5,32 (brs, 1H); 5,43 (dd, 1H); 5,58 (dd, 1H); 6,00 (d, 1H); 6,38 (d, 1H)

**Beispiel 21**

(5*Z*,7*E*,22*E*)-(1*S*,3*R*,24*R*)-25-(3-Ethyl-1,2,4-oxadiazol-5-yl)-26,27-cyclo-9,10-secocholesta-5,7,10(19),22-tetraen-1,3,24-triol **96b**

Ausgehend von (5*Z*,7*E*,22*E*)-(1*S*,3*R*,24*R*)-1,3-Bis-[[dimethyl(1,1-dimethylethyl)silyl]oxyl]-24-hydroxy-26,27-cyclo-9,10-secocholesta-5,7,10(19),22-tetraen-25-carbonsäuremethylester **94b** (Herstellung siehe DE 42 34 382) wird analog Beispiel 20 mit Propionamidoxim [K.P. Flora et al. *Cancer Res.* 1291 (1978)] die Titelverbindung **96b** als farbloser Schaum erhalten.

$^1$H-NMR (300 MHz, $CDCl_3$): δ= 0,52 ppm (s, 3H); 1,02 (d, 3H); 1,20 (m, 4H); 1,32 (t, 3H); 2,75 (q, 2H); 4,23 (m, 2H); 4,43 (m, 1H); 5,00 (brs, 1H); 5,32 (brs, 1H); 5,43 (dd, 1H); 5,58 (dd, 1H); 6,00 (d, 1H); 6,38 (d, 1H)

**Ausgangsmaterialien für die 25-Pyridyl-Reihe**

2-(1-Bromcyclopropyl)-pyridin **100**

Man löst 10,0 g 1-Bromcyclopropancarbonsäure **4,** 7,0 g 2-Pyridinthiol und 13,8 g N,N'-Dicyclohexylcarbodiimid in 150 ml Essigester und rührt 2 h unter Stickstoff bei Raumtemperatur. Nach Zugabe von Natriumchlorid-Lösung extrahiert man mit Essigester, wäscht die organische Phase mit gesättigter Natriumchlorid-Lösung, trocknet über Natriumsulfat und engt ein. Der ölige Rückstand wird an Kieselgel mit Essigester/Hexan chromatographiert, wobei 8,25 g 1-Brom-S-(2-pyridyl)-cyclopropanthioat **97** als gelbes Öl erhalten werden. 10,11 g Thioester **97** in 26 ml THF werden bei 0°C mit 3-(1,3-Dioxolan-2-yl)-propylmagnesiumbromid **98** [D. Wenkert et al. *J. Org. Chem.* **50,** 4114 (1985)] tropfenweise versetzt. Nach 3 h bei Raumtemperatur wird die Suspension in Ammoniumchlorid-Lösung eingerührt und mit Essigester extrahiert. Man trocknet über Natriumsulfat, engt ein und chromatographiert den Rückstand an Kieselgel mit Essigester/Hexan, wobei man 7,92 g 1-(1-Bromcyclopropyl)-4-(1-dioxolan-2-yl)-butan-1-on **99** als farbloses Öl erhält. 6,68 g des Ketons **99** in 76 ml Essigsäure werden mit 5,08 g Hydroxylamin Hydrochlorid 1 h unter Stickstoff zum Sieden erhitzt. Nach Verdampfung der Essigsäure wird der ölige Rückstand mit Eis versetzt und mit ca. 20%iger Natronlauge basisch gestellt. Anschließend wird mit Natriumchlorid-Lösung verdünnt und mit Essigester extrahiert. Die organische Phase trocknet man über Natriumsulfat und engt ein. Der Rückstand wird an Kieselgel mit Essigester/Hexan chromatographiert, wobei man 2,73 g der Titelverbindung **100** als hellgelbes Öl erhält.

$^1$H-NMR (300 MHz, $CDCl_3$): δ= 1,55 ppm (m, 2H); 1,68 (m, 2H); 7,10 (m, 1H); 7,65 (m, 1H); 7,75 (m, 1H); 8,48 (m, 1H)

2-(1-Bromcyclopropyl)-6-methylpyridin **103**

Ausgehend von 1-(1-Bromcyclopropyl)-5,5-ethylendioxyhexan-1-on **102**, welches durch Reaktion des Thioesters **97** mit dem Grignard-Reagenz **101** [S. Borrelly, L.A. Paquette *J. Am. Chem. Soc.* **118**, 727 (1996), T.E. Bellas et al. *Tetrahedron* **25**, 5149 (1969)] erhalten wird, stellt man die Titelverbindung **103** in Analogie zu **99** her.

$^1$H-NMR (300 MHz, $CDCl_3$): δ= 1,50 ppm (m, 2H); 1,65 (m, 2H); 2,50 (s, 3H); 6,98 (m, 1H); 7,52 (m, 2H)

**Beispiel 22**

(5*Z*,7*E*,22*E*)-(1*S*,3*R*,24*S*)-25-(2-Pyridyl)-26,27-cyclo-9,10-secocholesta-5,7,10(19),22-tetraen-1,3,24-trios **105a** und (5*Z*,7*E*,22*E*)-(1*S*,3*R*,24*R*)-25-(2-Pyridyl)-26,27-cyclo-9,10-secocholesta-5,7,10(19),22-tetraen-1,3,24-triol **105b**

Bei -20°C werden unter Stickstoff zu 24 ml Diethylether 8,5 ml *tert*.-Butyllithium (1.7 M in Pentan) gegeben. Bei -78°C werden 1,34 g **100** in 3,2 ml Diethylether zugetropft und 30 min bei -78°C gerührt. Danach tropft man 1,34 g des Aldehydes **3** in 8,3 ml Diethylether zu, rührt weitere 30 min bei -78°C und läßt dann in 90 min auf 0°C kommen. Anschließend quencht man mit Ammoniumchlorid-Lösung, extrahiert mit Essigester, trocknet über Natriumsulfat und engt ein. Der Rückstand wird an Kieselgel mit Essigester/Hexan chromatographiert, wobei in der Elutionsreihenfolge 120 mg (5*Z*,7*E*,22*E*)-(1*S*,3*R*,24*S*)-1,3-Bis-[[dimethyl(1,1-dimethylethyl)silyl]oxy]-25-(2-pyridyl)-26,27-cyclo-9,10-secocholesta-5,7,10(19),22-tetraen-24-ol **104a** und 100 mg (5*Z*,7*E*,22*E*)-(1*S*,3*R*,24*R*)-1,3-Bis-[[dimethyl(1,1-dimethylethyl)-silyl]oxy]-25-(2-pyridyl)]26,27-cyclo-9,10-secocholesta-5,7,10(19),22-tetraen-24-ol **104b** jeweils als farblose Schäume erhalten werden.
120 mg der Disilylverbindung **104a** werden in 4,8 ml THF gelöst und mit 257 mg Tetrabutylammoniumfluorid (Trihydrat) unter Stickstoff über Nacht bei Raumtemperatur gerührt. Man gießt die Reaktionsmischung anschließend in ein Gemisch aus Natriumchloridund Natriumhydrogencarbonat-Lösung, extrahiert mit Essigester, wäscht die organische Phase mit Natriumchlorid-Lösung, trocknet über Natriumsulfat und engt ein. Der Rückstand wird an Kieselgel mit Essigester/Hexan chromatographiert, wobei 19 mg der Titelverbindung **105a** als farbloser Schaum anfallen.
**105a:** $^1$H-NMR (300 MHz, $CDCl_3$): δ= 0,48 ppm (s, 3H); 0,88 (m, 4H); 0,95 (d, 3H), 3,88 (d, 1H); 4,23 (m, 2H); 4,43 (m, 1H); 4,98 (brs, 1H); 5,32 (brs, 1H); 5,38 (dd, 1H); 5,50 (dd, 1H); 5,98 (d, 1H); 6,38 (d, 1H); 6,98 (d, 1H); 7,12 (brt, 1H); 7,60 (brt, 1H); 8,45 (d, 1H)
Analog wird der Disilylether **104b** mit Tetrabutylammoniumfluorid (Trihydrat) behandelt, wobei man die Titelverbindung **105b** als farblosen Schaum erhält.
**105b**: $^1$H-NMR (300 MHz, $CDCl_3$): δ= 0,50 ppm (s, 3H); 0,88 (m, 4H); 0,95 (d, 3H); 3,88 (d, 1H); 4,23 (m, 2H); 4,43 (m, 1H); 4,98 (brs, 1H); 5,32 (brs, 1H); 5,40 (m, 2H); 6,00 (d, 1H); 6,38 (d, 1H); 6,98 (d, 1H); 7,12 (brt, 1H); 7,60 (brt, 1H); 8,45 (d, 1H)

**Beispiel 23**

(5*Z*,7*E*,22*E*)-(1*S*,3*R*,24*R*)-25-(6-Methyl-2-pyridyl)-26,27-cyclo-9,10-secocholesta-5,7,10(19),22-tetraen-1,3,24-triol **106b**

Ausgehend vom Aldehyd **3** wird analog Beispiel 22 mit dem Pyridin-Derivat **103** die Titelverbindung **106b** als farbloser Schaum erhalten.
$^1$H-NMR (300 MHz, $CDCl_3$): δ= 0,50 ppm (s, 3H); 0,85 (m, 4H); 0,95 (d, 3H); 2,51 (s, 3H); 3,80 (d, 1H); 4,23 (m, 2H); 4,43 (m, 1H); 4,98 (brs, 1H); 5,32 (brs, 1H); 5,40 (m, 2H); 5,48 (d, 1H); 6,38 (d, 1H); 6,45 (d, 1H), 6,70 (d, 1H); 7,48 (t, 1H)

**Ausgangsmaterialien in der 25-Oxazolin-Reihe**

2-(1-Bromcyclopropyl)-5,5-dimethyl-2-oxazolin **109**

Man versetzt 47 g der Carbonsäure **4** unter Stickstoff mit 78 ml Thionylchlorid und rührt über Nacht bei Raumtemperatur. Anschließend destilliert man das überschüssige Thionylchlorid ab und fraktioniert den Rückstand im Ölpumpenvakuum, wobei 44,74 g 1-Bromcyclopropan-carbonsäurechlorid **107** als farbloses Öl (Sdp. 35-37°C, 0,05mm) anfallen.
$^1$H-NMR (300 MHz, $CDCl_3$): δ= 1,69 ppm (m, 2H); 2,09 (m, 2H)
Man legt 1,69 g 2-Amino-2-methyl-1-propanol in 85 ml Methylenchlorid unter Stickstoff bei Raumtemperatur vor und gibt 2,38 g Natriumcarbonat in 38 ml Wasser und anschließend 3,6 g des Säurechlorides **107** zu. Es wird über Nacht bei Raumtemperatur gerührt und danach mit methanolischer 2 N Natronlauge behandelt. Man trennt die organische Phase ab, trocket über Natriumsulfat und engt ein. Der Rückstand wird an Kieselgel mit Essigester/Hexan chromatographiert, wobei 3,83 g 1-Bromcyclopropancarbonsäure-1,1-dimethyl-2-hydroxy-ethylamid **108** als farbloses Ölerhalten werden.
$^1$H-NMR (300 MHz, $CDCl_3$): δ= 1,32 ppm (m, 2H); 1,33 (s, 6H); 1,68 (m, 2H); 3,61 (d, 2H); 4,25 (t, 1H); 6,87 (brs, 1H)
Man löst 800 mg des Amides **107** in 8 ml Toluol und tropft bei Raumtemperatur unter Stickstoff 3 ml Phosphoroxychlorid hinzu. Nach 30 min wird eingeengt, der Rückstand in Methylenchlorid aufgenommen und mit 10 ml

Natriumcarbonat-Lösung (10%) für 30 min kräftig gerührt. Man trennt die Phasen, trocknet die organische Phase über Natriumsulfat, filtriert und engt ein, wobei 750 mg der Titelverbindung **109** als farbloses Öl anfallen.

$^1$H-NMR (300 MHz, $CDCl_3$): δ= 1,29 ppm (s, 6H); 1,38 (m, 2H); 1,58 (m, 2H); 4,01 (s, 2H)

(5*R*)-2-(1-Bromcyclopropyl)-5-phenyl-2-oxazolin 111

Man löst 739 mg D-(-)-α-Phenylglycinol in 27 ml Methylenchlorid und gibt bei Raumtemperatur 678 mg Natriumcarbonat in 11 ml Wasser sowie 1,09 g des Säurechlorides **107** zu. Es wird über Nacht gerührt und danach mit methanolischer Natronlauge versetzt, die organische Phase abgetrennt, über Natriumsulfat getrocknet und eingeengt. Der Rückstand wird an Kieselgel mit Essigester/Hexan chromatographiert, wobei 1,09 g (1*R*)-1-Bromcyclopropancarbonsäure-2-hydroxy-1-phenylethylamid **110** als farbloses Öl isoliert werden.

$^1$H-NMR (300 MHz, $CDCl_3$): δ= 1,35 ppm (s, 2H); 1,70 (m, 2H); 2,30 (t, 1H); 3,90 (t, 2H); 5,03 (m, 1H); 7,37 (m, 5H); 7,52 (brs, 1H)

Das Amid **110** wird in Analogie zu **107** in die Titelverbindung **111** überführt.

$^1$H-NMR (300 MHz, $CDCl_3$): δ= 1,46 ppm (m, 2H); 1,69 (m, 2H); 4,21 (t, 1H); 4,72 (dd, 1H); 5,23 (dd, 1H); 7,30 (m, 5H)

2-(1-Bromcyclopropyl)-5-methyl-2-oxazolin **113**

Man setzt das 2,18 g des Säurechlorides **107** mit 780 mg DL-2-Amino-1-propanol in Analogie zur Darstellung vom Amid **110** um, wobei man 1,7 g 1-Bromcyclopropancarbonsäure-2-hydroxy-1-methylethylamid **112** als farbloses Öl erhält.

$^1$H-NMR (300 MHz, $CDCl_3$): δ= 1,22 ppm (d, 3H); 1,32 (m, 2H); 1,70 (m, 2H); 2,60 (t, 1H); 3,58 (m, 1H); 3,69 (m, 1H); 4,03 (m, 1H); 6,92 (brs, 1H)

Das Amid **112** wird in Analogie zu **107** in die Titelverbindung **113** überführt.

$^1$H-NMR (300 MHz, $CDCl_3$): δ= 1,28 ppm (d, 3H); 1,40 (m, 2H); 1,59 (m, 2H); 3,87 (t, 2H); 4,22 (m, 1H); 4,44 (dd, 1H)

5(*R*)-2-(1-Bromcyclopropyl)-5-ethyl-2-oxazolin **115**

Man setzt das 2,18 g des Säurechlorides **107** mit 962 mg (-)-2-Amino-1-butanol in Analogie zur Darstellung vom Amid **110** um, wobei man 2,3 g (1*R*)-1-Bromcyclopropancarbonsaure-2-hydroxy-1-ethylethylamid **114** als farbloses Öl erhält.

$^1$H-NMR (300 MHz, $CDCl_3$): δ= 0,98 ppm (t, 3H); 1,32 (m, 2H); 1,70 (m, 2H); 2,60 (t, 1H); 3,58 (m, 1H); 3,69 (m, 1H); 4,03 (m, 1H); 6,92 (brs, 1H)

Das Amid **114** wird in Analogie zu **107** in die Titelverbindung **115** überführt.

$^1$H-NMR (300 MHz, $CDCl_3$): δ= 0,92 ppm (t, 3H); 1,38 (m, 2H); 1,58 (m, 2H); 1,70 (m, 2H); 3,98 (t, 1H); 4,38 (dd, 1H); 6,90 (brs, 1H)

**25-Oxazolin-Reihe**

**Beispiel 24**

(5*Z*,7*E*,22*E*)-(1*S*,3*R*,24*S*)-25-(5,5-Dimethyl-2-oxazolin-2-yl)-26,27-cyclo-9,10-secocholesta-5,7,10(19),22-tetraen-1,3,24-triol **117a** und (5*Z*,7*E*,22*E*)-(1*S*,3*R*,24*R*)-25-(5,5-Dimethyl-2-oxazolin-2-yl)-26,27-cyclo-9,10-secocholesta-5,7,10(19),22-tetraen-1,3,24-triol **117b**

Man legt 2,16 ml *tert*.-Butyllithium unter Stickstoff in 6 ml Diethylether vor und kühlt auf -78°C. Es werden 430 mg des Oxazolins **109** in 1 ml Diethylether zugetropft und 5 min nachgerührt. Anschließend erfolgt die Zugabe von 400 mg des Aldehydes **3** in 1 ml Diethylether. Man läßt innerhalb von 1,5 h auf 0°C kommen und hydrolysiert mit Ammoniumchlorid-Lösung. Nach Extraktion mit Essigester und Trocknen über Natriumsulfat wird eingeengt und der Rückstand an Kieselgel mit Essigester/Hexan chromatographiert, wobei nacheinander 90 mg (5*Z*,7*E*,22*E*)-(1*S*,3*R*,24*S*)-1,3-Bis-[[dimethyl(1,1-dimethylethyl)silyl]oxy]-25-(5,5-dimethyl-2-oxazolin-2-yl)-26,27-cyclo-9,10-secocholesta-5,7,10(19),22-tetraen-24-ol **116a** und 50 mg (5*Z*,7*E*,22*E*)-(1*S*,3*R*,24*R*)-1,3-Bis-[[dimethyl(1,1-dimethylethyl)-silyl]oxy]-25-(5,5-dimethyl-2-oxazolin-2-yl)-26,27-cyclo-9,10-secocholesta-5,7,10(19),22-tetraen-24-ol **116b** als farblose Schäume erhalten werden.

**116a**: $^1$H-NMR (300 MHz, $CDCl_3$): δ= 0,07 ppm (s, 6H); 0,53 (s, 3H); 0,88 (s, 18H); 1,02 (d, 3H); 1,28 (s, 6H); 3,80 (d, 1H); 3,86 (d, 1H); 3,97 (d, 1H); 4,18 (m, 1H); 4,38 (m, 1H); 4,88 (brs, 1H); 4,98 (brs, 1H); 5,19 (brs, 1H); 5,40 (dd, 1H);

5,56 (dd, 1H); 6,01 (d, 1H); 6,23 (d, 1H)
**116b**: $^1$H-NMR (300 MHz, $CDCl_3$): δ= 0,07 ppm (s, 6H); 0,53 (s, 3H); 0,88 (s, 18H); 1,03 (d, 3H); 1,28 (s, 3H); 3,80 (d, 1H); 3,86 (d, 1H); 3,94 (d, 1H); 4,18 (m, 1H); 4,38 (m, 1H); 4,88 (brs, 1H); 4,98 (brs, 1H); 5,19 (brs, 1H); 5,40 (dd, 1H); 5,50 (dd, 1H); 6,01 (d, 1H); 6,23 (d, 1H)

Man löst 90 mg des Disilylethers in 10 ml THF, gibt 156 mg Tetrabutylammoniumfluorid (Trihydrat) hinzu und rührt unter Stickstoff fiir 12 h bei Raumtemperatur. Das Reaktionsgemisch wird in Wasser gegossen, mit Essigester extrahiert, über Natriumsulfat getrocknet und eingeengt. Der Rückstand wird an Kieselgel mit Essigester/Hexan chromatographiert, wobei 36 mg der Titelverbindung **117a** als farbloser Schaum erhalten werden.

$^1$H-NMR (300 MHz, $CDCl_3$): δ= 0,58 ppm (s, 3H); 0,78 (m, 2H); 0,84 (m, 2H); 1,02 (d, 3H); 1,28 (s, 6H); 3,80 (d, 1H); 3,84 (d, 1H); 3,97 (d, 1H); 4,22 (m, 1H); 4,42 (m, 1H); 5,00 (brs, 1H); 5,33 (brs, 1H); 5,39 (dd, 1H); 5,55 (dd, 1H); 6,01 (d, 1H); 6,38 (d, 1H)

Analog wird der Disilylether **116b** in die Titelverbindung **117b** überführt.

$^1$H-NMR (300 MHz, $CDCl_3$): δ= 0,58 ppm (s, 3H), 0,78 (m, 2H); 0,84 (m, 2H); 1,03 (d, 1H); 1,28 (s, 6H); 3,80 (d, 1H); 3,84 (d, 1H); 3,92 (d, 1H); 4,22 (m, 1H); 4,42 (m, 1H); 5,00 (brs, 1H); 5,32 (brs, 1H); 5,40 (dd, 1H); 5,50 (dd, 1H); 6,01 (d, 1H); 6,38 (d, 1H)

**Beispiel 25**

(5*Z*,7*E*,22*E*)-[1*S*,3*R*,24*S*,25(*R*)]-25-(5-Phenyl-2-oxazolin-2-yl)-26,27-cyclo-9,10-secocholesta-5,7,10(19),22-tetraen-1,3,24-triol **119a** und (5*Z*,7*E*,22*E*)-[1*S*,3*R*,24*R*,25(*R*)]-25-(5-Phenyl-2-oxazolin-2-yl)-26,27-cyclo-9,10-secocholesta-5,7,10(19),22-tetraen-1,3,24-triol **119b**

Man legt 1,84 ml *tert*.-Butyllithium unter Stickstoff in 5 ml Diethylether vor und kühlt auf -78°C. Es werden 450 mg des Oxazolins **111** in 1 ml Diethylether zugetropft und 5 min nachgerührt. Anschließend erfolgt die Zugabe von 337 mg des Aldehydes **3** in 1 ml Diethylether. Man läßt innerhalb von 1,5 h auf 0°C kommen und hydrolysiert mit Ammoniumchlorid-Lösung. Nach Extraktion mit Essigester und Trocknen über Natriumsulfat wird eingeengt und der Rückstand an Kieselgel mit Essigester/Hexan chromatographiert, wobei nacheinander 110 mg (5*Z*,7*E*,22*E*)-[1*S*,3*R*,24*S*,25(*R*)]-1,3-Bis-[[dimethyl(1,1-dimethylethyl)silyl]oxy]-25-(5-phenyl-2-oxazolin-2-yl)-26,27-cyclo-9,10-secocholesta-5,7,10(19),22-tetraen-24-ol **118a** und 100 mg (5*Z*,7*E*,22*E*)-[1*S*,3*R*,24*R*,25(*R*))-1,3-Bis-[[dimethyl(1,1-dimethylethyl)-silyl]oxy]-25-(5-phenyl-2-oxazolin-2-yl)-26,27-cyclo-9,10-secocholesta-5,7,10(19),22-tetraen-24-ol **118b** als farblose Schäume erhalten werden.
Man löst 110 mg des Disilylethers **118a** in 15 ml THF, gibt 182 mg Tetrabutylammoniumfluorid (Trihydrat) hinzu und rührt unter Stickstoff für 12 h bei Raumtemperatur. Das Reaktionsgemisch wird in Wasser gegossen, mit Essigester extrahiert, über Natriumsulfat getrocknet und eingeengt. Der Rückstand wird an Kieselgel mit Essigester/Hexan chromatographiert, wobei 36 mg der Titelverbindung **119a** als farbloser Schaum erhalten werden.

$^1$H-NMR (300 MHz, $CDCl_3$): δ= 0,58 ppm (s, 3H); 0,88 (m, 2H); 0,92 (m, 2H); 1,06 (d, 3H); 4,00 (t, 1H); 4,01 (m, 1H); 4,22 (m, 1H); 4,42 (m, 1H); 4,53 (dd, 1H); 4,82 (brs, 1H); 5,00 (brs, 1H); 5,22 (dd, 1H); 5,33 (brs, 1H); 5,50 (dd, 1H); 5,62 (dd, 1H); 6,01 (d, 1H); 6,38 (d, 1H); 7,30 (m, 5H)

Analog wird der Disilylether **118b** in die Titelverbindung **119b** überführt.

$^1$H-NMR (300 MHz, $CDCl_3$): δ= 0,58 ppm (s, 3H); 0,88 (m, 2H); 0,94 (m, 2H); 1,07 (d, 3H); 3,98 (t, 1H); 4,02 (m, 1H); 4,22 (m, 1H); 4,42 (m, 1H); 4,55 (dd, 1H); 4,90 (brs, 1H); 5,00 (brs, 1H); 5,22 (dd, 1H); 5,32 (brs, 1H); 5,50 (m, 2H); 6,01 (d, 1H); 6,38 (d, 1H); 7,30 (m, 5H)

**Beispiel 26**

(5*Z*,7*E*,22*E*)-(1*S*,3*R*,24*S*)-25-(5-Methyl-2-oxazolin-2-yl)-26,27-cyclo-9,10-secocholesta-5,7,10(19),22-tetraen-1,3,24-triol **121a** und (5*Z*,7*E*,22*E*)-(1*S*,3*R*,24*R*)-25-(5-Methyl-2-oxazolin-2-yl)-26,27-cyclo-9,10-secocholesta-5,7,10(19),22-tetraen-1,3,24-triol **121b**

Man legt 1,84 ml *tert*.-Butyllithium unter Stickstoff in 5 ml Diethylether vor und kühlt auf -78°C. Es werden 350 mg des Oxazolins **113** in 1 ml Diethylether zugetropft und 5 min nachgerührt. Anschließend erfolgt die Zugabe von 337 mg des Aldehydes **3** in 1 ml Diethylether. Man läßt innerhalb von 1,5 h auf 0°C kommen und hydrolysiert mit Ammoniumchlorid-Lösung. Nach Extraktion mit Essigester und Trocknen über Natriumsulfat wird eingeengt und der Rückstand an Kieselgel mit Essigester/Hexanb chromatographiert, wobei nacheinander 110 mg (5*Z*,7*E*,22*E*)-(1*S*,3*R*,24*S*)-1,3-Bis-[[dimethyl(1,1-dimethylethyl)silyl]oxy]-25-(5-methyl-2-oxazolin-2-yl)-26,27-cyclo-9,10-secocholesta-5,7,10(19),22-tetraen-24-ol **120a** und 100 mg (5*Z*,7*E*,22*E*)-(1*S*,3*R*,24*R*)-1,3-Bis-[[dimethyl(1,1-dimethylethyl)-silyl]oxy]-25-(5-methyl-2-oxazolin-2-yl)-26,27-cyclo-9,10-secocholesta-5,7,10(19),22-tetraen-24-ol **120b** als farblose Schäume erhalten werden.

Man löst 90 mg des Disilylethers **120a** in 12 ml THF, gibt 156 mg Tetrabutylammoniumfluorid (Trihydrat) hinzu und rührt unter Stickstoff fiir 12 h bei Raumtemperatur. Das Reaktionsgemisch wird in Wasser gegossen, mit Essigester extrahiert, über Natriumsulfat getrocknet und eingeengt. Der Rückstand wird an Kieselgel mit Essigester/Hexan chromatographiert, wobei 41 mg der Titelverbindung **121a** als farbloser Schaum erhalten werden.

$^1$H-NMR (300 MHz, $CDCl_3$); δ= 0,56 ppm (s, 3H); 0,87 (m, 4H); 1,04 (d, 3H); 1,28 (d, 3H); 3,69 (t, 1H); 3,99 (d, 1H); 4,22 (m, 3H); 4,42 (m, 1H); 4,53 (dd, 1H); 5,00 (brs, 1H); 5,32 (brs, 1H); 5,39 (dd, 1H); 5,54 (dd, 1H); 6,01 (d, 1H); 6,38 (d, 1H)

Analog wird der Disilylether **120b** in die Titelverbindung **121b** überführt.

$^1$H-NMR (300 MHz, $CDCl_3$): δ= 0,56 ppm (s, 3H); 0,87 (m, 4H); 1,03 (d, 3H); 1,26 (d, 3H); 3,67 (t, 1H); 3,95 (d, 1H); 4,22 (m, 3H); 4,42 (m, 1H); 4,53 (dd, 1H); 5,00 (brs, 1H); 5,32 (brs, 1H); 5,45 (m, 2H); 6,01 (d, 1H); 6,38 (d, 1H)

**Beispiel 27**

(5*Z*,7*E*,22*E*)-[1*S*,3*R*,24*S*,25(*R*)]-25-(5-Ethyl-2-oxazolin-2-yl)-26,27-cyclo-9,10-secocholesta-5,7,10(19),22-tetraen-1,3,24-triol **123a** und (5*Z*,7*E*,22*E*)-[1*S*,3*R*,24*R*,25(*R*)]-25-(5-Ethyl-2-oxazolin-2-yl)-26,27-cyclo-9,10-secocholesta-5,7,10(19),22-tetraen-1,3,24-triol **123b**

Man legt 2,17 ml *tert*.-Butyllithium unter Stickstoff in 6 ml Diethylether vor und kühlt auf -78°C. Es werden 436 mg des Oxazolins **113** in 1 ml Diethylether zugetropft und 5 min nachgerührt. Anschließend erfolgt die Zugabe von 400 mg des Aldehydes **3** in 1 ml Diethylether. Man läßt innerhalb von 1,5 h auf 0°C kommen und hydrolysiert mit Ammoniumchlorid-Lösung. Nach Extraktion mit Essigester und Trocknen über Natriumsulfat wird eingeengt und der Rückstand an Kieselgel mit Essigester/Hexan chromatographiert, wobei nacheinander 70 mg (5*Z*,7*E*,22*E*)-[1*S*,3*R*,24*S*,25(*R*)]-1,3-Bis-[[dimethyl(1,1-dimethylethyl)silyl]oxy)-25-(5-ethyl-2-oxazolin-2-yl)-26,27-cyclo-9,10-secocholesta-5,7,10(19),22-tetraen-24-ol **122a** und 40 mg (5*Z*,7*E*,22*E*)-[1*S*,3*R*,24*R*,25(*R*)]-1,3-Bis-[[dimethyl(1,1-dimethylethyl)-silyl]oxy]-25-(5-ethyl-2-oxazolin-2-yl)-26,27-cyclo-9,10-secocholesta-5,7,10(19),22-tetraen-24-ol **122b** als farblose Schäume erhalten werden.

Man löst 70 mg des Disilylethers **122a** in 10 ml THF, gibt 122 mg Tetrabutylammoniumfluorid (Trihydrat) hinzu und rührt unter Stickstoff für 12 h bei Raumtemperatur. Das Reaktionsgemisch wird in Wasser gegossen, mit Essigester extrahiert, über Natriumsulfat getrocknet und eingeengt. Der Rückstand wird an Kieselgel mit Essigester/Hexan chromatographiert, wobei 36 mg der Titelverbindung **123a** als farbloser Schaum erhalten werden.

$^1$H-NMR (300 MHz, $CDCl_3$): δ= 0,57 ppm (s, 3H), 0,79 (m, 2H); 0,85 (m, 2H); 0,96 (t, 3H); 1,03 (d, 3H); 3,78 (t, 1H); 3,99 (d, 1H); 4,13 (m, 2H); 4,22 (m, 1H); 4,42 (m, 1H); 5,00 (brs, 1H); 5,32 (brs, 1H); 5,39 (dd, 1H); 5,56 (dd, 1H); 6,01 (d, 1H); 6,39 (d, 1H)

Analog wird der Disilylether **122b** in die Titelverbindung **123b** überführt.

$^1$H-NMR (300 MHz, $CDCl_3$): δ= 0,56 ppm (s, 3H); 0,79 (m, 2H); 0,85 (m, 2H); 0,94 (t, 3H); 1,02 (d, 3H); 3,78 (t, 1H); 3,93 (d, 1H); 4,13 (m, 2H); 4,22 (m, 1H); 4,42 (m, 1H); 5,00 (brs, 1H); 5,32 (brs, 1H); 5,45 (m, 2H); 6,01 (d, 1H); 6,39 (d, 1H)

**20-Methyl-Reihe**

**Beispiel 28**

(5*Z*,7*E*,22*E*)-(1*S*,3*R*,24*S*)-20-Methyl-25-(4-methylthiazol-2-yl)-26,27-cyclo-9,10-secocholesta-5,7,10(19),22-tetraen-1,3,24-triol **126a** und (5*Z*,7*E*,22*E*)-(1*S*,3*R*,24*R*)-20-Methyl-25-(4-methylthiazol-2-yl)-26,27-cyclo-9,10-secocholesta-5,7,10(19),22-tetraen-1,3,24-triol **126b**

Man behandelt 640 mg (5*E*,7*E*,22*E*)-(1*S*,3*R*)-1,3-Bis[[dimethyl(1,1-dimethylethyl)silyl]oxy]-20-methyl-9,10-secochola-5,7,10(19),22-tetraen-24-al **124** (WO 94/07853) analog dem Aldehyd **29** und erhält 620 mg (5*Z*,7*E*,22*E*)-(1*S*,3*R*)-1,3-Bis[[dimethyl(1,1-dimethylethyl)silyl]oxy]-20-methyl-9,10-secochola-5,7,10(19),22-tetraen-24-al **125** als farblosen Schaum.

$^1$H-NMR (300 MHz, $CDCl_3$): δ= 0,05 ppm (s, 12H); 0,52 (s, 3H); 0,87 (s, 3H); 1,10 (s, 3H); 1,17 (s, 3H); 4,18 (m, 1H); 4,38 (m, 1H); 4,82 (brs, 1H); 5,18 (brs, 1H); 5,99 (d, 1H); 6,02 (dd, 1H); 6,20 (d, 1H); 7,00 (d, 1H); 9,52 (d, 1H)

Der Aldehyd **125** wird analog Beispiel 5 mit 2-(1-Bromcyclopropyl)-4-methylthiazol **18** in die Titelverbindung **126a** und **126b** überführt, die nach chromatographischer Trennung als farblose Schäume anfallen.

**126a:** $^1$H-NMR (300 MHz, $CDCl_3$): δ= 0,50 ppm (s, 3H); 0,97 (s, 3H); 1,04 (s, 3H), 2,39 (s, 1H), 3,98 (d, 1H); 4,22 (m, 1H); 4,43 (m, 1H); 5,00 (brs, 1H); 5,32 (dd, 1H); 5,33 (brs, 1H); 5,86 (d, 1H); 5,98 (d, 1H); 6,48 (d, 1H); 6,64 (s, 1H)

**126b**: $^1$H-NMR (300 MHz, $CDCl_3$): δ= 0,49 ppm (s, 3H); 0,96 (s, 3H); 1,05 (s, 3H); 2,39 (s, 1H); 3,98 (d, 1H);

4,22 (m, 1H); 4,43 (m, 1H); 5,00 (brs, 1H); 5,31 (dd, 1H); 5,33 (brs, 1H); 5,86 (d, 1H); 5,98 (d, 1H); 6,48 (d, 1H); 6,64 (s, 1H)

**20-Epi-Reihe**

**Beispiel 29**

(5*Z*,7*E*,22*E*)-(1*S*,3*R*,20*S*,24*S*)-25-(5-Butyloxazol-2-yl)-26,27-cyclo-9,10-secocholesta-5,7,10(19),22-tetraen-1,3,24-triol **131a** und (5*Z*,7*E*,22*E*)-(1*S*,3*R*,20*S*,24*R*)-25-(5-Butyloxazol-2-yl)-26,27-cyclo-9,10-secocholesta-5,7,10(19),22-tetraen-1,3,24-triol **131b**

Man behandelt 2,1 g (5*E*,7*E*)-(1*S*,3*R*,20*R*)-1,3-Bis[[dimethyl(1,1-dimethylethyl)silyl]oxy]-9,10-secopregna-5,7,10(19)-trien-20-carbaldehyd **127** [M.J. Calverley, L. Binderup *Bioorg. Med. Chem. Lett*. **3**, 1845 (1993)] analog dem Aldehyd **29** und erhält 2,0 g (5*Z*,7*E*)-(1*S*,3*R*,20*R*)-1,3-Bis[[dimethyl(1,1-dimethylethyl)silyl]oxy]-9,10-secopregna-5,7,10(19)-trien-20-carbaldehyd **128** als farblosen Schaum.

$^1$H-NMR (300 MHz, $CDCl_3$): δ= 0,05 ppm (s, 12H); 0,56 (s, 3H); 0,87 (s, 3H); 1,17 (d, 3H); 4,18 (m, 1H); 4,38 (m, 1H); 4,83 (brs, 1H); 5,18 (brs, 1H); 6,00 (d, 1H); 6,22 (d, 1H); 9,54 (d, 1H)

Man legt 1,9 g des Aldehydes **128** in 28 ml Toluol vor, gibt unter Stickstoff 2,6 g N-Methoxy-N-methyl-2-(triphenylphosphoranyliden)acetamid [D. A. Evans et al. *J. Am. Chem. Soc.* **112,** 7001 (1990)] und erhitzt 3 d auf 80°C. Nach dem Abkühlen wird in Wasser gegossen, mit Essigester extrahiert, über Natriumsulfat getrocknet und eingeengt. Der Rückstand wird an Kieselgel mit Essigester/Hexan chromatographiert, wobei 1,6 g (5*Z*,7*E*,22*E*)-(1*S*,3*R*,20*R*)-1,3-Bis[[dimethyl(1,1-dimethylethyl)silyl]oxy]-N-methyl-N-methoxy-9,10-secochola-5,7,10(19),22-tetraen-24-amid **129** als farbloser Schaum anfallen. 1,4 g des Amides **129** werden in 25 ml THF gelöst und unter Stickstoff auf -78°C gekühlt. Man tropft 10 ml Diisobutylaluminium-Lösung (1 M in Hexan) hinzu und rührt 1 h nach. Nun tropft man 0,8 ml Methanol zu und läßt die Mischung auf Raumtemperatur kommen. Das Reaktionsgemisch wird nun in Kalium-Natriumtartrat-Lösung gegossen, mit Essigester extrahiert, die organische Phase über Natriumsulfat getrocknet und eingeengt. Der Rückstand wird an Kieselgel mit Essigester/Hexan chromatographiert, wobei 1,1 g (5*Z*,7*E*,22*E*)-(1*S*,3*R*,20*R*)-1,3-Bis[[dimethyl(1,1-dimethylethyl)silyl]oxy]-9,10-secochola-5,7,10(19),22-tetraen-24-al **130** als farbloser Schaum erhalten werden.

$^1$H-NMR (300 MHz, $CDCl_3$): δ= 0,05 ppm (s, 12H); 0,53 (s, 3H); 0,87 (s, 3H); 1,10 (d, 3H); 4,20 (m, 1H); 4,39 (m, 1H), 4,85 (brs, 1H); 5,20 (brs, 1H); 6,00 (d, 1H); 6,10 (dd, 1H); 6,22 (d, 1H); 6,79 (dd, 1H); 9,53 (d, 1H)

Der Aldehyd **130** wird analog Beispiel 16 mit 2-(1-Bromcyclopropyl)-5-butyloxazol **87** in die Titelverbindung **131a** und **131b** überführt, die nach chromatographischer Trennung als farblose Schäume anfallen.

**131a:** $^1$H-NMR (300 MHZ, $CDCl_3$): δ= 0,56 ppm (s, 3H); 0,90 (t, 3H); 0,96 (m, 2H); 1,11 (d, 3H); 1,15 (m, 2H); 2,57 (s, 3H); 4,10 (d, 1H); 4,22 (m, 1H); 4,43 (m, 1H); 5,00 (brs, 1H); 5,33 (brs, 1H); 5,41 (dd, 1H), 5,58 (dd, 1H); 6,00 (d, 1H); 6,37 (d, 1H); 6,57 (s, 1H)

**131a:** $^1$H-NMR (300 MHz, $CDCl_3$): δ= 0,57 ppm (s, 3H); 0,90 (t, 3H); 0,97 (m, 2H); 1,10 (d, 3H); 1,15 (m, 2H); 2,58 (s, 3H); 4,10 (d, 1H); 4,22 (m, 1H); 4,43 (m, 1H); 5,00 (brs, 1H); 5,33 (brs, 1H); 5,42 (dd, 1H). 5,53 (dd, 1H); 6,00 (d, 1H); 6,38 (d, 1H); 6,58 (s, 1H)

## Ausgangsmaterialien in der 5-Alkyloxazol-Reihe

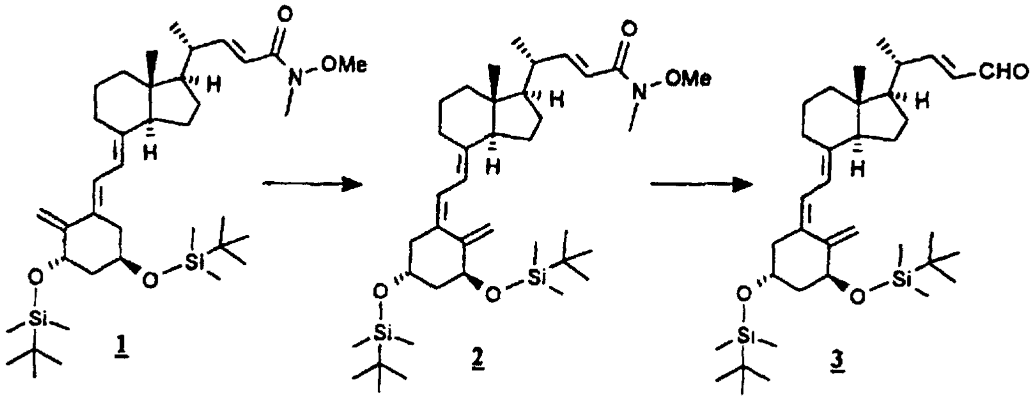

1 2 3

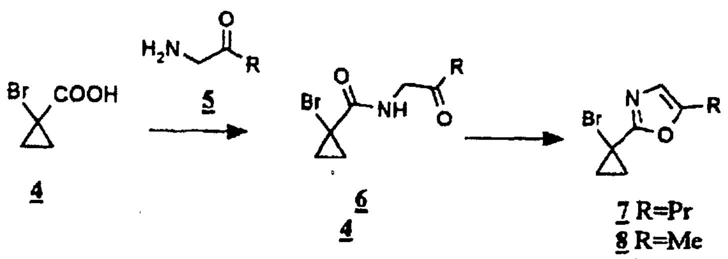

5

4 6 4

7 R=Pr
8 R=Me
9 R=Et
10 R=Pent

## 5-Alkyloxazol-Derivate

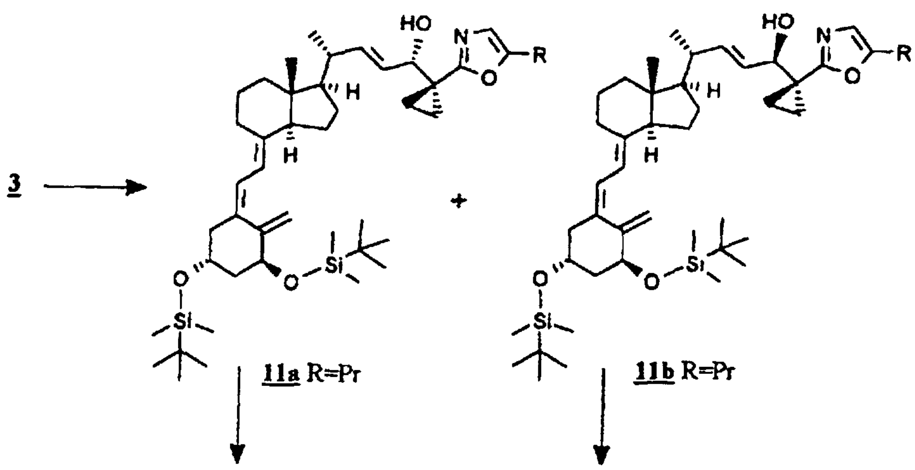

3 +

11a R=Pr

11b R=Pr

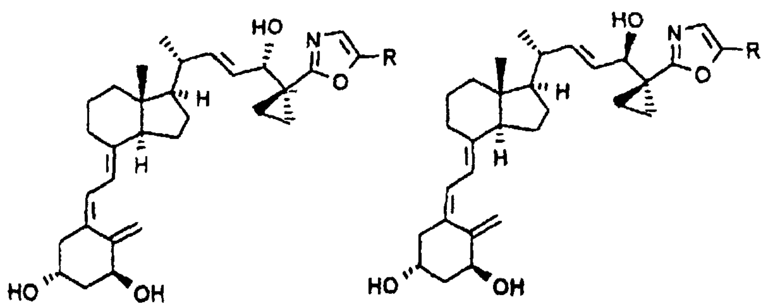

**12a** R=Pr **12b** R=Pr

3

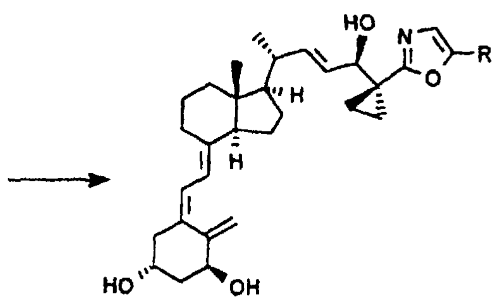

**13b** R=Me
**14b** R=Et
**15b** R=Pent

## Ausgangsmaterialien in der 4-Alkylthiazol-Reihe

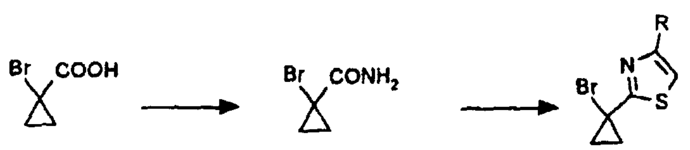

4 16 4

**17** R=Et
**18** R=Me
**19** R=Pr
**20** R=Bu

## 4-Alkylthiazol-Derivate

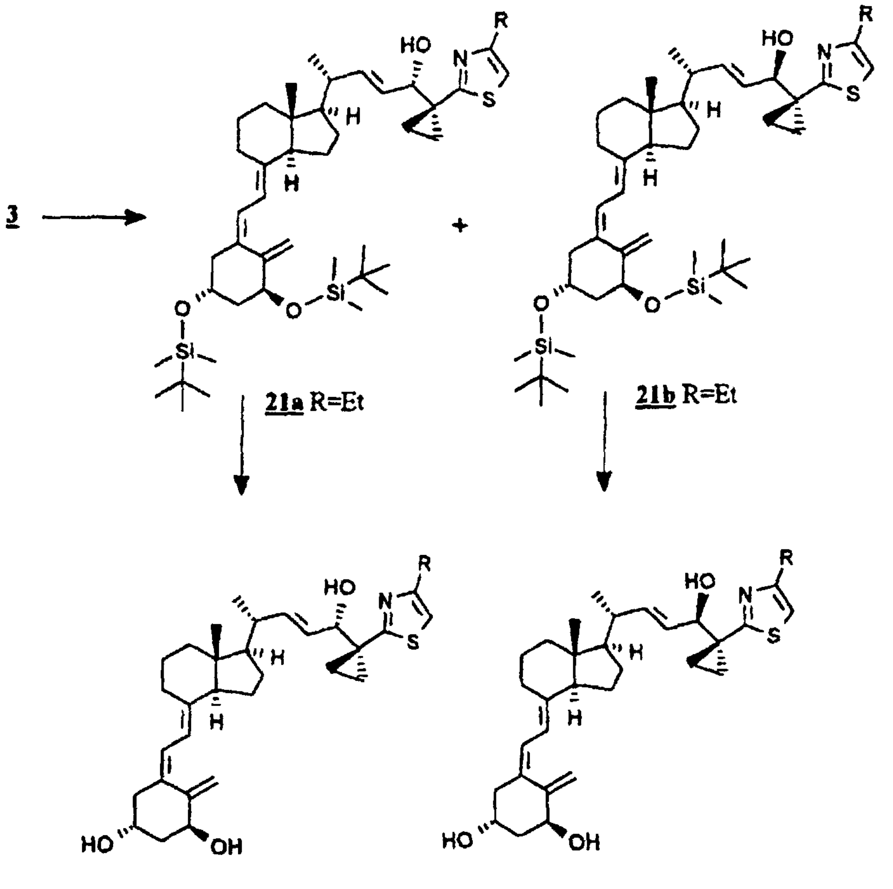

3

21a R=Et

21b R=Et

22a R=Et

22b R=Et

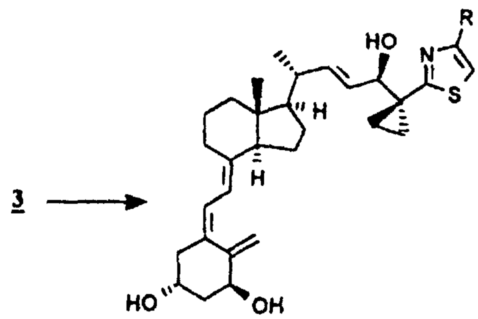

3

23b R=Me
24b R=Pr
25b R=Bu

## Ausgangmaterialien in der Phenyl-Reihe

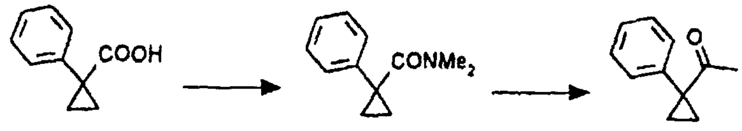

26 27 4 28

## Phenyl-Derivate

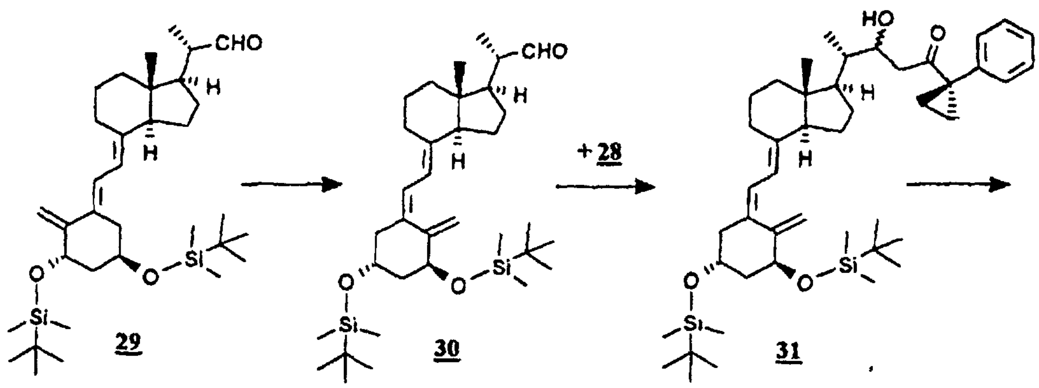


29 30 31

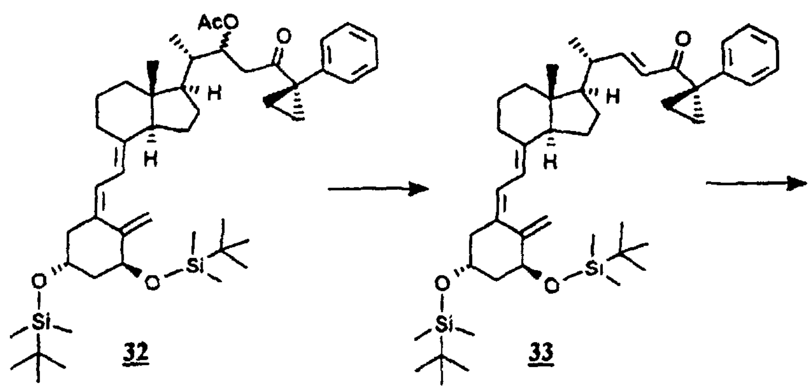

32 33

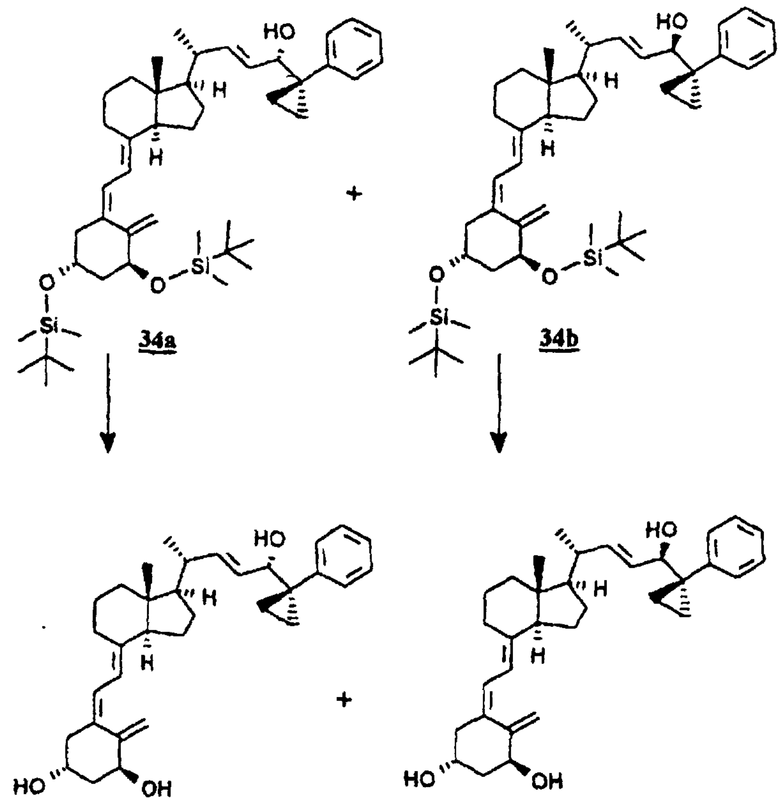

34a + 34b

35a + 35b

## Ausgangsmaterialien in der 4-Alkylphenyl-Reihe

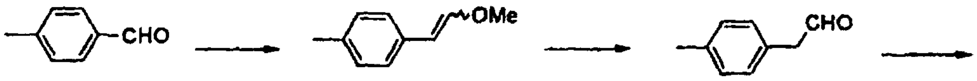

36 37

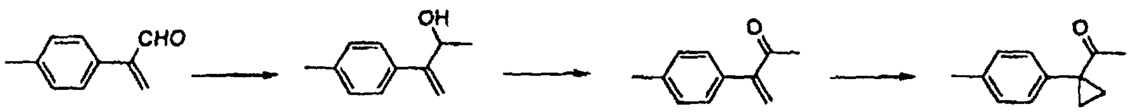

38 39 40 41

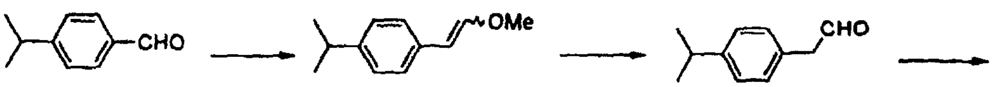

42 43

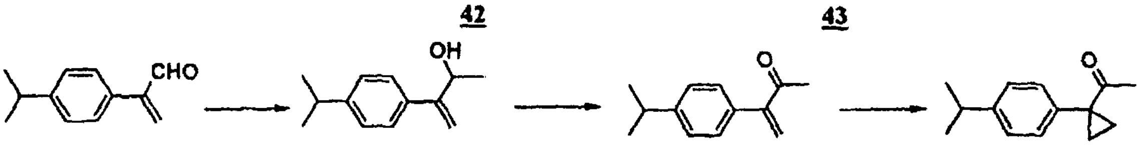

44 45 46 47

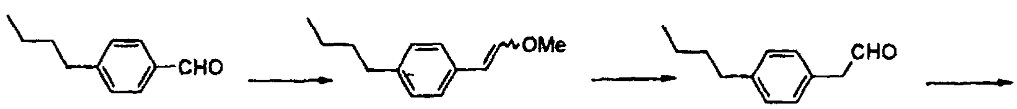

48 49

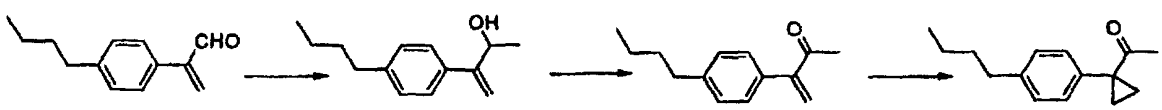

50 51 52 53

## 4-Alkylphenyl-Derivate

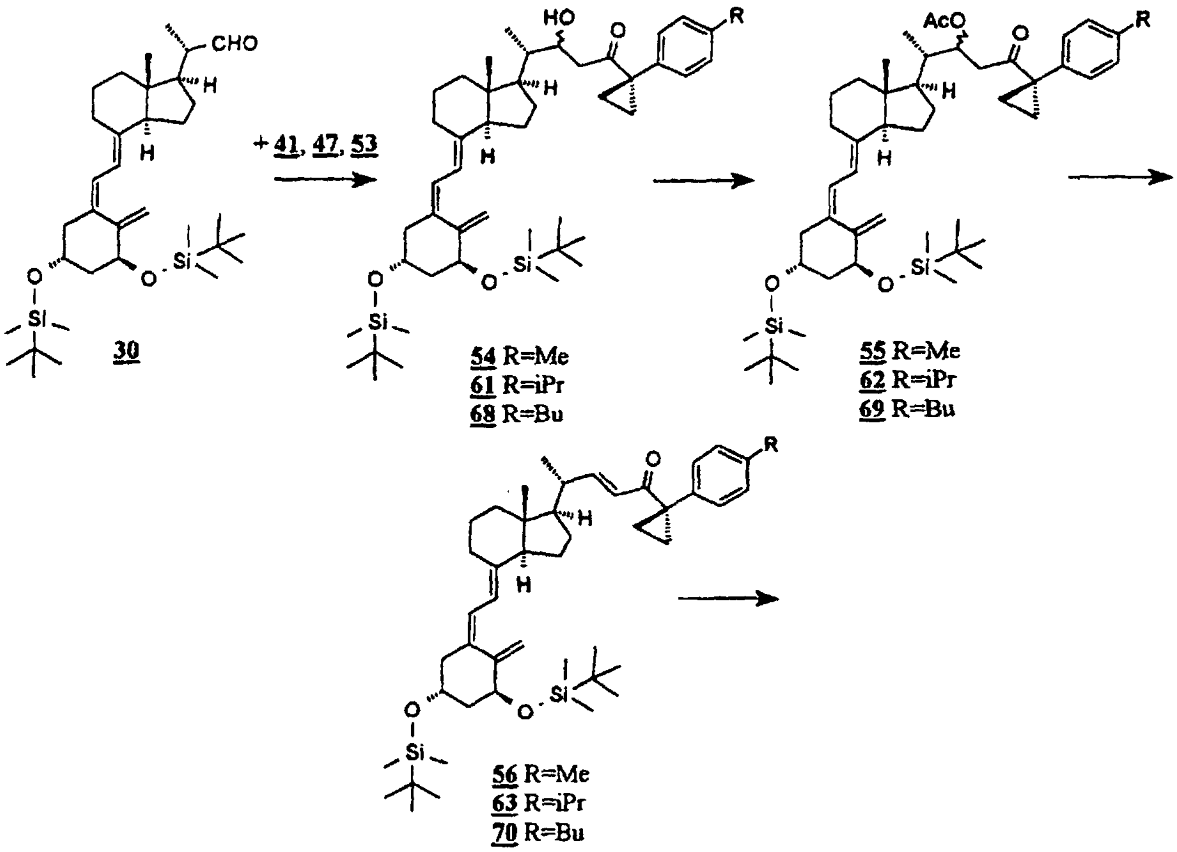


30

54 R=Me
61 R=iPr
68 R=Bu

55 R=Me
62 R=iPr
69 R=Bu

56 R=Me
63 R=iPr
70 R=Bu

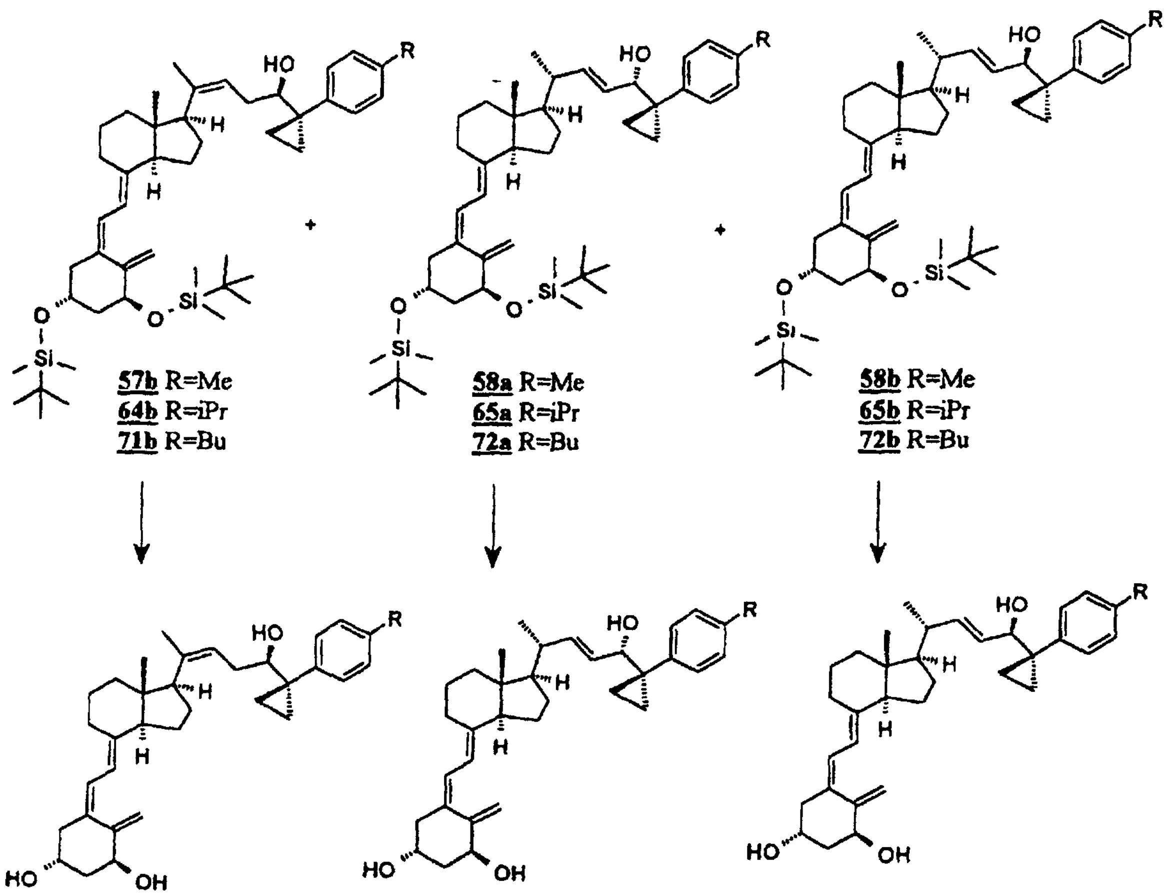

57b R=Me
64b R=iPr
71b R=Bu

58a R=Me
65a R=iPr
72a R=Bu

58b R=Me
65b R=iPr
72b R=Bu

59b R=Me
66b R=iPr
73b R=Bu

60a R=Me
67a R=iPr
74a R=Bu

60b R=Me
67b R=iPr
74b R=Bu

## Ausgangsmaterialien in der 4-Alkyloxazol-Reihe

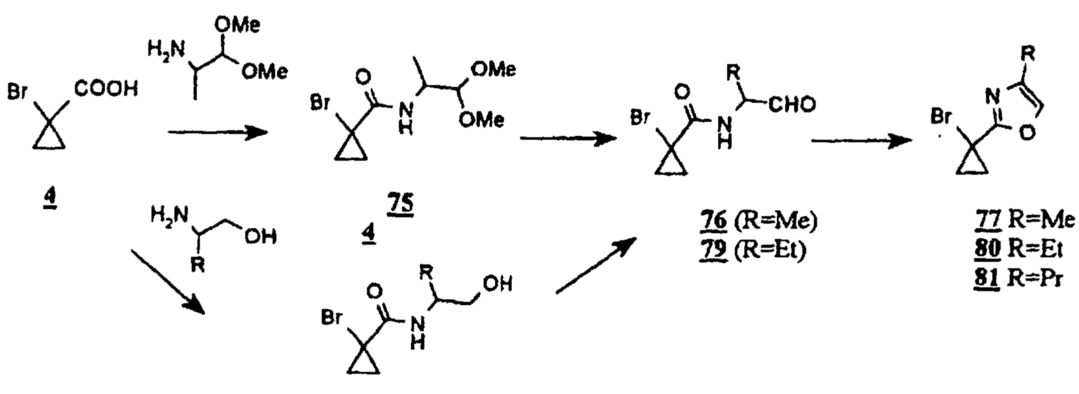

4

75

4

76 (R=Me)
79 (R=Et)

77 R=Me
80 R=Et
81 R=Pr

R=Et (78), Pr

## 4-Alkyloxazol-Reihe

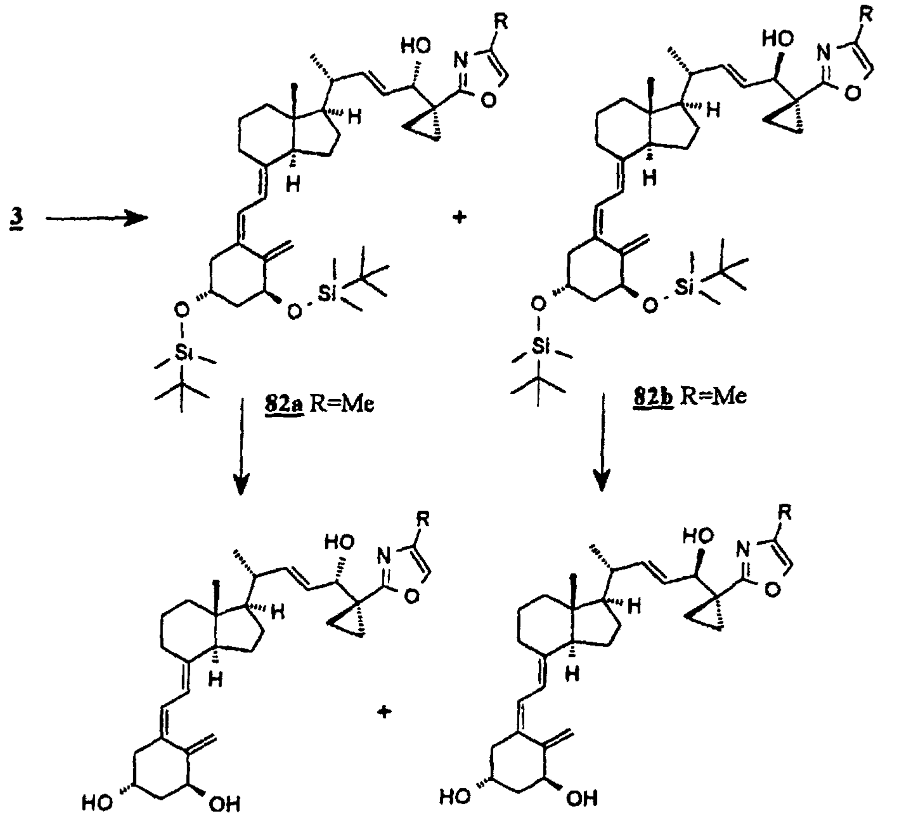

3

82a R=Me

82b R=Me

83a R=Me
84a R=Et
85a R=Pr

83b R=Me
84b R=Et
85b R=Pr

## 5-Butyloxazol-Derivat

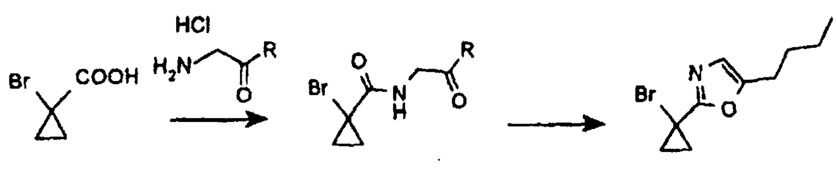

4

86 R=Bu
4

87

3

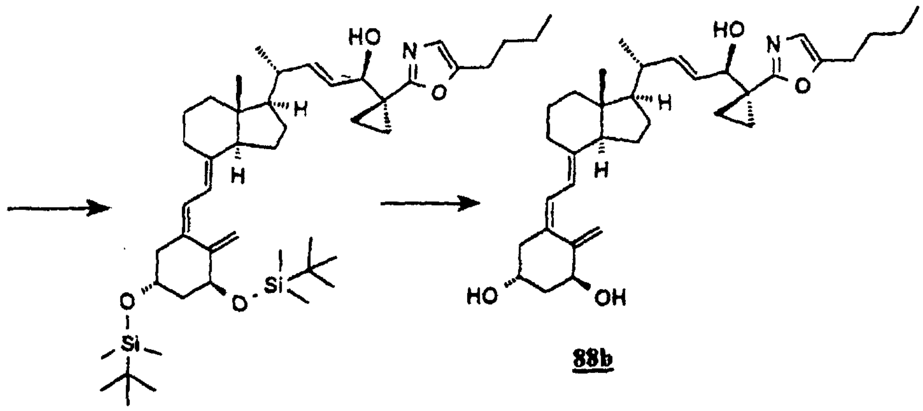

88b

## Ausgangsmaterialien für die 5-Alkylthiazol-Reihe

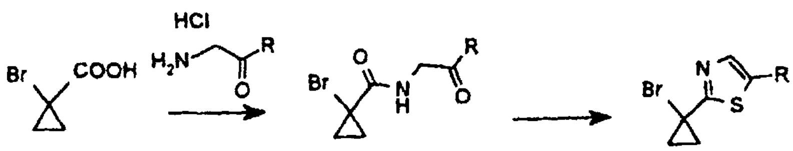


4

86 R=Bu
92 R=Et

89 R=Bu
90 R=Et

## 5-Alkylthiazol-Reihe

3

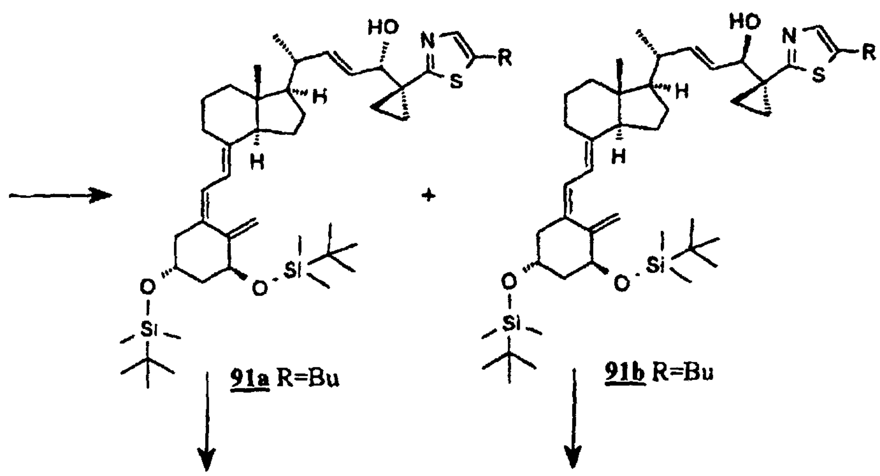

+

91a R=Bu

91b R=Bu

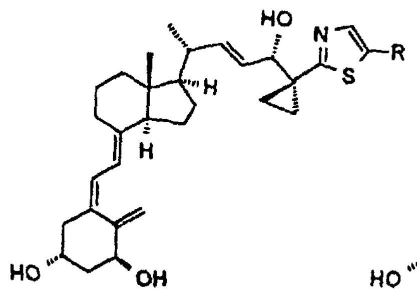

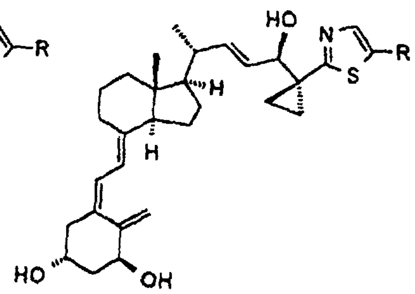

92a R=Bu
93a R=Et

92b R=Bu
93b R=Et

## 1,2,4-Oxadiazol-Reihe

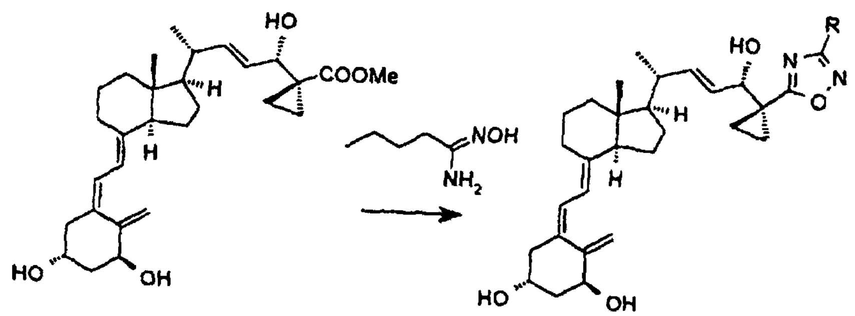

94a

95a R=Bu

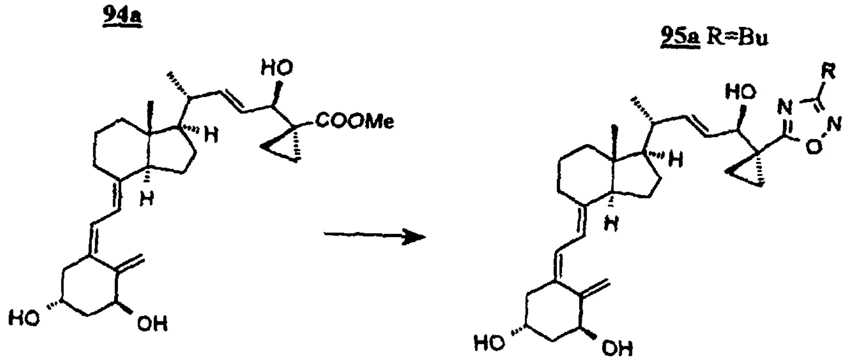

94b

95b R=Bu
96b R=Et

## Ausgangsmaterialien für die 25-Pyridyl-Reihe

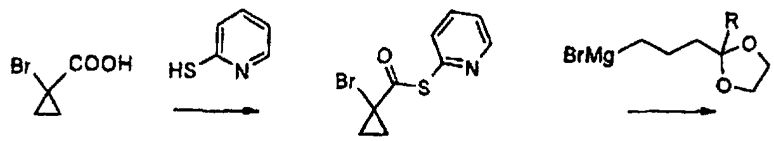

4 97 98 R=H 101 R=Me

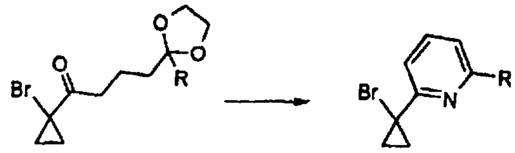

99 R=H 102 R=Me

100 R=H 103 R=Me

## 25-Pyridyl-Reihe

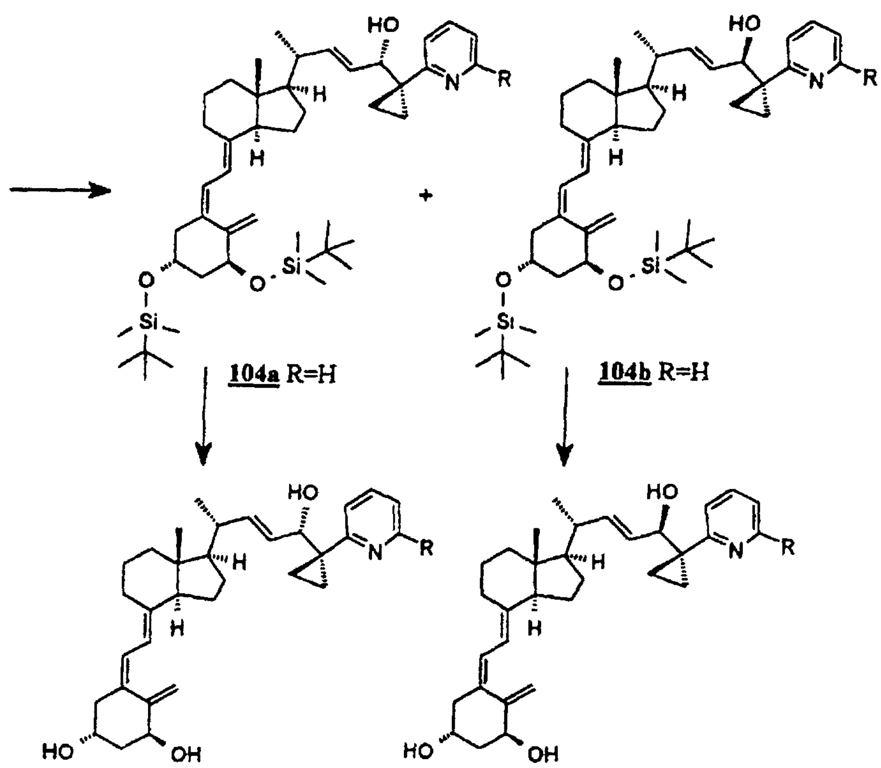

3

104a R=H

104b R=H

105a R=H

105b R=H
106b R=Me

## Ausgangsmaterialien in der 25-Oxazolin-Reihe

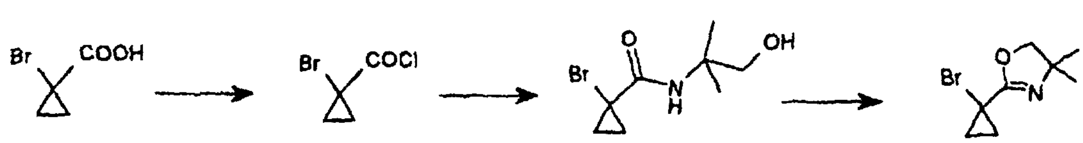

4 107 108 109

4

107 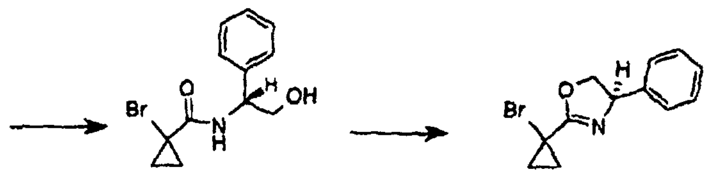

110 111

4

107 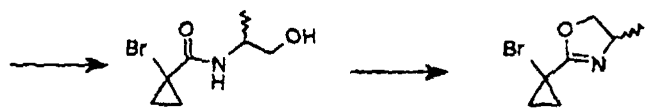

112 113

4

107 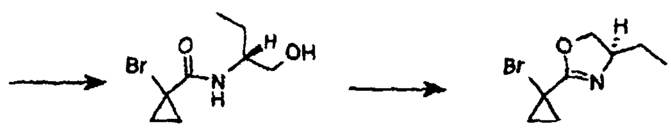

114 115

## 25-Oxazolin-Reihe

3 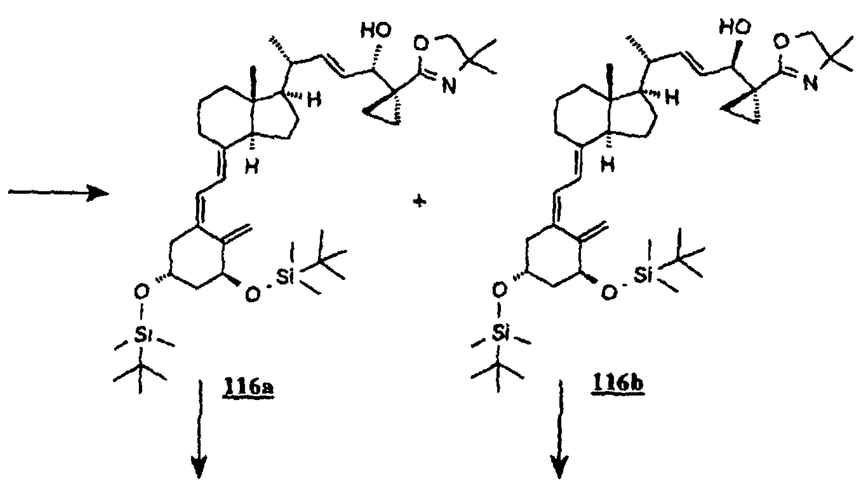

116a + 116b

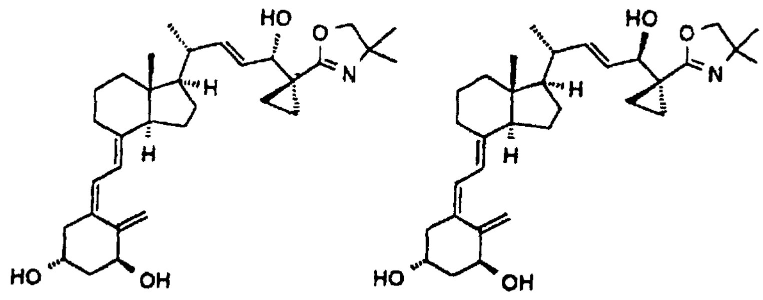

117a 117b

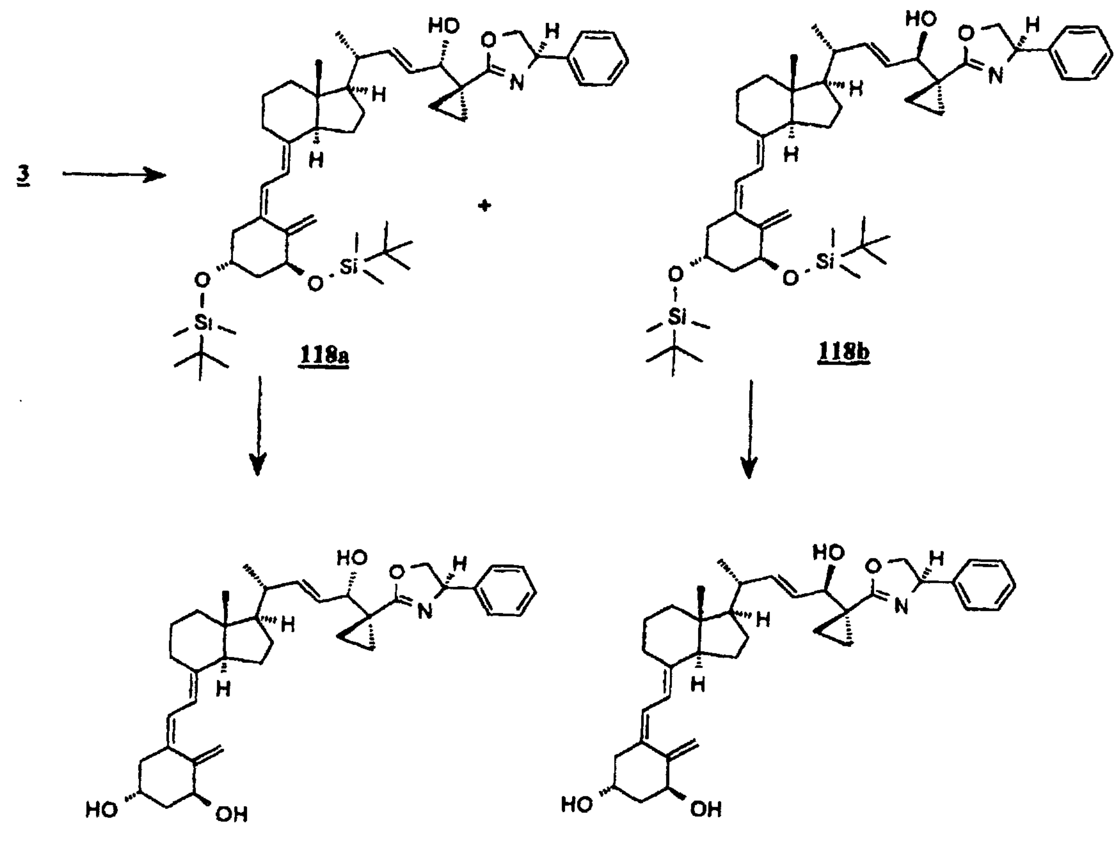

3

+

118a 118b

119a 119b

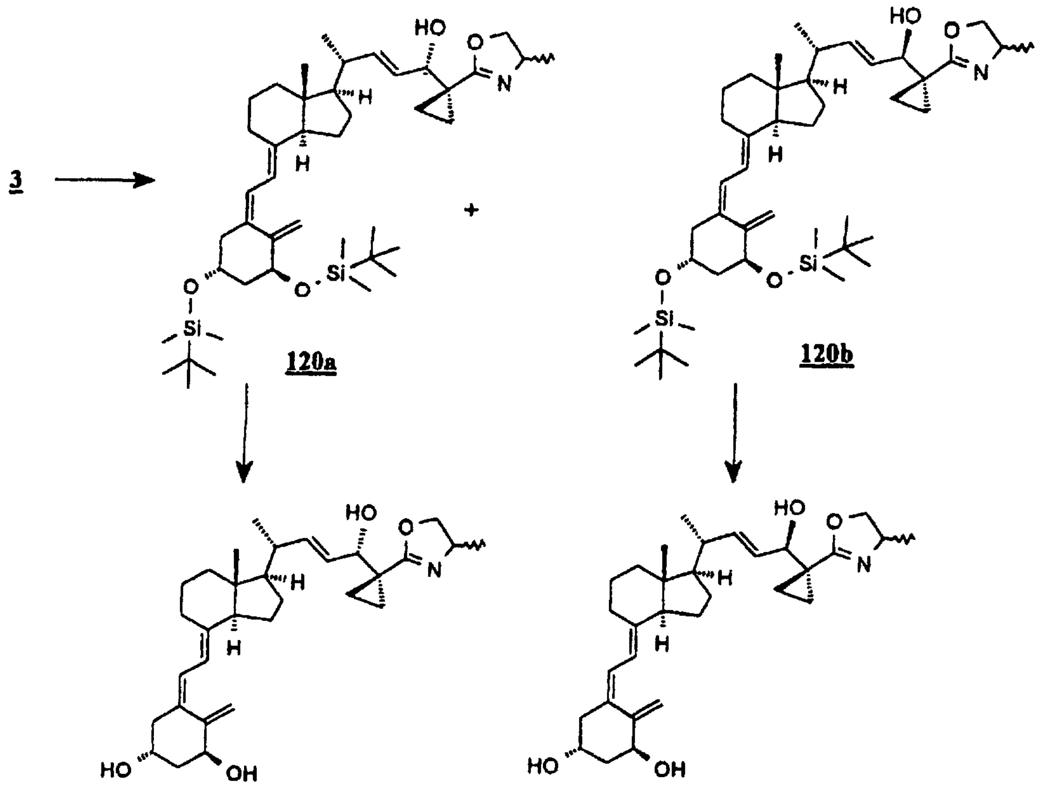

3

120a

120b

121a

121b

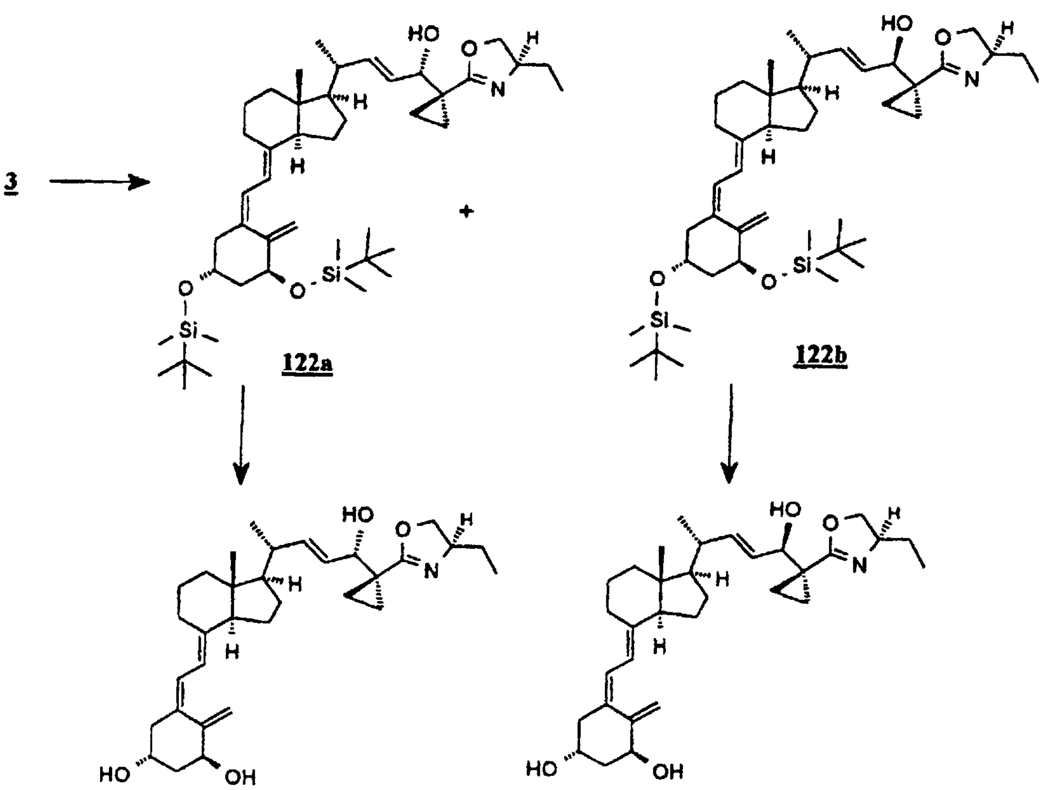

3

122a

122b

123a

123b

## 20-Methyl-Reihe

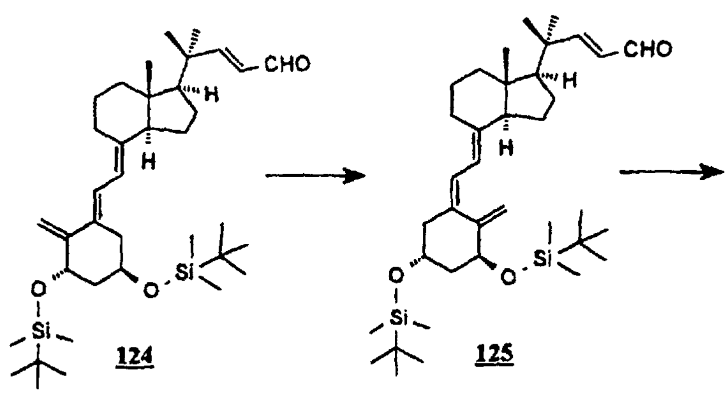

124 125

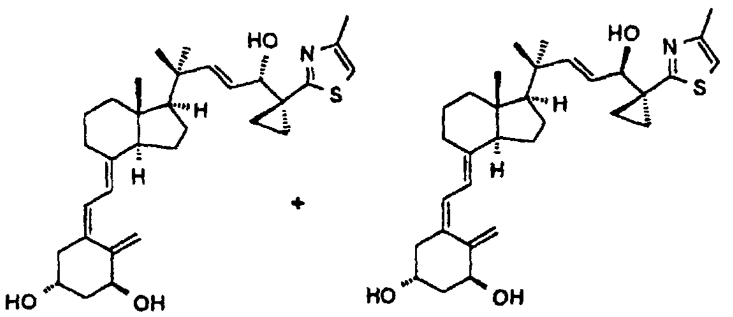

+

126a 126b

## 20-Epi-Reihe

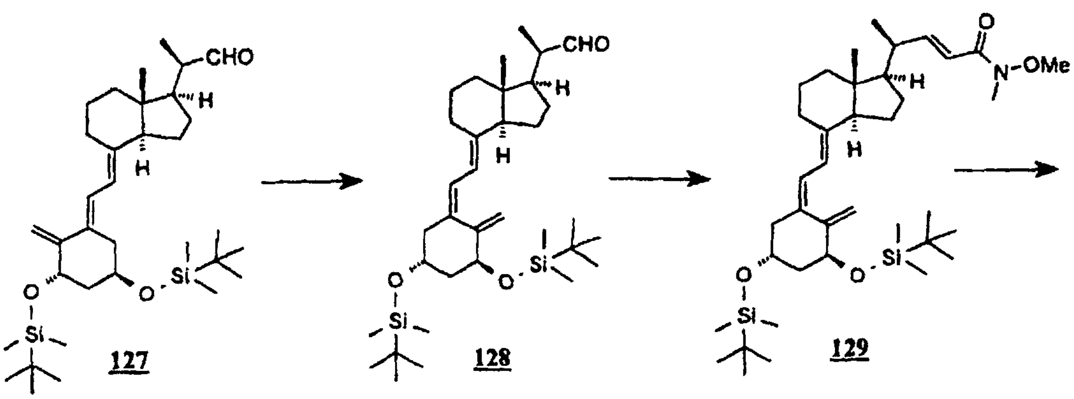

127 128 129

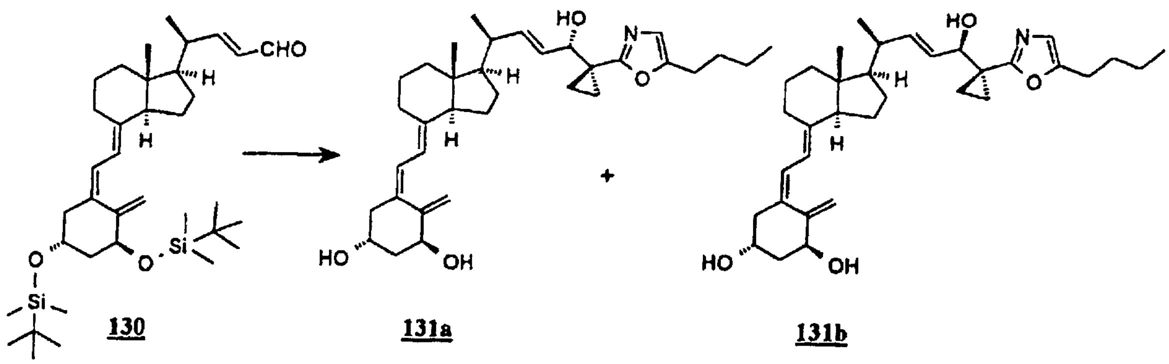

130 131a 131b

## Patentansprüche

1. Vitamin D-Derivate der allgemeinen Formel I

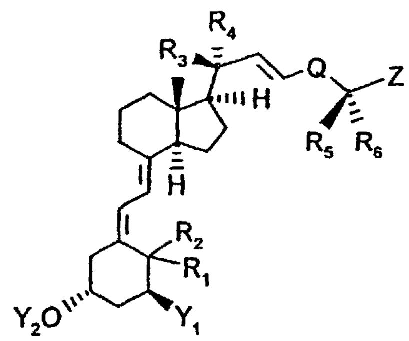

I

worin

$Y_1$ ein Wasserstoffatom, eine Hydroxylgruppe, eine Alkanoyloxygruppe mit 1 bis 12 C-Atomen oder eine Aroyloxygruppe,

$Y_2$ ein Wasserstoffatom oder eine Alkanoylgruppe mit 1 bis 12 C-Atomen oder eine Aroylgruppe,

$R_1$ und $R_2$ je ein Wasserstoffatom oder gemeinsam eine exocyclische Methylengruppe,

$R_3$ und $R_4$ unabhängig voneinander ein Wasserstoffatom, ein Chlor- oder Fluoratom, eine Alkylgruppe mit 1 bis 4 Kohlenstoffatomen, gemeinsam eine Methylengruppe oder gemeinsam mit dem quartären Kohlenstoffatom 20 einen 3-7-gliedrigen, gesättigten oder ungesättigten carbocyclischen Ring,

Q eine geradkettige oder verzweigte Kohlenstoffeinheit mit bis zu 10 Kohlenstoffatomen, die an beliebigen Positionen Hydroxylgruppen (α- oder β-ständig), die ihrerseits verethert oder verestert sein können, Ketogruppen, Aminogruppen oder Halogenatome aufweisen kann,

$R_5$ und $R_6$ gemeinsam mit dem Kohlenstoffatom 25 einen 3-7-gliedrigen, gesättigten oder ungesättigten carbocyclischen Ring und

Z einen fünf- oder sechsgliedrigen carbo- oder heterocyclischen Ring, der gesättigt, ungesättigt oder aromatisch sein kann und an beliebigen Positionen eine oder mehrere Alkylketten, die geradkettig oder verzweigt, gesättigt oder ungesättigt sein können und an beliebigen Stellen durch Oxa-, Thia- oder Azafunktionen (substituiert oder unsubstituiert) oder Sulfoxid oder Sulfongruppen unterbrochen sein können oder Substituenten (Hydroxygruppen, Halogenatome) tragen können,

bedeuten.

**2.** Vitamin D-Derivate der allgemeinen Formel I nach Anspruch 1, worin $R_5$ und $R_6$ gemeinsam mit dem Kohlenstoffatom C-25 einen Cyclopropylring und Z einen aromatischen oder heteroaromatischen Ring bedeuten.

**3.** Vitamin D-Derivate nach Anspruch 2, worin Z die Bedeutung eines gegebenenfalls substituierten Phenyl- oder Oxazol- oder Thiazolringes hat.

**4.** Vitamin D-Derivate der allgemeinen Formel I nach Anspruch 1, worin Q die Bedeutung einer Hydroxymethyl- oder Carbonylmethylgruppe hat.

**5.** Vitamin D-Derivate der allgemeinen Formel I nach Anspruch 1,
(5*Z*,7*E*,22*E*)-(1*S*,3*R*,24*R*)-25-(5-Propyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Propyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Methyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Methyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Ethyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Ethyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Butyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Butyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Pentyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Pentyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Propylthiazol-2-yl)-26,27-cyclo-9,10-secocholesta-5,7,10(19),22-tetraen-1,3,24-triol
(5*Z*,7*E*,2*E*)-(1*S*,3*R*,24*S*)-25-(5-Propylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Methylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Methylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Ethylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Ethylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Butylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Butylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Pentylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Pentylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Propylimidazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Propylimidazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Methylimidazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Methylimidazol-2-yl)-26,27-cyclo-9,10-secocholesta-5,7,10(19),22-tetraen-1,3,24-triol

(5*Z*,7*E*,22*E*)-(1*S*,3*R*,24*R*)-25-(5-Ethylimidazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Ethylimidazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Butylimidazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Butylimidazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Pentylimidazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Pentylimidazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Propylfuran-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Propylfuran-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Methylfuran-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Methylfuran-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Ethylfuran-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Ethylfuran-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Butylfuran-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Butylfuran-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Pentylfuran-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Pentylfuran-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Propylthiophen-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Propylthiophen-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Methylthiophen-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Methylthiophen-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Ethylthiophen-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Ethylthiophen-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Butylthiophen-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Butylthiophen-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Pentylthiophen-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Pentylthiophen-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Propylpyrrol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Propylpyrrol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Methylpyrrol-2-yl)-26,27-cyclo-9,10-secocholesta-5,7,10(19),22-tetraen-1,3,24-triol

(5*Z*,7*E*,22*E*)-(1*S*,3*R*,24*S*)-25-(5-Methylpyrrol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Ethylpyrrol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Ethylpyrrol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Butylpyrrol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Butylpyrrol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Pentylpyrrol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Pentylpyrrol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Propyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Propyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Methyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Methyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Ethyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Ethyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Butyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Butyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Pentyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Pentyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Propylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Propylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Methylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Methylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Ethylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Ethylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Butylthiazol-2-yl)-26,27-cyclo-9,10-secocholesta-5,7;10(19),22-tetraen-1,3,24-triol
(5*Z*,7*E*,22*E*)-(1*S*,3*R*,24*S*)-25-(4-Butylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Pentylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Pentylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Propylimidazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Propylimidazol-2-yl)-26,27-cyclo-9,10-secocholesta-5,7,10(19),22-tetraen-1,3,24-triol

(5*Z*,7*E*,22*E*)-(1*S*,3*R*,24*R*)-25-(4-Methylimidazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Methylimidazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Ethylimidazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Ethylimidazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Butylimidazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Butylimidazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Pentylimidazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Pentylimidazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Propylfuran-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Propylfuran-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Methylfuran-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Methylfuran-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Ethylfuran-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Ethylfuran-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Butylfuran-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Butylfuran-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Pentylfuran-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Pentylfuran-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Propylthiophen-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Propylthiophen-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Methylthiophen-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Methylthiophen-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Ethylthiophen-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Ethylthiophen-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Butylthiophen-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Butylthiophen-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Pentylthiophen-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Pentylthiophen-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Propylpyrrol-2-yl)-26,27-cyclo-9,10-secocholesta-5,7,10(19),22-tetraen-1,3,24-triol

(5*Z*,7*E*,22*E*)-(1*S*,3*R*,24*S*)-25-(4-Propylpyrrol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Methylpyrrol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Methylpyrrol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Ethylpyrrol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Ethylpyrrol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Butylpyrrol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Butylpyrrol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Pentylpyrrol-2-yl)-26,27-cyclo-9,10-secocholesta-5,7,10(19),22-tetraen-1,3,24-triol
(5*Z*,7*E*,22*E*)-(1*S*,3*R*,24*S*)-25-(4-Pentylpyrrol-2-yl)-26,27-cyclo-9,10-secocholesta-5,7,10(19),22-tetraen-1,3,24-triol
(5*Z*,7*E*,22*E*)-(1*S*,3*R*,24*R*)-25-(3-Methyl-1,2,4-oxadiazol-5-yl)-26,27-cyclo-9,10-secocholesta-5,7,10(19),22-tetraen-1,3,24-triol
(5*Z*,7*E*,22*E*)-(1*S*,3*R*,24*S*)-25-(3-Methyl-1,2,4-oxadiazol-5-yl)-26,27-cyclo-9,10-secocholesta-5,7,10(19),22-tetraen-1,3,24-triol (5*Z*,7*E*,22*E*)-(1*S*,3*R*,24*R*)-25-(3-Ethyl-1,2,4-oxadiazol-5-yl)-26,27-cyclo-9,10-secocholesta-5,7,10(19),22-tetraen-1,3,24-triol
(5*Z*,7*E*,22*E*)-(1*S*,3*R*,24*S*)-25-(3-Ethyl-1,2,4-oxadiazol-5-yl)-26,27-cyclo-9,10-secocholesta-5,7,10(19),22-tetraen-1,3,24-triol
(5*Z*,7*E*,22*E*)-(1*S*,3*R*,24*R*)-25-(3-Propyl-1,2,4-oxadiazol-5-yl)-26,27-cyclo-9,10-secocholesta-5,7,10(19),22-tetraen-1,3,24-triol
(5*Z*,7*E*,22*E*)-(1*S*,3*R*,24*S*)-25-(3-Propyl-1,2,4-oxadiazol-5-yl)-26,27-cyclo-9,10-secocholesta-5,7,10(19),22-tetraen-1,3,24-triol
(5*Z*,7*E*,22*E*)-(1*S*,3*R*,24*R*)-25-(3-Butyl-1,2,4-oxadiazol-5-yl)-26,27-cyclo-9,10-secocholesta-5,7,10(19),22-tetraen-1,3,24-triol
(5*Z*,7*E*,22*E*)-(1*S*,3*R*,24*S*)-25-(3-Butyl-1,2,4-oxadiazol-5-yl)-26,27-cyclo-9,10-secocholesta-5,7,10(19),22-tetraen-1,3,24-triol (5*Z*,7*E*,22*E*)-(1*S*,3*R*,24*R*)-25-(3-Pentyl-1,2,4-oxadiazol-5-yl)-26,27-cyclo-9,10-secocholesta-5,7,10(19),22-tetraen-1,3,24-triol
(5*Z*,7*E*,22*E*)-(1*S*,3*R*,24*S*)-25-(3-Pentyl-1,2,4-oxadiazol-5-yl)-26,27-cyclo-9,10-secocholesta-5,7,10(19),22-tetraen-1,3,24-triol (5*Z*,7*E*,22*E*)-(1*S*,3*R*,24*R*)-25-(5-Methyl-1,3,4-oxadiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Methyl-1,3,4-oxadiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Ethyl-1,3,4-oxadiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Ethyl-1,3,4-oxadiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Propyl-1,3,4-oxadiazol-2-yl)-26,27-cyclo-9,10-secocholesta-5,7,10(19),22-tetraen-1,3,24-triol (5*Z*,7*E*,22*E*)-(1*S*,3*R*,24*S*)-25-(5-Propyl-1,3,4-oxadiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Butyl-1,3,4-oxadiazol-2-yl)-26,27-cyclo-9,10-secocholesta-5, 7,10(19),22-tetraen-1,3,24-triol
(5*Z*,7*E*,22*E*)-(1*S*,3*R*,24*S*)-25-(5-Butyl-1,3,4-oxadiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Pentyl-1,3,4-oxadiazol-2-yl)-26,27-cyclo-9,10-secocholesta-5,7,10(19),22-tetraen-1,3,24-triol
(5*Z*,7*E*,22*E*)-(1*S*,3*R*,24*S*)-25-(5-Pentyl-1,3,4-oxadiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-Phenyl-26,27-cyclo-9,10-secocholesta-5,7,10(19),22-tetraen-1,3,24-triol
(5*Z*,7*E*,22*E*)-(1*S*,3*R*,24*S*)-25-Phenyl-26,27-cyclo-9,10-secocholesta-5,7,10(19),22-tetraen-1,3,24-triol
(5*Z*,7*E*,22*E*)-(1*S*,3*R*,24*R*)-25-(4-Methylphenyl)-26,27-cyclo-9,10-secocholesta-5,7,10(19),22-tetraen-1,3,24-triol
(5*Z*,7*E*,22*E*)-(1*S*,3*R*,24*S*)-25-(4-Methylphenyl)-26,27-cyclo-9,10-secocholesta-5,7,10(19),22-tetraen-1,3,24-triol

(5*Z*,7*E*,22*E*)-(1*S*,3*R*,24*R*)-25-(4-Ethylphenyl)-26,27-cyclo-9,10-secocholesta-5,7,10(19),22-tetraen-1,3,24-triol
(5*Z*,7*E*,22*E*)-(1*S*,3*R*,24*S*)-25-(4-Ethylphenyl)-26,27-cyclo-9,10-secocholesta-5,7,10(19),22-tetraen-1,3,24-triol
(5*Z*,7*E*,22*E*)-(1*S*,3*R*,24*R*)-25-(4-Propylphenyl)-26,27-cyclo-9,10-secocholesta-5,7,10(19),22-tetraen-1,3,24-triol
(5*Z*,7*E*,22*E*)-(1*S*,3*R*,24*S*)-25-(4-Propylphenyl)-26,27-cyclo-9,10-secocholesta-5,7,10(19),22-tetraen-1,3,24-triol
(5*Z*,7*E*,22*E*)-(1*S*,3*R*,24*R*)-25-(4-Butylphenyl)-26,27-cyclo-9,10-secocholesta-5,7,10(19),22-tetraen-1,3,24-triol
(5*Z*,7*E*,22*E*)-(1*S*,3*R*,24*S*)-25-(4-Butylphenyl)-26,27-cyclo-9,10-secocholesta-5,7,10(19),22-tetraen-1,3,24-triol
(5*Z*,7*E*,22*E*)-(1*S*,3*R*,24*R*)-25-(4-Pentylphenyl)-26,27-cyclo-9,10-secocholesta-5,7,10(19),22-tetraen-1,3,24-triol
(5*Z*,7*E*,22*E*)-(1*S*,3*R*,24*S*)-25-(4-Pentylphenyl)-26,27-cyclo-9,10-secocholesta-5,7,10(19),22-tetraen-1,3,24-triol
(5*Z*,7*E*,22*E*)-(1*S*,3*R*,24*R*)-25-(3-Methylphenyl)-26,27-cyclo-9,10-secocholesta-5,7,10(19),22-tetraen-1,3,24-triol
(5*Z*,7*E*,22*E*)-(1*S*,3*R*,24*S*)-25-(3-Methylphenyl)-26,27-cyclo-9,10-secocholesta-5,7,10(19),22-tetraen-1,3,24-triol
(5*Z*,7*E*,22*E*)-(1*S*,3*R*,24*R*)-25-(3-Ethylphenyl)-26,27-cyclo-9,10-secocholesta-5,7,10(19),22-tetraen-1,3,24-triol
(5*Z*,7*E*,22*E*)-(1*S*,3*R*,24*S*)-25-(3-Ethylphenyl)-26,27-cyclo-9,10-secocholesta-5,7,10(19),22-tetraen-1,3,24-triol
(5*Z*,7*E*,22*E*)-(1*S*,3*R*,24*R*)-25-(3-Propylphenyl)-26,27-cyclo-9,10-secocholesta-5,7,10(19),22-tetraen-1,3,24-triol
(5*Z*,7*E*,22*E*)-(1*S*,3*R*,24*S*)-25-(3-Propylphenyl)-26,27-cyclo-9,10-secocholesta-5,7,10(19),22-tetraen-1,3,24-triol
(5*Z*,7*E*,22*E*)-(1*S*,3*R*,24*R*)-25-(3-Butylphenyl)-26,27-cyclo-9,10-secocholesta-5,7,10(19),22-tetraen-1,3,24-triol
(5*Z*,7*E*,22*E*)-(1*S*,3*R*,24*S*)-25-(3-Butylphenyl)-26,27-cyclo-9,10-secocholesta-5,7,10(19),22-tetraen-1,3,24-triol
(5*Z*,7*E*,22*E*)-(1*S*,3*R*,24*R*)-25-(3-Pentylphenyl)-26,27-cyclo-9,10-secocholesta-5,7,10(19),22-tetraen-1,3,24-triol
(5*Z*,7*E*,22*E*)-(1*S*,3*R*,24*S*)-25-(3-Pentylphenyl)-26,27-cyclo-9,10-secocholesta-5,7,10(19),22-tetraen-1,3,24-triol
(5*Z*,7*E*,22*E*)-(1*S*,3*R*,24*R*)-25-[4-(1-Methylethyl)phenyl)-26,27-cyclo-9,10-secocholesta-5,7,10(19),22-tetraen-1,3,24-triol
(5*Z*,7*E*,22*E*)-(1*S*,3*R*,24*S*)-25-[4-(1-Methylethyl)phenyl)-26,27-cyclo-9,10-secocholesta-5,7,10(19),22-tetraen-1,3,24-triol
(5*Z*,7*E*,22*E*)-(1*S*,3*R*,24*R*)-25-[3-(1-Methylethyl)phenyl)-26,27-cyclo-9,10-secocholesta-5,7,10(19),22-tetraen-1,3,24-triol
(5*Z*,7*E*,22*E*)-(1*S*,3*R*,24*S*)-25-[3-(1-Methylethyl)phenyl)-26,27-cyclo-9,10-secocholesta-5,7,10(19),22-tetraen-1,3,24-triol
(5*Z*,7*E*,22*E*)-(1*S*,3*R*,24*R*)-25-(2-Pyridyl)-26,27-cyclo-9,10-secocholesta-5,7,10(19),22-tetraen-1,3,24-triol
(5*Z*,7*E*,22*E*)-(1*S*,3*R*,24*S*)-25-(2-Pyridyl)-26,27-cyclo-9,10-secocholesta-5,7,10(19),22-tetraen-1,3,24-triol
(5*Z*,7*E*,22*E*)-(1*S*,3*R*,24*R*)-25-(6-Methyl-2-pyridyl)-26,27-cyclo-9,10-secocholesta-5,7,10(19),22-tetraen-1,3,24-triol
(5*Z*,7*E*,22*E*)-(1*S*,3*R*,24*S*)-25-(6-Methyl-2-pyridyl)-26,27-cyclo-9,10-secocholesta-5,7,10(19),22-tetraen-1,3,24-triol
(5*Z*,7*E*,22*E*)-(1*S*,3*R*,24*R*)-25-(5-Methyl-2-pyridyl)-26,27-cyclo-9,10-secocholesta-5,7,10(19),22-tetraen-1,3,24-triol
(5*Z*,7*E*,22*E*)-(1*S*,3*R*,24*S*)-25-(5-Methyl-2-pyridyl)-26,27-cyclo-9,10-secocholesta-5,7,10(19),22-tetraen-1,3,24-triol
(5*Z*,7*E*,22*E*)-(1*S*,3*R*,24*R*)-25-(4-Methyl-2-pyridyl)-26,27-cyclo-9,10-secocholesta-5,7,10(19),22-tetraen-1,3,24-triol
(5*Z*,7*E*,22*E*)-(1*S*,3*R*,24*S*)-25-(4-Methyl-2-pyridyl)-26,27-cyclo-9,10-secocholesta-5,7,10(19),22-tetraen-1,3,24-triol
(5*Z*,7*E*,22*E*)-(1*S*,3*R*,24*R*)-25-(6-Ethyl-2-pyridyl)-26,27-cyclo-9,10-secocholesta-5,7,10(19),22-tetraen-1,3,24-triol
(5*Z*,7*E*,22*E*)-(1*S*,3*R*,24*S*)-25-(6-Ethyl-2-pyridyl)-26,27-cyclo-9,10-secocholesta-5,7,10(19),22-tetraen-1,3,24-triol
(5*Z*,7*E*,22*E*)-(1*S*,3*R*,24*R*)-25-(5-Ethyl-2-pyridyl)-26,27-cyclo-9,10-secocholesta-5,7,10(19),22-tetraen-1,3,24-triol
(5*Z*,7*E*,22*E*)-(1*S*,3*R*,24*S*)-25-(5-Ethyl-2-pyridyl)-26,27-cyclo-9,10-secocholesta-5,7,10(19),22-tetraen-1,3,24-triol
(5*Z*,7*E*,22*E*)-(1*S*,3*R*,24*R*)-25-(4-Ethyl-2-pyridyl)-26,27-cyclo-9,10-secocholesta-5,7,10(19),22-tetraen-1,3,24-triol
(5*Z*,7*E*,22*E*)-(1*S*,3*R*,24*S*)-25-(4-Ethyl-2-pyridyl)-26,27-cyclo-9,10-secocholesta-5,7,10(19),22-tetraen-1,3,24-triol
(5*Z*,7*E*,22*E*)-(1*S*,3*R*,24*R*)-25-(6-Propyl-2-pyridyl)-26,27-cyclo-9,10-secocholesta-5,7,10(19),22-tetraen-1,3,24-triol
(5*Z*,7*E*,22*E*)-(1*S*,3*R*,24*S*)-25-(6-Propyl-2-pyridyl)-26,27-cyclo-9,10-secocholesta-5,7,10(19),22-tetraen-1,3,24-triol
(5*Z*,7*E*,22*E*)-(1*S*,3*R*,24*R*)-25-(5-Propyl-2-pyridyl)-26,27-cyclo-9,10-secocholesta-5,7,10(19),22-tetraen-1,3,24-triol

(5*Z*,7*E*,22*E*)-(1*S*,3*R*,24*S*)-25-(5-Propyl-2-pyridyl)-26,27-cyclo-9,10-secocholesta-5,7,10(19),22-tetraen-1,3,24-triol
(5*Z*,7*E*,22*E*)-(1*S*,3*R*,24*R*)-25-(4-Propyl-2-pyridyl)-26,27-cyclo-9,10-secocholesta-5,7,10(19),22-tetraen-1,3,24-triol
(5*Z*,7*E*,22*E*)-(1*S*,3*R*,24*S*)-25-(4-Propyl-2-pyridyl)-26,27-cyclo-9,10-secocholesta-5,7,10(19),22-tetraen-1,3,24-triol
(5*Z*,7*E*,22*E*)-(1*S*,3*R*,24*R*)-25-(6-Butyl-2-pyridyl)-26,27-cyclo-9,10-secocholesta-5,7,10(19),22-tetraen-1,3,24-triol
(5*Z*,7*E*,22*E*)-(1*S*,3*R*,24*S*)-25-(6-Butyl-2-pyridyl)-26,27-cyclo-9,10-secocholesta-5,7,10(19),22-tetraen-1,3,24-triol
(5*Z*,7*E*,22*E*)-(1*S*,3*R*,24*R*)-25-(5-Butyl-2-pyridyl)-26,27-cyclo-9,10-secocholesta-5,7,10(19),22-tetraen-1,3,24-triol
(5*Z*,7*E*,22*E*)-(1*S*,3*R*,24*S*)-25-(5-Butyl-2-pyridyl)-26,27-cyclo-9,10-secocholesta-5,7,10(19),22-tetraen-1,3,24-triol
(5*Z*,7*E*,22*E*)-(1*S*,3*R*,24*R*)-25-(4-Butyl-2-pyridyl)-26,27-cyclo-9,10-secocholesta-5,7,10(19),22-tetraen-1,3,24-triol
(5*Z*,7*E*,22*E*)-(1*S*,3*R*,24*S*)-25-(4-Butyl-2-pyridyl)-26,27-cyclo-9,10-secocholesta-5,7,10(19),22-tetraen-1,3,24-triol
(5*Z*,7*E*,22*E*)-(1*S*,3*R*,24*R*)-25-(5,5-Dimethyl-2-oxazolin-2-yl)-26,27-cyclo-9,10-secocholesta-5,7,10(19),22-tetraen-1,3,24-triol
(5*Z*,7*E*,22*E*)-(1*S*,3*R*,24*S*)-25-(5,5-Dimethyl-2-oxazolin-2-yl)-26,27-cyclo-9,10-secocholesta-5,7,10(19),22-tetraen-1,3,24-triol
(5*Z*,7*E*,22*E*)-(1*S*,3*R*,24*R*)-25-(5,5-Diethyl-2-oxazolin-2-yl)-26,27-cyclo-9,10-secocholesta-5,7,10(19),22-tetraen-1,3,24-triol
(5*Z*,7*E*,22*E*)-(1*S*,3*R*,24*S*)-25-(5,5-Diethyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*R*,25(*R*)]-25-(5-Methyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*S*,25(*R*)]-25-(5-Methyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*R*,25(S)]-25-(5-Methyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*S*,25(*S*)]-25-(5-Methyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*R*,25(*R*)]-25-(5-Ethyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*S*,25(*R*)]-25-(5-Ethyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*R*,25(*S*)]-25-(5-Ethyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*S*,25(*S*)]-25-(5-Ethyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*R*,25(*R*)]-25-(5-Propyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*S*,25(*R*)]-25-(5-Propyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*R*,25(*S*)]-25-(5-Propyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*S*,25(*S*)]-25-(5-Propyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*R*,25(*R*)]-25-(5-Butyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*S*,25(*R*)]-25-(5-Butyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*R*,25(*S*)]-25-(5-Butyl-2-oxazolin-2-yl)-26,27-cyclo-9,10-secocholesta-5,7,10(19),22-tetraen-1,3,24-triol
(5*Z*,7*E*,22*E*)-[1*S*,3*R*,24*S*,25(*S*)]-25-(5-Butyl-2-oxazolin-2-yl)-26,27-cyclo-9,10-secocholesta-5,7,10(19),22-tetraen-1,3,24-triol

(5*Z*,7*E*,22*E*)-[1*S*,3*R*,24*R*,25(*R*)]-25-(5-Phenyl-2-oxazolin-2-yl)-26,27-cyclo-9,10-secocholesta-5,7,10(19),22-tetraen-1,3,24-triol

(5*Z*,7*E*,22*E*)-[1*S*,3*R*,24*S*,25(*R*)]-25-(5-Phenyl-2-oxazolin-2-yl)-26,27-cyclo-9,10-secocholesta-5,7,10(19),22-tetraen-1,3,24-triol

(5*Z*,7*E*,22*E*)-[1*S*,3*R*,24*R*,25(*S*)]-25-(5-Phenyl-2-oxazolin-2-yl)-26,27-cyclo-9,10-secocholesta-5,7,10(19),22-tetraen-1,3,24-triol

(5*Z*,7*E*,22*E*)-[1*S*,3*R*,24*S*,25(*S*)]-25-(5-Phenyl-2-oxazolin-2-yl)-26,27-cyclo-9,10-secocholesta-5,7,10(19),22-tetraen-1,3,24-triol

(5*Z*,7*E*,22*E*)-(1*S*,3*R*,20*S*,24*R*)-25-(5-Propyloxazol-2-yl)-26,27-cyclo-9,10-secocholesta-5,7,10(19),22-tetraen-1,3,24-triol

(5*Z*,7*E*,22*E*)-(1*S*,3*R*,20*S*,24*S*)-25-(5-Propyloxazol-2-yl)-26,27-cyclo-9,10-secocholesta-5,7,10(19),22-tetraen-1,3,24-triol

(5*Z*,7*E*,22*E*)-(1*S*,3*R*,20*S*,24*R*)-25-(5-Methyloxazol-2-yl)-26,27-cyclo-9,10-secocholesta-5,7,10(19),22-tetraen-1,3,24-triol

(5*Z*,7*E*,22*E*)-(1*S*,3*R*,20*S*,24*S*)-25-(5-Methyloxazol-2-yl)-26,27-cyclo-9,10-secocholesta-5,7,10(19),22-tetraen-1,3,24-triol

(5*Z*,7*E*,22*E*)-(1*S*,3*R*,20*S*,24*R*)-25-(5-Ethyloxazol-2-yl)-26,27-cyclo-9,10-secocholesta-5,7,10(19),22-tetraen-1,3,24-triol

(5*Z*,7*E*,22*E*)-(1*S*,3*R*,20*S*,24*S*)-25-(5-Ethyloxazol-2-yl)-26,27-cyclo-9,10-secocholesta-5,7,10(19),22-tetraen-1,3,24-triol

(5*Z*,7*E*,22*E*)-(1*S*,3*R*,20*S*,24*R*)-25-(5-Butyloxazol-2-yl)-26,27-cyclo-9,10-secocholesta-5,7,10(19),22-tetraen-1,3,24-triol

(5*Z*,7*E*,22*E*)-(1*S*,3*R*,20*S*,24*S*)-25-(5-Butyloxazol-2-yl)-26,27-cyclo-9,10-secocholesta-5,7,10(19),22-tetraen-1,3,24-triol

(5*Z*,7*E*,22*E*)-(1*S*,3*R*,20*S*,24*R*)-25-(5-Pentyloxazol-2-yl)-26,27-cyclo-9,10-secocholesta-5,7,10(19),22-tetraen-1,3,24-triol

(5*Z*,7*E*,22*E*)-(1*S*,3*R*,20*S*,24*S*)-25-(5-Pentyloxazol-2-yl)-26,27-cyclo-9,10-secocholesta-5,7,10(19),22-tetraen-1,3,24-triol

(5*Z*,7*E*,22*E*)-(1*S*,3*R*,20*S*,24*R*)-25-(5-Propylthiazol-2-yl)-26,27-cyclo-9,10-secocholesta-5,7,10(19),22-tetraen-1,3,24-triol

(5*Z*,7*E*,22*E*)-(1*S*,3*R*,20*S*,24*S*)-25-(5-Propylthiazol-2-yl)-26,27-cyclo-9,10-secocholesta-5,7,10(19),22-tetraen-1,3,24-triol

(5*Z*,7*E*,22*E*)-(1*S*,3*R*,20*S*,24*R*)-25-(5-Methylthiazol-2-yl)-26,27-cyclo-9,10-secocholesta-5,7,10(19),22-tetraen-1,3,24-triol

(5*Z*,7*E*,22*E*)-(1*S*,3*R*,20*S*,24*S*)-25-(5-Methylthiazol-2-yl)-26,27-cyclo-9,10-secocholesta-5,7,10(19),22-tetraen-1,3,24-triol

(5*Z*,7*E*,22*E*)-(1*S*,3*R*,20*S*,24*R*)-25-(5-Ethylthiazol-2-yl)-26,27-cyclo-9,10-secocholesta-5,7,10(19),22-tetraen-1,3,24-triol

(5*Z*,7*E*,22*E*)-(1*S*,3*R*,20*S*,24*S*)-25-(5-Ethylthiazol-2-yl)-26,27-cyclo-9,10-secocholesta-5,7,10(19),22-tetraen-1,3,24-triol

(5*Z*,7*E*,22*E*)-(1*S*,3*R*,20*S*,24*R*)-25-(5-Butylthiazol-2-yl)-26,27-cyclo-9,10-secocholesta-5,7,10(19),22-tetraen-1,3,24-triol

(5*Z*,7*E*,22*E*)-(1*S*,3*R*,20*S*,24*S*)-25-(5-Butylthiazol-2-yl)-26,27-cyclo-9,10-secocholesta-5,7,10(19),22-tetraen-1,3,24-triol

(5*Z*,7*E*,22*E*)-(1*S*,3*R*,20*S*,24*R*)-25-(5-Pentylthiazol-2-yl)-26,27-cyclo-9,10-secocholesta-5,7,10(19),22-tetraen-1,3,24-triol

(5*Z*,7*E*,22*E*)-(1*S*,3*R*,20*S*,24*S*)-25-(5-Pentylthiazol-2-yl)-26,27-cyclo-9,10-secocholesta-5,7,10(19),22-tetraen-1,3,24-triol

(5*Z*,7*E*,22*E*)-(1*S*,3*R*,20*S*,24*R*)-25-(4-Propyloxazol-2-yl)-26,27-cydo-9,10-secocholesta-5,7,10(19),22-tetraen-1,3,24-triol

(5*Z*,7*E*,22*E*)-(1*S*,3*R*,20*S*,24*S*)-25-(4-Propyloxazol-2-yl)-26,27-cyclo-9,10-secocholesta-5,7,10(19),22-tetraen-1,3,24-triol

(5*Z*,7*E*,22*E*)-(1*S*,3*R*,20*S*,24*R*)-25-(4-Methyloxazol-2-yl)-26,27-cyclo-9,10-secocholesta-5,7,10(19),22-tetraen-1,3,24-triol

(5*Z*,7*E*,22*E*)-(1*S*,3*R*,20*S*,24*S*)-25-(4-Methyloxazol-2-yl)-26,27-cyclo-9,10-secocholesta-5,7,10(19),22-tetraen-1,3,24-triol

(5*Z*,7*E*,22*E*)-(1*S*,3*R*,20*S*,24*R*)-25-(4-Ethyloxazol-2-yl)-26,27-cyclo-9,10-secocholesta-5,7,10(19),22-tetraen-1,3,24-triol

(5*Z*,7*E*,22*E*)-(1*S*,3*R*,20*S*,24*S*)-25-(4-Ethyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(4-Butyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(4-Butyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(4-Pentyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(4-Pentyloxazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(4-Propylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(4-Propylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(4-Methylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(4-Methylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(4-Ethylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(4-Ethylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(4-Butylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(4-Butylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*R*)-25-(4-Pentylthiazol-2-yl)-26,27-cyclo-9,10-secocholesta-5,7,10(19),22-tetraen-1,3,24-triol
(5*Z*,7*E*,22*E*)-(1*S*,3*R*,20*S*,24*S*)-25-(4-Pentylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(5-propyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(5-propyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(5-methyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(5-methyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(5-Ethyloxazol-2-yl)-20-methyl-26,27-cyclo-9,10-secocholesta-5, 7,10(19),22-tetraen-1,3,24-triol
(5*Z*,7*E*,22*E*)-(1*S*,3*R*,24*S*)-25-(5-Ethyloxazol-2-yl)-20-methyl-26,27-cyclo-9,10-secocholesta-5,7,10(19),22-tetraen-1,3,24-triol
(5*Z*,7*E*,22*E*)-(1*S*,3*R*,24*R*)-25-(5-Butyloxazol-2-yl)-20-methyl-26,27-cyclo-9,10-secocholesta-5,7,10(19),22-tetraen-1,3,24-triol
(5*Z*,7*E*,22*E*)-(1*S*,3*R*,24*S*)-25-(5-Butyloxazol-2-yl)-20-methyl-26,27-cyclo-9,10-secocholesta-5,7,10(19),22-tetraen-1,3,24-triol
(5*Z*,7*E*,22*E*)-(1*S*,3*R*,24*R*)-20-Methyl-25-(5-pentyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(5-pentyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(5-propylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(5-propylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(5-methylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(5-methylthiazol-2-yl)-26,27-cyclo-9,10-secocholesta-5,7,10(19),22-tetraen-1,3,24-triol

(5*Z*,7*E*,22*E*)-(1*S*,3*R*,24*R*)-25-(5-Ethylthiazol-2-yl)-20-methyl-26,27-cyclo-9,10-secocholesta-5,7,10(19),22-tetraen-1,3,24-triol
(5*Z*,7*E*,22*E*)-(1*S*,3*R*,24*S*)-25-(5-Ethylthiazol-2-yl)-20-methyl-26,27-cyclo-9,10-secocholesta-5,7,10(19),22-tetraen-1,3,24-triol
(5*Z*,7*E*,22*E*)-(1*S*,3*R*,24*R*)-25-(5-Butylthiazol-2-yl)-20-methyl-26,27-cyclo-9,10-secocholesta-5,7,10(19),22-tetraen-1,3,24-triol
(5*Z*,7*E*,22*E*)-(1*S*,3*R*,24*S*)-25-(5-Butylthiazol-2-yl)-20-methyl-26,27-cyclo-9,10-secocholesta-5,7,10(19),22-tetraen-1,3,24-triol
(5*Z*,7*E*,22*E*)-(1*S*,3*R*,24*R*)-25-(5-Pentylthiazol-2-yl)-20-methyl-26,27-cyclo-9,10-secocholesta-5,7,10(19),22-tetraen-1,3,24-triol
(5*Z*,7*E*,22*E*)-(1*S*,3*R*,24*S*)-25-(5-Pentylthiazol-2-yl)-20-methyl-26,27-cyclo-9,10-secocholesta-5,7,10(19),22-tetraen-1,3,24-triol
(5*Z*,7*E*,22*E*)-(1*S*,3*R*,24*R*)-20-Methyl-25-(4-propyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(4-propyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(4-methyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(4-methyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Ethyloxazol-2-yl)-20-methyl-26,27-cyclo-9,10-secocholesta-5,7,10(19),22-tetraen-1,3,24-triol
(5*Z*,7*E*,22*E*)-(1*S*,3*R*,24*S*)-25-(4-Ethyloxazol-2-yl)-20-methyl-26,27-cyclo-9,10-secocholesta-5,7,10(19),22-tetraen-1,3,24-triol
(5*Z*,7*E*,22*E*)-(1*S*,3*R*,24*R*)-25-(4-Butyloxazol-2-yl)-20-methyl-26,27-cyclo-9,10-secocholesta-5,7,10(19),22-tetraen-1,3,24-triol
(5*Z*,7*E*,22*E*)-(1*S*,3*R*,24*S*)-25-(4-Butyloxazol-2-yl)-20-methyl-26,27-cyclo-9,10-secocholesta-5,7,10(19),22-tetraen-1,3,24-triol
(5*Z*,7*E*,22*E*)-(1*S*,3*R*,24*R*)-20-Methyl-25-(4-pentyloxazol-2-yl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(4-pentyloxazol-2-yl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(4-propylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(4-propylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-20-Methyl-25-(4-methylthiazol-2-yl)-26,27-cyclo-9,10-secocholesta-5,7,10(19),22-tetraen-1,3,24-triol
(5*Z*,7*E*,22*E*)-(1*S*,3*R*,24*S*)-20-Methyl-25-(4-methylthiazol-2-yl)-26,27-cyclo-9,10-secocholesta-5,7,10(19),22-tetraen-1,3,24-triol
(5*Z*,7*E*,22*E*)-(1*S*,3*R*,24*R*)-25-(4-Ethylthiazol-2-yl)-20-methyl-26,27-cyclo-9,10-secocholesta-5,7,10(19),22-tetraen-1,3,24-triol
(5*Z*,7*E*,22*E*)-(1*S*,3*R*,24*S*)-25-(4-Ethylthiazol-2-yl)-20-methyl-26,27-cyclo-9,10-secocholesta-5,7,10(19),22-tetraen-1,3,24-triol
(5*Z*,7*E*,22*E*)-(1*S*,3*R*,24*R*)-25-(4-Butylthiazol-2-yl)-20-methyl-26,27-cyclo-9,10-secocholesta-5,7,10(19),22-tetraen-1,3,24-triol
(5*Z*,7*E*,22*E*)-(1*S*,3*R*,24*S*)-25-(4-Butylthiazol-2-yl)-20-methyl-26,27-cyclo-9,10-secocholesta-5,7,10(19),22-tetraen-1,3,24-triol
(5*Z*,7*E*,22*E*)-(1*S*,3*R*,24*R*)-25-(4-Pentylthiazol-2-yl)-20-methyl-26,27-cyclo-9,10-secocholesta-5,7,10(19),22-tetraen-1,3,24-triol
(5*Z*,7*E*,22*E*)-(1*S*,3*R*,24*S*)-25-(4-Pentylthiazol-2-yl)-20-methyl-26,27-cyclo-9,10-secocholesta-5,7,10(19),22-tetraen-1,3,24-triol

**6.** Verfahren zur Herstellung der Vitamin D-Derivate der allgemeinen Formel **I**, wobei eine Verbindung der allgemeinen Formel II

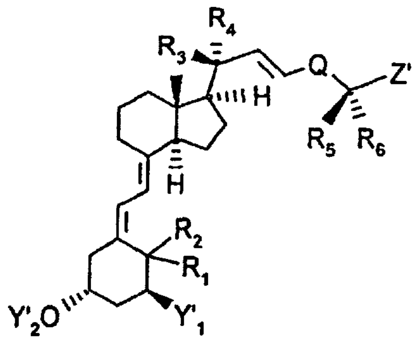

II

worin
Y'$_1$ ein Wasserstoffatom oder eine geschützte Hydroxygruppe und
Y'$_2$ eine Hydroxyschutzgruppe,
$R_1$ und $R_2$ je ein Wasserstoffatom oder gemeinsam eine exocyclische Methylengruppe,
$R_3$ und $R_4$ unabhängig voneinander ein Wasserstoffatom, ein Chlor- oder Fluoratom, eine Alkylgruppe mit 1 bis 4 Kohlenstoffatomen, gemeinsam eine Methylengruppe oder gemeinsam mit dem quartären Kohlenstoffatom 20 einen 3-7-gliedrigen, gesättigten oder ungesättigten carbocyclischen Ring,
Q eine geradkettige oder verzweigte Kohlenstoffeinheit mit bis zu 10 Kohlenstoffatomen, die an beliebigen Positionen Hydroxylgruppen ($\alpha$- oder $\beta$-ständig), die ihrerseits verethert oder verestert sein können, Ketogruppen, Aminogruppen oder Halogenatome aufweisen kann,
$R_5$ und $R_6$ gemeinsam mit dem Kohlenstoffatom 25 einen 3-7-gliedrigen, gesättigten oder ungesättigten carbocyclischen Ring und
Z' einen fünf- oder sechsgliedrigen carbo- oder heterocyclischen Ring, der gesättigt, ungesättigt oder aromatisch sein kann und an beliebigen Positionen eine oder mehrere Alkylketten, die geradkettig oder verzweigt, gesättigt oder ungesättigt sein können und an beliebigen Stellen durch Oxa-, Thia- oder Azafunktionen (substituiert oder unsubstituiert) oder Sulfoxid oder Sulfongruppen unterbrochen sein können oder Substituenten (Hydroxygruppen, Halogenatome) tragen können, wobei gegebenenfalls vorliegende Hydroxylgruppen in geschützter Form vorliegen können,
bedeuten, durch gleichzeitige oder sukzessive Abspaltung der Hydroxyschutzgruppen und gegebenenfalls durch partielle oder vollständige Veresterung(en) oder - Veretherung(en) der freien Hydroxygruppen umgesetzt werden.

7. Verwendung der Vitamin D-Derivate der allgemeinen Formel I zur Herstellung von Arzneimitteln.

8. Verwendung der Vitamin D-Derivate der allgemeinen Formel I nach Anspruch 7 zur Herstellung eines Arzneimittels zur Therapie von hyperproliferativen Hauterkrankungen (Psoriasis, Akne, Ichthyosis) sowie Tumorerkrankungen und Präkanzerosen (z. B. Darmtumoren, Mammakarzinom, Lungentumoren, Prostatakarzinom, Leukämien, T-Zell-Lymphome, aktinische Keratosen, Cervixdysplasien, weiterhin Autoimmunerkrankungen (multiple Sklerose, Diabetes mellitus Typ I, Myasthenia gravis, Lupus erythematodes), Abstoßungsreaktionen bei autologen, allogenen oder xenogenen Transplantaten sowie AIDS, daneben ist der therapeutische Einsatz bei atrophischer Haut oder Wundheilung möglich sowie der Therapie von sekundären Hyperparathyroidimus, renaler Osteodystrophie sowie seniler und postmenopausaler Osteoporose, Diabetes mellitus Typ II und der Therapie von degenativen Erkrankungen des peripheren und zentralen Nervensystems (der Alzheimerschen Krankheit und der amyotrophen Lateralsklerose) sowie auch der Regulation des Haarwachstums.

9. Verwendung von Vitamin D-Derivaten der allgemeinen Formel I nach Anspruch 7, die die Wirkung von Calcitriol in HL 60 - Zellen antagonisieren, zur Herstellung eines Arzneimittels zur Therapie von Hypercalcämien (Hypervitaminose D, Intoxikation mit Calcitriol oder dessen Analoga) oder granulomatösen Erkrankungen (Sarkoidose, Tuberkulose) sowie paraneoplastischer Hypercalcämien (so osteolytische Metastasen und Tumore mit erhöhter Synthese von Parathormon-related peptide) und Hypercalcämie bei Hyperparathyroidismus und zur Fertilitätskontrolle oder als Immunstimulantien sowie bei Hirsutismus und zur Therapie und Prophylaxe der Arteriosklerose, und weiter zur Therapie von entzündlichen Erkrankungen (rheumatische Arthritis, Morbus Crohn, Colitis ulcerosa und granulomatöse Erkrankungen).

**10.** Zwischenprodukte der allgemeinen Formeln XI, XII und XLIV innerhalb der Herstellung der Vitamin D-Derivate der allgemeinen Formel I nach Anspruch 1 :

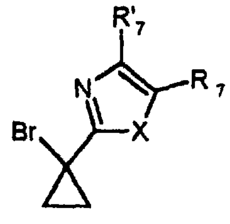

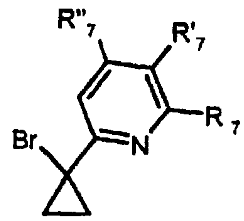

XI X=O
XIII X=S

XLIV

worin

$R_7$, $R'_7$ und $R''_7$ unabhängig voneinander ein Wasserstoffatom, einen geradkettigen oder verzweigten, gesättigten oder ungesättigten Alkylrest mit bis zu 12 Kohlenstoffatomen bedeuten, der an beliebigen Stellen durch Oxa-, Thia, oder Azafunktionen (substituiert oder unsubstituiert) oder Sulfoxid oder Sulfongruppen unterbrochen sein kann oder weitere Substituenten (freie oder geschützte Hydroxygruppen, Halogenatome) tragen kann.

## Claims

**1.** Vitamin D derivatives of general formula I

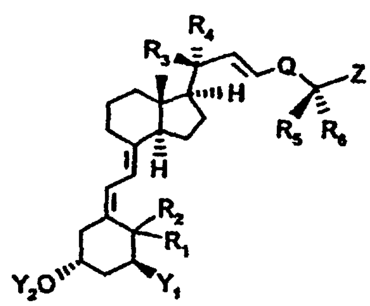

I

in which

| | |
|---|---|
| $Y^1$ | means a hydrogen atom, a hydroxyl group, an alkanoyloxy group with 1 to 12 C atoms or an aroyloxy group, |
| $Y^2$ | means a hydrogen atom or an alkanoyl group with 1 to 12 C atoms or an aroyl group, |
| $R_1$ and $R_2$ | each mean a hydrogen atom or together an exocyclic methylene group, |
| $R_3$ and $R_4$, | independently of one another, mean a hydrogen atom, a chlorine or fluorine atom, an alkyl group with 1 to 4 carbon atoms, together a methylene group or together with quaternary carbon atom 20 a 3 to 7-membered, saturated or unsaturated carbocyclic ring, |
| Q | means a straight-chain or branched carbon unit with up to 10 carbon atoms, which at any positions can have hydroxyl groups (in α or β-position), which in turn can be etherified or esterified, keto groups, amino groups or halogen atoms, |
| $R_5$ and $R_6$ | together with carbon atom 25 mean a 3-7-membered, saturated or unsaturated carbocyclic ring and |
| Z | means a five- or six-membered carbo- or heterocyclic ring, which can be saturated, unsaturated or aromatic, and can carry at any positions one or more alkyl chains, which can be straight-chain or branched, saturated or unsaturated, and at any points can be interrupted by oxa, thia or aza groups |

(substituted or unsubstituted) or sulfoxide or sulfone groups or can carry substituents (hydroxy groups, halogen atoms).

**2.** Vitamin D derivatives of general formula I according to claim 1, in which $R_5$ and $R_6$ together with carbon atom C-25 mean a cyclopropyl ring and Z means an aromatic or heteroaromatic ring.

**3.** Vitamin D derivatives according to claim 2, in which Z has the meaning of an optionally substituted phenyl or oxazole or thiazole ring.

**4.** Vitamin D derivatives of general formula I according to claim 1, in which Q has the meaning of a hydroxymethyl or carbonylmethyl group.

**5.** Vitamin D derivatives of general formula I according to claim 1, namely

(5Z,7E,22E)-(1S,3R,24R)-25-(5-propyloxazol-2-yl)-26,27-cyclo-9,10-secocholesta-5,7,10(19),22-tetraene-1,3,24-triol

(5Z,7E,22E)-(1S,3R,24S)-25-(5-propyloxazol-2-yl)-26,27-cyclo-9,10-secocholesta-5,7,10(19),22-tetraene-1,3,24-triol

(5Z,7E,22E)-(1S,3R,24R)-25-(5-methyloxazol-2-yl)-26,27-cyclo-9,10-secocholesta-5,7,10(19),22-tetraene-1,3,24-triol

(5Z,7E,22E)-(1S,3R,24S)-25-(5-methyloxazol-2-yl)-26,27-cyclo-9,10-secocholesta-5,7,10(19),22-tetraene-1,3,24-triol

(5Z,7E,22E)-(1S,3R,24R)-25-(5-ethyloxazol-2-yl)-26,27-cyclo-9,10-secocholesta-5,7,10(19),22-tetraene-1,3,24-triol

(5Z,7E,22E)-(1S,3R,24S)-25-(5-ethyloxazol-2-yl)-26,27-cyclo-9,10-secocholesta-5,7,10(19),22-tetraene-1,3,24-triol

(5Z,7E,22E)-(1S,3R,24R)-25-(5-butyloxazol-2-yl)-26,27-cyclo-9,10-secocholesta-5,7,10(19),22-tetraene-1,3,24-triol

(5Z,7E,22E)-(1S,3R,24S)-25-(5-butyloxazol-2-yl)-26,27-cyclo-9,10-secocholesta-5,7,10(19),22-tetraene-1,3,24-triol

(5Z,7E,22E)-(1S,3R,24R)-25-(5-pentyloxazol-2-yl)-26,27-cyclo-9,10-secocholesta-5,7,10(19),22-tetraene-1,3,24-triol

(5Z,7E,22E)-(1S,3R,24S)-25-(5-pentyloxazol-2-yl)-26,27-cyclo-9,10-secocholesta-5,7,10(19),22-tetraene-1,3,24-triol

(5Z,7E,22E)-(1S,3R,24R)-25-(5-propylthiazol-2-yl)-26,27-cyclo-9,10-secocholesta-5,7,10(19),22-tetraene-1,3,24-triol

(5Z,7E,22E)-(1S,3R,24S)-25-(5-propylthiazol-2-yl)-26,27-cycle-9,10-secocholesta-5,7,10(19),22-tetraene-1,3,24-triol

(5Z,7E,22E)-(1S,3R,24R)-25-(5-methylthiazol-2-yl)-26,27-cyclo-9,10-secocholesta-5,7,10(19),22-tetraene-1,3,24-triol

(5Z,7E,22E)-(1S,3R,24S)-25-(5-methylthiazol-2-yl)-26,27-cyclo-9,10-secocholesta-5,7,10(19),22-tetraene-1,3,24-triol

(5Z,7E,22E)-(1S,3R,24R)-25-(5-ethylthiazol-2-yl)-26,27-cyclo-9,10-secocholesta-5,7,10(19),22-tetraene-1,3,24-triol

(5Z,7E,22E)-(1S,3R,24S)-25-(5-ethylthiazol-2-yl)-26,27-cyclo-9,10-secocholesta-5,7,10(19),22-tetraene-1,3,24-triol

(5Z,7E,22E)-(1S,3R,24R)-25-(5-butylthiazol-2-yl)-26,27-cyclo-9,10-secocholesta-5,7,10(19),22-tetraene-1,3,24-triol

(5Z,7E,22E)-(1S,3R,24S)-25-(5-butylthiazol-2-yl)-26,27-cyclo-9,10-secocholesta-5,7,10(19),22-tetraene-1,3,24-triol

(5Z,7E,22E)-(1S,3R,24R)-25-(5-pentylthiazol-2-yl)-26,27-cyclo-9,10-secocholesta-5,7,10(19),22-tetraene-1,3,24-triol

(5Z,7E,22E)-(1S,3R,24S)-25-(5-pentylthiazol-2-yl)-26,27-cyclo-9,10-secocholesta-5,7,10(19),22-tetraene-1,3,24-triol

(5Z,7E,22E)-(1S,3R,24R)-25-(5-propylimidazol-2-yl)-26,27-cyclo-9,10-secocholesta-5,7,10(19), 22-tetraene-1,3,24-triol

(5Z,7E,22E)-(1S,3R,24S)-25-(5-propylimidazol-2-yl)-26,27-cyclo-9,10-secocholesta-5,7,10(19), 22-tetraene-1,3,24-triol

(5Z,7E,22E)-(1S,3R,24R)-25-(5-methylimidazol-2-yl)-26,27-cyclo-9,10-secocholesta-5,7,10(19),22-tetraene-1,3,24-triol

(5Z,7E,22E)-(1S,3R,24S)-25-(5-methylimidazol-2-yl)-26,27-cyclo-9,10-secocholesta-5,7,10(19),22-tetraene-1,3,24-triol

(5Z,7E,22E)-(1S,3R,24R)-25-(5-ethylimidazol-2-yl)-26,27-cyclo-9,10-secocholesta-5,7,10(19),22-tetraene-1,3,24-triol

(5Z,7E,22E)-(1S,3R,24S)-25-(5-ethylimidazol-2-yl)-26,27-cyclo-9,10-secocholesta-5,7,10(19),22-tetraene-1,3,24-triol

(5Z,7E,22E)-(1S,3R,24R)-25-(5-butylimidazol-2-yl)-26,27-cyclo-9,10-secocholesta-5,7,10(19),22-tetraene-1,3,24-triol

(5Z,7E,22E)-(1S,3R,24S)-25-(5-butylimidazol-2-yl)-26,27-cyclo-9,10-secocholesta-5,7,10(19),22-tetraene-1,3,24-triol

(5Z,7E,22E)-(1S,3R,24R)-25-(5-pentylimidazol-2-yl)-26,27-cyclo-9,10-secocholesta-5,7,10(19),22-tetraene-1,3,24-triol

(5Z,7E,22E)-(1S,3R,24S)-25-(5-pentylimidazol-2-yl)-26,27-cyclo-9,10-secocholesta-5,7,10(19),22-tetraene-1,3,24-triol

(5Z,7E,22E)-(1S,3R,24R)-25-(5-propylfuran-2-yl)-26,27-cyclo-9,10-secocholesta-5,7,10(19),22-tetraene-1,3,24-triol

(5Z,7E,22E)-(1S,3R,24S)-25-(5-propylfuran-2-yl)-26,27-cyclo-9,10-secocholesta-5,7,10(19),22-tetraene-1,3,24-triol

(5Z,7E,22E)-(1S,3R,24R)-25-(5-methylfuran-2-yl)-26,27-cyclo-9,10-secocholesta-5,7,10(19),22-tetraene-1,3,24-triol

(5Z,7E,22E)-(1S,3R,24S)-25-(5-methylfuran-2-yl)-26,27-cyclo-9,10-secocholesta-5,7,10(19),22-tetraene-1,3,24-triol

(5Z,7E,22E)-(1S,3R,24R)-25-(5-ethylfuran-2-yl)-26,27-cyclo-9,10-secocholesta-5,7,10(19),22-tetraene-1,3,24-triol

(5Z,7E,22E)-(1S,3R,24S)-25-(5-ethylfuran-2-yl)-26,27-cyclo-9,10-secocholesta-5,7,10(19),22-tetraene-1,3,24-triol

(5Z,7E,22E)-(1S,3R,24R)-25-(5-butylfuran-2-yl)-26,27-cyclo-9,10-secocholesta-5,7,10(19),22-tetraene-1,3,24-triol

(5Z,7E,22E)-(1S,3R,24S)-25-(5-butylfuran-2-yl)-26,27-cyclo-9,10-secocholesta-5,7,10(19),22-tetraene-1,3,24-triol

(5Z,7E,22E)-(1S,3R,24R)-25-(5-pentylfuran-2-yl)-26,27-cyclo-9,10-secocholesta-5,7,10(19),22-tetraene-1,3,24-triol

(5Z,7E,22E)-(1S,3R,24S)-25-(5-pentylfuran-2-yl)-26,27-cyclo-9,10-secocholesta-5,7,10(19),22-tetraene-1,3,24-triol

(5Z,7E,22E)-(1S,3R,24R)-25-(5-propylthiophen-2-yl)-26,27-cyclo-9,10-secocholesta-5,7,10(19),22-tetraene-1,3,24-triol

(5Z,7E,22E)-(1S,3R,24S)-25-(5-propylthiophen-2-yl)-26,27-cyclo-9,10-secocholesta-5,7,10(19),22-tetraene-1,3,24-triol

(5Z,7E,22E)-(1S,3R,24R)-25-(5-methylthiophen-2-yl)-26,27-cyclo-9,10-secocholesta-5,7,10(19),22-tetraene-1,3,24-triol

(5Z,7E,22E)-(1S,3R,24S)-25-(5-methylthiophen-2-yl)-26,27-cyclo-9,10-secocholesta-5,7,10(19),22-tetraene-1,3,24-triol

(5Z,7E,22E)-(1S,3R,24R)-25-(5-ethylthiophen-2-yl)-26,27-cyclo-9,10-secocholesta-5,7,10(19),22-tetraene-1,3,24-triol

(5Z,7E,22E)-(1S,3R,24S)-25-(5-ethylthiophen-2-yl)-26,27-cycle-9,10-secocholesta-5,7,10(19),22-tetraene-1,3,24-triol

(5Z,7E,22E)-(1S,3R,24R)-25-(5-butylthiophen-2-yl)-26,27-cyclo-9,10-secocholesta-5,7,10(19),22-tetraene-1,3,24-triol

(5Z,7E,22E)-(1S,3R,24S)-25-(5-butylthiophen-2-yl)-26,27-cyclo-9,10-secocholesta-5,7,10(19),22-tetraene-1,3,24-triol

(5Z,7E,22E)-(1S,3R,24R)-25-(5-pentylthiophen-2-yl)-26,27-cyclo-9,10-secocholesta-5,7,10(19),22-tetraene-1,3,24-triol

(5Z,7E,22E)-(1S,3R,24S)-25-(5-pentylthiophen-2-yl)-26,27-cyclo-9,10-secocholesta-5,7,10(19),22-tetraene-1,3,24-triol

(5Z,7E,22E)-(1S,3R,24R)-25-(5-propylpyrrol-2-yl)-26,27-cyclo-9,10-secocholesta-5,7,10(19),22-tetraene-1,3,24-triol

(5Z,7E,22E)-(1S,3R,24S)-25-(5-propylpyrrol-2-yl)-26,27-cyclo-9,10-secocholesta-5,7,10(19),22-tetraene-1,3,24-triol

(5Z,7E,22E)-(1S,3R,24R)-25-(5-methylpyrrol-2-yl)-26,27-cyclo-9,10-secocholesta-5,7,10(19),22-tetraene-1,3,24-triol

(5Z,7E,22E)-(1S,3R,24S)-25-(5-methylpyrrol-2-yl)-26,27-cyclo-9,10-secocholesta-5,7,10(19),22-tetraene-1,3,24-triol

(5Z,7E,22E)-(1S,3R,24R)-25-(5-ethylpyrrol-2-yl)-26,27-cycle-9,10-secocholesta-5,7,10(19),22-tetraene-1,3,24-triol

(5Z,7E,22E)-(1S,3R,24S)-25-(5-ethylpyrrol-2-yl)-26,27-cyclo-9,10-secocholesta-5,7,10(19),22-tetraene-1,3,24-triol

(5Z,7E,22E)-(1S,3R,24R)-25-(5-butylpyrrol-2-yl)-26,27-cyclo-9,10-secocholesta-5,7,10(19),22-tetraene-1,3,24-triol

(5Z,7E,22E)-(1S,3R,24S)-25-(5-butylpyrrol-2-yl)-26,27-cyclo-9,10-secocholesta-5,7,10(19),22-tetraene-1,3,24-triol

(5Z,7E,22E)-(1S,3R,24R)-25-(5-pentylpyrrol-2-yl)-26,27-cyclo-9,10-secocholesta-5,7,10(19),22-tetraene-1,3,24-triol

(5Z,7E,22E)-(1S,3R,24S)-25-(5-pentylpyrrol-2-yl)-26,27-cyclo-9,10-secocholesta-5,7,10(19),22-tetraene-1,3,24-triol

(5Z,7E,22E)-(1S,3R,24R)-25-(4-propyloxazol-2-yl)-26,27-cyclo-9,10-secocholesta-5,7,10(19),22-tetraene-1,3,24-triol

(5Z,7E,22E)-(1S,3R,24S)-25-(4-propyloxazol-2-yl)-26,27-cyclo-9,10-secocholesta-5,7,10(19),22-tetraene-1,3,24-triol

(5Z,7E,22E)-(1S,3R,24R)-25-(4-methyloxazol-2-yl)-26,27-cyclo-9,10-secocholesta-5,7,10(19),22-tetraene-1,3,24-triol

(5Z,7E,22E)-(1S,3R,24S)-25-(4-methyloxazol-2-yl)-26,27-cyclo-9,10-secocholesta-5,7,10(19),22-tetraene-1,3,24-triol

(5Z,7E,22E)-(1S,3R,24R)-25-(4-ethyloxazol-2-yl)-26,27-cyclo-9,10-secocholesta-5,7,10(19),22-tetraene-1,3,24-triol

(5Z,7E,22E)-(1S,3R,24S)-25-(4-ethyloxazol-2-yl)-26,27-cyclo-9,10-secocholesta-5,7,10(19),22-tetraene-1,3,24-triol

(5Z,7E,22E)-(1S,3R,24R)-25-(4-butyloxazol-2-yl)-26,27-cyclo-9,10-secocholesta-5,7,10(19),22-tetraene-1,3,24-triol

(5Z,7E,22E)-(1S,3R,24S)-25-(4-butyloxazol-2-yl)-26,27-cyclo-9,10-secocholesta-5,7,10(19),22-tetraene-1,3,24-triol

(5Z,7E,22E)-(1S,3R,24R)-25-(4-pentyloxazol-2-yl)-26,27-cyclo-9,10-secocholesta-5,7,10(19),22-tetraene-1,3,24-triol

(5Z,7E,22E)-(1S,3R,24S)-25-(4-pentyloxazol-2-yl)-26,27-cyclo-9,10-secocholesta-5,7,10(19),22-tetraene-1,3,24-triol

(5Z,7E,22E)-(1S,3R,24R)-25-(4-propylthiazol-2-yl)-26,27-cyclo-9,10-secocholesta-5,7,10(19),22-tetraene-1,3,24-triol

(5Z,7E,22E)-(1S,3R,24S)-25-(4-propylthiazol-2-yl)-26,27-cyclo-9,10-secocholesta-5,7,10(19),22-tetraene-1,3,24-triol

(5Z,7E,22E)-(1S,3R,24R)-25-(4-methylthiazol-2-yl)-26,27-cyclo-9,10-secocholesta-5,7,10(19),22-tetraene-1,3,24-triol

(5Z,7E,22E)-(1S,3R,24S)-25-(4-methylthiazol-2-yl)-26,27-cyclo-9,10-secocholesta-5,7,10(19),22-tetraene-1,3,24-triol

(5Z,7E,22E)-(1S,3R,24R)-25-(4-ethylthiazol-2-yl)-26,27-cyclo-9,10-secocholesta-5,7,10(19),22-tetraene-1,3,24-triol

(5Z,7E,22E)-(1S,3R,24S)-25-(4-ethylthiazol-2-yl)-26,27-cyclo-9,10-secocholesta-5,7,10(19),22-tetraene-1,3,24-triol

(5Z,7E,22E)-(1S,3R,24R)-25-(4-butylthiazol-2-yl)-26,27-cyclo-9,10-secocholesta-5,7,10(19),22-tetraene-1,3,24-triol

(5Z,7E,22E)-(1S,3R,24S)-25-(4-butylthiazol-2-yl)-26,27-cyclo-9,10-secocholesta-5,7,10(19),22-tetraene-1,3,24-triol

(5Z,7E,22E)-(1S,3R,24R)-25-(4-pentylthiazol-2-yl)-26,27-cyclo-9,10-secocholesta-5,7,10(19),22-tetraene-1,3,24-triol

(5Z,7E,22E)-(1S,3R,24S)-25-(4-pentylthiazol-2-yl)-26,27-cyclo-9,10-secocholesta-5,7,10(19),22-tetraene-1,3,24-triol

(5Z,7E,22E)-(1S,3R,24R)-25-(4-propylimidazol-2-yl)-26,27-cyclo-9,10-secocholesta-5,7,10(19),22-tetraene-1,3,24-triol

(5Z,7E,22E)-(1S,3R,24S)-25-(4-propylimidazol-2-yl)-26,27-cyclo-9,10-secocholesta-5,7,10(19),22-tetraene-1,3,24-triol

(5Z,7E,22E)-(1S,3R,24R)-25-(4-methylimidazol-2-yl)-26,27-cyclo-9,10-secocholesta-5,7,10(19),22-tetraene-1,3,24-triol

(5Z,7E,22E)-(1S,3R,24S)-25-(4-methylimidazol-2-yl)-26,27-cyclo-9,10-secocholesta-5,7,10(19),22-tetraene-1,3,24-triol

(5Z,7E,22E)-(1S,3R,24R)-25-(4-ethylimidazol-2-yl)-26,27-cyclo-9,10-secocholesta-5,7,10(19),22-tetraene-1,3,24-triol

(5Z,7E,22E)-(1S,3R,24S)-25-(4-ethylimidazol-2-yl)-26,27-cyclo-9,10-secocholesta-5,7,10(19),22-tetraene-1,3,24-triol

(5Z,7E,22E)-(1S,3R,24R)-25-(4-butylimidazol-2-yl)-26,27-cyclo-9,10-secocholesta-5,7,10(19),22-tetraene-1,3,24-triol

(5Z,7E,22E)-(1S,3R,24S)-25-(4-butylimidazol-2-yl)-26,27-cyclo-9,10-secocholesta-5,7,10(19),22-tetraene-1,3,24-triol

(5Z,7E,22E)-(1S,3R,24R)-25-(4-pentylimidazol-2-yl)-26,27-cyclo-9,10-secocholesta-5,7,10(19),22-tetraene-1,3,24-triol

(5Z,7E,22E)-(1S,3R,24S)-25-(4-pentylimidazol-2-yl)-26,27-cyclo-9,10-secocholesta-5,7,10(19),22-tetraene-1,3,24-triol

(5Z,7E,22E)-(1S,3R,24R)-25-(4-propylfuran-2-yl)-26,27-cyclo-9,10-secocholesta-5,7,10(19),22-tetraene-1,3,24-triol

(5Z,7E,22E)-(1S,3R,24S)-25-(4-propylfuran-2-yl)-26,27-cyclo-9,10-secocholesta-5,7,10(19),22-tetraene-1,3,24-triol

(5Z,7E,22E)-(1S,3R,24R)-25-(4-methylfuran-2-yl)-26,27-cyclo-9,10-secocholesta-5,7,10(19),22-tetraene-1,3,24-triol

(5Z,7E,22E)-(1S,3R,24S)-25-(4-methylfuran-2-yl)-26,27-cyclo-9,10-secocholesta-5,7,10(19),22-tetraene-1,3,24-triol

(5Z,7E,22E)-(1S,3R,24R)-25-(4-ethylfuran-2-yl)-26,27-cyclo-9,10-secocholesta-5,7,10(19),22-tetraene-1,3,24-triol

(5Z,7E,22E)-(1S,3R,24S)-25-(4-ethylfuran-2-yl)-26,27-cyclo-9,10-secocholesta-5,7,10(19),22-tetraene-1,3,24-triol

(5Z,7E,22E)-(1S,3R,24R)-25-(4-butylfuran-2-yl)-26,27-cyclo-9,10-secocholesta-5,7,10(19),22-tetraene-1,3,24-triol

(5Z,7E,22E)-(1S,3R,24S)-25-(4-butylfuran-2-yl)-26,27-cyclo-9,10-secocholesta-5,7,10(19),22-tetraene-1,3,24-triol

(5Z,7E,22E)-(1S,3R,24R)-25-(4-pentylfuran-2-yl)-26,27-cyclo-9,10-secocholesta-5,7,10(19),22-tetraene-1,3,24-triol

(5Z,7E,22E)-(1S,3R,24S)-25-(4-pentylfuran-2-yl)-26,27-cyclo-9,10-secocholesta-5,7,10(19),22-tetraene-1,3,24-triol

(5Z,7E,22E)-(1S,3R,24R)-25-(4-propylthiophen-2-yl)-26,27-cycle-9,10-secocholesta-5,7,10(19),22-tetraene-1,3,24-triol

(5Z,7E,22E)-(1S,3R,24S)-25-(4-propylthiophen-2-yl)-26,27-cyclo-9,10-secocholesta-5,7,10(19),22-tetraene-1,3,24-triol

(5Z,7E,22E)-(1S,3R,24R)-25-(4-methylthiophen-2-yl)-26,27-cyclo-9,10-secocholesta-5,7,10(19),22-tetraene-1,3,24-triol

(5Z,7E,22E)-(1S,3R,24S)-25-(4-methylthiophen-2-yl)-26,27-cyclo-9,10-secocholesta-5,7,10(19),22-tetraene-1,3,24-triol

(5Z,7E,22E)-(1S,3R,24R)-25-(4-ethylthiophen-2-yl)-26,27-cyclo-9,10-secocholesta-5,7,10(19),22-tetraene-1,3,24-triol

(5Z,7E,22E)-(1S,3R,24S)-25-(4-ethylthiophen-2-yl)-26,27-cyclo-9,10-secocholesta-5,7,10(19),22-tetraene-1,3,24-triol

(5Z,7E,22E)-(1S,3R,24R)-25-(4-butylthiophen-2-yl)-26,27-cyclo-9,10-secocholesta-5,7,10(19),22-tetraene-1,3,24-triol

(5Z,7E,22E)-(1S,3R,24S)-25-(4-butylthiophen-2-yl)-26,27-cyclo-9,10-secocholesta-5,7,10(19),22-tetraene-1,3,24-triol

(5Z,7E,22E)-(1S,3R,24R)-25-(4-pentylthiophen-2-yl)-26,27-cyclo-9,10-secocholesta-5,7,10(19),22-tetraene-1,3,24-triol

(5Z,7E,22E)-(1S,3R,24S)-25-(4-pentylthiophen-2-yl)-26,27-cyclo-9,10-secocholesta-5,7,10(19),22-tetraene-1,3,24-triol

(5Z,7E,22E)-(1S,3R,24R)-25-(4-propylpyrrol-2-yl)-26,27-cyclo-9,10-secocholesta-5,7,10(19),22-tetraene-1,3,24-triol

(5Z,7E,22E)-(1S,3R,24S)-25-(4-propylpyrrol-2-yl)-26,27-cyclo-9,10-secocholesta-5,7,10(19),22-tetraene-1,3,24-triol

(5Z,7E,22E)-(1S,3R,24R)-25-(4-methylpyrrol-2-yl)-26,27-cyclo-9,10-secocholesta-5,7,10(19),22-tetraene-1,3,24-triol

(5Z,7E,22E)-(1S,3R,24S)-25-(4-methylpyrrol-2-yl)-26,27-cyclo-9,10-secocholesta-5,7,10 (19),22-tetraene-1,3,24-triol

(5Z,7E,22E)-(1S,3R,24R)-25-(4-ethylpyrrol-2-yl)-26,27-cyclo-9,10-secocholesta-5,7,10(19),22-tetraene-1,3,24-triol

(5Z,7E,22E)-(1S,3R,24S)-25-(4-ethylpyrrol-2-yl)-26,27-cyclo-9,10-secocholesta-5,7,10(19),22-tetraene-1,3,24-triol

(5Z,7E,22E)-(1S,3R,24R)-25-(4-butylpyrrol-2-yl)-26,27-cyclo-9,10-secocholesta-5,7,10(19),22-tetraene-1,3,24-triol

(5Z,7E,22E)-(1S,3R,24S)-25-(4-butylpyrrol-2-yl)-26,27-cyclo-9,10-secocholesta-5,7,10(19),22-tetraene-1,3,24-triol

(5Z,7E,22E)-(1S,3R,24R)-25-(4-pentylpyrrol-2-yl)-26,27-cyclo-9,10-secocholesta-5,7,10(19),22-tetraene-1,3,24-triol

(5Z,7E,22E)-(1S,3R,24S)-25-(4-pentylpyrrol-2-yl)-26,27-cyclo-9,10-secocholesta-5,7,10(19),22-tetraene-1,3,24-triol

(5Z,7E,22E)-(1S,3R,24R)-25-(3-methyl-1,2,4-oxadiazol-5-yl)-26,27-cyclo-9,10-secocholesta-5,7,10(19), 22-tetraene-1,3,24-triol

(5Z,7E,22E)-(1S,3R,24S)-25-(3-methyl-1,2,4-oxadiazol-5-yl)-26,27-cyclo-9,10-secocholesta-5,7,10(19), 22-tetraene-1,3,24-triol

(5Z,7E,22E)-(1S,3R,24R)-25-(3-ethyl-1,2,4-oxadiazol-5-yl)-26,27-cyclo-9,10-secocholesta-5,7,10(19), 22-tetraene-1,3,24-triol

(5Z,7E,22E)-(1S,3R,24S)-25-(3-ethyl-1,2,4-oxadiazol-5-yl)-26,27-cyclo-9,10-secocholesta-5,7,10(19), 22-tetraene-1,3,24-triol

(5Z,7E,22E)-(1S,3R,24R)-25-(3-propyl-1,2,4-oxadiazol-5-yl)-26,27-cyclo-9,10-secocholesta-5,7,10(19), 22-tetraene-1,3,24-triol

(5Z,7E,22E)-(1S,3R,24S)-25-(3-propyl-1,2,4-oxadiazol-5-yl)-26,27-cyclo-9,10-secocholesta-5,7,10(19), 22-tetraene-1,3,24-triol

(5Z,7E,22E)-(1S,3R,24R)-25-(3-butyl-1,2,4-oxadiazol-5-yl)-26,27-cyclo-9,10-secocholesta-5,7,10(19), 22-tetraene-1,3,24-triol

(5Z,7E,22E)-(1S,3R,24S)-25-(3-butyl-1,2,4-oxadiazol-5-yl)-26,27-cyclo-9,10-secocholesta-5,7,10(19), 22-tetraene-1,3,24-triol

(5Z,7E,22E)-(1S,3R,24R)-25-(3-pentyl-1,2,4-oxadiazol-5-yl)-26,27-cyclo-9,10-secocholesta-5,7,10(19), 22-tetraene-1,3,24-triol

(5Z,7E,22E)-(1S,3R,24S)-25-(3-pentyl-1,2,4-oxadiazol-5-yl)-26,27-cyclo-9,10-secocholesta-5,7,10(19), 22-tetraene-1,3,24-triol

(5Z,7E,22E)-(1S,3R,24R)-25-(5-methyl-1,3,4-oxadiazol-2-yl)-26,27-cyclo-9,10-secocholesta-5,7,10(19), 22-tetraene-1,3,24-triol

(5Z,7E,22E)-(1S,3R,24S)-25-(5-methyl-1,3,4-oxadiazol-2-yl)-26,27-cyclo-9,10-secocholesta-5,7,10(19), 22-tetraene-1,3,24-triol

(5Z,7E,22E)-(1S,3R,24R)-25-(5-ethyl-1,3,4-oxadiazol-2-yl)-26,27-cyclo-9,10-secocholesta-5,7,10(19), 22-tetraene-1,3,24-triol

(5Z,7E,22E)-(1S,3R,24S)-25-(5-ethyl-1,3,4-oxadiazol-2-yl)-26,27-cyclo-9,10-secocholesta-5,7,10(19), 22-tetraene-1,3,24-triol

(5Z,7E,22E)-(1S,3R,24R)-25-(5-propyl-1,3,4-oxadiazol-2-yl)-26,27-cyclo-9,10-secocholesta-5,7,10(19), 22-tetraene-1,3,24-triol

(5Z,7E,22E)-(1S,3R,24S)-25-(5-propyl-1,3,4-oxadiazol-2-yl)-26,27-cyclo-9,10-secocholesta-5,7,10(19), 22-tetraene-1,3,24-triol

(5Z,7E,22E)-(1S,3R,24R)-25-(5-butyl-1,3,4-oxadiazol-2-yl)-26,27-cyclo-9,10-secocholesta-5,7,10(19), 22-tetraene-1,3,24-triol

(5Z,7E,22E)-(1S,3R,24S)-25-(5-butyl-1,3,4-oxadiazol-2-yl)-26,27-cyclo-9,10-secocholesta-5,7,10(19), 22-tetraene-1,3,24-triol

(5Z,7E,22E)-(1S,3R,24R)-25-(5-pentyl-1,3,4-oxadiazol-2-yl)-26,27-cyclo-9,10-secocholesta-5,7,10(19),22-tetraene-1,3,24-triol

(5Z,7E,22E)-(1S,3R,24S)-25-(5-pentyl-1,3,4-oxadiazol-2-yl)-26,27-cyclo-9,10-secocholesta-5,7,10(19), 22-tetraene-1,3,24-triol

(5Z,7E,22E)-(1S,3R,24R)-25-phenyl-26,27-cyclo-9,10-secocholesta-5,7,10(19),22-tetraene-1,3,24-triol

(5Z,7E,22E)-(1S,3R,24S)-25-phenyl-26,27-cyclo-9,10-secocholesta-5,7,10(19),22-tetraene-1,3,24-triol

(5Z,7E,22E)-(1S,3R,24R)-25-(4-methylphenyl)-26,27-cyclo-9,10-secocholesta-5,7,10(19),22-tetraene-1,3,24-triol

(5Z,7E,22E)-(1S,3R,24S)-25-(4-methylphenyl)-26,27-cyclo-9,10-secocholesta-5,7,10(19),22-tetraene-1,3,24-triol

(5Z,7E,22E)-(1S,3R,24R)-25-(4-ethylphenyl)-26,27-cyclo-9,10-secocholesta-5,7,10(19),22-tetraene-1,3,24-triol

(5Z,7E,22E)-(1S,3R,24S)-25-(4-ethylphenyl)-26,27-cyclo-9,10-secocholesta-5,7,10(19),22-tetraene-1,3,24-triol

(5Z,7E,22E)-(1S,3R,24R)-25-(4-propylphenyl)-26,27-cyclo-9,10-secocholesta-5,7,10(19),22-tetraene-1,3,24-triol

(5Z,7E,22E)-(1S,3R,24S)-25-(4-propylphenyl)-26,27-cyclo-9,10-secocholesta-5,7,10(19),22-tetraene-1,3,24-triol

(5Z,7E,22E)-(1S,3R,24R)-25-(4-butylphenyl)-26,27-cyclo-9,10-secocholesta-5,7,10(19),22-tetraene-1,3,24-triol

(5Z,7E,22E)-(1S,3R,24S)-25-(4-butylphenyl)-26,27-cyclo-9,10-secocholesta-5,7,10(19),22-tetraene-1,3,24-triol

(5Z,7E,22E)-(1S,3R,24R)-25-(4-pentylphenyl)-26,27-cyclo-9,10-secocholesta-5,7,10(19),22-tetraene-1,3,24-triol

(5Z,7E,22E)-(1S,3R,24S)-25-(4-pentylphenyl)-26,27-cyclo-9,10-secocholesta-5,7,10(19),22-tetraene-1,3,24-triol

(5Z,7E,22E)-(1S,3R,24R)-25-(3-methylphenyl)-26,27-cyclo-9,10-secocholesta-5,7,10(19),22-tetraene-1,3,24-triol

(5Z,7E,22E)-(1S,3R,24S)-25-(3-methylphenyl)-26,27-cyclo-9,10-secocholesta-5,7,10(19),22-tetraene-1,3,24-triol

(5Z,7E,22E)-(1S,3R,24R)-25-(3-ethylphenyl)-26,27-cyclo-9,10-secocholesta-5,7,10(19),22-tetraene-1,3,24-triol

(5Z,7E,22E)-(1S,3R,24S)-25-(3-ethylphenyl)-26,27-cyclo-9,10-secocholesta-5,7,10(19),22-tetraene-1,3,24-triol

(5Z,7E,22E)-(1S,3R,24R)-25-(3-propylphenyl)-26,27-cyclo-9,10-secocholesta-5,7,10(19),22-tetraene-1,3,24-triol

(5Z,7E,22E)-(1S,3R,24S)-25-(3-propylphenyl)-26,27-cyclo-9,10-secocholesta-5,7,10(19),22-tetraene-1,3,24-triol

(5Z,7E,22E)-(1S,3R,24R)-25-(3-butylphenyl)-26,27-cyclo-9,10-secocholesta-5,7,10(19),22-tetraene-1,3,24-triol

(5Z,7E,22E)-(1S,3R,24S)-25-(3-butylphenyl)-26,27-cyclo-9,10-secocholesta-5,7,10(19),22-tetraene-1,3,24-triol

(5Z,7E,22E)-(1S,3R,24R)-25-(3-pentylphenyl)-26,27-cyclo-9,10-secocholesta-5,7,10(19),22-tetraene-1,3,24-triol

(5Z,7E,22E)-(1S,3R,24S)-25-(3-pentylphenyl)-26,27-cyclo-9,10-secocholesta-5,7,10(19),22-tetraene-1,3,24-triol

(5Z,7E,22E)-(1S,3R,24R)-25-[4-(1-methylethyl)phenyl)-26,27-cyclo-9,10-secocholesta-5,7,10(19), 22-tetraene-1,3,24-triol

(5Z,7E,22E)-(1S,3R,24S)-25-[4-(1-methylethyl)phenyl)-26,27-cyclo-9,10-secocholesta-5,7,10(19), 22-tetraene-1,3,24-triol

(5Z,7E,22E)-(1S,3R,24R)-25-[3-(1-methylethyl)phenyl)-26,27-cyclo-9,10-secocholesta-5,7,10(19), 22-tetraene-1,3,24-triol

(5Z,7E,22E)-(1S,3R,24S)-25-[3-(1-methylethyl)phenyl)-26,27-cyclo-9,10-secocholesta-5,7,10(19), 22-tetraene-1,3,24-triol

(5Z,7E,22E)-(1S,3R,24R)-25-(2-pyridyl)-26,27-cyclo-9,10-secocholesta-5,7,10(19),22-tetraene-1,3,24-triol

(5Z,7E,22E)-(1S,3R,24S)-25-(2-pyridyl)-26,27-cyclo-9,10-secocholesta-5,7,10(19),22-tetraene-1,3,24-triol

(5Z,7E,22E)-(1S,3R,24R)-25-(6-methyl-2-pyridyl)-26,27-cyclo-9,10-secocholesta-5,7,10(19),22-tetraene-1,3,24-triol

(5Z,7E,22E)-(1S,3R,24S)-25-(6-methyl-2-pyridyl)-26,27-cyclo-9,10-secocholesta-5,7,10(19),22-tetraene-1,3,24-triol

(5Z,7E,22E)-(1S,3R,24R)-25-(5-methyl-2-pyridyl)-26,27-cyclo-9,10-secocholesta-5,7,10(19),22-tetraene-1,3,24-triol

(5Z,7E,22E)-(1S,3R,24S)-25-(5-methyl-2-pyridyl)-26,27-cyclo-9,10-secocholesta-5,7, 10(19),22-tetraene-1,3,24-triol

(5Z,7E,22E)-(1S,3R,24R)-25-(4-methyl-2-pyridyl)-26,27-cyclo-9,10-secocholesta-5,7,10(19),22-tetraene-1,3,24-triol

(5Z,7E,22E)-(1S,3R,24S)-25-(4-methyl-2-pyridyl)-26,27-cyclo-9,10-secocholesta-5,7,10(19),22-tetraene-1,3,24-triol

(5Z,7E,22E)-(1S,3R,24R)-25-(6-ethyl-2-pyridyl)-26,27-cyclo-9,10-secocholesta-5,7,10(19),22-tetraene-1,3,24-triol

(5Z,7E,22E)-(1S,3R,24S)-25-(6-ethyl-2-pyridyl)-26,27-cyclo-9,10-secocholesta-5,7,10(19),22-tetraene-1,3,24-triol

(5Z,7E,22E)-(1S,3R,24R)-25-(5-ethyl-2-pyridyl)-26,27-cyclo-9,10-secocholesta-5,7,10(19),22-tetraene-1,3,24-triol

(5Z,7E,22E)-(1S,3R,24S)-25-(5-ethyl-2-pyridyl)-26,27-cyclo-9,10-secocholesta-5,7,10(19),22-tetraene-1,3,24-triol

(5Z,7E,22E)-(1S,3R,24R)-25-(4-ethyl-2-pyridyl)-26,27-cyclo-9,10-secocholesta-5,7,10(19),22-tetraene-1,3,24-triol

(5Z,7E,22E)-(1S,3R,24S)-25-(4-ethyl-2-pyridyl)-26,27-cyclo-9,10-secocholesta-5,7,10(19),22-tetraene-1,3,24-triol

(5Z,7E,22E)-(1S,3R,24R)-25-(6-propyl-2-pyridyl)-26,27-cyclo-9,10-secocholesta-5,7,10(19),22-tetraene-1,3,24-triol

(5Z,7E,22E)-(1S,3R,24S)-25-(6-propyl-2-pyridyl)-26,27-cyclo-9,10-secocholesta-5,7,10(19),22-tetraene-1,3,24-triol

(5Z,7E,22E)-(1S,3R,24R)-25-(5-propyl-2-pyridyl)-26,27-cyclo-9,10-secocholesta-5,7,10(19),22-tetraene-1,3,24-triol

(5Z,7E,22E)-(1S,3R,24S)-25-(5-propyl-2-pyridyl)-26,27-cyclo-9,10-secocholesta-5,7,10(19),22-tetraene-1,3,24-triol

(5Z,7E,22E)-(1S,3R,24R)-25(4-propyl-2-pyridyl)-26,27-cyclo-9,10-secocholesta-5,7,10(19),22-tetraene-1,3,24-triol

(52,7E,22E)-(1S,3R,24S)-25-(4-propyl-2-pyridyl)-26,27-cyclo-9,10-secocholesta-5,7,10(19),22-tetraene-1,3,24-triol

(5Z,7E,22E)-(1S,3R,24R)-25-(6-butyl-2-pyridyl)-26,27-cyclo-9,10-secocholesta-5,7,10(19),22-tetraene-1,3,24-triol

(5Z,7E,22E)-(1S,3R,24S)-25-(6-butyl-2-pyridyl)-26,27-cyclo-9,10-secocholesta-5,7,10(19),22-tetraene-1,3,24-triol

(5Z,7E,22E)-(1S,3R,24R)-25-(5-butyl-2-pyridyl)-26,27-cyclo-9,10-secocholesta-5,7,10(19),22-tetraene-1,3,24-triol

(5Z,7E,22E)-(1S,3R,24S)-25-(5-butyl-2-pyridyl)-26,27-cyclo-9,10-secocholesta-5,7,10(19),22-tetraene-1,3,24-triol

(5Z,7E,22E)-(1S,3R,24R)-25-(4-butyl-2-pyridyl)-26,27-cyclo-9,10-secocholesta-5,7,10(19),22-tetraene-1,3,24-triol

(5Z,7E,22E)-(1S,3R,24S)-25-(4-butyl-2-pyridyl)-26,27-cyclo-9,10-secocholesta-5,7,10(19),22-tetraene-1,3,24-triol

(5Z,7E,22E)-(1S,3R,24R)-25-(5,5-dimethyl-2-oxazolin-2-yl)-26,27-cyclo-9,10-secocholesta-5,7,10(19), 22-tetraene-1,3,24-triol

(5Z,7E,22E)-(1S,3R,24S)-25-(5,5-dimethyl-2-oxazolin-2-yl)-26,27-cyclo-9,10-secocholesta-5,7,10(19), 22-tetraene-1,3,24-triol

(5Z,7E,22E)-(1S,3R,24R)-25-(5,5-diethyl-2-oxazolin-2-yl)-26,27-cyclo-9,10-secocholesta-5,7,10(19), 22-tetraene-1,3,24-triol

(5Z,7E,22E)-(1S,3R,24S)-25-(5,5-diethyl-2-oxazolin-2-yl)-26,27-cyclo-9,10-secocholesta-5,7,10(19), 22-tetraene-1,3,24-triol

(5Z,7E,22E)-[1S,3R,24R,25(R)]-25-(5-methyl-2-oxazolin-2-yl)-26,27-cyclo-9,10-secocholesta-5,7,10(19), 22-tetraene-1,3,24-triol

(5Z,7E,22E)-[1S,3R,24S,25(R)]-25-(5-methyl-2-oxazolin-2-yl)-26,27-cyclo-9,10-secocholesta-5,7,10(19), 22-tetraene-1,3,24-triol

(5Z,7E,22E)-[1S,3R,24R,25(S)]-25-(5-methyl-2-oxazolin-2-yl)-26,27-cyclo-9,10-secocholesta-5,7,10(19),22-tetraene-1,3,24-triol

(5Z,7E,22E)-[1S,3R,24S,25(S)]-25-(5-methyl-2-oxazolin-2-yl)-26,27-cyclo-9,10-secocholesta-5,7,10(19), 22-tetraene-1,3,24-triol

(5Z,7E,22E)-[1S,3R,24R,25(R)]-25-(5-ethyl-2-oxazolin-2-yl)-26,27-cyclo-9,10-secocholesta-5,7,10(19), 22-tetraene-1,3,24-triol

(5Z,7E,22E)-(1S,3R,24S,25(R)]-25-(5-ethyl-2-oxazolin-2-yl)-26,27-cyclo-9,10-secocholesta-5,7,10(19), 22-tetraene-1,3,24-triol

(5Z,7E,22E)-[1S,3R,24R,25(S)]-25-(5-ethyl-2-oxazolin-2-yl)-26,27-cyclo-9,10-secocholesta-5,7,10(19), 22-tetraene-1,3,24-triol

(5Z,7E,22E)-[1S,3R,24S,25(S)]-25-(5-ethyl-2-oxazolin-2-yl)-26,27-cyclo-9,10-secocholesta-5,7,10(19), 22-tetraene-1,3,24-triol

(5Z,7E,22E)-[1S,3R,24R,25(R)]-25-(5-propyl-2-oxazolin-2-yl)-26,27-cyclo-9,10-secocholesta-5,7,10(19), 22-tetraene-1,3,24-triol

(5Z,7E,22E)-[1S,3R,24S,25(R)]-25-(5-propyl-2-oxazolin-2-yl)-26,27-cyclo-9,10-secocholesta-5,7,10(19), 22-tetraene-1,3,24-triol

(5Z,7E,22E)-[1S,3R,24R,25(S)]-25-(5-propyl-2-oxazolin-2-yl)-26,27-cyclo-9,10-secocholesta-5,7,10(19), 22-tetraene-1,3,24-triol

(5Z,7E,22E)-[1S,3R,24S,25(S)]-25-(5-propyl-2-oxazolin-2-yl)-26,27-cyclo-9,10-secocholesta-5,7,10(19), 22-tetraene-1,3,24-triol

(5Z,7E,22E)-[1S,3R,24R,25(R)]-25-(5-butyl-2-oxazolin-2-yl)-26,27-cyclo-9,10-secocholesta-5,7,10(19), 22-tetraene-1,3,24-triol

(5Z,7E,22E)-[1S,3R,24S,25(R)]-25-(5-butyl-2-oxazolin-2-yl)-26,27-cyclo-9,10-secocholesta-5,7,10(19), 22-tetraene-1,3,24-triol

(5Z,7E,22E)-[1S,3R,24R,25(S)]-25-(5-butyl-2-oxazolin-2-yl)-26,27-cyclo-9,10-secocholesta-5,7,10(19), 22-tetraene-1,3,24-triol

(5Z,7E,22E)-[1S,3R,24S,25(S)]-25-(5-butyl-2-oxazolin-2-yl)-26,27-cyclo-9,10-secocholesta-5,7,10(19), 22-tetraene-1,3,24-triol

(5Z,7E,22E)-[1S,3R,24R,25(R)]-25-(5-phenyl-2-oxazolin-2-yl)-26,27-cyclo-9,10-secocholesta-5,7,10(19), 22-tetraene-1,3,24-triol

(5Z,7E,22E)-[1S,3R,24S,25(R)]-25-(5-phenyl-2-oxazolin-2-yl)-26,27-cyclo-9,10-secocholesta-5,7,10(19), 22-tetraene-1,3,24-triol

(5Z,7E,22E)-[1S,3R,24R,25(S)]-25-(5-phenyl-2-oxazolin-2-yl)-26,27-cyclo-9,10-secocholesta-5,7,10(19), 22-tetraene-1,3,24-triol

(5Z,7E,22E)-[1S,3R,24S,25(S)]-25-(5-phenyl-2-oxazolin-2-yl)-26,27-cyclo-9,10-secocholesta-5,7,10(19), 22-tetraene-1,3,24-triol

(5Z,7E,22E)-(1S,3R,20S,24R)-25-(5-propyloxazol-2-yl)-26,27-cyclo-9,10-secocholesta-5,7,10(19), 22-tetraene-1,3,24-triol

(5Z,7E,22E)-(1S,3R,20S,24S)-25-(5-propyloxazol-2-yl)-26,27-cyclo-9,10-secocholesta-5,7,10(19), 22-tetraene-1,3,24-triol

(5Z,7E,22E)-(1S,3R,20S,24R)-25-(5-methyloxazol-2-yl)-26,27-cyclo-9,10-secocholesta-5,7,10(19), 22-tetraene-1,3,24-triol

(5Z,7E,22E)-(1S,3R,20S,24S)-25-(5-methyloxazol-2-yl)-26,27-cyclo-9,10-secocholesta-5,7,10(19), 22-tetraene-1,3,24-triol

(5Z,7E,22E)-(1S,3R,20S,24R)-25-(5-ethyloxazol-2-yl)-26,27-cyclo-9,10-secocholesta-5,7,10(19), 22-tetraene-1,3,24-triol

(5Z,7E,22E)-(1S,3R,20S,24S)-25-(5-ethyloxazol-2-yl)-26,27-cyclo-9,10-secocholesta-5,7,10(19), 22-tetraene-1,3,24-triol

(5Z,7E,22E)-(1S,3R,20S,24R)-25-(5-butyloxazol-2-yl)-26,27-cyclo-9,10-secocholesta-5,7,10(19), 22-tetraene-1,3,24-triol

(5Z,7E,22E)-(1S,3R,20S,24S)-25-(5-butyloxazol-2-yl)-26,27-cyclo-9,10-secocholesta-5,7,10(19), 22-tetraene-1,3,24-triol

(5Z,7E,22E)-(1S,3R,20S,24R)-25-(5-pentyloxazol-2-yl)-26,27-cyclo-9,10-secocholesta-5,7,10(19), 22-tetraene-1,3,24-triol

(5Z,7E,22E)-(1S,3R,20S,24S)-25-(5-pentyloxazol-2-yl)-26,27-cyclo-9,10-secocholesta-5,7,10(19), 22-tetraene-1,3,24-triol

(5Z,7E,22E)-(1S,3R,20S,24R)-25-(5-propylthiazol-2-yl)-26,27-cyclo-9,10-secocholesta-5,7,10(19), 22-tetraene-1,3,24-triol

(5Z,7E,22E)-(1S,3R,20S,24S)-25-(5-propylthiazol-2-yl)-26,27-cyclo-9,10-secocholesta-5,7,10(19),22-tetraene-1,3,24-triol

(5Z,7E,22E)-(1S,3R,20S,24R)-25-(5-methylthiazol-2-yl)-26,27-cycle-9,10-secocholesta-5,7,10(19), 22-tetraene-1,3,24-triol

(5Z,7E,22E)-(1S,3R,20S,24S)-25-(5-methylthiazol-2-yl)-26,27-cyclo-9,10-secocholesta-5,7,10(19), 22-tetraene-1,3,24-triol

(5Z,7E,22E)-(1S,3R,20S,24R)-25-(5-ethylthiazol-2-yl)-26,27-cyclo-9,10-secocholesta-5,7,10(19), 22-tetraene-1,3,24-triol

(5Z,7E,22E)-(1S,3R,20S,24S)-25-(5-ethylthiazol-2-yl)-26,27-cyclo-9,10-secocholesta-5,7,10(19), 22-tetraene-1,3,24-triol

(5Z,7E,22E)-(1S,3R,20S,24R)-25-(5-butylthiazol-2-yl)-26,27-cyclo-9,10-secocholesta-5,7,10(19), 22-tetraene-1,3,24-triol

(5Z,7E,22E)-(1S,3R,20S,24S)-25-(5-butylthiazol-2-yl)-26,27-cyclo-9,10-secocholesta-5,7,10(19), 22-tetraene-1,3,24-triol

(52,7E,22E)-(1S,3R,20S,24R)-25-(5-pentylthiazol-2-yl)-26,27-cyclo-9,10-secocholesta-5,7,10(19), 22-tetraene-1,3,24-triol

(5Z,7E,22E)-(1S,3R,20S,24S)-25-(5-pentylthiazol-2-yl)-26,27-cyclo-9,10-secocholesta-5,7,10(19), 22-tetraene-1,3,24-triol

(5Z,7E,22E)-(1S,3R,20S,24R)-25-(4-propyloxazol-2-yl)-26,27-cyclo-9,10-secocholesta-5,7,10(19), 22-tetraene-1,3,24-triol

(5Z,7E,22E)-(1S,3R,20S,24S)-25-(4-propyloxazol-2-yl)-26,27-cyclo-9,10-secocholesta-5,7,10(19), 22-tetraene-1,3,24-triol

(5Z,7E,22E)-(1S,3R,20S,24R)-25-(4-methyloxazol-2-yl)-26,27-cyclo-9,10-secocholesta-5,7,10(19), 22-tetraene-1,3,24-triol

(5Z,7E,22E)-(1S,3R,20S,24S)-25-(4-methyloxazol-2-yl)-26,27-cyclo-9,10-secocholesta-5,7,10(19), 22-tetraene-1,3,24-triol

(5Z,7E,22E)-(1S,3R,20S,24R)-25-(4-ethyloxazol-2-yl)-26,27-cyclo-9,10-secocholesta-5,7,10(19), 22-tetraene-1,3,24-triol

(5Z,7E,22E)-(1S,3R,20S,24S)-25-(4-ethyloxazol-2-yl)-26,27-cyclo-9,10-secocholesta-5,7,10(19), 22-tetraene-1,3,24-triol

(5Z,7E,22E)-(1S,3R,20S,24R)-25-(4-butyloxazol-2-yl)-26,27-cyclo-9,10-secocholesta-5,7,10(19), 22-tetraene-1,3,24-triol

(5Z,7E,22E)-(1S,3R,20S,24S)-25-(4-butyloxazol-2-yl)-26,27-cyclo-9,10-secocholesta-5,7,10(19), 22-tetraene-1,3,24-triol

(5Z,7E,22E)-(1S,3R,20S,24R)-25-(4-pentyloxazol-2-yl)-26,27-cyclo-9,10-secocholesta-5,7,10(19), 22-tetraene-1,3,24-triol

(5Z,7E,22E)-(1S,3R,20S,24S)-25-(4-pentyloxazol-2-yl)-26,27-cyclo-9,10-secocholesta-5,7,10(19), 22-tetraene-1,3,24-triol

(5Z,7E,22E)-(1S,3R,20S,24R)-25-(4-propylthiazol-2-yl)-26,27-cyclo-9,10-secocholesta-5,7,10(19), 22-tetraene-1,3,24-triol

(5Z,7E,22E)-(1S,3R,20S,24S)-25-(4-propylthiazol-2-yl)-26,27-cyclo-9,10-secocholesta-5,7,10(19), 22-tetraene-1,3,24-triol

(5Z,7E,22E)-(1S,3R,20S,24R)-25-(4-methylthiazol-2-yl)-26,27-cyclo-9,10-secocholesta-5,7,10(19), 22-tetraene-1,3,24-triol

(5Z,7E,22E)-(1S,3R,20S,24S)-25-(4-methylthiazol-2-yl)-26,27-cyclo-9,10-secocholesta-5,7,10(19), 22-tetraene-1,3,24-triol

(5Z,7E,22E)-(1S,3R,20S,24R)-25-(4-ethylthiazol-2-yl)-26,27-cyclo-9,10-secocholesta-5,7,10(19), 22-tetraene-1,3,24-triol

(5Z,7E,22E)-(1S,3R,20S,24S)-25-(4-ethylthiazol-2-yl)-26,27-cyclo-9,10-secocholesta-5,7,10(19), 22-tetraene-1,3,24-triol

(5Z,7E,22E)-(1S,3R,20S,24R)-25-(4-butylthiazol-2-yl)-26,27-cyclo-9,10-secocholesta-5,7,10(19), 22-tetraene-1,3,24-triol

(5Z,7E,22E)-(1S,3R,20S,24S)-25-(4-butylthiazol-2-yl)-26,27-cyclo-9,10-secocholesta-5,7,10(19), 22-tetraene-1,3,24-triol

(5Z,7E,22E)-(1S,3R,20S,24R)-25-(4-pentylthiazol-2-yl)-26,27-cyclo-9,10-secocholesta-5,7,10(19), 22-tetraene-1,3,24-triol

(5Z,7E,22E)-(1S,3R,20S,24S)-25-(4-pentylthiazol-2-yl)-26,27-cyclo-9,10-secocholesta-5,7,10(19), 22-tetraene-1,3,24-triol

(5Z,7E,22E)-(1S,3R,24R)-20-methyl-25-(5-propyloxazol-2-yl)-26,27-cyclo-9,10-secocholesta-5,7,10(19),22-tetraene-1,3,24-triol

(5Z,7E,22E)-(1S,3R,24S)-20-methyl-25-(5-propyloxazol-2-yl)-26,27-cyclo-9,10-secocholesta-5,7,10(19), 22-tetraene-1,3,24-triol

(5Z,7E,22E)-(1S,3R,24R)-20-methyl-25-(5-methyloxazol-2-yl)-26,27-cyclo-9,10-secocholesta-5,7,10(19), 22-tetraene-1,3,24-triol

(5Z,7E,22E)-(1S,3R,24S)-20-methyl-25-(5-methyloxazol-2-yl)-26,27-cyclo-9,10-secocholesta-5,7,10(19), 22-tetraene-1,3,24-triol

(5Z,7E,22E)-(1S,3R,24R)-25-(5-ethyloxazol-2-yl)-20-methyl-26,27-cyclo-9,10-secocholesta-5,7,10(19), 22-tetraene-1,3,24-triol

(5Z,7E,22E)-(1S,3R,24S)-25-(5-ethyloxazol-2-yl)-20-methyl-26,27-cyclo-9,10-secocholesta-5,7,10(19), 22-tetraene-1,3,24-triol

(5Z,7E,22E)-(1S,3R,24R)-25-(5-butyloxazol-2-yl)-20-methyl-26,27-cyclo-9,10-secocholesta-5,7,10(19), 22-tetraene-1,3,24-triol

(5Z,7E,22E)-(1S,3R,24S)-25-(5-butyloxazol-2-yl)-20-methyl-26,27-cyclo-9,10-secocholesta-5,7,10(19), 22-tetraene-1,3,24-triol

(5Z,7E,22E)-(1S,3R,24R)-20-methyl-25-(5-pentyloxazol-2-yl)-26,27-cyclo-9,10-secocholesta-5,7,10(19), 22-tetraene-1,3,24-triol

(5Z,7E,22E)-(1S,3R,24S)-20-methyl-25-(5-pentyloxazol-2-yl)-26,27-cyclo-9,10-secocholesta-5,7,10(19), 22-tetraene-1,3,24-triol

(5Z,7E,22E)-(1S,3R,24R)-20-methyl-25-(5-propylthiazol-2-yl)-26,27-cyclo-9,10-secocholesta-5,7,10(19), 22-tetraene-1,3,24-triol

(5Z,7E,22E)-(1S,3R,24S)-20-methyl-25-(5-propylthiazol-2-yl)-26,27-cyclo-9,10-secocholesta-5,7,10(19), 22-tetraene-1,3,24-triol

(5Z,7E,22E)-(1S,3R,24R)-20-methyl-25-(5-methylthiazol-2-yl)-26,27-cyclo-9,10-secocholesta-5,7,10(19), 22-tetraene-1,3,24-triol

(5Z,7E,22E)-(1S,3R,24S)-20-methyl-25-(5-methylthiazol-2-yl)-26,27-cyclo-9,10-secocholesta-5,7,10(19), 22-tetraene-1,3,24-triol

(5Z,7E,22E)-(1S,3R,24R)-25-(5-ethylthiazol-2-yl)-20-methyl-26,27-cyclo-9,10-secocholesta-5,7,10(19), 22-tetraene-1,3,24-triol

(5Z,7E,22E)-(1S,3R,24S)-25-(5-ethylthiazol-2-yl)-20-methyl-26,27-cyclo-9,10-secocholesta-5,7,10(19), 22-tetraene-1,3,24-triol

(5Z,7E,22E)-(1S,3R,24R)-25-(5-butylthiazol-2-yl)-20-methyl-26,27-cyclo-9,10-secocholesta-5,7,10(19), 22-tetraene-1,3,24-triol

(5Z,7E,22E)-(1S,3R,24S)-25-(5-butylthiazol-2-yl)-20-methyl-26,27-cyclo-9,10-secocholesta-5,7,10(19), 22-tetraene-1,3,24-triol

(5Z,7E,22E)-(1S,3R,24R)-25-(5-pentylthiazol-2-yl)-20-methyl-26,27-cyclo-9,10-secocholesta-5,7,10(19), 22-tetraene-1,3,24-triol

(5Z,7E,22E)-(1S,3R,24S)-25-(5-pentylthiazol-2-yl)-20-methyl-26,27-cyclo-9,10-secocholesta-5,7,10(19), 22-tetraene-1,3,24-triol

(5Z,7E,22E)-(1S,3R,24R)-20-methyl-25-(4-propyloxazol-2-yl)-26,27-cyclo-9,10-secocholesta-5,7,10(19), 22-tetraene-1,3,24-triol

(5Z,7E,22E)-(1S,3R,24S)-20-methyl-25-(4-propyloxazol-2-yl)-26,27-cyclo-9,10-secocholesta-5,7,10(19), 22-tetraene-1,3,24-triol

(5Z,7E,22E)-(1S,3R,24R)-20-methyl-25-(4-methyloxazol-2-yl)-26,27-cyclo-9,10-secocholesta-5,7,10(19), 22-tetraene-1,3,24-triol

(5Z,7E,22E)-(1S,3R,24S)-20-methyl-25-(4-methyloxazol-2-yl)-26,27-cyclo-9,10-secocholesta-5,7,10(19), 22-tetraene-1,3,24-triol

(5Z,7E,22E)-(1S,3R,24R)-25-(4-ethyloxazol-2-yl)-20-methyl-26,27-cyclo-9,10-secocholesta-5,7,10(19), 22-tetraene-1,3,24-triol

(5Z,7E,22E)-(1S,3R,24S)-25-(4-ethyloxazol-2-yl)-20-methyl-26,27-cyclo-9,10-secocholesta-5,7,10(19), 22-tetraene-1,3,24-triol

(5Z,7E,22E)-(1S,3R,24R)-25-(4-butyloxazol-2-yl)-20-methyl-26,27-cyclo-9,10-secocholesta-5,7,10(19), 22-tetraene-1,3,24-triol

(5Z,7E,22E)-(1S,3R,24S)-25-(4-butyloxazol-2-yl)-20-methyl-26,27-cyclo-9,10-secocholesta-5,7,10(19), 22-tetraene-1,3,24-triol

(5Z,7E,22E)-(1S,3R,24R)-20-methyl-25-(4-pentyloxazol-2-yl)-26,27-cyclo-9,10-secocholesta-5,7,10(19), 22-tetraene-1,3,24-triol

(5Z,7E,22E)-(1S,3R,24S)-20-methyl-25-(4-pentyloxazol-2-yl)-26,27-cyclo-9,10-secocholesta-5,7,10(19),22-tetraene-1,3,24-triol

(5Z,7E,22E)-(1S,3R,24R)-20-methyl-25-(4-propylthiazol-2-yl)-26,27-cyclo-9,10-secocholesta-5,7,10(19), 22-tetraene-1,3,24-triol

(5Z,7E,22E)-(1S,3R,24S)-20-methyl-25-(4-propylthiazol-2-yl)-26,27-cyclo-9,10-secocholesta-5,7,10(19), 22-tetraene-1,3,24-triol

(5Z,7E,22E)-(1S,3R,24R)-20-methyl-25-(4-methylthiazol-2-yl)-26,27-cyclo-9,10-secocholesta-5,7,10(19), 22-tetraene-1,3,24-triol

(5Z,7E,22E)-(1S,3R,24S)-20-methyl-25-(4-methylthiazol-2-yl)-26,27-cyclo-9,10-secocholesta-5,7,10(19), 22-tetraene-1,3,24-triol

(5Z,7E,22E)-(1S,3R,24R)-25-(4-ethylthiazol-2-yl)-20-methyl-26,27-cyclo-9,10-secocholesta-5,7,10(19), 22-tetraene-1,3,24-triol

(5Z,7E,22E)-(1S,3R,24S)-25-(4-ethylthiazol-2-yl)-20-methyl-26,27-cyclo-9,10-secocholesta-5,7,10(19), 22-tetraene-1,3,24-triol

(5Z,7E,22E)-(1S,3R,24R)-25-(4-butylthiazol-2-yl)-20-methyl-26,27-cyclo-9,10-secocholesta-5,7,10(19), 22-tetraene-1,3,24-triol

(5Z,7E,22E)-(1S,3R,24S)-25-(4-butylthiazol-2-yl)-20-methyl-26,27-cyclo-9,10-secocholesta-5,7,10(19), 22-tetraene-1,3,24-triol

(5Z,7E,22E)-(1S,3R,24R)-25-(4-pentylthiazol-2-yl)-20-methyl-26,27-cyclo-9,10-secocholesta-5,7,10(19), 22-tetraene-1,3,24-triol

(5Z,7E,22E)-(1S,3R,24S)-25-(4-pentylthiazol-2-yl)-20-methyl-26,27-cyclo-9,10-secocholesta-5,7,10(19), 22-tetraene-1,3,24-triol.

**6.** Process for the production of vitamin D derivatives of general formula I, where a compound of general formula II

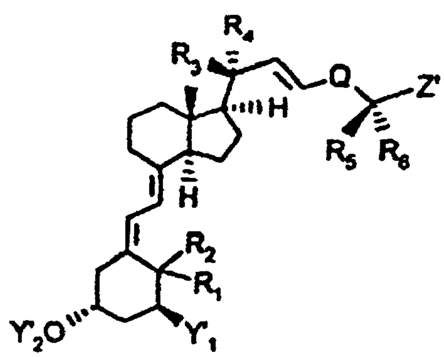

II

in which

| | |
|---|---|
| $Y'_1$ | means a hydrogen atom or a protected hydroxy group and |
| $Y'_2$ | means a hydroxy protective group, |
| $R_1$ and $R_2$ | each mean a hydrogen atom or together an exocyclic methylene group, |
| $R_3$ and $R_4$, | independently of one another, mean a hydrogen atom, a chlorine or fluorine atom, an alkyl group with 1 to 4 carbon atoms, together a methylene group or together with quaternary carbon atom 20 a 3- to 7-membered, saturated or unsaturated carbocyclic ring, |
| Q | means a straight-chain or branched carbon unit with up to 10 carbon atoms, which at any positions can have hydroxyl groups (in α- or β- position), which in turn can be etherified or esterified, keto groups, amino groups or halogen atoms, |
| $R_5$ and $R_6$ | together with carbon atom 25 mean a 3- to 7-membered, saturated or unsaturated carbocyclic ring and |
| Z' | means a five- or six-membered carbo- or heterocyclic ring, which can be saturated, unsaturated or aromatic, and can carry at any positions one or more alkyl chains, which can be straight-chain or branched, saturated or unsaturated, and at any points can be interrupted by oxa, thia or aza groups (substituted or unsubstituted) or sulfoxide or sulfone groups or can carry substituents (hydroxy groups, halogen atoms), where optionally present hydroxyl groups can be present in protected form, |

is reacted by simultaneous or successive cleavage of the hydroxy protective groups and optionally by partial or complete esterification(s) or etherification(s) of free hydroxy groups.

**7.** Use of the vitamin D derivatives of general formula I for the production of pharmaceutical agents.

**8.** Use of the vitamin D derivatives of general formula I according to claim 7 for the production of a pharmaceutical agent for the therapy of hyperproliferative diseases of the skin (psoriasis, acne, ichthyosis) as well as tumor diseases and precancerous stages (e.g., tumors of the intestines, carcinomas of the breast, lung tumors, prostate carcinomas, leukemias, T-cell lymphomas, actinic keratoses, cervix dysplasias, also auto-immune diseases (multiple sclerosis, diabetes mellitus type I, myasthenia gravis, lupus erythematosus), rejection reactions in the case of autologous, allogeneic or xenogeneic transplants, as well as AIDS; in addition, therapeutic use in the case of atrophic skin or wound healing is possible, as well as the therapy of secondary hyperparathyroidism, renal osteodystrophia, as well as senile and postmenopausal osteoporosis, diabetes mellitus type II and the therapy of degenerative diseases of the peripheral and central nervous system (Alzheimer's disease and amyotrophic lateral sclerosis) as well as the regulation of hair growth.

**9.** Use of vitamin D derivatives of general formula I according to claim 7, which antagonize the action of calcitriol in HL 60 cells, for the production of a pharmaceutical agent for the therapy of hypercalcemias (hypervitaminosis D, intoxication with calcitriol or its analogues) or granulomatous diseases (sarcoidosis, tuberculosis), as well as paraneoplastic hypercalcemias (thus osteolytic metastases and tumors with increased synthesis of parathormone-related peptides) and hypercalcemias in hyperparathyroidism and for birth control or as immunostimulants, as well as in hirsutism, and for the therapy and prophylaxis of arteriosclerosis, as well as for the therapy of inflammatory diseases (rheumatoid arthritis, Crohn's disease, ulcerative colitis, and granulomatous diseases).

**10.** Intermediate products of general formula XI, XII, and XLIV within the production of vitamin D derivatives of general formula I according to claim 1:

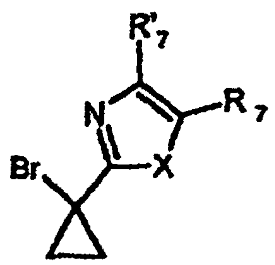

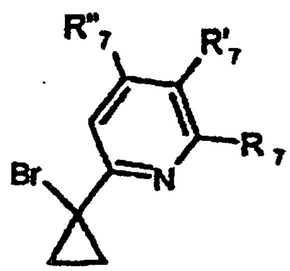

XI X=O
XIII X=S

XLIV

in which

$R_7$, $R'_7$ and $R''_7$, independently of one another, mean a hydrogen atom, a straight-chain or branched, saturated or unsaturated alkyl radical with up to 12 carbon atoms, which at any points can be interrupted by oxa, thia or aza groups (substituted or unsubstituted) or sulfoxide or sulfone groups or can carry other substituents (free or protected hydroxy groups, halogen atoms).

**Revendications**

**1.** Dérivés de vitamine D de formule générale I

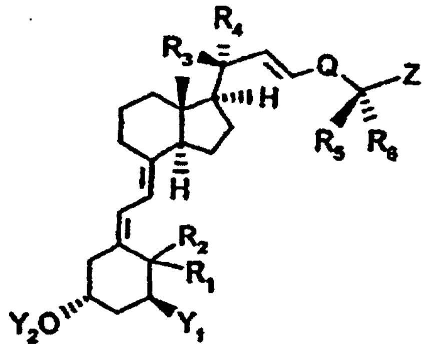

I

dans laquelle
$Y_1$ signifie un atome d'hydrogène, un groupement hydroxyle, un groupement alcanoyloxy comprenant 1 à 12 atomes de carbone ou un groupement aroyloxy,
$Y_2$ signifie un atome d'hydrogène ou un groupement alcanoyle comprenant 1 à 12 atomes de carbone ou un groupement aroyle,
$R_1$ et $R_2$ signifient chacun un atome d'hydrogène ou ensemble un groupement méthylène exocyclique,
$R_3$ et $R_4$ signifient, indépendamment l'un de l'autre, un atome d'hydrogène, un atome de chlore ou de fluor, un groupement alkyle comprenant 1 à 4 atomes de carbone, ensemble un groupement méthylène ou ensemble avec l'atome de carbone quaternaire 20 un cycle carbocyclique de 3 jusqu'à 7 membres, saturé ou insaturé,
Q signifie une unité carbonée linéaire ou ramifiée comprenant jusqu'à 10 atomes de carbone, qui peut présenter en des positions quelconques des groupements hydroxyle (en position $\alpha$ ou $\beta$), qui peuvent à leur tour être éthérifiés ou estérifiés, des groupements céto, des groupements amino ou des atomes d'halogène,
$R_5$ et $R_6$ signifient ensemble avec l'atome de carbone 25 un cycle carbocyclique de 3 jusqu'à 7 membres, saturé ou insaturé et
Z signifie un cycle carbocyclique ou hétérocyclique à cinq ou six membres, qui peut être saturé, insaturé ou aromatique, et qui peut présenter en des positions quelconques une ou plusieurs chaînes alkyle, qui peuvent être linéaires ou ramifiées, saturées ou insaturées et qui peuvent être interrompues en des positions quelconques par des fonctions oxa, thia ou aza (substituées ou non substituées) ou par un sulfoxyde ou des groupements sulfone ou qui peuvent porter des substituants (groupements hydroxy, atomes d'halogène).

2. Dérivés de la vitamine D de formule générale I selon la revendication 1, dans lesquels $R_5$ et $R_6$ signifient ensemble avec l'atome de carbone C-25 un cycle cyclopropyle et Z un cycle aromatique ou hétéroaromatique.

3. Dérivés de la vitamine D selon la revendication 2, dans lesquels Z a la signification d'un cycle phényle, oxazole ou thiazole, le cas échéant substitué.

4. Dérivés de la vitamine D de formule générale I selon la revendication 1, dans laquelle Q a la signification d'un groupement hydroxyméthyle ou carbonylméthyle.

5. Dérivés de la vitamine D de formule générale I selon la revendication 1,
(5Z,7E,22E)-(1S,3R,24R)-25-(5-propyloxazol-2-yl)-26,27-cyclo-9,10-secocholesta-5,7,10(29),22-tétraène-1,3,24-triol,
(5Z,7E,22E)-(1S,3R,24S)-25-(5-propyloxazol-2-yl)-26,27-cyclo-9,10-secocholesta-5,7,10(29),22-tétraène-1,3,24-triol,
(5Z,7E,22E)-(1S,3R,24R)-25-(5-méthyloxazol-2-yl)-26,27-cyclo-9,10-secocholesta-5,7,10(19),22-tétraène-1,3,24-triol,
(5Z,7E,22E)-(1S,3R,24S)-25-(5-méthyloxazol-2-yl)-26,27-cyclo-9,10-secocholesta-5,7,10(19),22-tétraène-1,3,24-triol,
(5Z,7E,22E)-(1S,3R,24R)-25-(5-éthyloxazol-2-yl)-26,27-cyclo-9,10-secocholesta-5,7,10(19),22-tétraène-1,3,24-triol,
(5Z,7E,22E)-(1S,3R,24S)-25-(5-éthyloxazol-2-yl)-26,27-cyclo-9,10-secocholesta-5,7,10(19),22-tétraène-1,3,24-triol,
(5Z,7E,22E)-(1S,3R,24R)-25-(5-butyloxazol-2-yl)-26,27-cyclo-9,10-secocholesta-5,7,10(19),22-tétraène-1,3,24-triol,
(5Z,7E,22E)-(1S,3R,24S)-25-(5-butyloxazol-2-yl)-26,27-cyclo-9,10-secocholesta-5,7,10(29),22-tétraène-1,3,24-triol,
(5Z,7E,22E)-(1S,3R,24R)-25-(5-Pentyloxazol-2-yl)-26,27-cyclo-9,20-secocholesta-5,7,10(19),22-tétraène-1,3,24-triol,
(5Z,7E,22E)-(1S,3R,24S)-25-(5-pentyloxazol-2-yl)-26,27-cyclo-9,10-secocholesta-5,7,10(19),22-tétraène-1,3,24-triol,
(5Z,7E,22E)-(1S,3R,24R)-25-(5-propylthiazol-2-yl)-26,27-cyclo-9,10-secocholesta-5,7,10(19),22-tétraène-1,3,24-triol,
(5Z,7E,22E)-(1S,3R,24S)-25-(5-propylthiazol-2-yl)-26,27-cyclo-9,10-secocholesta-5,7,10(19),22-tétraène-1,3,24-triol,
(5Z,7E,22E)-(1S,3R,24R)-25-(5-méthylthiazol-2-yl)-26,27-cyclo-9,10-secocholesta-5,7,10(19),22-tétraène-2,3,24-triol,
(5Z,7E,22E)-(1S,3R,24S)-25-(5-méthylthiazol-2-yl)-26,27-cyclo-9,10-secocholesta-5,7,10(19),22-tétraène-1,3,24-triol,
(5Z,7E,22E)-(1S,3R,24R)-25-(5-éthylthiazol-2-yl)-26,27-cyclo-9,10-secocholesta-5,7,10(19),22-tétraène-1,3,24-triol,
(5Z,7E,22E)-(1S,3R,24S)-25-(5-éthylthiazol-2-yl)-26,27-cyclo-9,10-secocholesta-5,7,10(19),22-tétraène-1,3,24-triol,
(5Z,7E,22E)-(1S,3R,24R)-25-(5-butylthiazol-2-yl)-26,27-cyclo-9,10-secocholesta-5,7,10(19),22-tétraène-1,3,24-triol,
(5Z,7E,22E)-(1S,3R,24S)-25-(5-butylthiazol-2-yl)-26,27-cyclo-9,10-secocholesta-5,7,10(19),22-tétraène-1,3,24-triol,
(5Z,7E,22E)-(1S,3R,24R)-25-(5-pentylthiazol-2-yl)-26,27-cyclo-9,10-secocholesta-5,7,10(19),22-tétraène-1,3,24-triol,
(5Z,7E,22E)-(1S,3R,24S)-25-(5-pentylthiazol-2-yl)-26,27-cyclo-9,10-secocholesta-5,7,10(19),22-tétraène-1,3,24-triol,
(5Z,7E,22E)-(1S,3R,24R)-25-(5-propylimidazol-2-yl)-26,27-cyclo-9,10-secocholesta-5,7,10(19),22-tétraène-1,3,24-triol,
(5Z,7E,22E)-(1S,3R,24S)-25-(5-propylimidazol-2-yl)-26,27-cyclo-9,10-secocholesta-5,7,10(19),22-tétraène-1,3,24-triol,
(5Z,7E,22E)-(1S,3R,24R)-25-(5-méthylimidazol-2-yl)-26,27-cyclo-9,10-secocholesta-5,7,10(19),22-tétraène-1,3,24-triol,
(5Z,7E,22E)-(1S,3R,24S)-25-(5-méthylimidazol-2-yl)-26,27-cyclo-9,10-secocholesta-5,7,10(19),22-tétraène-1,3,24-triol,
(5Z,7E,22E)-(1S,3R,24R)-25-(5-éthylimidazol-2-yl)-26,27-cyclo-9,10-secocholesta-5,7,10(19),22-tétraène-1,3,24-triol,
(5Z,7E,22E)-(1S,3R,24S)-25-(5-éthylimidazol-2-yl)-26,27-cyclo-9,10-secocholesta-5,7,10(19),22-tétraène-1,3,24-triol,
(5Z,7E,22E)-(1S,3R,24R)-25-(5-butylimidazol-2-yl)-26,27-cyclo-9,10-secocholesta-5,7,10(19),22-tétraène-1,3,24-triol,
(5Z,7E,22E)-(1S,3R,24S)-25-(5-butylimidazol-2-yl)-26,27-cyclo-9,10-secocholesta-5,7,10(19),22-tétraène-1,3,24-triol,
(5Z,7E,22E)-(1S,3R,24R)-25-(5-pentylimidazol-2-yl)-26,27-cyclo-9,10-secocholesta-5,7,10(19),22-tétraène-1,3,24-triol,
(5Z,7E,22E)-(1S,3R,24S)-25-(5-pentylimidazol-2-yl)-26,27-cyclo-9,10-secocholesta-5,7,10(19),22-tétraène-1,3,24-triol,
(5Z,7E,22E)-(1S,3R,24R)-25-(5-propylfurann-2-yl)-26,27-cyclo-9,10-secocholesta-5,7,10(19),22-tétraène-1,3,24-triol,
(5Z,7E,22E)-(1S,3R,24S)-25-(5-propylfurann-2-yl)-26,27-cyclo-9,10-secocholesta-5,7,10(19),22-tétraène-1,3,24-triol,
(5Z,7E,22E)-(1S,3R,24R)-25-(5-méthylfurann-2-yl)-26,27-cyclo-9,10-secocholesta-5,7,10(19),22-tétraène-1,3,24-triol,
(5Z,7E,22E)-(1S,3R,24S)-25-(5-méthylfurann-2-yl)-26,27-cyclo-9,10-secocholesta-5,7,10(19),22-tétraène-1,3,24-triol,
(5Z,7E,22E)-(1S,3R,24R)-25-(5-éthylfurann-2-yl)-26,27-cyclo-9,10-secocholesta-5,7,10(19),22-tétraène-1,3,24-triol,
(5Z,7E,22E)-(1S,3R,24S)-25-(5-éthylfurann-2-yl)-26,27-cyclo-9,10-secocholesta-5,7,10(19),22-tétraène-1,3,24-triol,
(5Z,7E,22E)-(1S,3R,24R)-25-(5-butylfurann-2-yl)-26,27-cyclo-9,10-secocholesta-5,7,10(19),22-tétraène-1,3,24-triol,
(5Z,7E,22E)-(1S,3R,24S)-25-(5-butylfurann-2-yl)-26,27-cyclo-9,10-secocholesta-5,7,10(19),22-tétraène-1,3,24-triol,
(5Z,7E,22E)-(1S,3R,24R)-25-(5-pentylfurann-2-yl)-26,27-cyclo-9,10-secocholesta-5,7,10(19),22-tétraène-1,3,24-triol,
(5Z,7E,22E)-(1S,3R,24S)-25-(5-pentylfurann-2-yl)-26,27-cyclo-9,10-secocholesta-5,7,10(19),22-tétraène-1,3,24-triol,
(5Z,7E,22E)-(1S,3R,24R)-25-(5-propylthiophén-2-yl)-26,27-cyclo-9,10-secocholesta-5,7,10(19),22-tétraène-1,3,24-triol,
(5Z,7E,22E)-(1S,3R,24S)-25-(5-propylthiophén-2-yl)-26,27-cyclo-9,10-secocholesta-5,7,10(19),22-tétraène-1,3,24-triol,
(5Z,7E,22E)-(1S,3R,24R)-25-(5-méthylthiophén-2-yl)-26,27-cyclo-9,10-secocholesta-5,7,10(19),22-tétraène-1,3,24-triol,
(5Z,7E,22E)-(1S,3R,24S)-25-(5-méthylthiophén-2-yl)-26,27-cyclo-9,10-secocholesta-5,7,10(19),22-tétraène-1,3,24-triol,
(5Z,7E,22E)-(1S,3R,24R)-25-(5-éthylthiophén-2-yl)-26,27-cyclo-9,10-secocholesta-5,7,10(19),22-tétraène-1,3,24-triol,
(5Z,7E,22E)-(1S,3R,24S)-25-(5-éthylthiophén-2-yl)-26,27-cyclo-9,10-secocholesta-5,7,10(19),22-tétraène-1,3,24-triol,
(5Z,7E,22E)-(1S,3R,24R)-25-(5-butylthiophén-2-yl)-26,27-cyclo-9,10-secocholesta-5,7,10(19),22-tétraène-1,3,24-triol,
(5Z,7E,22E)-(1S,3R,24S)-25-(5-butylthiophén-2-yl)-26,27-cyclo-9,10-secocholesta-5,7,10(19),22-tétraène-1,3,24-triol,
(5Z,7E,22E)-(1S,3R,24R)-25-(5-pentylthiophén-2-yl)-26,27-cyclo-9,10-secocholesta-5,7,20(19),22-tétraène-1,3,24-triol,
(5Z,7E,22E)-(1S,3R,24S)-25-(5-pentylthiophén-2-yl)-26,27-cyclo-9,10-secocholesta-5,7,10(19),22-tétraène-1,3,24-triol,
(5Z,7E,22E)-(1S,3R,24R)-25-(5-propylpyrrol-2-yl)-26,27-cyclo-9,10-secocholesta-5,7,10(19),22-tétraène-1,3,24-triol,
(5Z,7E,22E)-(1S,3R,24S)-25-(5-propylpyrrol-2-yl)-26,27-cyclo-9,10-secocholesta-5,7,10(19),22-tétraène-1,3,24-triol,
(5Z,7E,22E)-(1S,3R,24R)-25-(5-méthylpyrrol-2-yl)-26,27-cyclo-9,10-secocholesta-5,7,10(19),22-tétraène-1,3,24-triol,
(5Z,7E,22E)-(1S,3R,24S)-25-(5-méthylpyrrol-2-yl)-26,27-cyclo-9,10-secocholesta-5,7,10(19),22-tétraène-1,3,24-triol,
(5Z,7E,22E)-(1S,3R,24R)-25-(5-éthylpyrrol-2-yl)-26,27-cyclo-9,10-secocholesta-5,7,10(19),22-tétraène-1,3,24-triol,
(5Z,7E,22E)-(1S,3R,24S)-25-(5-éthylpyrrol-2-yl)-26,27-cyclo-9,10-secocholesta-5,7,10(19),22-tétraène-1,3,24-triol,
(5Z,7E,22E)-(1S,3R,24R)-25-(5-butylpyrrol-2-yl)-26,27-cyclo-9,10-secocholesta-5,7,10(19),22-tétraène-1,3,24-triol,
(5Z,7E,22E)-(1S,3R,24S)-25-(5-butylpyrrol-2-yl)-26,27-cyclo-9,10-secocholesta-5,7,10(19),22-tétraène-1,3,24-triol,
(5Z,7E,22E)-(2S,3R,24R)-25-(5-pentylpyrrol-2-yl)-26,27-cyclo-9,10-secocholesta-5,7,10(19),22-tétraène-1,3,24-triol,
(5Z,7E,22E)-(1S,3R,24S)-25-(5-pentylpyrrol-2-yl)-26,27-cyclo-9,10-secocholesta-5,7,10(19),22-tétraène-1,3,24-triol,
(5Z,7E,22E)-(1S,3R,24R)-25-(4-propyloxazol-2-yl)-26,27-cyclo-9,10-secocholesta-5,7,10(19),22-tétraène-1,3,24-triol,
(5Z,7E,22E)-(1S,3R,24S)-25-(4-propyloxazol-2-yl)-26,27-cyclo-9,10-secocholesta-5,7,10(19),22-tétraène-1,3,24-triol,
(5Z,7E,22E)-(1S,3R,24R)-25-(4-méthyloxazol-2-yl)-26,27-cyclo-9,10-secocholesta-5,7,10(19),22-tétraène-1,3,24-triol,
(5Z,7E,22E)-(1S,3R,24S)-25-(4-méthyloxazol-2-yl)-26,27-cyclo-9,10-secocholesta-5,7,10(19),22-tétraène-1,3,24-triol,
(5Z,7E,22E)-(1S,3R,24R)-25-(4-éthyloxazol-2-yl)-26,27-cyclo-9,10-secocholesta-5,7,10(19),22-tétraène-1,3,24-triol,
(5Z,7E,22E)-(1S,3R,24S)-25-(4-éthyloxazol-2-yl)-26,27-cyclo-9,10-secocholesta-5,7,10(19),22-tétraène-1,3,24-triol,
(5Z,7E,22E)-(1S,3R,24R)-25-(4-butyloxazol-2-yl)-26,27-cyclo-9,10-secocholesta-5,7,10(19),22-tétraène-1,3,24-triol,
(5Z,7E,22E)-(2S,3R,24S)-25-(4-butyloxazol-2-yl)-26,27-cyclo-9,10-secocholesta-5,7,10(19),22-tétraène-1,3,24-triol,
(5Z,7E,22E)-(1S,3R,24R)-25-(4-pentyloxazol-2-yl)-26,27-cyclo-9,10-secocholesta-5,7,10(19),22-tétraène-1,3,24-triol,
(5Z,7E,22E)-(1S,3R,24S)-25-(4-pentyloxazol-2-yl)-26,27-cyclo-9,10-secocholesta-5,7,10(19),22-tétraène-1,3,24-triol,
(5Z,7E,22E)-(1S,3R,24R)-25-(4-propylthiazol-2-yl)-26,27-cyclo-9,10-secocholesta-5,7,10(19),22-tétraène-1,3,24-triol,
(5Z,7E,22E)-(1S,3R,24S)-25-(4-propylthiazol-2-yl)-26,27-cyclo-9,10-secocholesta-5,7,10(19),22-tétraène-1,3,24-triol,
(5Z,7E,22E)-(1S,3R,24R)-25-(4-méthylthiazol-2-yl)-26,27-cyclo-9,10-secocholesta-5,7,10(19),22-tétraène-1,3,24-triol,
(5Z,7E,22E)-(1S,3R,24S)-25-(4-méthylthiazol-2-yl)-26,27-cyclo-9,10-secocholesta-5,7,10(19),22-tétraène-1,3,24-triol,
(5Z,7E,22E)-(1S,3R,24R)-25-(4-éthylthiazol-2-yl)-26,27-cyclo-9,10-secocholesta-5,7,10(19),22-tétraène-1,3,24-triol,
(5Z,7E,22E)-(1S,3R,24S)-25-(4-éthylthiazol-2-yl)-26,27-cyclo-9,10-secocholesta-5,7,10(19),22-tétraène-1,3,24-triol,
(5Z,7E,22E)-(1S,3R,24R)-25-(4-butylthiazol-2-yl)-26,27-cyclo-9,10-secocholesta-5,7,10(19),22-tétraène-1,3,24-triol,
(5Z,7E,22E)-(1S,3R,24S)-25-(4-butylthiazol-2-yl)-26,27-cyclo-9,10-secocholesta-5,7,10(19),22-tétraène-1,3,24-triol,
(5Z,7E,22E)-(1S,3R,24R)-25-(4-pentylthiazol-2-yl)-26,27-cyclo-9,10-secocholesta-5,7,10(29),22-tétraène-1,3,24-triol,
(5Z,7E,22E)-(1S,3R,24S)-25-(4-pentylthiazol-2-yl)-26,27-cyclo-9,10-secocholesta-5,7,10(19),22-tétraène-1,3,24-triol,
(5Z,7E,22E)-(1S,3R,24R)-25-(4-propylimidazol-2-yl)-26,27-cyclo-9,10-secocholesta-5,7,10(19),22-tétraène-1,3,24-triol,
(5Z,7E,22E)-(1S,3R,24S)-25-(4-propylimidazol-2-yl)-26,27-cycle-9,10-secocholesta-5,7,10(19),22-tétraène-1,3,24-triol,
(5Z,7E,22E)-(1S,3R,24R)-25-(4-méthylimidazol-2-yl)-26,27-cyclo-9,10-secocholesta-5,7,10(19),22-tétraène-1,3,24-triol,
(5Z,7E,22E)-(1S,3R,24S)-25-(4-méthylimidazol-2-yl)-26,27-cyclo-9,10-secocholesta-5,7,10(19),22-tétraène-1,3,24-triol,
(5Z,7E,22E)-(1S,3R,24R)-25-(4-éthylimidazol-2-yl)-26,27-cyclo-9,10-secocholesta-5,7,10(19),22-tétraène-1,3,24-triol,
(5Z,7E,22E)-(1S,3R,24S)-25-(4-éthylimidazol-2-yl)-26,27-cyclo-9,10-secocholesta-5,7,10(19),22-tétraène-1,3,24-triol,
(5Z,7E,22E)-(1S,3R,24R)-25-(4-butylimidazol-2-yl)-26,27-cyclo-9,10-secocholesta-5,7,10(19),22-tétraène-1,3,24-triol,
(5Z,7E,22E)-(1S,3R,24S)-25-(4-butylimidazol-2-yl)-26,27-cyclo-9,10-secocholesta-5,7,10(19),22-tétraène-1,3,24-triol,
(5Z,7E,22E)-(1S,3R,24R)-25-(4-pentylimidazol-2-yl)-26,27-cyclo-9,10-secocholesta-5,7,10(19),22-tétraène-1,3,24-triol,
(5Z,7E,22E)-(1S,3R,24S)-25-(4-pentylimidazol-2-yl)-26,27-cyclo-9,10-secocholesta-5,7,10(19),22-tétraène-1,3,24-triol,
(5Z,7E,22E)-(1S,3R,24R)-25-(4-propylfurann-2-yl)-26,27-cyclo-9,10-secocholesta-5,7,10(19),22-tétraène-1,3,24-triol,
(5Z,7E,22E)-(1S,3R,24S)-25-(4-propylfurann-2-yl)-26,27-cyclo-9,10-secocholesta-5,7,10(19),22-tétraène-1,3,24-triol,
(5Z,7E,22E)-(1S,3R,24R)-25-(4-méthylfurann-2-yl)-26,27-cyclo-9,10-secocholesta-5,7,10(19),22-tétraène-1,3,24-triol,
(5Z,7E,22E)-(1S,3R,24S)-25-(4-méthylfurann-2-yl)-26,27-cyclo-9,10-secocholesta-5,7,10(19),22-tétraène-1,3,24-triol,
(5Z,7E,22E)-(1S,3R,24R)-25-(4-éthylfurann-2-yl)-26,27-cyclo-9,10-secocholesta-5,7,10(19),22-tétraène-1,3,24-triol,
(5Z,7E,22E)-(1S,3R,24S)-25-(4-éthylfurann-2-yl)-26,27-cyclo-9,10-secocholesta-5,7,10(19),22-tétraène-1,3,24-triol,
(5Z,7E,22E)-(1S,3R,24R)-25-(4-butylfurann-2-yl)-26,27-cyclo-9,10-secocholesta-5,7,10(19),22-tétraène-1,3,24-triol,
(5Z,7E,22E)-(1S,3R,24S)-25-(4-butylfurann-2-yl)-26,27-cyclo-9,10-secocholesta-5,7,10(19),22-tétraène-1,3,24-triol,
(5Z,7E,22E)-(1S,3R,24R)-25-(4-pentylfurann-2-yl)-26,27-cyclo-9,10-secocholesta-5,7,10(19),22-tétraène-1,3,24-triol,
(5Z,7E,22E)-(1S,3R,24S)-25-(4-pentylfurann-2-yl)-26,27-cyclo-9,10-secocholesta-5,7,10(19),22-tétraène-1,3,24-triol,
(5Z,7E,22E)-(1S,3R,24R)-25-(4-propylthiophén-2-yl)-26,27-cyclo-9,10-secocholesta-5,7,10(19),22-tétraène-1,3,24-triol,
(5Z,7E,22E)-(1S,3R,24S)-25-(4-propylthiophén-2-yl)-26,27-cyclo-9,10-secocholesta-5,7,10(19),22-tétraène-1,3,24-triol,
(5Z,7E,22E)-(2S,3R,24R)-25-(4-méthylthiophén-2-yl)-26,27-cyclo-9,10-secocholesta-5,7,10(19),22-tétraène-1,3,24-triol,
(5Z,7E,22E)-(1S,3R,24S)-25-(4-méthylthiophén-2-yl)-26,27-cyclo-9,10-secocholesta-5,7,10(19),22-tétraène-1,3,24-triol,
(5Z,7E,22E)-(1S,3R,24R)-25-(4-éthylthiophén-2-yl)-26,27-cyclo-9,10-secocholesta-5,7,10(19),22-tétraène-1,3,24-triol,
(5Z,7E,22E)-(1S,3R,24S)-25-(4-éthylthiophén-2-yl)-26,27-cyclo-9,10-secocholesta-5,7,10(19),22-tétraène-1,3,24-triol,
(5Z,7E,22E)-(1S,3R,24R)-25-(4-butylthiophén-2-yl)-26,27-cyclo-9,10-secocholesta-5,7,10(19),22-tétraène-1,3,24-triol,
(5Z,7E,22E)-(1S,3R,24S)-25-(4-butylthiophén-2-yl)-26,27-cyclo-9,10-secocholesta-5,7,10(19),22-tétraène-1,3,24-triol,
(5Z,7E,22E)-(1S,3R,24R)-25-(4-pentylthiophén-2-yl)-26,27-cyclo-9,10-secocholesta-5,7,10(19),22-tétraène-1,3,24-triol,
(5Z,7E,22E)-(1S,3R,24S)-25-(4-pentylthiophén-2-yl)-26,27-cyclo-9,10-secocholesta-5,7,10(19),22-tétraène-1,3,24-triol,
(5Z,7E,22E)-(1S,3R,24R)-25-(4-propylpyrrol-2-yl)-26,27-cyclo-9,10-secocholesta-5,7,10(19),22-tétraène-1,3,24-triol,
(5Z,7E,22E)-(1S,3R,24S)-25-(4-propylpyrrol-2-yl)-26,27-cyclo-9,20-secocholesta-5,7,20(19),22-tétraène-1,3,24-triol,
(5Z,7E,22E)-(1S,3R,24R)-25-(4-méthylpyrrol-2-yl)-26,27-cyclo-9,10-secocholesta-5,7,10(19),22-tétraène-1,3,24-triol,
(5Z,7E,22E)-(1S,3R,24S)-25-(4-méthylpyrrol-2-yl)-26,27-cyclo-9,10-secocholesta-5,7,10(19),22-tétraène-1,3,24-triol,
(5Z,7E,22E)-(1S,3R,24R)-25-(4-éthylpyrrol-2-yl)-26,27-cyclo-9,10-secocholesta-5,7,10(19),22-tétraène-1,3,24-triol,
(5Z,7E,22E)-(1S,3R,24S)-25-(4-éthylpyrrol-2-yl)-26,27-cyclo-9,20-secocholesta-5,7,10(29),22-tétraène-1,3,24-triol,
(5Z,7E,22E)-(1S,3R,24R)-25-(4-butylpyrrol-2-yl)-26,27-cyclo-9,10-secocholesta-5,7,10(19),22-tétraène-1,3,24-triol,
(5Z,7E,22E)-(1S,3R,24S)-25-(4-butylpyrrol-2-yl)-26,27-cyclo-9,10-secocholesta-5,7,10(19),22-tétraène-1,3,24-triol,
(5Z,7E,22E)-(1S,3R,24R)-25-(4-pentylpyrrol-2-yl)-26,27-cyclo-9,10-secocholesta-5,7,10(19),22-tétraène-1,3,24-triol,
(5Z,7E,22E)-(1S,3R,24S)-25-(4-pentylpyrrol-2-yl)-26,27-cyclo-9,10-secocholesta-5,7,10(19),22-tétraène-1,3,24-triol,
(5Z,7E,22E)-(1S,3R,24R)-25-(3-méthyl-1,2,4-oxadiazol-5-yl)-26,27-cycle-9,10-secocholesta-5,7,10(19),22-tétraène-1,3,24-triol,
(5Z,7E,22E)-(1S,3R,24S)-25-(3-méthyl-1,2,4-oxadiazol-5-yl)-26,27-cycle-9,10-secocholesta-5,7,10(19),22-tétraène-1,3,24-triol,
(5Z,7E,22E)-(1S,3R,24R)-25-(3-éthyl-1,2,4-oxadiazol-5-yl)-26,27-cyclo-9,10-secocholesta-5,7,10(19),22-tétraène-1,3,24-triol,
(5Z,7E,22E)-(1S,3R,24S)-25-(3-éthyl-1,2,4-oxadiazol-5-yl)-26,27-cyclo-9,10-secocholesta-5,7,10(19),22-tétraène-1,3,24-triol,
(5Z,7E,22E)-(1S,3R,24R)-25-(3-propyl-1,2,4-oxadiazol-5-yl)-26,27-cyclo-9,10-secocholesta-5,7,10(19),22-tétraène-1,3,24-triol,
(5Z,7E,22E)-(1S,3R,24S)-25-(3-propyl-1,2,4-oxadiazol-5-yl)-26,27-cyclo-9,10-secocholesta-5,7,10(19),22-tétraène-1,3,24-triol,
(5Z,7E,22E)-(1S,3R,24R)-25-(3-butyl-1,2,4-oxadiazol-5-yl)-26,27-cyclo-9,10-secocholesta-5,7,10(19),22-tétraène-1,3,24-triol,
(5Z,7E,22E)-(1S,3R,24S)-25-(3-butyl-1,2,4-oxadiazol-5-yl)-26,27-cyclo-9,10-secocholesta-5,7,10(19),22-tétraène-1,3,24-triol,
(5Z,7E,22E)-(1S,3R,24R)-25-(3-pentyl-1,2,4-oxadiazol-5-yl)-26,27-cyclo-9,10-secocholesta-5,7,10(19),22-tétraène-1,3,24-triol,
(5Z,7E,22E)-(1S,3R,24S)-25-(3-pentyl-1,2,4-oxadiazol-5-yl)-26,27-cyclo-9,10-secocholesta-5,7,10(19),22-tétraène-1,3,24-triol,
(5Z,7E,22E)-(1S,3R,24R)-25-(5-méthyl-1,3,4-oxadiazol-2-yl)-26,27-cyclo-9,10-secocholesta-5,7,20(19),22-tétraène-1,3,24-triol,
(5Z,7E,22E)-(1S,3R,24S)-25-(5-méthyl-1,3,4-oxadiazol-2-yl)-26,27-cyclo-9,10-secocholesta-5,7,10(19),22-tétraène-1,3,24-triol,
(5Z,7E,22E)-(1S,3R,24R)-25-(5-éthyl-1,3,4-oxadiazol-2-yl)-26,27-cyclo-9,10-secocholesta-5,7,10(19),22-tétraène-1,3,24-triol,
(5Z,7E,22E)-(1S,3R,24S)-25-(5-éthyl-1,3,4-oxadiazol-2-yl)-26,27-cyclo-9,10-secocholesta-5,7,10(19),22-tétraène-1,3,24-triol,
(5Z,7E,22E)-(1S,3R,24R)-25-(5-propyl-1,3,4-oxadiazol-2-yl)-26,27-cyclo-9,10-secocholesta-5,7,10(19),22-tétraène-1,3,24-triol,
(5Z,7E,22E)-(2S,3R,24S)-25-(5-propyl-1,3,4-oxadiazol-2-yl)-26,27-cyclo-9,10-secocholesta-5,7,10(19),22-tétraène-1,3,24-triol,
(5Z,7E,22E)-(1S,3R,24R)-25-(5-butyl-1,3,4-oxadiazol-2-yl)-26,27-cyclo-9,10-secocholesta-5,7,10(19),22-tétraène-1,3,24-triol,
(5Z,7E,22E)-(1S,3R,24S)-25-(5-butyl-1,3,4-oxadiazol-2-yl)-26,27-cyclo-9,10-secocholesta-5,7,10(19),22-tétraène-1,3,24-triol,
(5Z,7E,22E)-(1S,3R,24R)-25-(5-pentyl-1,3,4-oxadiazol-2-yl)-26,27-cyclo-9,10-secocholesta-5,7,10(19),22-tétraène-1,3,24-triol,
(5Z,7E,22E)-(1S,3R,24S)-25-(5-pentyl-1,3,4-oxadiazol-2-yl)-26,27-cyclo-9,10-secocholesta-5,7,20(19),22-tétraène-1,3,24-triol,
(5Z,7E,22E)-(1S,3R,24R)-25-phényl-26,27-cyclo-9,10-secocholesta-5,7,10(19),22-tétraène-1,3,24-triol,
(5Z,7E,22E)-(1S,3R,24S)-25-phényl-26,27-cyclo-9,10-secocholesta-5,7,10(19),22-tétraène-1,3,24-triol,
(5Z,7E,22E)-(1S,3R,24R)-25-(4-méthylphényl)-26,27-cyclo-9,10-secocholesta-5,7,10(19),22-tétraène-1,3,24-triol,
(5Z,7E,22E)-(1S,3R,24S)-25-(4-méthylphényl)-26,27-cyclo-9,10-secocholesta-5,7,10(19),22-tétraène-1,3,24-triol,
(5Z,7E,22E)-(1S,3R,24R)-25-(4-éthylphényl)-26,27-cyclo-9,10-secocholesta-5,7,10(19),22-tétraène-1,3,24-triol,
(5Z,7E,22E)-(1S,3R,24S)-25-(4-éthylphényl)-26,27-cyclo-9,10-secocholesta-5,7,10(19),22-tétraène-1,3,24-triol,
(5Z,7E,22E)-(1S,3R,24R)-25-(4-propylphényl)-26,27-cyclo-9,10-secocholesta-5,7,10(19),22-tétraène-1,3,24-triol,
(5Z,7E,22E)-(1S,3R,24S)-25-(4-propylphényl)-26,27-cyclo-9,10-secocholesta-5,7,10(19),22-tétraène-1,3,24-triol,
(5Z,7E,22E)-(1S,3R,24R)-25-(4-butylphényl)-26,27-cyclo-9,10-secocholesta-5,7,10(19),22-tétraène-1,3,24-triol,
(5Z,7E,22E)-(1S,3R,24S)-25-(4-butylphényl)-26,27-cyclo-9,10-secocholesta-5,7,10(19),22-tétraène-1,3,24-triol,
(5Z,7E,22E)-(1S,3R,24R)-25-(4-pentylphényl)-26,27-cyclo-9,10-secocholesta-5,7,10(19),22-tétraène-1,3,24-triol,
(5Z,7E,22E)-(1S,3R,24S)-25-(4-pentylphényl)-26,27-cyclo-9,10-secocholesta-5,7,10(19),22-tétraène-1,3,24-triol,
(5Z,7E,22E)-(1S,3R,24R)-25-(3-méthylphényl)-26,27-cyclo-9,10-secocholesta-5,7,10(19),22-tétraène-1,3,24-triol,
(5Z,7E,22E)-(1S,3R,24S)-25-(3-méthylphényl)-26,27-cyclo-9,10-secocholesta-5,7,10(19),22-tétraène-1,3,24-triol,
(5Z,7E,22E)-(1S,3R,24R)-25-(3-éthylphényl)-26,27-cyclo-9,10-secocholesta-5,7,10(19),22-tétraène-1,3,24-triol,

(5Z,7E,22E)-(1S,3R,24S)-25-(3-éthylphényl)-26,27-cyclo-9,10-secocholesta-5,7,10(19),22-tétraène-1,3,24-triol,
(5Z,7E,22E)-(1S,3R,24R)-25-(3-propylphényl)-26,27-cyclo-9,20-secocholesta-5,7,10(19),22-tétraène-1,3,24-triol,
(5Z,7E,22E)-(1S,3R,24S)-25-(3-propylphényl)-26,27-cyclo-9,10-secocholesta-5,7,10(19),22-tétraène-1,3,24-triol,
(5Z,7E,22E)-(1S,3R,24R)-25-(3-butylphényl)-26,27-cyclo-9,10-secocholesta-5,7,10(19),22-tétraène-1,3,24-triol,
(5Z,7E,22E)-(1S,3R,24S)-25-(3-butylphényl)-26,27-cyclo-9,10-secocholesta-5,7,10(19),22-tétraène-1,3,24-triol,
(5Z,7E,22E)-(1S,3R,24R)-25-(3-pentylphényl)-26,27-cyclo-9,10-secocholesta-5,7,10(19),22-tétraène-1,3,24-triol,
(5Z,7E,22E)-(1S,3R,24S)-25-(3-pentylphényl)-26,27-cyclo-9,10-secocholesta-5,7,10(19),22-tétraène-1,3,24-triol,
(5Z,7E,22E)-(1S,3R,24R)-25-[4-(1-méthyléthyl)phényl]-26,27-cyclo-9,10-secocholesta-5,7,10(19),22-tétraène-1,3,24-triol,
(5Z,7E,22E)-(1S,3R,24S)-25-[4-(1-méthyléthyl)phényl]-26,27-cyclo-9,10-secocholesta-5,7,10(19),22-tétraène-1,3,24-triol,
(5Z,7E,22E)-(1S,3R,24R)-25-[3-(1-méthyléthyl)phényl]-26,27-cyclo-9,10-secocholesta-5,7,10(19),22-tétraène-1,3,24-triol,
(5Z,7E,22E)-(1S,3R,24S)-25-[3-(1-méthyléthyl)phényl]-26,27-cyclo-9,10-secocholesta-5,7,10(19),22-tétraène-1,3,24-triol,
(5Z,7E,22E)-(1S,3R,24R)-25-(2-pyridyl)-26,27-cyclo-9,10-secocholesta-5,7,10(19),22-tétraène-1,3,24-triol,
(5Z,7E,22E)-(1S,3R,24S)-25-(2-pyridyl)-26,27-cyclo-9,10-secocholesta-5,7,10(19),22-tétraène-1,3,24-triol,
(5Z,7E,22E)-(1S,3R,24R)-25-(6-méthyl-2-pyridyl)-26,27-cyclo-9,10-secocholesta-5,7,10(19),22-tétraène-1,3,24-triol,
(5Z,7E,22E)-(1S,3R,24S)-25-(6-méthyl-2-pyridyl)-26,27-cyclo-9,10-secocholesta-5,7,20(19),22-tétraène-1,3,24-triol,
(5Z,7E,22E)-(1S,3R,24R)-25-(5-méthyl-2-pyridyl)-26,27-cyclo-9,10-secocholesta-5,7,10(19),22-tétraène-1,3,24-triol,
(5Z,7E,22E)-(1S,3R,24S)-25-(5-méthyl-2-pyridyl)-26,27-cyclo-9,10-secocholesta-5,7,10(19),22-tétraène-1,3,24-triol,
(5Z,7E,22E)-(1S,3R,24R)-25-(4-méthyl-2-pyridyl)-26,27-cyclo-9,10-secocholesta-5,7,10(19),22-tétraène-1,3,24-triol,
(5Z,7E,22E)-(1S,3R,24S)-25-(4-méthyl-2-pyridyl)-26,27-cyclo-9,10-secocholesta-5,7,10(19),22-tétraène-1,3,24-triol,
(5Z,7E,22E)-(1S,3R,24R)-25-(6-éthyl-2-pyridyl)-26,27-cyclo-9,10-secocholesta-5,7,10(19),22-tétraène-1,3,24-triol,
(5Z,7E,22E)-(1S,3R,24S)-25-(6-éthyl-2-pyridyl)-26,27-cyclo-9,10-secocholesta-5,7,10(19),22-tétraène-1,3,24-triol,
(5Z,7E,22E)-(1S,3R,24R)-25-(5-éthyl-2-pyridyl)-26,27-cyclo-9,10-secocholesta-5,7,10(19),22-tétraène-1,3,24-triol,
(5Z,7E,22E)-(1S,3R,24S)-25-(5-éthyl-2-pyridyl)-26,27-cyclo-9,10-secocholesta-5,7,10(19),22-tétraène-1,3,24-triol,
(5Z,7E,22E)-(1S,3R,24R)-25-(4-éthyl-2-pyridyl)-26,27-cyclo-9,10-secocholesta-5,7,10(19),22-tétraène-1,3,24-triol,
(5Z,7E,22E)-(1S,3R,24S)-25-(4-éthyl-2-pyridyl)-26,27-cyclo-9,10-secocholesta-5,7,10(19),22-tétraène-1,3,24-triol,
(5Z,7E,22E)-(1S,3R,24R)-25-(6-propyl-2-pyridyl)-26,27-cyclo-9,10-secocholesta-5,7,10(19),22-tétraène-1,3,24-triol,
(5Z,7E,22E)-(1S,3R,24S)-25-(6-propyl-2-pyridyl)-26,27-cyclo-9,10-secocholesta-5,7,10(19),22-tétraène-1,3,24-triol,
(5Z,7E,22E)-(1S,3R,24R)-25-(5-propyl-2-pyridyl)-26,27-cyclo-9,10-secocholesta-5,7,10(19),22-tétraène-1,3,24-triol,
(5Z,7E,22E)-(1S,3R,24S)-25-(5-propyl-2-pyridyl)-26,27-cyclo-9,10-secocholesta-5,7,10(19),22-tétraène-1,3,24-triol,
(5Z,7E,22E)-(1S,3R,24R)-25-(4-propyl-2-pyridyl)-26,27-cyclo-9,10-secocholesta-5,7,10(19),22-tétraène-2,3,24-triol,
(5Z,7E,22E)-(1S,3R,24S)-25-(4-propyl-2-pyridyl)-26,27-cyclo-9,10-secocholesta-5,7,10(19),22-tétraène-1,3,24-triol,
(5Z,7E,22E)-(1S,3R,24R)-25-(6-butyl-2-pyridyl)-26,27-cyclo-9,10-secocholesta-5,7,10(19),22-tétraène-1,3,24-triol,
(5Z,7E,22E)-(1S,3R,24S)-25-(6-butyl-2-pyridyl)-26,27-cyclo-9,10-secocholesta-5,7,10(19),22-tétraène-1,3,24-triol,
(5Z,7E,22E)-(1S,3R,24R)-25-(5-butyl-2-pyridyl)-26,27-cyclo-9,10-secocholesta-5,7,10(19),22-tétraène-1,3,24-triol,
(5Z,7E,22E)-(1S,3R,24S)-25-(5-butyl-2-pyridyl)-26,27-cyclo-9,10-secocholesta-5,7,10(19),22-tétraène-1,3,24-triol,
(5Z,7E,22E)-(1S,3R,24R)-25-(4-butyl-2-pyridyl)-26,27-cyclo-9,10-secocholesta-5,7,10(19),22-tétraène-1,3,24-triol,
(5Z,7E,22E)-(1S,3R,24S)-25-(4-butyl-2-pyridyl)-26,27-cyclo-9,10-secocholesta-5,7,10(19),22-tétraène-1,3,24-triol,
(5Z,7E,22E)-(1S,3R,24R)-25-(5,5-diméthyl-2-oxazolin-2-yl)-26,27-cyclo-9,10-secocholesta-5,7,10(19),22-tétraène-1,3,24-triol,
(5Z,7E,22E)-(1S,3R,24S)-25-(5,5-diméthyl-2-oxazolin-2-yl)-26,27-cyclo-9,20-secocholesta-5,7,10(19),22-tétraène-1,3,24-triol,
(5Z,7E,22E)-(1S,3R,24R)-25-(5,5-diéthyl-2-oxazolin-2-yl)-26,27-cyclo-9,10-secocholesta-5,7,20(29),22-tétraène-1,3,24-triol,
(5Z,7E,22E)-(1S,3R,24S)-25-(5,5-diéthyl-2-oxazolin-2-yl)-26,27-cyclo-9,10-secocholesta-5,7,10(19),22-tétraène-1,3,24-triol,
(5Z,7E,22E)-[1S,3R,24R,25(R)]-25-(5-méthyl-2-oxazolin-2-yl)-26,27-cyclo-9,10-secocholesta-5,7,10(19),22-tétraène-1,3,24-triol,
(5Z,7E,22E)-[1S,3R,24S,25(R)]-25-(5-méthyl-2-oxazolin-2-yl)-26,27-cyclo-9,10-secocholesta-5,7,10(19),22-tétraène-1,3,24-triol,
(5Z,7E,22E)-[1S,3R,24R,25(S)]-25-(5-méthyl-2-oxazolin-2-yl)-26,27-cyclo-9,10-secocholesta-5,7,10(19),22-tétraène-1,3,24-triol,
(5Z,7E,22E)-[1S,3R,24S,25(S)]-25-(5-méthyl-2-oxazolin-2-yl)-26,27-cycle-9,10-secocholesta-5,7,10(19),22-tétraène-1,3,24-triol,
(5Z,7E,22E)-[1S,3R,24R,25(R)]-25-(5-éthyl-2-oxazolin-2-yl)-26,27-cyclo-9,10-secocholesta-5,7,10(19),22-tétraène-1,3,24-triol,
(5Z,7E,22E)-[1S,3R,24S,25(R)]-25-(5-éthyl-2-oxazolin-2-yl)-26,27-cyclo-9,10-secocholesta-5,7,10(19),22-tétraène-1,3,24-triol,
(5Z,7E,22E)-[1S,3R,24R,25(S)]-25-(5-éthyl-2-oxazolin-2-yl)-26,27-cyclo-9,10-secocholesta-5,7,10(19),22-tétraène-1,3,24-triol,
(5Z,7E,22E)-[1S,3R,24S,25(S)]-25-(5-éthyl-2-oxazolin-2-yl)-26,27-cyclo-9,10-secocholesta-5,7,10(19),22-tétraène-1,3,24-triol,
(5Z,7E,22E)-[1S,3R,24R,25(R)]-25-(5-propyl-2-oxazolin-2-yl)-26,27-cyclo-9,10-secocholesta-5,7,10(19),22-tétraène-1,3,24-triol,
(5Z,7E,22E)-(1S,3R,24S,25(R)]-25-(5-propyl-2-oxazolin-2-yl)-26,27-cyclo-9,10-secocholesta-5,7,10(19),22-tétraène-1,3,24-triol,
(5Z,7E,22E)-[1S,3R,24R,25(S)]-25-(5-propyl-2-oxazolin-2-yl)-26,27-cyclo-9,10-secocholesta-5,7,10(19),22-tétraène-1,3,24-triol,
(5Z,7E,22E)-[1S,3R,24S,25(S)]-25-(5-propyl-2-oxazolin-2-yl)-26,27-cyclo-9,10-secocholesta-5,7,10(19),22-tétraène-1,3,24-triol,
(5Z,7E,22E)-[1S,3R,24R,25(R)]-25-(5-butyl-2-oxazolin-2-yl)-26,27-cyclo-9,10-secocholesta-5,7,10(19),22-tétraène-1,3,24-triol,
(5Z,7E,22E)-[1S,3R,24S,25(R)]-25-(5-butyl-2-oxazolin-2-yl)-26,27-cyclo-9,10-secocholesta-5,7,10(29),22-tétraène-1,3,24-triol,
(5Z,7E,22E)-[1S,3R,24R,25(S)]-25-(5-butyl-2-oxazolin-2-yl)-26,27-cyclo-9,10-secocholesta-5,7,10(19),22-tétraène-1,3,24-triol,
(5Z,7E,22E)-[1S,3R,24S,25(S)]-25-(5-butyl-2-oxazolin-2-yl)-26,27-cyclo-9,10-secocholesta-5,7,10(19),22-tétraène-1,3,24-triol,
(5Z,7E,22E)-[1S,3R,24R,25(R)]-25-(5-phényl-2-oxazolin-2-yl)-26,27-cyclo-9,10-secocholesta-5,7,10(19),22-tétraène-1,3,24-triol,
(5Z,7E,22E)-[1S,3R,24S,25(R)]-25-(5-phényl-2-oxazolin-2-yl)-26,27-cyclo-9,10-secocholesta-5,7,10(19),22-tétraène-1,3,24-triol,
(5Z,7E,22E)-[1S,3R,24R,25(S)]-25-(5-phényl-2-oxazolin-2-yl)-26,27-cyclo-9,10-secocholesta-5,7,10(19),22-tétraène-1,3,24-triol,
(5Z,7E,22E)-[1S,3R,24S,25(S)]-25-(5-phényl-2-oxazolin-2-yl)-26,27-cyclo-9,20-secocholesta-5,7,10(19),22-tétraène-1,3,24-triol,
(5Z,7E,22E)-(1S,3R,20S,24R)-25-(5-propyloxazol-2-yl)-26,27-cyclo-9,10-secocholesta-5,7,10(19),22-tétraène-1,3,24-triol,
(5Z,7E,22E)-(1S,3R,20S,24S)-25-(5-propyloxazol-2-yl)-26,27-cyclo-9,10-secocholesta-5,7,10(19),22-tétraène-1,3,24-triol,
(5Z,7E,22E)-(1S,3R,20S,24R)-25-(5-méthyloxazol-2-yl)-26,27-cyclo-9,10-secocholesta-5,7,10(19),22-tétraène-1,3,24-triol,
(5Z,7E,22E)-(1S,3R,20S,24S)-25-(5-méthyloxazol-2-yl)-26,27-cyclo-9,10-secocholesta-5,7,10(19),22-tétraène-1,3,24-triol,
(5Z,7E,22E)-(1S,3R,20S,24R)-25-(5-éthyloxazol-2-yl)-26,27-cyclo-9,10-secocholesta-5,7,10(19),22-tétraène-1,3,24-triol,
(5Z,7E,22E)-(1S,3R,20S,24S)-25-(5-éthyloxazol-2-yl)-26,27-cyclo-9,10-secocholesta-5,7,20(19),22-tétraène-1,3,24-triol,
(5Z,7E,22E)-(1S,3R,20S,24R)-25-(5-butyloxazol-2-yl)-26,27-cyclo-9,10-secocholesta-5,7,10(19),22-tétraène-1,3,24-triol,
(5Z,7E,22E)-(1S,3R,20S,24S)-25-(5-butyloxazol-2-yl)-26,27-cyclo-9,10-secocholesta-5,7,10(19),22-tétraène-1,3,24-triol,
(5Z,7E,22E)-(1S,3R,20S,24R)-25-(5-pentyloxazol-2-yl)-26,27-cyclo-9,10-secocholesta-5,7,10(19),22-tétraène-1,3,24-triol,
(5Z,7E,22E)-(1S,3R,20S,24S)-25-(5-pentyloxazol-2-yl)-26,27-cycle-9,10-secocholesta-5,7,10(19),22-tétraène-1,3,24-triol,
(5Z,7E,22E)-(1S,3R,20S,24R)-25-(5-propylthiazol-2-yl)-26,27-cyclo-9,10-secocholesta-5,7,10(19),22-tétraène-1,3,24-triol,
(5Z,7E,22E)-(1S,3R,20S,24S)-25-(5-propylthiazol-2-yl)-26,27-cyclo-9,10-secocholesta-5,7,10(19),22-tétraène-1,3,24-triol,
(5Z,7E,22E)-(1S,3R,20S,24R)-25-(5-méthylthiazol-2-yl)-26,27-cyclo-9,10-secocholesta-5,7,10(19),22-tétraène-1,3,24-triol,
(5Z,7E,22E)-(1S,3R,20S,24S)-25-(5-méthylthiazol-2-yl)-26,27-cyclo-9,20-secocholesta-5,7,20(29),22-tétraène-1,3,24-triol,
(5Z,7E,22E)-(1S,3R,20S,24R)-25-(5-éthylthiazol-2-yl)-26,27-cyclo-9,10-secocholesta-5,7,10(19),22-tétraène-1,3,24-triol,
(5Z,7E,22E)-(1S,3R,20S,24S)-25-(5-éthylthiazol-2-yl)-26,27-cyclo-9,10-secocholesta-5,7,10(Z9),22-tétraène-1,3,24-triol,
(5Z,7E,22E)-(1S,3R,20S,24R)-25-(5-butylthiazol-2-yl)-26,27-cyclo-9,10-secocholesta-5,7,10(19),22-tétraène-1,3,24-triol,
(5Z,7E,22E)-(1S,3R,20S,24S)-25-(5-butylthiazol-2-yl)-26,27-cyclo-9,10-secocholesta-5,7,10(19),22-tétraène-1,3,24-triol,
(5Z,7E,22E)-(1S,3R,20S,24R)-25-(5-pentylthiazol-2-yl)-26,27-cyclo-9,10-secocholesta-5,7,10(19),22-tétraène-1,3,24-triol,
(5Z,7E,22E)-(1S,3R,20S,24S)-25-(5-pentylthiazol-2-yl)-26,27-cyclo-9,10-secocholesta-5,7,10(19),22-tétraène-1,3,24-triol,
(5Z,7E,22E)-(1S,3R,20S,24R)-25-(4-propyloxazol-2-yl)-26,27-cyclo-9,20-secocholesta-5,7,10(19),22-tétraène-1,3,24-triol,
(5Z,7E,22E)-(1S,3R,20S,24S)-25-(4-propyloxazol-2-yl)-26,27-cyclo-9,10-secocholesta-5,7,10(19),22-tétraène-1,3,24-triol,
(5Z,7E,22E)-(1S,3R,20S,24R)-25-(4-méthyloxazol-2-yl)-26,27-cyclo-9,10-secocholesta-5,7,10(19),22-tétraène-1,3,24-triol,
(5Z,7E,22E)-(1S,3R,20S,24S)-25-(4-méthyloxazol-2-yl)-26,27-cyclo-9,10-secocholesta-5,7,10(19),22-tétraène-1,3,24-triol,
(5Z,7E,22E)-(1S,3R,20S,24R)-25-(4-éthyloxazol-2-yl)-26,27-cycle-9,10-secocholesta-5,7,10(19),22-tétraène-1,3,24-triol,
(5Z,7E,22E)-(1S,3R,20S,24S)-25-(4-éthyloxazol-2-yl)-26,27-cyclo-9,10-secocholesta-5,7,10(19),22-tétraène-1,3,24-triol,
(5Z,7E,22E)-(1S,3R,20S,24R)-25-(4-butyloxazol-2-yl)-26,27-cyclo-9,10-secocholesta-5,7,10(19),22-tétraène-1,3,24-triol,
(5Z,7E,22E)-(1S,3R,20S,24S)-25-(4-butyloxazol-2-yl)-26,27-cyclo-9,10-secocholesta-5,7,10(19),22-tétraène-1,3,24-triol,
(5Z,7E,22E)-(1S,3R,20S,24R)-25-(4-pentyloxazol-2-yl)-26,27-cyclo-9,10-secocholesta-5,7,10(19),22-tétraène-1,3,24-triol,
(5Z,7E,22E)-(1S,3R,20S,24S)-25-(4-pentyloxazol-2-yl)-26,27-cyclo-9,10-secocholesta-5,7,10(19),22-tétraène-1,3,24-triol,
(5Z,7E,22E)-(1S,3R,20S,24R)-25-(4-propylthiazol-2-yl)-26,27-cyclo-9,10-secocholesta-5,7,20(19),22-tétraène-1,3,24-triol,
(5Z,7E,22E)-(1S,3R,20S,24S)-25-(4-propylthiazol-2-yl)-26,27-cyclo-9,10-secocholesta-5,7,10(19),22-tétraène-1,3,24-triol,
(5Z,7E,22E)-(1S,3R,20S,24R)-25-(4-méthylthiazol-2-yl)-26,27-cyclo-9,10-secocholesta-5,7,10(19),22-tétraène-1,3,24-triol,
(5Z,7E,22E)-(1S,3R,20S,24S)-25-(4-méthylthiazol-2-yl)-26,27-cyclo-9,10-secocholesta-5,7,10(19),22-tétraène-1,3,24-triol,
(5Z,7E,22E)-(1S,3R,20S,24R)-25-(4-éthylthiazol-2-yl)-26,27-cyclo-9,10-secocholesta-5,7,10(19),22-tétraène-1,3,24-triol,
(5Z,7E,22E)-(1S,3R,20S,24S)-25-(4-éthylthiazol-2-yl)-26,27-cyclo-9,10-secocholesta-5,7,10(19),22-tétraène-1,3,24-triol,
(5Z,7E,22E)-(1S,3R,20S,24R)-25-(4-butylthiazol-2-yl)-26,27-cyclo-9,10-secocholesta-5,7,10(19),22-tétraène-1,3,24-triol,
(5Z,7E,22E)-(1S,3R,20S,24S)-25-(4-butylthiazol-2-yl)-26,27-cyclo-9,10-secocholesta-5,7,10(19),22-tétraène-1,3,24-triol,
(5Z,7E,22E)-(1S,3R,20S,24R)-25-(4-pentylthiazol-2-yl)-26,27-cyclo-9,10-secocholesta-5,7,10(19),22-tétraène-1,3,24-triol,
(5Z,7E,22E)-(2S,3R,20S,24S)-25-(4-pentylthiazol-2-yl)-26,27-cyclo-9,10-secocholesta-5,7,10(19),22-tétraène-1,3,24-triol,
(5Z,7E,22E)-(1S,3R,24R)-20-méthyl-25-(5-propyloxazol-2-yl)-26,27-cyclo-9,10-secocholesta-5,7,10(19),22-tétraène-1,3,24-triol,
(5Z,7E,22E)-(1S,3R,24S)-20-méthyl-25-(5-propyloxazol-2-yl)-26,27-cyclo-9,10-secocholesta-5,7,10(19),22-tétraène-1,3,24-triol,
(5Z,7E,22E)-(1S,3R,24R)-20-méthyl-25-(5-méthyloxazol-2-yl)-26,27-cyclo-9,10-secocholesta-5,7,10(19),22-tétraène-1,3,24-triol,
(5Z,7E,22E)-(1S,3R,24S)-20-méthyl-25-(5-méthyloxazol-2-yl)-26,27-cyclo-9,10-secocholesta-5,7,10(19),22-tétraène-1,3,24-triol,
(5Z,7E,22E)-(1S,3R,24R)-25-(5-éthyloxazol-2-yl)-20-méthyl-26,27-cyclo-9,10-secocholesta-5,7,10(19),22-tétraène-1,3,24-triol,
(5Z,7E,22E)-(1S,3R,24S)-25-(5-éthyloxazol-2-yl)-20-méthyl-26,27-cyclo-9,10-secocholesta-5,7,10(19),22-tétraène-1,3,24-triol,
(5Z,7E,22E)-(1S,3R,24R)-25-(5-butyloxazol-2-yl)-20-méthyl-26,27-cyclo-9,20-secocholesta-5,7,10(19),22-tétraène-1,3,24-trial,
(5Z,7E,22E)-(1S,3R,24S)-25-(5-butyloxazol-2-yl)-20-méthyl-26,27-cyclo-9,10-secocholesta-5,7,10(19),22-tétraène-1,3,24-triol,
(5Z,7E,22E)-(1S,3R,24R)-20-méthyl-25-(5-pentyloxazol-2-yl)-26,27-cyclo-9,10-secocholesta-5,7,10(19),22-tétraène-1,3,24-triol,
(5Z,7E,22E)-(1S,3R,24S)-20-méthyl-25-(5-pentyloxazol-2-yl)-26,27-cyclo-9,10-secocholesta-5,7,10(19),22-tétraène-1,3,24-triol,
(5Z,7E,22E)-(1S,3R,24R)-20-méthyl-25-(5-propylthiazol-2-yl)-26,27-cyclo-9,10-secocholesta-5,7,10(19),22-tétraène-1,3,24-triol,
(5Z,7E,22E)-(1S,3R,24S)-20-méthyl-25-(5-propylthiazol-2-yl)-26,27-cyclo-9,10-secocholesta-5,7,10(19),22-tétraène-2,3,24-triol,
(5Z,7E,22E)-(1S,3R,24R)-20-méthyl-25-(5-méthylthiazol-2-yl)-26,27-cyclo-9,10-secocholesta-5,7,10(19),22-tétraène-1,3,24-triol,
(5Z,7E,22E)-(1S,3R,24S)-20-méthyl-25-(5-méthylthiazol-2-yl)-26,27-cyclo-9,10-secocholesta-5,7,10(19),22-tétraène-1,3,24-triol,
(5Z,7E,22E)-(1S,3R,24R)-25-(5-éthylthiazol-2-yl)-20-méthyl-26,27-cyclo-9,10-secocholesta-5,7,10(19),22-tétraène-1,3,24-triol,
(5Z,7E,22E)-(1S,3R,24S)-25-(5-éthylthiazol-2-yl)-20-méthyl-26,27-cyclo-9,10-secocholesta-5,7,10(19),22-tétraène-1,3,24-triol,
(5Z,7E,22E)-(1S,3R,24R)-25-(5-butylthiazol-2-yl)-20-méthyl-26,27-cyclo-9,10-secocholesta-5,7,10(19),22-tétraène-1,3,24-triol,
(5Z,7E,22E)-(1S,3R,24S)-25-(5-butylthiazol-2-yl)-20-méthyl-26,27-cyclo-9,10-secocholesta-5,7,10(19),22-tétraène-1,3,24-triol,
(5Z,7E,22E)-(1S,3R,24R)-25-(5-pentylthiazol-2-yl)-20-méthyl-26,27-cyclo-9,10-secocholesta-5,7,10(19),22-tétraène-1,3,24-triol,
(5Z,7E,22E)-(1S,3R,24S)-25-(5-pentylthiazol-2-yl)-20-méthyl-26,27-cyclo-9,10-secocholesta-5,7,10(19),22-tétraène-1,3,24-triol,
(5Z,7E,22E)-(1S,3R,24R)-20-méthyl-25-(4-propyloxazol-2-yl)-26,27-cyclo-9,10-secocholesta-5,7,10(19),22-tétraène-1,3,24-triol,
(5Z,7E,22E)-(1S,3R,24S)-20-méthyl-25-(4-propyloxazol-2-yl)-26,27-cyclo-9,10-secocholesta-5,7,10(19),22-tétraène-1,3,24-triol,
(5Z,7E,22E)-(1S,3R,24R)-20-méthyl-25-(4-méthyloxazol-2-yl)-26,27-cyclo-9,10-secocholesta-5,7,10(19),22-tétraène-1,3,24-triol,
(5Z,7E,22E)-(1S,3R,24S)-20-méthyl-25-(4-méthyloxazol-2-yl)-26,27-cyclo-9,10-secocholesta-5,7,10(19),22-tétraène-1,3,24-triol,
(5Z,7E,22E)-(1S,3R,24R)-25-(4-éthyloxazol-2-yl)-20-méthyl-26,27-cyclo-9,10-secocholesta-5,7,10(19),22-tétraène-1,3,24-triol,
(5Z,7E,22E)-(1S,3R,24S)-25-(4-éthyloxazol-2-yl)-20-méthyl-26,27-cyclo-9,10-secocholesta-5,7,10(29),22-tétraène-1,3,24-triol,
(5Z,7E,22E)-(1S,3R,24R)-25-(4-butyloxazol-2-yl)-20-méthyl-26,27-cyclo-9,10-secocholesta-5,7,10(19),22-tétraène-1,3,24-triol,
(5Z,7E,22E)-(1S,3R,24S)-25-(4-butyloxazol-2-yl)-20-méthyl-26,27-cyclo-9,10-secocholesta-5,7,10(19),22-tétraène-1,3,24-triol,
(5Z,7E,22E)-(1S,3R,24R)-20-méthyl-25-(4-pentyloxazol-2-yl)-26,27-cyclo-9,10-secocholesta-5,7,10(19),22-tétraène-1,3,24-triol,
(5Z,7E,22E)-(1S,3R,24S)-20-méthyl-25-(4-pentyloxazol-2-yl)-26,27-cyclo-9,10-secocholesta-5,7,10(19),22-tétraène-1,3,24-triol,
(5Z,7E,22E)-(1S,3R,24R)-20-méthyl-25-(4-propylthiazol-2-yl)-26,27-cyclo-9,10-secocholesta-5,7,10(19),22-tétraène-1,3,24-triol,
(5Z,7E,22E)-(1S,3R,24S)-20-méthyl-25-(4-propylthiazol-2-yl)-26,27-cyclo-9,10-secocholesta-5,7,10(19),22-tétraène-1,3,24-triol,
(5Z,7E,22E)-(1S,3R,24R)-20-méthyl-25-(4-méthylthiazol-2-yl)-26,27-cyclo-9,10-secocholesta-5,7,10(19),22-tétraène-1,3,24-triol,
(5Z,7E,22E)-(1S,3R,24S)-20-méthyl-25-(4-méthylthiazol-2-yl)-26,27-cyclo-9,10-secocholesta-5,7,10(19),22-tétraène-1,3,24-triol,
(5Z,7E,22E)-(1S,3R,24R)-25-(4-éthylthiazol-2-yl)-20-méthyl-26,27-cyclo-9,10-secocholesta-5,7,10(19),22-tétraène-1,3,24-triol,
(5Z,7E,22E)-(1S,3R,24S)-25-(4-éthylthiazol-2-yl)-20-méthyl-26,27-cyclo-9,10-secocholesta-5,7,10(19),22-tétraène-1,3,24-triol,
(5Z,7E,22E)-(1S,3R,24R)-25-(4-butylthiazol-2-yl)-20-méthyl-26,27-cyclo-9,10-secocholesta-5,7,10(19),22-tétraène-1,3,24-triol,
(5Z,7E,22E)-(1S,3R,24S)-25-(4-butylthiazol-2-yl)-20-méthyl-26,27-cyclo-9,10-secocholesta-5,7,10(19),22-tétraène-1,3,24-triol,
(5Z,7E,22E)-(1S,3R,24R)-25-(4-pentylthiazol-2-yl)-20-méthyl-26,27-cyclo-9,10-secocholesta-5,7,10(19),22-tétraène-1,3,24-triol,
(5Z,7E,22E)-(1S,3R,24S)-25-(4-pentylthiazol-2-yl)-20-méthyl-26,27-cyclo-9,10-secocholesta-5,7,10(19),22-tétraène-1,3,24-triol.

**6.** Procédé pour la préparation des dérivés de la vitamine D de formule générale I, un composé de formule générale II,

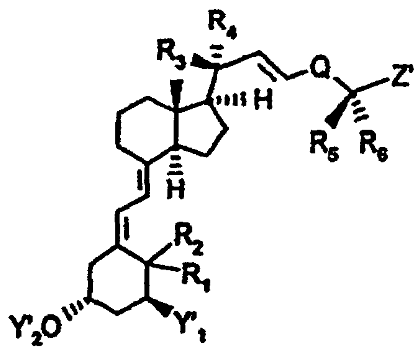

II

dans laquelle
Y'$_1$ signifie un atome d'hydrogène ou un groupement hydroxy protégé,
Y'$_2$ signifie un groupement de protection d'un hydroxy,
$R_1$ et $R_2$ signifient chacun un atome d'hydrogène ou, ensemble, un groupement méthylène exocyclique,
$R_3$ et $R_4$ signifient, indépendamment l'un de l'autre, un atome d'hydrogène, un atome de chlore ou de fluor, un groupement alkyle comprenant 1 à 4 atomes de carbone, ensemble un groupement méthylène ou ensemble avec l'atome de carbone quaternaire 20, un cycle carbocyclique de 3 jusqu'à 7 membres, saturé ou insaturé,
Q signifie une unité carbonée linéaire ou ramifiée comprenant jusqu'à 10 atomes de carbone, qui peut présenter en des positions quelconques des groupements hydroxyle (en position $\alpha$ ou $\beta$), qui peuvent à leur tour être éthérifiés ou estérifiés, des groupements céto, amino ou des atomes d'halogène;
$R_5$ et $R_6$ signifient ensemble avec l'atome de carbone 25 un cycle carbocyclique saturé ou insaturé de 3 jusqu'à 7 membres et
Z' signifie un cycle carbocyclique ou hétérocyclique à cinq ou six membres, qui peut être saturé, insaturé ou aromatique et qui peut porter en des positions quelconques une ou plusieurs chaînes alkyle, qui peuvent être linéaires ou ramifiées, saturées ou insaturées et qui peuvent être interrompues en des endroits quelconques par des fonctions oxa, thia ou aza (substituées ou non substituées) ou par un sulfoxyde ou par des groupements sulfone ou qui peuvent porter des substituants (groupements hydroxy, atomes d'halogène), les groupements hydroxyle éventuellement présents pouvant se trouver sous forme protégée,
étant transformé par dissociation simultanée ou successive des groupements de protection d'hydroxy et, le cas échéant, par une ou des estérifications ou éthérifications partielles ou complètes des groupements hydroxy libres.

7. Utilisation des dérivés de la vitamine D de formule générale I pour la préparation de médicaments.

8. Utilisation des dérivés de la vitamine D de formule générale I selon la revendication 7 pour la préparation d'un médicament pour la thérapie de maladies de la peau hyperproliférantes (psoriasis, acné, ichthyose) ainsi que des maladies tumorales et précancéreuses (par exemple des tumeurs intestinales, le carcinome mammaire, des tumeurs pulmonaires, le carcinome de la prostate, des leucémies, des lymphomes des cellules T, des kératoses actiniques, des dysplasies du col de l'utérus, de plus des maladies auto-immures (la sclérose en plaques, le diabète mellitus de type I, la myasthénie gravis, le lupus érythémateux), des réactions de rejet dans le cas de transplantations autologues, allogéniques ou xénogéniques ainsi que du SIDA, par ailleurs l'utilisation thérapeutique dans le cas de la peau atrophique ou la cicatrisation de plaies est également possible, ainsi que la thérapie de l'hyperparathyroïdie secondaire, l'ostéodystrophie rénale ainsi que l'ostéoporose post-ménopausique, le diabète mellitus de type II et la thérapie de maladies dégénératives du système nerveux périphérique et central (la maladie d'Alzheimer et la sclérose latérale amyotrophe) ainsi que la régulation de la croissance des cheveux.

9. Utilisation des dérivés de la vitamine D de formule générale I selon la revendication 7 qui sont antagonistes de l'action du calcitrol dans des cellules HL60 pour la préparation d'un médicament pour la thérapie d'hypercalcémies (hypervitaminose D, intoxication avec du calcitrol ou ses analogues) ou des maladies granulomatoses (sarcoïdose, tuberculose) ainsi que d'hypercalcémies paranéoplasiques (c'est-à-dire des métastases ostéolytiques et des tumeurs avec une synthèse accrue de peptides associés aux parathormones) et de l'hypercalcémie dans le cas de l'hyperparathyroïdie et pour le contrôle de la fertilité ou comme immuno-stimulants ainsi que dans le cas de l'hirsutisme et pour la thérapie et la prophylaxie de l'artériosclérose ainsi que par ailleurs pour la thérapie de maladies inflammatoires (arthrite rhumatoïde, maladie de Crohn, la colite ulcéreuse et les maladies granulomatoses).

**10.** Produits intermédiaires de formule générale XI, XII et XLIV dans la préparation des dérivés de la vitamine D de formule générale I selon la revendication 1,

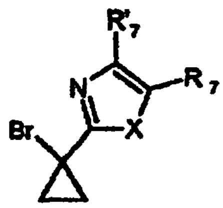

XI X=O
XIII X=S

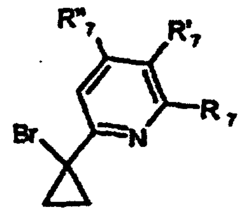

XLIV

dans laquelle
$R_7$, $R'_7$ et $R''_7$ signifient indépendamment l'un de l'autre un atome d'hydrogène, un radical alkyle linéaire ou ramifié, saturé ou insaturé comprenant jusqu'à 12 atomes de carbone, qui peut être interrompu en des endroits quelconques par des fonctions oxa, thia ou aza (substituées ou non substituées) ou par un sulfoxyde ou des groupements sulfone ou qui peut porter d'autres substituants (des groupements hydroxy libres ou protégés, des atomes d'halogène).